# EUROPEAN PATENT SPECIFICATION

(11) **EP 4 452 984 B1**
(45) Date of publication and mention of the grant of the patent: **02.12.2026**
(21) Application number: 22840329.1
(22) Date of filing: 22.12.2022
(51) Int. Cl.: C07D 495/04, A61P 3/00, A61P 9/00, A61P 11/00, A61P 25/28, A61P 37/00, A61K 31/4365

(54) **DEUBIQUITINASE INHIBITORS AND METHODS OF USE THEREOF**
DEUBIQUITINASEHEMMER UND VERFAHREN ZUR VERWENDUNG DAVON
INHIBITEURS DE LA DÉSUBIQUITINASE ET LEURS PROCÉDÉS D'UTILISATION

(30) Priority: 23.12.2021 PL 43997921; 23.12.2021 US 202163293205 P; 05.01.2022 PL 44008322; 05.01.2022 US 202263296570 P
(43) Date of publication of application: 30.10.2024
(73) Proprietor: Molecure S.A., 02-089 Warszawa (PL)
(72) Inventor: CHRZANOWSKI, Jacek, 90-539 Lodz (PL); NOWICKA, Julita, 95-070 Wola Grzymkowa (PL); JOACHIMIAK, Lukasz, 93-120 Lodz (PL); KORALEWSKI, Robert, 91-848 Lodz (PL); OLEJNICZAK, Sylwia, 94-104 Lodz (PL); MAZUR, Marzena, 91-158 Lodz (PL); BOREK, Bartlomiej, 93-519 Lodz (PL); GZIK, Anna, 99-220 Wartkowice (PL); MATYSZEWSKI, Krzysztof, 93-160 Lodz (PL); CIASTEK-ISKRZYCKA, Sylwia, 93-115 Lodz (PL); NIEDZIEJKO, Piotr, 00-168 Warszawa (PL); MUCHOWICZ, Angelika, 02-497 Warszawa (PL); ZASLONA, Zbigniew, 02-625 Warszawa (PL); BLASZCZYK, Roman, 94-109 Lodz (PL); OLCZAK, Jacek, 92-701 Lodz (PL); GOLEBIOWSKI, Adam, Madison, Connecticut 06443 (US)
(74) Representative: Patpol Kancelaria Patentowa Sp. z o.o.
(86) International application number: PCT/IB2022/062697
(87) International publication number: WO 2023/119233

(56) References cited:
- WO-A1-2020/068600
- US-B2- 10 722 514
- LEGER PAUL R. ET AL: "Discovery of Potent, Selective, and Orally Bioavailable Inhibitors of USP7 with In Vivo Antitumor Activity", JOURNAL OF MEDICINAL CHEMISTRY, vol. 63, no. 10, 17 April 2020 (2020-04-17), US, pages 5398 - 5420, XP055909131, ISSN: 0022-2623, DOI: 10.1021/acs.jmedchem.0c00245

## Description

### RELATED APPLICATIONS

This application claims the benefit of priority to Polish Patent Application number P.439979, filed December 23, 2021, United States Provisional Patent Application serial number 63/293,205, filed December 23, 2021, Polish Patent Application number P.440083, filed January 5, 2022, and United States Provisional Patent Application serial number 63/296570, filed January 5, 2022.

### BACKGROUND OF THE INVENTION

### Field of the Invention

This disclosure relates to small molecule therapeutic inhibitors of deubiquitinases.

### Description of Related Art

Ubiquitin system is a major post-translational regulator of protein levels and regulates essential cellular processes in a cell. Ubiquitination is a covalent modification of a given protein that directs it towards proteasomal degradation. Ubiquitination as well as deubiquitination is tightly controlled by E1, E2 or E3 ligases and deubiquitinases (DUBs) respectively. Ubiquitination is usually done by formation of isopeptide bond on a protein's lysine side-chain. This process is further regulated by polyubiquitination during which a ubiquitin molecule's C-terminal Gly is conjugated with one of the seven Lys residues on another ubiquitin (Lys6, Lys11, Lys27, Lys29, Lys33, Lys48, or Lys63) or with the N-terminus to form linear chains. It is called "the ubiquitin code". The ubiquitination code is related to diverse aspects of cellular biology and therefore disruption of this balance contributes to many diseases.

The ubiquitination is a reversible process and the deubiquitination is catalyzed by enzymes called deubiquitinases (DUBs). There are approximately 100 DUBs known in human genome, any of which could be playing a key role in the ubiquitin proteasome system as well as other biological processes. The DUBs are subdivided into five families: UCHs, USPs, OTUs, Josephin, and JAM/MPN+. Except the last one (which is metalloprotease), all DUBs are cysteine proteases consisting catalytic triad with cysteine residue as nucleophile. It is prone to covalent modifications by electrophilic fragments in small molecules and this has interfered with screening methodology and lead to identification of many low quality DUBs inhibitors in the past. However, recent years brought increasing appreciation of this family of targets once more thorough characterization of small molecule inhibitors of DUBs was performed. Deubiquitinases generally prevent protein degradation by cleaving the ubiquitin molecule or editing the polyubiquitin chains. Inhibition of DUBs therefore increases the rate of degradation of DUB-targeted protein. Regulation of DUBs enzymes with their inhibitors allow to indirectly affect the levels of protein of interest. Moreover the protein degradation is getting increasing attention as it might be an alternative strategy to overcome protein mutations and resistance to direct inhibitors.

Among USPs, a ubiquitin specific protease 7 - USP7 has been intensively studied in recent years and numerous inhibitors of USP7 have been identified. Widely known function of USP7 is genetically validated interaction with an E3 ligase MDM2, which ubiquitinates and thus directs for degradation the known oncosupressor p53. MDM2 is deubiquitinated by USP7 resulting in its stabilization. Inhibition of USP7 has proven to restore ubiquitinylation of MDM2 and subsequent proteasomal degradation. This results in accumulation of p53 and promotes cell cycle arrest and apoptosis. On the other hand USP7 can stabilize p53 itself. [US11084829B2 or WO2022170198A1] Highly potent, specific, reversible, orally bioavailable USP7 inhibitors demonstrate marked tumor growth inhibition in both p53-wild type and p53-mutant tumors, indicating that USP7 inhibition can suppress tumor growth *in vivo* through both p53 dependent and independent mechanisms [Leger, P. L. et al. J. Med. Chem. 2020, 63, 5398-5420].

The further studies implicate numerous other partners for USP7 that are linked to DNA-damage response, cancer, immunotherapy, diabetes and viral infections [Nicholson B., Suresh Kumar K.G. The multifaceted roles of USP7; new therapeutic opportunities. *Cell Biochemistry and Biophysics* 2011, 60: 61-68.]. PTEN is thought to be a tumor suppressor in the nucleus. Its location is regulated by mono-ubiquitination. USP7 catalyzes the deubiquitylation nuclear PTEN which results in nuclear exclusion, blocking apoptosis in prostate cancer cells. That suggest that inhibition of USP7 triggers accumulation PTEN in nucleus and apoptosis. Other studies have uncovered a link between USP7 and Polycomb mediated silencing of genes. In particular, USP7 was shown to regulate the function of Ring1B and BMI1, which are essential core components of Polycomb Repressive Complex 1 and 2 [Gagarina, V. et al. J. Mol. Biol. 2020, 432, 4, 897 - 912.]. PRC 1 and 2 complexes catalyze the mono-, di- and trimethylation of lysine 27 of histone H3 (H3K27me1, H3K27me2 and H3K27me3) and is bound to CpG islands. H3K27me3 is a hallmark of PcG-associated transcriptional silencing and is thought to result in gene repression. The histone methyl transferase activity of PRC2 is mediated by one of the two catalytic subunits, enhancer of zeste homologue 1 (EZH1) or EZH2. Interaction between USP7 and PRC complexes, including EZH2 in particular but also other PRC components has been reported by several groups [De Bie, P. et al. Biochemical and Biophysical Research Communications 2010, 400, 3, 389-395.; Lecona, E. et al. Molecular and Cellular Biology 2015, 35, 7, 1157 - 1168.; Gagarina, V. et al. J. Mol. Biol. 2020, 432, 4, 897 - 912.]. Another epigenetic regulator - LSD1 - was also reported to interact with USP7. Overexpression of LSD1 has been proved in numerous cancers, and high level of LSD1 aggressiveness and poor prognosis in lung, prostate, colon and breast cancers. The study shows that another oncogene - CARM1-dependendent methylation of LSD1 promotes deubiquitylation of LSD1 by USP7 [Liu, J. et al. EMBO Rep. 2020, 21(2), e48597.]. Wnt/beta-catenin/axin pathway plays important roles in many important biological processes and aberrant Wnt/β-catenin signaling has been associated with many human diseases, such as degenerative diseases and cancer. It was previously postulated that USP7 activates Wnt signaling and therefore inhibition of USP7 triggers Wnt attenuation. It was shown that USP7 stabilizes beta-catenin and once inhibited, beta-catenin is degraded and the canonical wnt pathway is inhibited [Novellasdemunt, L et al. Cell Reports 2017, 21(3), 612 - 627.]. However, recent study published by NIBR in Nat. Comm. shows that USP7 actually inhibits Wnt-induced beta-catenin accumulation [ Ji, B. et al. Nature Communications 2019, 10, 4184.]. Both, genetic inhibition and pharmacological intervention on USP7 enhanced Wnt/B-catenin signaling with downstream effects on osteoblasts and adipocyte differentiation.

Importantly, USP7 plays a key role in determining half-life of crucial proteins involved in the regulation of immune response, namely FOXP3 and PD-L1 resulting in preservation of immunosuppressive functions of Tregs and causing escape of cancer cells from cytotoxic immune cells respectively. Evidence that USP7 by de-ubiquitylating of FOXP3 increases T Treg numbers and mediates suppression of tumor-infiltrating T effector cells has been elegantly demonstrated [Van Loosdregt, J. et al. Immunity 2013, 22, 39 (2), 259 - 271.]. This presented USP7 as an attractive immunoregulatory target. Indeed, USP7 deletion resulted in improved clinical outcome for many solid tumors. The observation that the accumulation of FOXP3+ Treg cells at the tumor or in draining lymph nodes signals poor prognosis highlights the significance of oncogenic mechanism of USP7.

As a part of immunotherapy, checkpoint blockades (PD-1/PD-L1) have acquired clinical success, antibody treatment has several limitations and need for small molecule is evident. Therefore the fact that USP7 has been demonstrated to be responsible for PD-L1 protein stabilization has serious therapeutic implications [Wang, Z. et al. Acta Pharm. Sin. B. 2021, 11, 3, 694 - 707.].

Overall it has been demonstrated that USP7 plays a critical role in affecting the tumor microenvironment. The effects of USP7 inhibition demonstrated promoting remodeling of the extracellular matrix (ECM), thereby promoting tumor invasion and metastasis. USP7 also affects angiogenesis and VEGF levels both systemically and in the tumor microenvironment (TME). Moreover, USP7 inhibition, particularly in the fibroblast compartment of the TME, leads to a significant decrease in both cell invasion and angiogenesis. USP7 inhibition also results in modulation of the tumor immune environment (*e.g*., by promoting infiltration of CD8 T cells). *In vivo* USP7 inhibition inhibits tumor growth in cell models that are not affected by direct inhibition by USP7 inhibitors *in vitro.* [WO2021161047A1]. Antitumor efficacy in the MC38 colon cancer syngeneic mouse model, through upregulating the tumor infiltration of CD8+ T, NK, and NKT cells and downregulating that of Tregs and MDSCs was recently published [Li X. et al. J. Med. Chem., DOI: 10.1021/acs.jmedchem.2c01444].

Overall USP7 was found to be involved in many pathways that are aberrant in cancer and immune-oncology. Thus, inhibitors of USP7 can exert *in vivo* antitumor activity by: 1) directly inhibiting tumor cell proliferation via Hdm2 and other targets; 2) suppressing T regulatory cells via FOXP3, thereby facilitating the antitumor function of T effector cells; 3) inhibition of PD-L1 expression which sensitizes tumor cells towards cytotoxic effect of immune cells. First generation of USP7 inhibitors was often covalent (*i.e*., P5091 or HBX19818) or not selective. Next generation of allosteric and non-covalent inhibitors of USP7 was reported such as GNE-6640 and FT671 which exhibited *in vivo* efficacy.

Over the last years a potential of modulating immune system for the treatment of cancer has been described. A clinical success of immune checkpoint inhibitors, particularly those targeting the PD-1 axis, has set new trends in cancer therapy. Unfortunately, there is still unmet need and many patients do not improve after checkpoint blockade or other existing immunotherapies. T cell activation is crucial for the efficacy of immune checkpoint inhibitor therapies. Our experiments have been designed to highlight the effect of our compound T cell activation, particularly IFN-gamma production. IFN-gamma is known to have anti-cancer properties, such as antiproliferative effect and induction of necroptosis in apoptosis-resistant cancer cells, regression of the tumor vasculature, activation antigen-presenting cells, as well as enhancement Th1 differentiation and cytotoxic T lymphocyte function.

### SUMMARY OF THE INVENTION

In one aspect, the invention provides a compound of structural Formula (Ia): 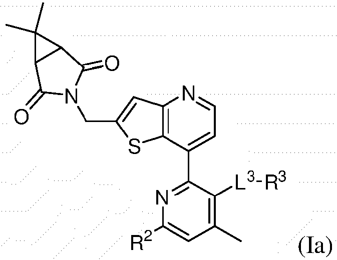 wherein:
R² is selected from hydrogen, methyl, ethyl, chloro, -C≡N, and -CF₃;
L³ represents a single bond, -O-, -CH₂-, -C(=O)-, -NH-, or -NH-C(=O)-; and
R³ is selected from (4-6) membered heterocycloalkyl containing 1 to 2 nitrogen heteroatoms or 1 nitrogen heteroatom and 1 oxygen heteroatom, and optionally substituted with 1 or 2 substituents selected from fluoro, methyl, ethyl, amino, methylamino, dimethylamino, ethylamino, isopropylamino, hydroxy, oxo, aminomethyl, 1-aminoethyl, (isopropylamino)methyl, ureido, alanylamino, valylamino, fluoromethyl, difluoromethyl, trifluoromethyl, and 2,2,2-trifluoroacetyl;
or a tautomer, stereoisomer, a racemic or scalemic mixture of stereoisomers, a pharmaceutically acceptable salt, ester, or solvate thereof.

Also provided herein is a pharmaceutical composition comprising (i) a therapeutically effective amount of at least one compound of the invention, or a pharmaceutically acceptable salt, or solvate thereof; and (ii) a pharmaceutically acceptable carrier, vehicle or excipient therefor.

In another aspect, the invention provides a compound according to the invention, or a pharmaceutically acceptable salt, or solvate thereof, for use in a method for inhibiting USP7 in a cell or a tissue, comprising contacting the cell or the tissue with at least one compound according to the invention, or a pharmaceutically acceptable salt, or solvate thereof.

In another aspect, the invention provides use of at least one compound according to the invention, or a pharmaceutically acceptable salt, or solvate thereof, in the manufacturing of a medicament for the treatment of a disease, disorder, or condition associated with expression of USP7.

In further aspect, the invention provides a compound according to the invention, or a pharmaceutically acceptable salt, or solvate thereof, for use in a method for the treatment or prevention of a disease, disorder, or condition associated with aberrant expression or activity of USP7, comprising administering to the subject in need thereof a therapeutically effective amount of at least one compound according to the invention, or a pharmaceutically acceptable salt, or solvate thereof, or a pharmaceutical composition according to the invention.

In another aspect, the invention provides at least one compound according to the invention, or a pharmaceutically acceptable salt, or solvate thereof, for use in the treatment or prevention of a disease, disorder, or condition selected from the group consisting of cardiovascular disorders, pulmonary disorders, autoimmune disorders, immune disorders, immunoregulatory disorders, neurodegenerative disorders, metabolic disorders, hemolytic disorders, gastrointestinal disorders, sexual disorders, infections, wound healing disorders, and cancers.

In further aspect, the invention provides a compound according to the invention for use in a method for activating cytokine release in T lymphocyte cell *in vitro* or *ex vivo,* comprising contacting the cell with at least one compound according to the invention, or a pharmaceutically acceptable salt, or solvate thereof.

In another aspect, the invention provides use of a compound according to the invention, or a pharmaceutically acceptable salt, or solvate thereof, for protecting an organ during transport.

### BRIEF DESCRIPTION OF THE DRAWINGS

FIGS. 1A-1C are graphs showing results of assays comparing cell viability for compound Example 77 (denoted USP7i in the Figures) and a reference compound from RAPT Therapeutics.
FIGS. 2A and 2B show TNF-α and IFN-γ levels in supernatants obtained from three independent ELISA experiments.
FIGS. 3A-3D show the effect of Example 77 on CD4⁺T cell activation and proinflammatory cytokine release and, in particular, on levels of IFN, GM-CSF, TNF and IL-10.
FIGS. 4A-4D show the in vivo and ex vivo immunomodulatory effects of Example 77 in BALB/c mice.
FIGS. 5A and 5B shows the effect of 2 µM Example 77 on CD4⁺ T cell viability as determined in a LDH-Glo and a CellTiter Glo Assay.
FIG. 6A shows the antitumor efficacy effect of Example 77 in the subcutaneous CT26 model of colon carcinoma.
FIG. 6B shows the effect of Example 77 in a and killing assay measuring splenocyte cytotoxicity to tumor cells.
FIG. 6C shows the effect of Example 77 on % of CD4 and CD8 T lymphocytes in tumor by cytometric analysis.
FIG. 6D shows the effect of Example 77 on granzyme B production in CD8+ T cells at varying concentrations against vehicle.
FIG. 7A shows result of evaluating the antitumor efficacy of Example 77 in combination with anti-PD-1 antibodies in the subcutaneous CT26 model of colon carcinoma in a Man-Whitney test.
FIG. 7B shows result of evaluating the antitumor efficacy of Example 77 in combination with anti-PD-1 antibodies in the subcutaneous CT26 model of colon carcinoma in a log-rank test.
FIG. 8 shows the effect of Example 124 of U.S. Patent No. 11, 084,829 on antitumor activity in a CT26 model alone and in combination with anti-PD-1 therapy.
FIG. 9 compares the cytotoxic effect of Example 77 against the effect of Example 124 of U.S. Patent No. 11,084,829 in mouse CD8+ T cells.
FIG. 10 shows the rate of survival after treatment with Example 77 and CD8-depleting antibodies.

### DETAILED DESCRIPTION

The present invention is based on a surprising finding that some small molecule USP7 inhibitors possess very high activity accompanied by superior pharmacokinetics.

### Definitions

The articles "a" and "an" are used herein to refer to one or to more than one *(i.e.,* to at least one) of the grammatical object of the article. By way of example, "an element" means one element or more than one element.

The terms used herein may be preceded and/or followed by a single dash "-", or a double dash "=", to indicate the bond order of the bond between the named substituent and its parent moiety; a single dash indicates a single bond and a double dash indicates a double bond. In the absence of a single or double dash, it is understood that a single bond is formed between the substituent and its parent moiety; further, substituents are intended to be read "from left to right," unless a dash indicates otherwise. For example, (C₁-C₆)-alkoxycarbonyloxy and - OC(O)O(C₁-C₆)alkyl indicate the same functionality; similarly arylalkyl and -alkylaryl indicate the same functionality.

The terms "hydrogen", "hydrogen atom", and symbol "H", as used herein in the context of substituents to Markush formulas, such as Formula (Ia), denote a hydrogen atom attached to the remaining part of the molecule or group in question. For the sake of simplicity, hydrogen atoms attached to carbon atoms are not shown in the structural formulas; each carbon atom is understood to be associated with enough hydrogen atoms to give the carbon atom four bonds.

The term "alkyl" as used herein is a term of art and refers to saturated aliphatic groups, including straight-chain alkyl groups, branched-chain alkyl groups, cycloalkyl (alicyclic) groups, alkyl substituted cycloalkyl groups, and cycloalkyl substituted alkyl groups. In certain embodiments, a straight-chain or branched-chain alkyl has about 30 or fewer carbon atoms in its backbone (*e.g*., C₁-C₃₀ for straight chain, C₃-C₃₀ for branched chain), and alternatively, about 20 or fewer, 10 or fewer. Preferred alkyl groups have 1-6 carbons. Representative examples of alkyl include, but are not limited to, methyl, ethyl, *n*-propyl, isopropyl, *n*-butyl, *sec*-butyl, isobutyl, *tert*-butyl, *n*-pentyl, isopentyl, neopentyl, and *n*-hexyl. Alkyl groups are independently optionally substituted with at least one substituent independently selected from the group consisting of oxo, fluoro, chloro, bromo, iodo, -CF₃, -CCl₃, -CBr₃, -CI₃, -CHF₂, -CHCl₂, -CHBr₂, -CHI₂, -CH₂F, - CH₂Cl, -CH₂Br, -CH₂I, -CN, -OH, -NH₂, -CO₂H, -CONH₂, -NO₂, -SH, -SO₃H, -SO₄H, - SO₂NH₂, -NHNH₂, -ONH₂, -NHC(=O)NHNH₂, -NHC(=O)NH₂, -NHSO₂H, -NHC(=O)H, - NHOH, -OCF₃, -OCCl₃, -OCBr₃, -OCI₃, -OCHF₂, -OCHCl₂, -OCHBr₂, -OCHI₂, -OCH₂F, - OCH₂Cl, -OCH₂Br, -OCH₂I, (C₁-C₆)alkoxy, (C₁-C₆)haloalkoxy, (C₃-C₈)cycloalkyl, and (C₆-C₁₀)aryl.

The term "cycloalkyl" means monocyclic saturated or partially saturated carbocyclic rings, having from 3-6 carbon atoms in their ring structure. Examples of monocyclic cycloalkyls include cyclopropyl, cyclobutyl, cyclopentyl, cyclopentenyl, cyclohexyl, and cyclohexenyl. Cycloalkyl groups are independently optionally substituted by at least one substituent independently selected from the group consisting of oxo, fluoro, chloro, bromo, iodo, -CF₃, - CCl₃, -CBr₃, -CI₃, -CHF₂, -CHCl₂, -CHBr₂, -CHI₂, -CH₂F, -CH₂Cl, -CH₂Br, -CH₂I, -CN, -OH, - NH₂, -CO₂H, -CONH₂, -NO₂, -SH, -SO₃H, -SO₄H, -SO₂NH₂, -NHNH₂, -ONH₂, - NHC(=O)NHNH₂, -NHC(=O)NH₂, -NHSO₂H, -NHC(=O)H, -NHOH, -OCF₃, -OCCl₃, -OCBr₃, - OCI₃, -OCHF₂, -OCHCl₂, -OCHBr₂, -OCHI₂, -OCH₂F, -OCH₂Cl, -OCH₂Br, -OCH₂I, (C₁-C₆)alkyl, (C₁-C₆)alkoxy, (C₁-C₆)haloalkoxy, (C₃-C₈)cycloalkyl, and (C₆-C₁₀)aryl.

The term "heterocyclyl" as used herein refers to a radical of a non-aromatic ring system, including, monocyclic rings, which can be completely saturated or which can contain one or more units of unsaturation, for the avoidance of doubt, the degree of unsaturation does not result in an aromatic ring system, and having 3 to 7 atoms including at least one heteroatom, such as nitrogen, oxygen, or sulfur. More preferred heterocycloalkyl groups have from 5-7 ring members where from 1-4 of the ring members are hetero atoms selected from the group comprising O, N, and S, the remaining ring atoms being C. For purposes of exemplification, which should not be construed as limiting the scope of this invention, the following are examples of heterocyclic rings: aziridinyl, azirinyl, oxiranyl, thiiranyl, thiirenyl, dioxiranyl, diazirinyl, diazepanyl, 1,3-dioxanyl, 1,3-dioxolanyl, 1,3-dithiolanyl, 1,3-dithianyl, imidazolinyl, imidazolidinyl, isothiazolinyl, isothiazolidinyl, isoxazolinyl, isoxazolidinyl, azetyl, oxetanyl, oxetyl, thietanyl, thietyl, diazetidinyl, dioxetanyl, dioxetenyl, dithietanyl, dithietyl, furyl, dioxalanyl, pyrrolyl, oxazolyl, thiazolyl, imidazolyl, oxadiazolyl, thiadiazolyl, triazolyl, triazinyl, isothiazolyl, isoxazolyl, thiophenyl, pyrazolyl, tetrazolyl, pyridyl, pyridazinyl, pyrimidinyl, pyrazinyl, triazinyl, tetrazinyl, azepinyl, azepanyl, azetidinyl, morpholinyl, oxadiazolinyl, oxadiazolidinyl, oxazolinyl, oxazolidinyl, oxopiperidinyl, oxopyrrolidinyl, piperazinyl, piperidinyl, pyranyl, pyrazolinyl, pyrazolidinyl, pyrrolinyl, pyrrolidinyl, thiomorpholinyl, tetrahydropyranyl, tetrahydrofuranyl, tetrahydrothienyl, thiadiazolinyl, thiadiazolidinyl, thiazolinyl, thiazolidinyl, thiomorpholinyl, 1,1-dioxidothiomorpholinyl (thiomorpholine sulfone), thiopyranyl, and trithianyl. A heterocyclyl group is optionally substituted by one or more substituents independently selected from the group consisting of oxo, fluoro, chloro, bromo, iodo, -CF₃, -CCl₃, -CBr₃, -CI₃, -CHF₂, -CHCl₂, -CHBr₂, -CHI₂, -CH₂F, -CH₂Cl, -CH₂Br, -CH₂I, -CN, -OH, -NH₂, -CO₂H, - CONH₂, -NO₂, -SH, -SO₃H, -SO₄H, -SO₂NH₂, -NHNH₂, -ONH₂, -NHC(=O)NHNH₂, - NHC(=O)NH₂, -NHSO₂H, -NHC(=O)H, -NHC(=O)(C₁-C₆)alkyl, -NH(C₁-C₆)alkyl, -N(C₁-C₆)alkyl(C₁-C₆)alkyl, -NHOH, -OCF₃, -OCCl₃, -OCBr₃, -OCI₃, -OCHF₂, -OCHCl₂, -OCHBr₂, - OCHI₂, -OCH₂F, -OCH₂Cl, -OCH₂Br, -OCH₂I, (C₁-C₆)alkyl, (C₁-C₆)alkoxy, (C₁-C₆)haloalkoxy, (C₃-C₈)cycloalkyl, and (C₆-C₁₀)aryl.

The term "heteroatom" is art-recognized, and includes an atom of any element other than carbon or hydrogen. Illustrative heteroatoms include oxygen, nitrogen and sulfur.

The term "cycloalkylalkyl" as used herein refers to an alkyl group substituted with one or more cycloalkyl groups.

The term "heterocycloalkylalkyl" as used herein refers to an alkyl group substituted with one or more heterocycloalkyl (*i.e*., heterocyclyl) groups.

The term "alkenyl" as used herein means a straight-chain or branched chain hydrocarbon radical containing from 2 to 6 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, and 5-hexenyl. The unsaturated bond(s) of the alkenyl group can be located anywhere in the moiety and can have either the (Z) or the (E) configuration about the double bond(s).

The term "amino" is a term of art and as used herein refers to both unsubstituted and substituted amines, *e.g*., a moiety that may be represented by the general formulas: 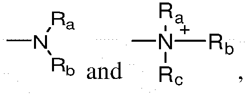 wherein Rₐ, R_{b}, and R_{c} each independently represent a hydrogen, an alkyl, an alkenyl, -(CH₂)ₓ-R_{d}, or Rₐ and R_{b}, taken together with the N atom to which they are attached complete a heterocycle having from 4 to 8 atoms in the ring structure; R_{d} represents an aryl, a cycloalkyl, a cycloalkenyl, or a heterocyclyl; and x is zero or an integer in the range of 1 to 8. In certain embodiments, only one of Rₐ or R_{b} may be a carbonyl, *e.g*., Rₐ, R_{b}, and the nitrogen together do not form an imide. In other embodiments, Rₐ and R_{b} (and optionally R_{c}) each independently represent a hydrogen, an alkyl, an alkenyl, or -(CH₂)ₓ-R_{d}.

In certain embodiments, the term "amino" refers to -NH₂.

The term "alpha-amino acid" is a term of art, including, but not limited to, any one of 20 canonical alpha-amino acids, including their D- and L-isomers; and also glycine, as well as D- and L-proline (being, in fact, imino acids). The three-letter codes, trivial names, and systematic names of residues of these amino acids (L isomer series assumed) are as follows:

| | | |
|---|---|---|
| Ala | alanyl | (*S*)-2-aminopropanoyl; |
| Arg | arginyl | (*S*)-2-amino-5-guanidinopentanoyl; |
| Asn | asparaginyl | (*S*)-2,4-diamino-4-oxobutanoyl; |
| Asp | aspartyl | (*S*)-2-amino-3-carboxypropanoyl; |
| Cys | cysteinyl | (*R*)-2-amino-3-mercaptopropanoyl; |
| Gln | glutaminyl | (*S*)-2,5-diamino-5-oxopentanoyl; |
| Glu | glutamyl | (*S*)-2-amino-4-carboxybutanoyl; |
| Gly | glycyl | 2-aminoacetyl; |
| His | histidyl | (*S*)-2-amino-3-(1*H*-imidazol-4-yl)propanoyl; |
| Ile | isoleucyl | (2*S*,3*S*)-2-amino-3-methylpentanoyl; |
| Leu | leucyl | (*S*)-2-amino-4-methylpentanoyl; |
| Lys | lysyl | (*S*)-2,6-diaminohexanoyl; |
| Met | methionyl | (*S*)-2-amino-4-(methylthio)butanoyl; |
| Phe | phenylalanyl | (*S*)-2-amino-3-phenylpropanoyl; |
| Pro | prolyl | (*S*)-pyrrolidine-2-carboxyl; |
| Ser | seryl | (*S*)-2-amino-3-hydroxypropanoyl; |
| Thr | threonyl | (2*S*,3*R*)-2-amino-3-hydroxybutanoyl; |
| Trp | tryptophanyl | (*S*)-2-amino-3-(1*H*-indol-3-yl)propanoyl; |
| Tyr | tyrosyl | (*S*)-2-amino-3-(4-hydroxyphenyl)propanoyl; |
| Val | valyl | (*S*)-2-amino-3-methylbutanoyl. |

Other alpha-amino acid residues mentioned in this disclosure are, for example,

| | | |
|---|---|---|
| *tert-Leu* | *tert*-leucyl | (*S*)-2-amino-3,3-dimethylbutanoyl; |
| Nva | norvalyl | (*S*)-2-aminopentanoyl; |
| Orn | ornithyl | (*S*)-2,5-diaminopentanoyl; |
| Pyl | pyrrolysyl | (*S*)-2-amino-6-{[(2*R*,3*R*)-3-methyl-3,4-dihydro-2*H*-pyrrol-2-yl]carbonylamino}hexanoyl; |
| Sec | selenocysteinyl | (*R*)-2-amino-3-selanylpropanoyl; |
| SeMet | selenomethionyl | (*S*)-2-amino-4-(methylselanyl)butanoyl; |
| Cit | citrullinyl | (*S*)2-amino-5-(carbamoylamino)pentanoyl; |
| | | (*S*)-2-amino-2-cyclopentylacetyl; |
| | | (*S*)-2-amino-3-hydroxy-3-methylbutanoyl; and |
| | | (*S*)-2-amino-2,3-dimethylbutanoyl. |

The term "amido", as used herein, means -NHC(=O)-, wherein the amido group is bound to the parent molecular moiety through the nitrogen. Examples of amido include alkylamido such as CH₃C(=O)N(H)- and CH₃CH₂C(=O)N(H)-.

The term "acyl" is a term of art and as used herein refers to any group or radical of the form RCO- where R is any organic group, *e.g.,* alkyl, aryl, heteroaryl, aralkyl, and heteroaralkyl. Representative acyl groups include acetyl, benzoyl, and malonyl.

The term "aminoalkyl" as used herein refers to an alkyl group substituted with one or more one amino groups. In one embodiment, the term "aminoalkyl" refers to an aminomethyl group.

The term "aminoacyl" is a term of art and as used herein refers to an acyl group substituted with one or more amino groups.

The term "azide" or "azido", as used herein, means an -N₃ group.

The term "oxo" refers to the =O radical.

The term "carbonyl" as used herein refers to -C(=O)-.

The term "thiocarbonyl" as used herein refers to -C(=S)-.

The term "alkylthio" as used herein refers to alkyl-S-.

The term "carboxy", as used herein, means a -CO₂H group.

The term "aryl" is a term of art and as used herein refers to includes monocyclic, bicyclic and polycyclic aromatic hydrocarbon groups, for example, benzene, naphthalene, anthracene, 1,2,3,4-tetrahydronaphthalene, indene, 2,3-dihydroindene, and pyrene. The aromatic ring may be substituted at one or more ring positions with one or more substituents, such as halogen, azide, alkyl, aralkyl, alkenyl, alkynyl, cycloalkyl, cycloalkylalkyl, (cycloalkyl)alkoxyl, hydroxyl, alkoxyl, amino, nitro, sulfhydryl, imino, amido, phosphonate, phosphinate, carbonyl, carboxyl, silyl, ether, alkylthio, sulfonyl, aminosulfonyl, sulfonamido, ketone, aldehyde, ester, heterocyclyl, heterocyclylalkyl, aromatic or heteroaromatic moieties, aminoalkyl, haloalkyl, fluoroalkyl (such as trifluoromethyl), haloalkoxyl, cyano, or the like. The term "aryl" also includes polycyclic ring systems having two or more cyclic rings in which two or more carbons are common to two adjoining rings (the rings are "fused rings") wherein at least one of the rings is an aromatic hydrocarbon, *e.g*., the other cyclic rings may be cycloalkyls, cycloalkenyls, cycloalkynyls, aryls, heteroaryls, and/or heterocyclyls. Representative examples of the polycyclic aryl ring systems include, but are not limited to, azulenyl, naphthyl, dihydroinden-1-yl, dihydroinden-2-yl, dihydroinden-3-yl, dihydroinden-4-yl, 2,3-dihydroindol-4-yl, 2,3-dihydroindol-5-yl, 2,3-dihydroindol-6-yl, 2,3-dihydroindol-7-yl, inden-1-yl, inden-2-yl, inden-3-yl, inden-4-yl, dihydronaphthalen-2-yl, dihydronaphthalen-3-yl, dihydronaphthalen-4-yl, dihydronaphthalen-1-yl, 5,6,7,8-tetrahydronaphthalen-1-yl, 5,6,7,8-tetrahydronaphthalen-2-yl, 2,3-dihydrobenzofuran-4-yl, 2,3-dihydrobenzofuran-5-yl, 2,3-dihydrobenzofuran-6-yl, 2,3-dihydrobenzofuran-7-yl, benzo[d][1,3]dioxol-4-yl, benzo[d][1,3]dioxol-5-yl, 2*H*-chromen-2-on-5-yl, 2*H*-chromen-2-on-6-yl, 2*H*-chromen-2-on-7-yl, 2*H*-chromen-2-on-8-yl, isoindoline-1,3-dion-4-yl, isoindoline-1,3-dion-5-yl, inden-1-on-4-yl, inden-1-on-5-yl, inden-1-on-6-yl, inden-1-on-6-yl, 2,3-dihydrobenzo[b][1,4]dioxan-5-yl, 2,3-dihydrobenzo[b][1,4]dioxan-6-yl, 2*H-*benzo[b][1,4]oxazin3(4*H*)-on-5-yl, 2*H*-benzo[b][1,4]oxazin3(4*H*)-on-6-yl, 2*H-*benzo[b][1,4]oxazin3(4*H*)-on-7-yl, 2*H*-benzo[b][1,4]oxazin3(4*H*)-on-8-yl, benzo[d]oxazin-2(3*H*)-on-5-yl, benzo[d]oxazin-2(3*H*)-on-6-yl, benzo[d]oxazin-2(3*H*)-on-7-yl, benzo[d]oxazin-2(3*H*)-on-8-yl, quinazolin-4(3*H*)-on-5-yl, quinazolin-4(3*H*)-on-6-yl, quinazolin-4(3*H*)-on-7-yl, quinazolin-4(3*H*)-on-8-yl, quinoxalin-2(1*H*)-on-5-yl, quinoxalin-2(1*H*)-on-6-yl, quinoxalin-2(1*H*)-on-7-yl, quinoxalin-2(1*H*)-on-8-yl, benzo[d]thiazol-2(3*H*)-on-4-yl, benzo[d]thiazol-2(3*H*)-on-5-yl, benzo[d]thiazol-2(3*H*)-on-6-yl, and, benzo[d]thiazol-2(3*H*)-on-7-yl. In certain embodiments, the bicyclic aryl is (i) naphthyl, or (ii) a phenyl ring fused to either a 5 or 6 membered monocyclic cycloalkyl, a 5 or 6 membered monocyclic cycloalkenyl, or a 5 or 6 membered monocyclic heterocyclyl, wherein the fused cycloalkyl, cycloalkenyl, and heterocyclyl groups are optionally substituted. In certain embodiments, the term "aryl" refers to a phenyl group.

Aryl groups are optionally substituted by one or more substituents independently selected from the group consisting of fluoro, chloro, bromo, iodo, -CF₃, -CCl₃, -CBr₃, -CI₃, -CHF₂, -CHCl₂, - CHBr₂, -CHI₂, -CH₂F, -CH₂Cl, -CH₂Br, -CH₂I, -CN, -OH, -NH₂, -CO₂H, -CONH₂, -NO₂, -SH, - SO₃H, -SO₄H, -SO₂NH₂, -NHNH₂, -ONH₂, -NHC(=O)NHNH₂, -NHC(=O)NH₂, -NHSO₂H, - NHC(=O)H, -NHC(=O)(C₁-C₆)alkyl, -NH(C₁-C₆)alkyl, -N(C₁-C₆)alkyl(C₁-C₆)alkyl, -NHOH, - OCF₃, -OCCl₃, -OCBr₃, -OCI₃, -OCHF₂, -OCHCl₂, -OCHBr₂, -OCHI₂, -OCH₂F, -OCH₂Cl, - OCH₂Br, -OCH₂I, (C₁-C₆)alkyl, (C₁-C₆)alkoxy, (C₁-C₆)haloalkoxy, (C₃-C₈)cycloalkyl, and (C₆-C₁₀)aryl.

The term "heteroaryl" is a term of art and as used herein refers to a monocyclic, bicyclic, and polycyclic aromatic group having 3 to 14, 5 to 14, or 3 to 12 total atoms including one or more heteroatoms such as nitrogen, oxygen, or sulfur in the ring structure. More preferred heteroaryl groups have from 5-10 ring members where from 1-4 of the ring members are hetero atoms selected from the group comprising O, N, and S. Exemplary heteroaryl groups include, for example, azaindolyl, benzo(b)thienyl, benzimidazolyl, benzofuranyl, benzoxazolyl, benzothiazolyl, benzothiadiazolyl, benzotriazolyl, benzoxadiazolyl, furanyl, imidazolyl, imidazopyridinyl, indolyl, indolinyl, indazolyl, isoindolinyl, isoxazolyl, isothiazolyl, isoquinolinyl, oxadiazolyl, oxazolyl, purinyl, pyranyl, pyrazinyl, pyrazolyl, pyridinyl, pyrimidinyl, pyrrolyl, pyrrolo[2,3-d]pyrimidinyl, pyrazolo[3,4-d]pyrimidinyl, quinolinyl, quinazolinyl, triazolyl, thiazolyl, thiophenyl, tetrahydroindolyl, tetrazolyl, thiadiazolyl, thienyl, thiomorpholinyl, triazolyl or tropanyl, and the like. The "heteroaryl" may be substituted at one or more ring positions with one or more substituents such as halogen, azido, alkyl, aralkyl, alkenyl, alkynyl, cycloalkyl, hydroxyl, alkoxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, fluoroalkyl (such as trifluromethyl), cyano, or the like. The term "heteroaryl" also includes polycyclic ring systems having two or more cyclic rings in which two or more carbons are common to two adjoining rings (the rings are "fused rings") wherein at least one of the rings is an aromatic group having one or more heteroatoms in the ring structure, *e.g*., the other cyclic rings may be cycloalkyls, cycloalkenyls, cycloalkynyls, aryls, heteroaryls, and/or heterocyclyls. Representative examples of bicyclic heteroaryl include, but are not limited to, benzimidazolyl, benzofuranyl, benzothienyl, benzoxadiazolyl, benzoxathiadiazolyl, benzothiazolyl, cinnolinyl, 5,6-dihydroquinolin-2-yl, 5,6-dihydroisoquinolin-1-yl, furopyridinyl, indazolyl, indolyl, isoquinolinyl, naphthyridinyl, quinolinyl, purinyl, 5,6,7,8-tetrahydroquinolin-2-yl, 5,6,7,8-tetrahydroquinolin-3-yl, 5,6,7,8-tetrahydroquinolin-4-yl, 5,6,7,8-tetrahydroisoquinolin-1-yl, thienopyridinyl, 4,5,6,7-tetrahydrobenzo[c][1,2,5]oxadiazolyl, and 6,7-dihydrobenzo[c][1,2,5]oxadiazol-4(5*H*)-onyl. Any bicyclic heteroaryl can be optionally substituted as detailed for "heteroaryl" above.

The term "aralkyl", "arylalkyl", or "aryl(C₁-C₆)alkyl" is a term of art and as used herein refers to an alkyl group, for example a C₁-C₆ alkyl group, substituted with an aryl group, wherein the moiety is appended to the parent molecule through the alkyl group.

The term "heteroaralkyl", "heteroarylalkyl", or "heteroaryl(C₁-C₆)alkyl" is a term of art and as used herein refers to an alkyl group, for example a C₁-C₆ alkyl group, substituted with a heteroaryl group, appended to the parent molecular moiety through the alkyl group.

The term "alkoxy" or "alkoxyl" as used herein means an alkyl group, as defined herein, appended to the parent molecular moiety through an oxygen atom. Representative examples of alkoxy include, but are not limited to, methoxy, ethoxy, propoxy, 2-propoxy, butoxy, *tert*-butoxy, pentyloxy, and hexyloxy.

The term "alkoxycarbonyl" means an alkoxy group, as defined herein, appended to the parent molecular moiety through a carbonyl group, represented by -C(=O)-, as defined herein. Representative examples of alkoxycarbonyl include, but are not limited to, methoxycarbonyl, ethoxycarbonyl, and *tert*-butoxycarbonyl.

The term "alkylcarbonyl", as used herein, means an alkyl group, as defined herein, appended to the parent molecular moiety through a carbonyl group, as defined herein. Representative examples of alkylcarbonyl include, but are not limited to, acetyl, 1-oxopropyl, 2,2-dimethyl-1-oxopropyl, 1-oxobutyl, and 1-oxopentyl.

The term "arylcarbonyl", as used herein, means an aryl group, as defined herein, appended to the parent molecular moiety through a carbonyl group, as defined herein. Representative examples of arylcarbonyl include, but are not limited to, benzoyl and (2-pyridinyl)carbonyl.

The term "alkylcarbonyloxy" and "arylcarbonyloxy", as used herein, means an alkylcarbonyl or arylcarbonyl group, as defined herein, appended to the parent molecular moiety through an oxygen atom. Representative examples of alkylcarbonyloxy include, but are not limited to, acetyloxy, ethylcarbonyloxy, and *tert*-butylcarbonyloxy. Representative examples of arylcarbonyloxy include, but are not limited to phenylcarbonyloxy.

The term "alkenoxy" or "alkenoxyl" means an alkenyl group, as defined herein, appended to the parent molecular moiety through an oxygen atom. Representative examples of alkenoxyl include, but are not limited to, 2-propen-1-oxyl (*i.e*., CH₂=CH-CH₂-O-) and vinyloxy (*i.e*., CH₂=CH-O-).

The term "aryloxy" as used herein means an aryl group, as defined herein, appended to the parent molecular moiety through an oxygen atom.

The term "heteroaryloxy" as used herein means a heteroaryl group, as defined herein, appended to the parent molecular moiety through an oxygen atom.

The terms "cyano" and "nitrile" are terms of art and as used herein refer to -CN.

The term "nitro", as used herein, means -NO₂.

The terms "halo" and "halogen" are terms of art and as used herein refer to -F, -Cl, -Br, or -I.

The term "haloalkyl" as used herein refers to an alkyl group, as defined herein, wherein some or all of the hydrogens are replaced with halogen atoms. The term "haloalkoxyl" refers to an alkoxy group, as defined herein, wherein some or all of the hydrogens are replaced with halogen atoms. An exemplary haloalkyl group is trifluoromethyl.

The terms "hydroxy" and "hydroxyl" are a term of art and as used herein refer to -OH.

The term "hydroxyalkyl", as used herein, means at least one hydroxy group, as defined herein, is appended to the parent molecular moiety through an alkyl group, as defined herein. Representative examples of hydroxyalkyl include, but are not limited to, hydroxymethyl, 2-hydroxyethyl, 3-hydroxypropyl, 2,3-dihydroxypentyl, and 2-ethyl-4-hydroxyheptyl.

The term "polyol", as used herein, denotes an organic compound containing more than one hydroxy group, an no other functional groups. Representative examples of polyols include, but are not limited to, glycerol, erythritol, xylitol, sorbitol, mannitol, and pinanediol.

Certain compounds contained in compositions of the present invention may exist in particular geometrical isomer or stereoisomeric forms. In addition, compounds of the present invention may also be optically active. The present invention contemplates all such compounds, including *cis-* and *trans*-isomers, (*R*)*-* and (*S*)-enantiomers, diastereoisomers, D-isomers, L-isomers, the racemic mixtures thereof, and other mixtures thereof, as falling within the scope of the invention. Additional asymmetric carbon atoms may be present in a substituent such as an alkyl group. All such isomers, as well as mixtures thereof, are intended to be included in this invention.

If, for instance, a particular enantiomer of compound of the present invention is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically-active acid or base, followed by resolution of the diastereomers thus formed by fractional crystallization or chromatographic means well known in the art, and subsequent recovery of the pure enantiomers.

"Optional" or "optionally" means that a subsequently described event or circumstance may or may not occur and that the description includes instances when the event or circumstance occurs and instances in which it does not. For example, "optionally substituted aryl" means that the aryl radical may or may not be substituted and that the description includes both substituted aryl radicals and aryl radicals having no substitution.

It will be understood that "substitution" or "substituted with" includes the implicit proviso that such substitution is in accordance with permitted valence of the substituted atom and the substituent, and that the substitution results in a stable compound, *e.g*., which does not spontaneously undergo transformation such as by rearrangement, fragmentation, decomposition, cyclization, elimination, or other reaction.

The term "substituted" is also contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, halogen, azide, alkyl, aralkyl, alkenyl, alkynyl, cycloalkyl, cycloalkylalkyl, (cycloalkyl)alkoxyl, hydroxyl, alkoxyl, amino, nitro, sulfhydryl, imino, amido, phosphonate, phosphinate, carbonyl, carboxyl, silyl, ether, alkylthio, sulfonyl, aminosulfonyl, sulfonamido, ketone, aldehyde, ester, heterocyclyl, heterocyclylalkyl, aromatic or heteroaromatic moieties, aminoalkyl, haloalkyl, fluoroalkyl (such as trifluoromethyl), haloalkoxyl, cyano, or other substituents described above. The permissible substituents may be one or more and the same or different for appropriate organic compounds. For purposes of this invention, the heteroatoms such as nitrogen may have hydrogen substituents and/or any permissible substituents of organic compounds described herein which satisfy the valences of the heteroatoms. This invention is not intended to be limited in any manner by the permissible substituents of organic compounds.

The phrase "protecting group", as used herein, means temporary substituents which protect a potentially reactive functional group from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M. Protective Groups in Organic Synthesis, 2nd ed.; Wiley: New York, 1991).

A "saturated" or "fully saturated" compound means that the referenced chemical structure does not contain any multiple carbon-carbon bonds. For example, a saturated cycloalkyl group as defined herein includes cyclohexyl, cyclopropyl, and the like.

An "unsaturated" or "partially saturated" compound means that the referenced chemical structure may contains on or more multiple carbon-carbon bonds, but is not aromatic. For example, an unsaturated cycloalkyl group as defined herein includes cyclohexenyl, cyclopentenyl, cyclohexadienyl, and the like.

For purposes of the invention, the chemical elements are identified in accordance with the Periodic Table of the Elements, CAS version, Handbook of Chemistry and Physics, 67th ed., 1986-87, inside cover.

It will be apparent to one skilled in the art that certain compounds of this disclosure may exist in tautomeric forms, all such tautomeric forms of the compounds being within the scope of the disclosure. A "tautomer" refers to a molecule wherein a proton shift from one atom of a molecule to another atom of the same molecule is possible. In certain embodiments, the compounds presented herein exist as tautomers. In circumstances where tautomerization is possible, a chemical equilibrium of tautomers will exist.

Unless otherwise stated, structures depicted herein are also meant to include all stereochemical forms of the structure; *i.e.,* the *R* and *S* configurations for each asymmetric center. Therefore, single stereochemical isomers as well as enantiomeric and diastereomeric mixtures of the present compounds are within the scope of the disclosure. Both the *R* and the *S* stereochemical isomers, as well as all mixtures thereof, are included within the scope of the disclosure. A "racemic mixture" or "racemate" denotes equimolar mixture of two enantiomers, whereas a "scalemic mixture" denotes non-racemic mixture of two enantiomers.

The chemical structure of examples that are a mixture of diastereoisomers or a single diastereoisomer but with unknown relative configuration are drawn and named without defined stereochemical configuration.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The term "pharmaceutically acceptable salt" as used herein includes salts derived from inorganic or organic acids including, for example, hydrochloric, hydrobromic, sulfuric, nitric, perchloric, phosphoric, formic, acetic, lactic, maleic, fumaric, pamoic (embonic), succinic, tartaric, glycolic, salicylic, citric, methanesulfonic, benzenesulfonic, benzoic, malonic, trifluoroacetic, trichloroacetic, naphthalene-2-sulfonic, and other acids. Pharmaceutically acceptable salt forms can include forms wherein the ratio of molecules comprising the salt is not 1:1. For example, the salt may comprise more than one inorganic or organic acid molecule per molecule of base, such as two hydrochloric acid molecules per molecule of compound of Formula (Ia). As another example, the salt may comprise less than one inorganic or organic acid molecule per molecule of base, such as two molecules of compound of Formula (Ia) per molecule of tartaric acid.

The compounds according to the invention can form solvates with a stoichiometric or non-stoichiometric amount of one or more solvents, such as water, ethanol, diethyl ether, or ethyl acetate. The solvates formed with water ale called hydrates.

As used herein, a protic solvent is a solvent that has a hydrogen atom bound to an oxygen atom (as in a hydroxyl group) or a nitrogen atom (as in an amine group). In general terms, any solvent that contains labile H⁺ is called a protic solvent. The molecules of such solvents readily donate protons (H⁺) to reagents. In contrast, an aprotic solvent is a solvent that does not have a hydrogen atom bound to an oxygen (as in a hydroxyl group) or a nitrogen (as in an amine group), and it cannot donate hydrogen.

As used herein, a polar protic solvent is a protic solvent that will dissolve many salts. In general, these solvents have high dielectric constants and high polarity. Non-limiting examples of polar protic solvents include acetic acid, ammonia, ethanol, formic acid, isopropanol, methanol, *n*-butanol, nitromethane, *n*-propanol, *tert*-butanol, and water.

As used herein, a polar aprotic solvent is a solvent that will dissolve many salts, but lacks an acidic hydrogen; these solvents generally have intermediate to high dielectric constants and polarity. Non-limiting examples of polar aprotic solvents include acetone, acetonitrile, dichloromethane (DCM), dimethyl sulfoxide (DMSO), ethyl acetate, hexamethylphosphoric triamide (HMPT), *N,N*-dimethylformamide (DMF), and tetrahydrofuran (THF).

As used herein, a nonpolar aprotic solvent is a solvent that will dissolve many salts, but lacks an acidic hydrogen; these solvents generally have low dielectric constants and polarity. Non-limiting examples of nonpolar aprotic solvents include benzene, chloroform, cyclohexane, diethyl ether, hexane, pentane, and toluene.

A physician or veterinarian having ordinary skill in the art can readily determine and prescribe the therapeutically effective amount of the pharmaceutical composition required. For example, the physician or veterinarian could start doses of the pharmaceutical composition or compound at levels lower than that required in order to achieve the desired therapeutic effect and gradually increase the dosage until the desired effect is achieved. By "therapeutically effective amount" is meant the concentration of a compound that is sufficient to elicit the desired therapeutic effect. It is generally understood that the effective amount of the compound will vary according to the weight, sex, age, and medical history of the subject. Other factors which influence the effective amount may include, but are not limited to, the severity of the patient's condition, the disorder being treated, the stability of the compound, the mode of administration, the bioavailability of the particular compound, and, if desired, another type of therapeutic agent being administered with the compound of the invention. A larger total dose can be delivered by multiple administrations of the agent. Methods to determine efficacy and dosage are known to those skilled in the art (Isselbacher et al. (1996) Harrison's Principles of Internal Medicine 13 ed., 1814-1882).

"Modulating" or "modulate" refers to the treating, prevention, suppression, enhancement or induction of a function, condition or disorder.

The term "treating" includes prophylactic and/or therapeutic treatments. The term "prophylactic or therapeutic" treatment is art-recognized and includes administration to the host of one or more of the subject compositions. If it is administered prior to clinical manifestation of the unwanted condition (*e.g*., disease or other unwanted state of the host animal) then the treatment is prophylactic, in other words - preventive, (*i.e*., it protects the host against developing the unwanted condition), whereas if it is administered after manifestation of the unwanted condition, the treatment is therapeutic, (*i.e*., it is intended to diminish, ameliorate, or stabilize the existing unwanted condition or side effects thereof). Thus, in certain aspects, the disclosure encompasses non-prophylactic treatment, i.e., therapeutic treatment.

As used herein, "subject" refers to a warm blooded animal such as a mammal, preferably a human, or a human child, which is afflicted with, or has the potential to be afflicted with one or more diseases and disorders described herein.

"EC₅₀" refers to a dosage, concentration or amount of a particular test compound that elicits a dose-dependent response at 50% of maximal expression of a particular response that is induced, provoked or potentiated by the particular test compound.

"IC₅₀" refers to an amount, concentration or dosage of a particular test compound that achieves a 50% inhibition of a maximal response in an assay that measures such response.

### Compounds of the Invention

In one aspect, the invention provides a compound of structural Formula (Ia): 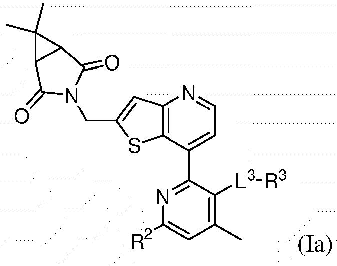 wherein:
R² is selected from hydrogen, methyl, ethyl, chloro, -C≡N, and -CF₃;
L³ represents a single bond, -O-, -CH₂-, -C(=O)-, -NH-, or -NH-C(=O)-; and
R³ is selected from (4-6) membered heterocycloalkyl containing 1 to 2 nitrogen heteroatoms or 1 nitrogen heteroatom and 1 oxygen heteroatom, and optionally substituted with 1 or 2 substituents selected from fluoro, methyl, ethyl, amino, methylamino, dimethylamino, ethylamino, isopropylamino, hydroxy, oxo, aminomethyl, 1-aminoethyl, (isopropylamino)methyl, ureido, alanylamino, valylamino, fluoromethyl, difluoromethyl, trifluoromethyl, and 2,2,2-trifluoroacetyl;
or a tautomer, stereoisomer, a racemic or scalemic mixture of stereoisomers, a pharmaceutically acceptable salt, ester, or solvate thereof.

In certain embodiments, the compound according to the invention is:
6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile;
3-((7-(6-chloro-4-methyl-3-(pyrrolidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(((*S*)-piperidin-3-yl)oxy)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-(pyrrolidin-3-ylamino)picolinonitrile;
5-(azetidin-3-ylamino)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
*N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide;
6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione;
3-((7-(3-((3,6-dioxopiperazin-2-yl)methyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-((3-oxomorpholino)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((2-oxopyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((4-methyl-2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-5-(3-(dimethylamino)azetidine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-((*S*)-3-(dimethylamino)pyrrolidine-1-carbonyl)-4-methylpicolinonitrile;
6,6-dimethyl-3-((7-(4-methyl-3-(piperazine-1-carbonyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(piperazin-1-ylmethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-((*S*)-2-methylpiperazine-1-carbonyl)picolinonitrile;
6,6-dimethyl-3-((7-(4-methyl-3-(morpholine-4-carbonyl)pyridin-2-yl)thieno[3,2-*b*]pyridin-2-yl)methyl)-3-azabicyclo[3.1.0]hexane-2,4-dione;
3-((7-(3-(3,3-difluoropyrrolidine-1-carbonyl)-4-methylpyridin-2-yl)thieno[3,2-*b]*pyridin-2-yl)methyl)-6,6-dimethyl-3-azabicyclo[3.1.0]hexane-2,4-dione;
3-((7-(3-(3,3-difluoroazetidine-1-carbonyl)-4-methylpyridin-2-yl)thieno[3,2-*b*]pyridin-2-yl)methyl)-6,6-dimethyl-3-azabicyclo[3.1.0]hexane-2,4-dione;
5-(3,3-difluoroazetidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-((*S*)-2,4-dimethylpiperazine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-(3,3-dimethylpiperazine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-((*R*)-2-methylpiperazine-1-carbonyl)picolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-(piperazine-1-carbonyl)picolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-(morpholine-4-carbonyl)picolinonitrile;
5-(3,3-difluoropyrrolidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
3-((7-(3-((*S*)-3-(ethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(isopropylamino)pyrrolidine- 1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperazine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((*S*)-3-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((*S*)-2-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-2,4-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(4-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-3-aminopyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(dimethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3,3-difluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(morpholine-4-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-(3,3-difluoroazetidine-1-carbonyl)-4,6-dimethylpyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitrile;
6,6-dimethyl-3-((7-(4-methyl-3-(((*S*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*R*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*S*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*R*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-3-(difluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(fluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*S*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*R*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
(2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)-3-methylbutanamide;
(2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)propanamide;
3-((7-(3-(4-aminopiperidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-hydroxypyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*R*)-3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*R*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(2-oxotetrahydropyrimidin- 1(2*H*)-yl)pyridin-2-yl)thieno[3,2-*b*]pyridin-2-yl)methyl)-6,6-dimethyl-3-azabicyclo[3.1.0]hexane-2,4-dione;
3-((7-(3-(azetidin-3-ylamino)-6-chloro-4-methylpyridin-2-yl)thieno[3,2-*b*]pyridin-2-yl)methyl)-6,6-dimethyl-3-azabicyclo[3.1.0]hexane-2,4-dione;
3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-4-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-4-ylamino)-6-(trifluoromethyl)pyridin-2-yl)thieno[3,2-*b*]pyridin-2-yl)methyl)-3-azabicyclo[3.1.0]hexane-2,4-dione;
3-((7-(6-chloro-4-methyl-3-(piperidin-3-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-3-ylamino)-6-(trifluoromethyl)pyridin-2-yl)thieno[3,2-*b*]pyridin-2-yl)methyl)-3-azabicyclo[3.1.0]hexane-2,4-dione;
*N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)azetidine-3-carboxamide;
*N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)piperidine-4-carboxamide;
3-((7-(6-chloro-4-methyl-3-(piperazine-1-carbonyl)pyridin-2-yl)thieno[3,2-*b*]pyridin-2-yl)methyl)-6,6-dimethyl-3-azabicyclo[3.1.0]hexane-2,4-dione;
1-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)urea;
3-((7-(3-(3-amino-3-methylpyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((3*S*)-3-(1-aminoethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3-((isopropylamino)methyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((2*S*,6*S*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((2*R*,6*R*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(4-ethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione; or
6,6-dimethyl-3-((7-(4-methyl-3-((*S*)-3-(methylamino)piperidine-1-carbonyl)-6-(trifluoromethyl)pyridin-2-yl)thieno[3,2-*b*]pyridin-2-yl)methyl)-3-azabicyclo[3.1.0]hexane-2,4-dione;
or a tautomer, stereoisomer, a racemic or scalemic mixture of stereoisomers, a pharmaceutically acceptable salt, ester, or solvate thereof.

In certain embodiments, the compound according to the invention is:
6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile;
3-((7-(6-chloro-4-methyl-3-(pyrrolidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(((*S*)-piperidin-3-yl)oxy)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-(pyrrolidin-3-ylamino)picolinonitrile;
5-(azetidin-3-ylamino)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
*N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide;
6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-((*S*)-2-methylpiperazine-1-carbonyl)picolinonitrile;
5-(3,3-difluoroazetidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-((*S*)-2,4-dimethylpiperazine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-(piperazine-1-carbonyl)picolinonitrile;
5-(3,3-difluoropyrrolidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
3-((7-(3-((*S*)-3-(ethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(isopropylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperazine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((*S*)-3-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((*S*)-2-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-2,4-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(4-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-3-aminopyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(dimethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3,3-difluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione; 6-(2-((6,6-dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitrile;
6,6-dimethyl-3-((7-(4-methyl-3-(((*S*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*R*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*R*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-3-(difluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(fluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*S*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*R*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
(2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)-3-methylbutanamide;
3-((7-(3-(4-aminopiperidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-hydroxypyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*R*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(azetidin-3-ylamino)-6-chloro-4-methylpyridin-2-yl)thieno[3,2-*b*]pyridin-2-yl)methyl)-6,6-dimethyl-3-azabicyclo[3.1.0]hexane-2,4-dione;
3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-4-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-4-ylamino)-6-(trifluoromethyl)pyridin-2-yl)thieno[3,2-*b*]pyridin-2-yl)methyl)-3-azabicyclo[3.1.0]hexane-2,4-dione;
3-((7-(6-chloro-4-methyl-3-(piperidin-3-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-3-ylamino)-6-(trifluoromethyl)pyridin-2-yl)thieno[3,2-*b*]pyridin-2-yl)methyl)-3-azabicyclo[3.1.0]hexane-2,4-dione;
3-((7-(3-(3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3-((isopropylamino)methyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((2*S*,6*S*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione; or
3-((7-(3-(4-ethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
or a tautomer, stereoisomer, a racemic or scalemic mixture of stereoisomers, a pharmaceutically acceptable salt, ester, or solvate thereof.

In certain embodiments, the compound according to the invention is:
6,6-dimethyl-3-((7-(4-methyl-3-(((*S*)-3-(methylamino)pyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((3-oxopiperazin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((3*R*,4*S*)-3-methyl-4-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((3*R*,4*S*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*S*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione; or
3-((7-(3-((3*S*,4*R*)-3-(dimethylamino)-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
or a tautomer, stereoisomer, a racemic or scalemic mixture of stereoisomers, a pharmaceutically acceptable salt, ester, or solvate thereof.

The following representative structures of compounds of Formula (Ia), mainly in the form of salts thereof, are disclosed herein.

| Ex. No. | Structure | IUPAC Name |
|---|---|---|
| 1. | 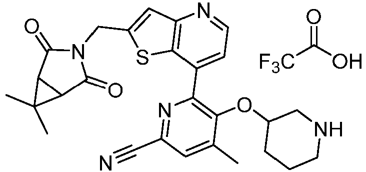 | 6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile 2,2,2-trifluoroacetate |
| 2. | 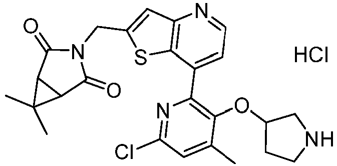 | 3-((7-(6-chloro-4-methyl-3-(pyrrolidin-3-yloxy)pyridin-2-yl)thieno[3,2-*b*]pyridin-2-yl)methyl)-6,6-dimethyl-3-azabicyclo[3.1.0]hexane-2,4-dione hydrochloride |
| 3. | 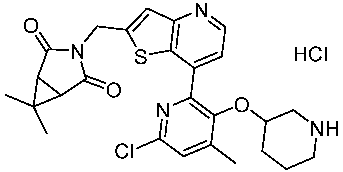 Racemate | 3-((7-(6-chloro-4-methyl-3-(piperidin-3-yloxy)pyridin-2-yl)thieno[3,2-*b*]pyridin-2-yl)methyl)-6,6-dimethyl-3-azabicyclo[3.1.0]hexane-2,4-dione hydrochloride |
| 4. | 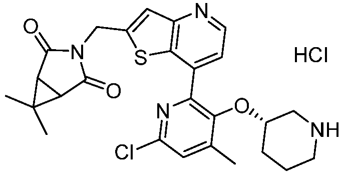 Single enantiomer of Ex. 3 | 3-((7-(6-chloro-4-methyl-3-(((*S*)-piperidin-3-yl)oxy)pyridin-2-yl)thieno[3,2-*b*]pyridin-2-yl)methyl)-6,6-dimethyl-3-azabicyclo[3.1.0]hexane-2,4-dione hydrochloride |
| 18. | 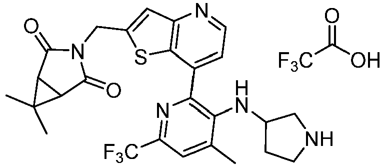 | 6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione 2,2,2-trifluoroacetate |
| 20. | 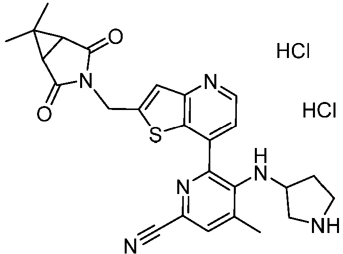 | 6-(2-((6,6-dimethyl-2,4-dioxo-3-azabicyclo[3.1.0]hexan-3-yl)methyl)thieno[3,2-*b*]pyridin-7-yl)-4-methyl-5-(pyrrolidin-3-ylamino)picolinonitrile dihydrochloride |
| 24. | 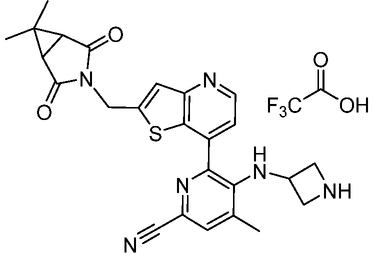 | 5-(azetidin-3-ylamino)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile 2,2,2-trifluoroacetate |
| 28. | 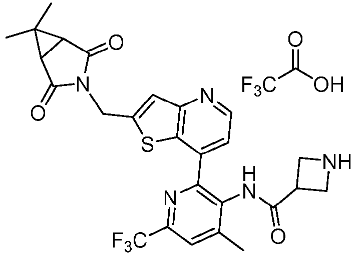 | *N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide 2,2,2-trifluoroacetate |
| 35. | 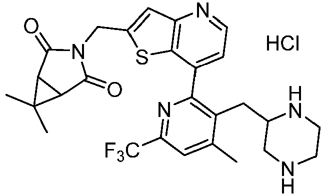 Racemate | 6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione hydrochloride |
| 37. | 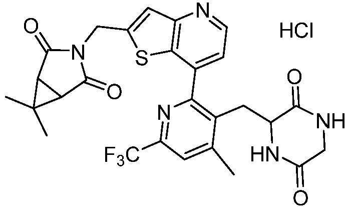 | 3-((7-(3-((3,6-dioxopiperazin-2-yl)methyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 38. | 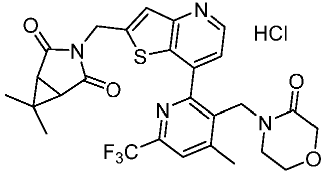 | 6,6-dimethyl-3-((7-(4-methyl-3-((3-oxomorpholino)methyl)-6-(trifluoromethyl)pyridin-2-yl)thieno[3,2-*b*]pyridin-2-yl)methyl)-3-azabicyclo[3.1.0]hexane-2,4-dione hydrochloride |
| 39. | 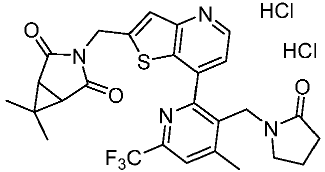 | 6,6-dimethyl-3-((7-(4-methyl-3-((2-oxopyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 40. | 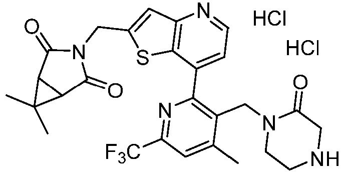 | 6,6-dimethyl-3-((7-(4-methyl-3-((2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 41. | 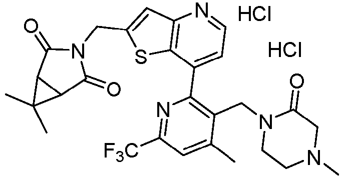 | 6,6-dimethyl-3-((7-(4-methyl-3-((4-methyl-2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 43. | 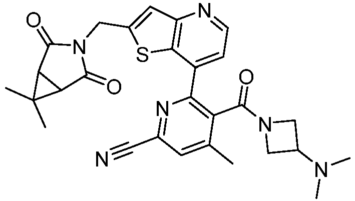 | 6-(2-((6,6-dimethyl-2,4-dioxo-3-azabicyclo[3.1.0]hexan-3-yl)methyl)thieno[3,2-*b*]pyridin-7-yl)-5-(3-(dimethylamino)azetidine-1-carbonyl)-4-methylpicolinonitrile |
| 44. | 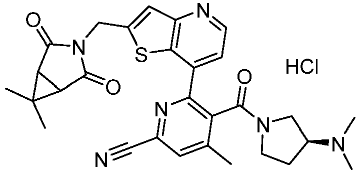 | 6-(2-((6,6-dimethyl-2,4-dioxo-3-azabicyclo[3.1.0]hexan-3-yl)methyl)thieno[3,2-*b*]pyridin-7-yl)-5-((S)-3-(dimethylamino)pyrrolidine-1-carbonyl)-4-methylpicolinonitrile hydrochloride |
| 45. | 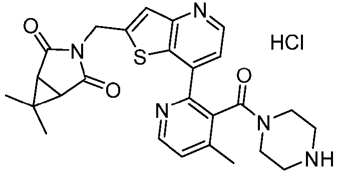 | 6,6-dimethyl-3-((7-(4-methyl-3-(piperazine-1-carbonyl)pyridin-2-yl)thieno[3,2-*b*]pyridin-2-yl)methyl)-3-azabicyclo[3.1.0]hexane-2,4-dione hydrochloride |
| 46. | 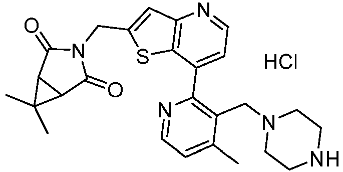 | 6,6-dimethyl-3-((7-(4-methyl-3-(piperazin-1-ylmethyl)pyridin-2-yl)thieno[3,2-*b*]pyridin-2-yl)methyl)-3-azabicyclo[3.1.0]hexane-2,4-dione hydrochloride |
| 47. | 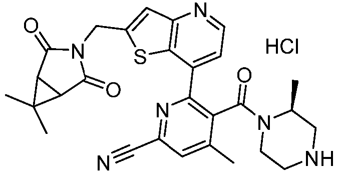 | 6-(2-((6,6-dimethyl-2,4-dioxo-3-azabicyclo[3.1.0]hexan-3-yl)methyl)thieno[3,2-*b*]pyridin-7-yl)-4-methyl-5-((*S*)-2-methylpiperazine-1-carbonyl)picolinonitrile hydrochloride |
| 48. | 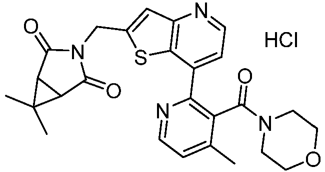 | 6,6-dimethyl-3-((7-(4-methyl-3-(morpholine-4-carbonyl)pyridin-2-yl)thieno[3,2-*b*]pyridin-2-yl)methyl)-3-azabicyclo[3.1.0]hexane-2,4-dione hydrochloride |
| 49. | 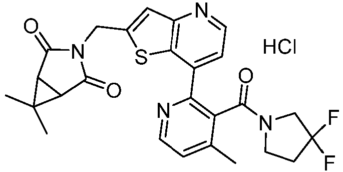 | 3-((7-(3-(3,3-difluoropyrrolidine-1-carbonyl)-4-methylpyridin-2-yl)thieno[3,2-*b*]pyridin-2-yl)methyl)-6,6-dimethyl-3-azabicyclo[3.1.0]hexane-2,4-dione hydrochloride |
| 50. | 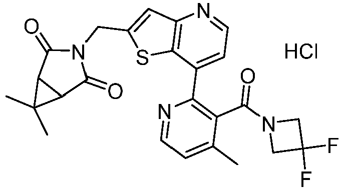 | 3-((7-(3-(3,3-difluoroazetidine-1-carbonyl)-4-methylpyridin-2-yl)thieno[3,2-*b*]pyridin-2-yl)methyl)-6,6-dimethyl-3-azabicyclo[3.1.0]hexane-2,4-dione hydrochloride |
| 51. | 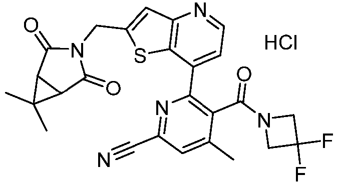 | 5-(3,3-difluoroazetidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile hydrochloride |
| 52. | 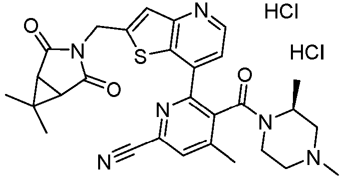 | 6-(2-((6,6-dimethyl-2,4-dioxo-3-azabicyclo[3.1.0]hexan-3-yl)methyl)thieno[3,2-*b*]pyridin-7-yl)-5-((*S*)-2,4-dimethylpiperazine-1-carbonyl)-4-methylpicolinonitrile dihydrochloride |
| 53. | 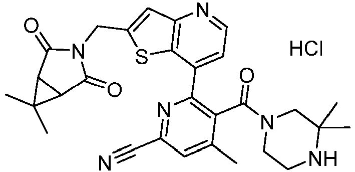 | 6-(2-((6,6-dimethyl-2,4-dioxo-3-azabicyclo[3.1.0]hexan-3-yl)methyl)thieno[3,2-*b*]pyridin-7-yl)-5-(3,3-dimethylpiperazine-1-carbonyl)-4-methylpicolinonitrile hydrochloride |
| 54. | 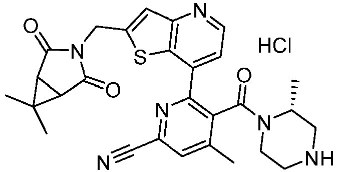 | 6-(2-((6,6-dimethyl-2,4-dioxo-3-azabicyclo[3.1.0]hexan-3-yl)methyl)thieno[3,2-*b*]pyridin-7-yl)-4-methyl-5-((*R*)-2-methylpiperazine-1-carbonyl)picolinonitrile hydrochloride |
| 55. | 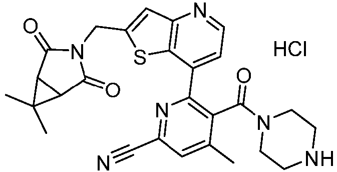 | 6-(2-((6,6-dimethyl-2,4-dioxo-3-azabicyclo[3.1.0]hexan-3-yl)methyl)thieno[3,2-*b*]pyridin-7-yl)-4-methyl-5-(piperazine-1-carbonyl)picolinonitrile hydrochloride |
| 58. | 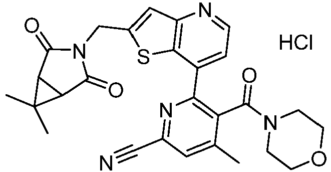 | 6-(2-((6,6-dimethyl-2,4-dioxo-3-azabicyclo[3.1.0]hexan-3-yl)methyl)thieno[3,2-*b*]pyridin-7-yl)-4-methyl-5-(morpholine-4-carbonyl)picolinonitrile hydrochloride |
| 59. | 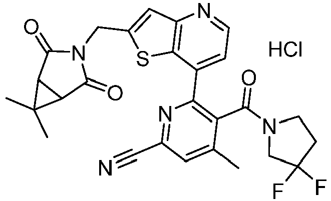 | 5-(3,3-difluoropyrrolidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile hydrochloride |
| 73. | 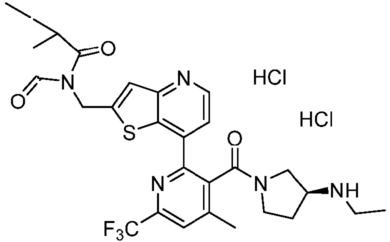 | 3-((7-(3-((*S*)-3-(ethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 74. | 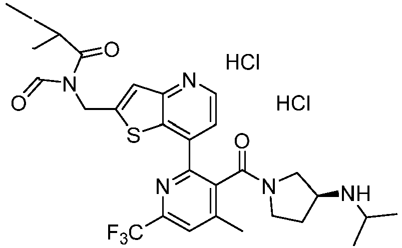 | 3-((7-(3-((*S*)-3-(isopropylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 76. | 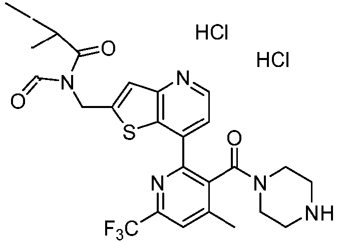 | 6,6-dimethyl-3-((7-(4-methyl-3-(piperazine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride |
| 77. | 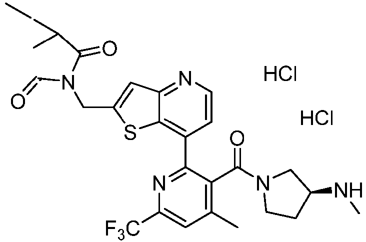 | 6,6-dimethyl-3-((7-(4-methyl-3-((*S*)-3-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride |
| 78. | 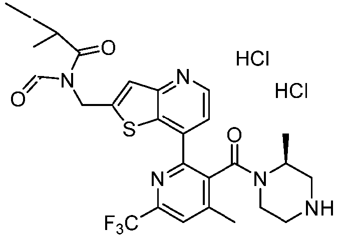 | 6,6-dimethyl-3-((7-(4-methyl-3-((*S*)-2-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride |
| 79. | 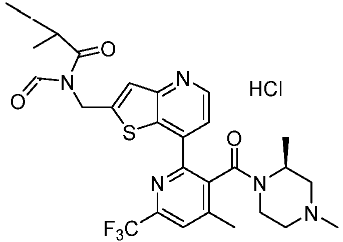 | 3-((7-(3-((*S*)-2,4-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 80. | 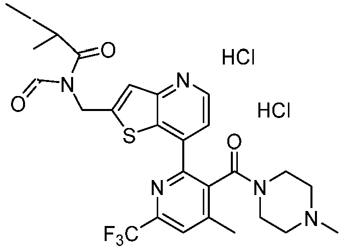 | 6,6-dimethyl-3-((7-(4-methyl-3-(4-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride |
| 81. | 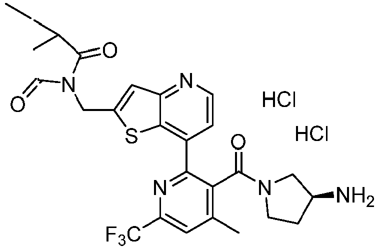 | 3-((7-(3-((*S*)-3-aminopyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 82. | 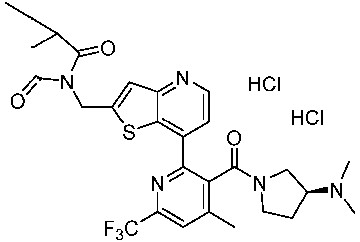 | 3-((7-(3-((*S*)-3-(dimethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 83. | 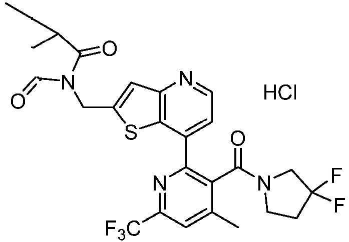 | 3-((7-(3-(3,3-difluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 84. | 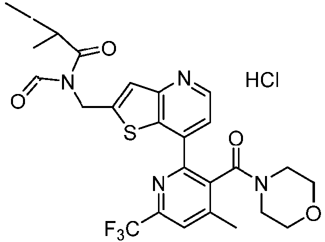 | 6,6-dimethyl-3-((7-(4-methyl-3-(morpholine-4-carbonyl)-6-(trifluoromethyl)pyridin-2-yl)thieno[3,2-*b*]pyridin-2-yl)methyl)-3-azabicyclo[3.1.0]hexane-2,4-dione hydrochloride |
| 85. | 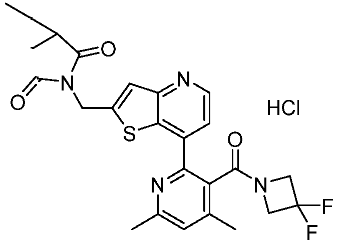 | 3-((7-(3-(3,3-difluoroazetidine-1-carbonyl)-4,6-dimethylpyridin-2-yl)thieno[3,2-*b*]pyridin-2-yl)methyl)-6,6-dimethyl-3-azabicyclo[3.1.0]hexane-2,4-dione hydrochloride |
| 92. | 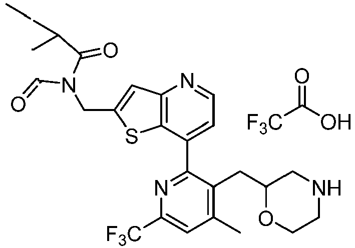 Racemate | 6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione 2,2,2-trifluoroacetate |
| 94. | 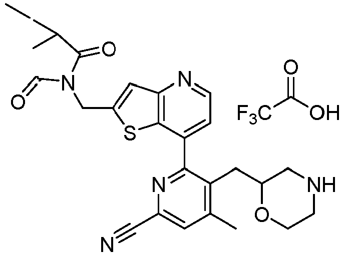 Racemate | 6-(2-((6,6-dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitrile 2,2,2-trifluoroacetate |
| 95. | 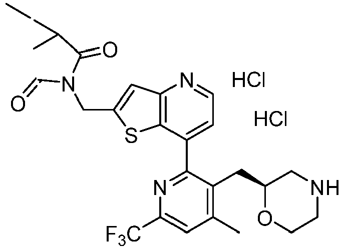 | 6,6-dimethyl-3-((7-(4-methyl-3-(((S)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 96. | 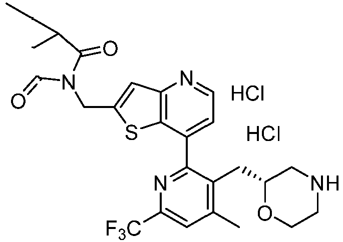 | 6,6-dimethyl-3-((7-(4-methyl-3-(((*R*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 97. | 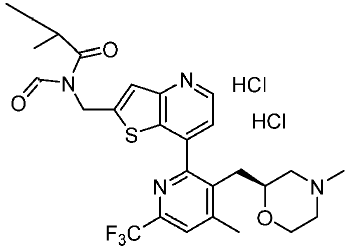 | 6,6-dimethyl-3-((7-(4-methyl-3-(((*S*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 98. | 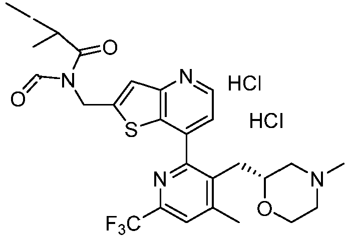 | 6,6-dimethyl-3-((7-(4-methyl-3-(((*R*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride |
| 104. | 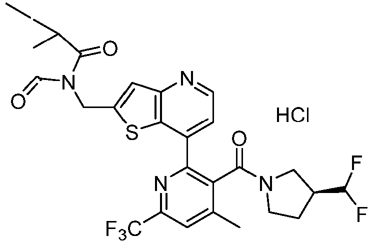 | 3-((7-(3-((*S*)-3-(difluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 105. | 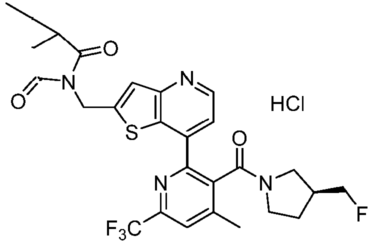 | 3-((7-(3-((*S*)-3-(fluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 106. | 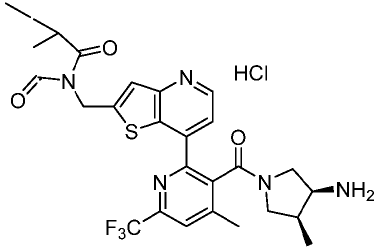 | 3-((7-(3-((3*S*,4*S*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 107. | 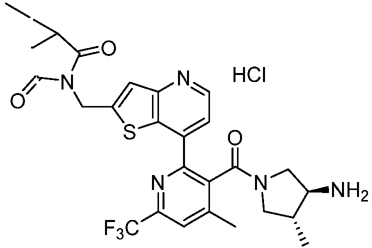 | 3-((7-(3-((3*S*,4*R*)-3-amino-4-methylpyrrolidine-1-carbonyl)-4-methyl-6-(trifluoromethyl)pyridin-2-yl)thieno[3,2-b*]*pyridin-2-yl)methyl)-6,6-dimethyl-3-azabicyclo[3.1.0]hexane-2,4-dione hydrochloride |
| 109. | 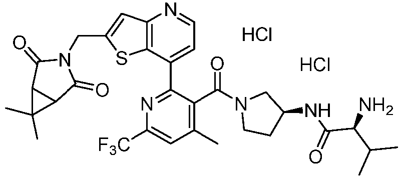 | (2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)-3-methylbutanamide dihydrochloride |
| 110. | 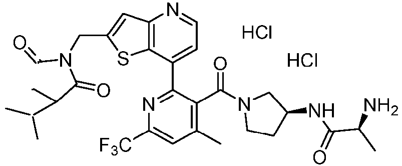 | (2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-vl)propanamide dihydrochloride |
| 112. | 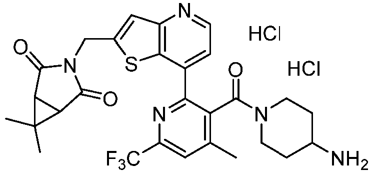 | 3-((7-(3-(4-aminopiperidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 113. | 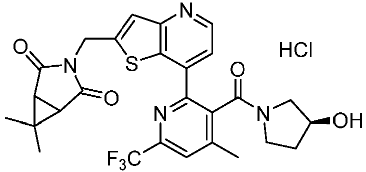 | 3-((7-(3-((*S*)-3-hydroxypyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 114. | 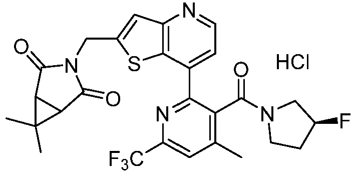 | 3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 115. | 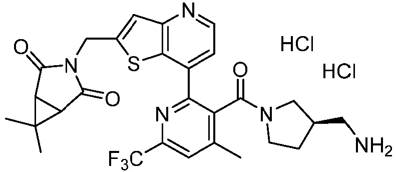 | 3-((7-(3-((*R*)-3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 117. | 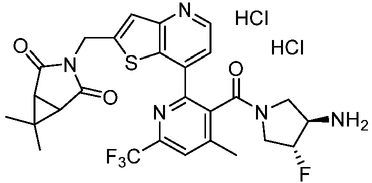 | 3-((7-(3-((3*R*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride |
| 121. | 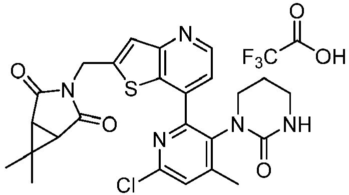 | 3-((7-(6-chloro-4-methyl-3-(2-oxotetrahydropyrimidin-1(2*H*)-yl)pyridin-2-yl)thieno[3,2-*b*]pyridin-2-yl)methyl)-6,6-dimethyl-3-azabicyclo[3.1.0]hexane-2,4-dione 2,2,2-trifluoroacetate |
| 122. | 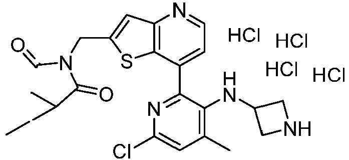 | 3-((7-(3-(azetidin-3-ylamino)-6-chloro-4-methylpyridin-2-yl)thieno[3,2-*b*]pyridin-2-yl)methyl)-6,6-dimethyl-3-azabicyclo[3.1.0]hexane-2,4-dione tetrahydrochloride |
| 124. | 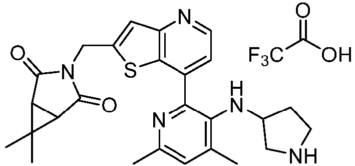 Racemate | 3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione 2,2,2-trifluoroacetate |
| 126. | 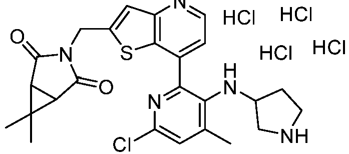 Racemate | 3-((7-(6-chloro-4-methyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione tetrahydrochloride |
| 127. | 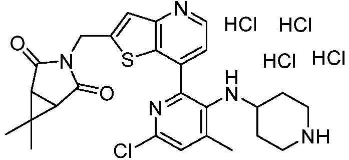 | 3-((7-(6-chloro-4-methyl-3-(piperidin-4-ylamino)pyridin-2-yl)thieno[3,2-*b*]pyridin-2-yl)methyl)-6,6-dimethyl-3-azabicyclo[3.1.0]hexane-2,4-dione tetrahydrochloride |
| 128. | 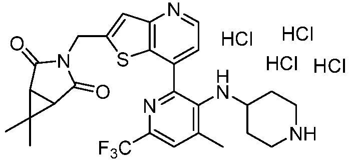 | 6,6-dimethyl-3-((7-(4-methyl-3-(piperidin-4-ylamino)-6-(trifluoromethyl)pyridin-2-yl)thieno[3,2-*b*]pyridin-2-yl)methyl)-3-azabicyclo[3.1.0]hexane-2,4-dione tetrahydrochloride |
| 129. | 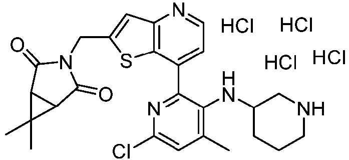 Racemate | 3-((7-(6-chloro-4-methyl-3-(piperidin-3-ylamino)pyridin-2-yl)thieno[3,2-*b*]pyridin-2-yl)methyl)-6,6-dimethyl-3-azabicyclo[3.1.0]hexane-2,4-dione tetrahydrochloride |
| 130. | 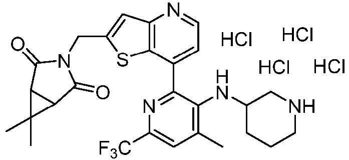 Racemate | 6,6-dimethyl-3-((7-(4-methyl-3-(piperidin-3-ylamino)-6-(trifluoromethyl)pyridin-2-yl)thieno[3,2-*b*]pyridin-2-yl)methyl)-3-azabicyclo[3.1.0]hexane-2,4-dione tetrahydrochloride |
| 133. | 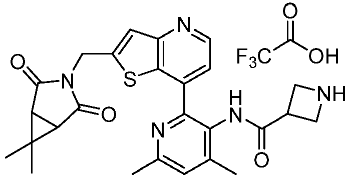 | *N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)azetidine-3-carboxamide 2,2,2-trifluoroacetate |
| 134. | 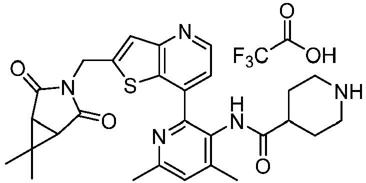 | *N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)piperidine-4-carboxamide 2,2,2-trifluoroacetate |
| 135. | 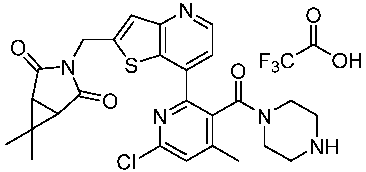 | 3-((7-(6-chloro-4-methyl-3-(piperazine-1-carbonyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate |
| 136. | 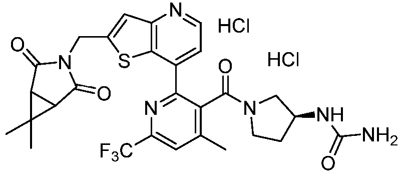 | 1-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)urea dihydrochloride |
| 137. | 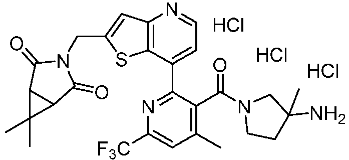 Racemate | 3-((7-(3-(3-amino-3-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride |
| 138. | 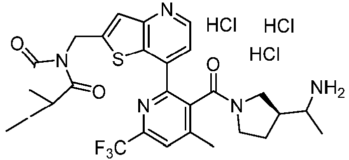 One diastereoisomer | 3-((7-(3-((3*S*)-3-(1-aminoethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride |
| 139. | 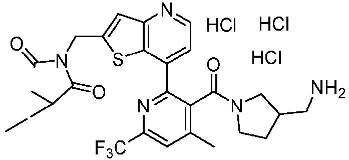 Racemate | 3-((7-(3-(3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride |
| 140. | 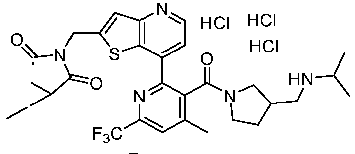 Racemate | 3-((7-(3-(3-((isopropylamino)methyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride |
| 141. | 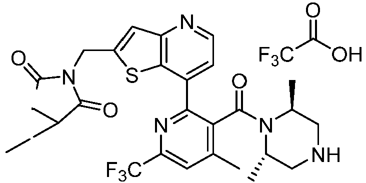 | 3-((7-(3-((2*S*,6*S*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate |
| 142. | 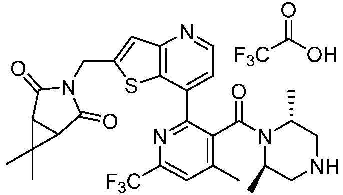 | 3-((7-(3-((2*R*,6*R*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate |
| 143. | 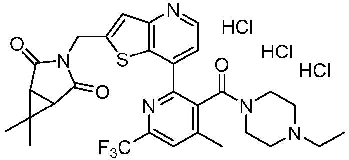 | 3-((7-(3-(4-ethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride |
| 144. | 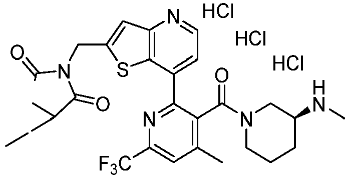 | 6,6-dimethyl-3-((7-(4-methyl-3-((*S*)-3-(methylamino)piperidine-1-carbonyl)-6-(trifluoromethyl)pyridin-2-yl)thieno[3,2-*b*]pyridin-2-yl)methyl)-3-azabicyclo[3.1.0]hexane-2,4-dione trihydrochloride |
| 160. | 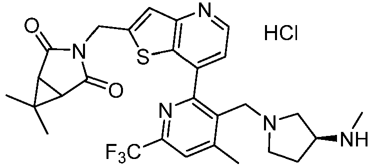 | 6,6-dimethyl-3-((7-(4-methyl-3-(((*S*)-3-(methylamino)pyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione hydrochloride |
| 165. | 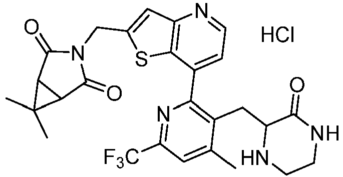 | 6,6-dimethyl-3-((7-(4-methyl-3-((3-oxopiperazin-2-yl)methyl)-6-(trifluoromethyl)pyridin-2-yl)thieno[3,2-*b*]pyridin-2-yl)methyl)-3-azabicyclo[3.1.0]hexane-2,4-dione hydrochloride |
| 168. | | 6,6-dimethyl-3-((7-(4-methyl-3-((3*R*,4*S*)-3-methyl-4-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione hydrochloride |
| 169. | | 3-((7-(3-((3*R*,4*S*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 170. | | 3-((7-(3-((3*S*,4*S*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 171. | | 3-((7-(3-((3*S*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |
| 172. | | 3-((7-(3-((3*S*,4*R*)-3-(dimethylamino)-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride |

### Pharmaceutical Compositions of the Invention

In another aspect, the invention provides a pharmaceutical composition comprising (i) a therapeutically effective amount of at least one compound according to the invention, or a pharmaceutically acceptable salt, or solvate thereof; and (ii) a pharmaceutically acceptable carrier, vehicle or excipient therefor. In general, pharmaceutical compositions comprise (i) a therapeutically effective amount of at least one active compound, or a tautomer, stereoisomer, pharmaceutically acceptable salt, and/or a solvate thereof; and (ii) a pharmaceutically acceptable carrier, vehicle or excipient therefor, including, but not limited to, bioavailability enhancers, penetration enhancers, biopolymers, PLGA-based nanoparticles, sugar-based nanoparticles, coating to avoid the deleterious effects of the stomach environment, either by protection of the compound of the invention or derivative thereof, or by release of the biologically active material beyond the stomach environment, such as in the intestine.

The exact nature of the carrier, or, for example excipient or diluent, will depend upon the desired use for the composition, and may be suitable or acceptable for veterinary use and/or suitable or acceptable for human use. The composition may optionally include one or more additional compounds, including one or more additional therapeutic agents.

Compounds of the invention can be combined with other therapeutic agents. The compound of the invention and other therapeutic agent may be administered simultaneously or sequentially. When the other therapeutic agents are administered simultaneously, they can be administered in the same or separate formulations, but they are administered substantially at the same time. The other therapeutic agents are administered sequentially with one another and with compound of the invention, when the administration of the other therapeutic agents and the compound of the invention is temporally separated. The separation in time between the administration of these compounds may be a matter of minutes or it may be longer.

Examples of other therapeutic agents that may be administered with the compounds of the invention include steroids, membrane stabilizers, 5LO inhibitors, leukotriene synthesis and receptor inhibitors, inhibitors of IgE isotype switching or IgE synthesis, IgG isotype switching or IgG synthesis, β-agonists, tryptase inhibitors, aspirin, COX inhibitors, methotrexate, anti-TNF drugs, rituximab, p38 inhibitors, PDE4 inhibitors, and antihistamines, immunotherapeutic agents, including checkpoint inhibitors such as PD-1, PD-L1, CTLA-4, LAG-3, TIM-3, TIGIT, VISTA inhibitors, IDO/TDO inhibitors, Arg1 and Arg2 inhibitors, adenosine A2A receptor antagonists, ectonucleotidase (CD73 and CD39) inhibitors, immunosuppressants, agents affecting interleukins, cytokines and chemokines, kinase inhibitors, chemotherapeutic agents including alkylating antineoplastic agents, antimetabolites, anti-microtubule agents, topoisomerase inhibitors, cytotoxic antibiotics or targeted therapies such as antibodies, antibodies drug conjugates, cell-based immunotherapies, nanoparticles, anti-cancer vaccines and radiotherapy.

The one or more additional chemotherapeutic agents includes aminoglutethimide, amsacrine, anastrozole, asparaginase, AZD5363, Bacillus Calmette-Guerin vaccine (BCG), bicalutamide, bleomycin, bortezomib, buserelin, busulfan, camptothecin, capecitabine, carboplatin, carfilzomib, carmustine, chlorambucil, chloroquine, cisplatin, cladribine, clodronate, cobimetinib, colchicine, cyclophosphamide, cyproterone, cytarabine, dacarbazine, dactinomycin, daunorubicin, demethoxyviridin, dexamethasone, dichloroacetate, dienestrol, diethylstilbestrol, docetaxel, doxorubicin, epirubicin, erlotinib, estradiol, estramustine, etoposide, everolimus, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, lenalidomide, letrozole, leucovorin, leuprolide, levamisole, lomustine, lonidamine, mechlorethamine, medroxyprogesterone, megestrol, melphalan, mercaptopurine, mesna, metformin, methotrexate, miltefosine, mitomycin, mitotane, mitoxantrone, MK-2206, nilutamide, nocodazole, octreotide, olaparib, oxaliplatin, paclitaxel, pamidronate, pazopanib, pentostatin, perifosine, plicamycin, pomalidomide, porfimer, procarbazine, raltitrexed, rituximab, rucaparib, selumetinib, sorafenib, streptozocin, sunitinib, suramin, talazoparib, tamoxifen, temozolomide, temsirolimus, teniposide, testosterone, thalidomide, thioguanine, thiotepa, titanocene dichloride, topotecan, trametinib, trastuzumab, tretinoin, veliparib, vinblastine, vincristine, vindesine, or vinorelbine.

In some embodiments, the one or more additional chemotherapeutic agents include abagovomab, adecatumumab, afutuzumab, anatumomab mafenatox, apolizumab, avelumab, blinatumomab, catumaxomab, durvalumab, epratuzumab, inotuzumab ozogamicin, intelumumab, ipilimumab, isatuximab, lambrolizumab, nivolumab, ocaratuzumab, olatatumab, pembrolizumab, pidilizumab, ticilimumab, samalizumab, tremelimumab, and BMS-936559.

In other embodiments, the method further comprises administering one or more non-chemical methods of cancer treatment, such as radiation therapy, surgery, thermoablation, focused ultrasound therapy, cryotherapy, or a combination thereof.

As stated above, an "effective amount" refers to any amount that is sufficient to achieve a desired biological effect. Combined with the teachings provided herein, by choosing among the various active compounds and weighing factors such as potency, relative bioavailability, patient body weight, severity of adverse side-effects and preferred mode of administration, an effective prophylactic or therapeutic treatment regimen can be planned which does not cause substantial unwanted toxicity and yet is effective to treat the particular subject. The effective amount for any particular application can vary depending on such factors as the disease or condition being treated, the particular compound of the invention being administered, the size of the subject, or the severity of the disease or condition. One of ordinary skill in the art can empirically determine the effective amount of a particular compound of the invention and/or other therapeutic agent without necessitating undue experimentation. It is preferred generally that a maximum dose be used, that is, the highest safe dose according to some medical judgment. Multiple doses per day may be contemplated to achieve appropriate systemic levels of compounds. Appropriate systemic levels can be determined by, for example, measurement of the patient's peak or sustained plasma level of the drug. "Dose" and "dosage" are used interchangeably herein.

Generally, daily oral doses of active compounds will be, for human subjects, from about 0.0001 milligrams/kg per day, 0.001 milligrams/kg per day, or 0.01 milligrams/kg per day to about 100 milligrams/kg per day or 1000 milligrams/kg per day. It is expected that oral doses in the range of 0.5 to 50 milligrams/kg, in one or several administrations per day, will yield the desired results. Dosage may be adjusted appropriately to achieve desired drug levels sufficient to achieve or maintain a desired therapeutic effect, local or systemic, depending upon the mode of administration. For example, it is expected that intravenous administration would be from one order to several orders of magnitude lower dose per day. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of compounds. The compounds may be administered once per week, several times per week (*e.g*., every other day), once per day or multiple times per day, depending upon, among other things, the mode of administration, the specific indication being treated and the judgment of the prescribing physician.

In one embodiment, intravenous administration of a compound of the invention may typically be from 0.1 mg/kg/day to 20 mg/kg/day.

Determination of an effective dosage of a compound for a particular use and mode of administration is well within the capabilities of those skilled in the art. Effective dosages may be estimated initially from *in vitro* activity and metabolism assays. For example, an initial dosage of compound for use in animals may be formulated to achieve a circulating blood or serum concentration of the metabolite active compound that is at or above an IC₅₀ of the particular compound as measured in as *in vitro* assay. Calculating dosages to achieve such circulating blood or serum concentrations taking into account the bioavailability of the particular compound *via* the desired route of administration is well within the capabilities of skilled artisans. Initial dosages of compound can also be estimated from *in vivo* data, such as animal models. For any compound described herein the therapeutically effective amount can be initially determined from animal models. A therapeutically effective dose can also be determined from human data for compounds of the invention which have been tested in humans and for compounds which are known to exhibit similar pharmacological activities, such as other related active agents. Higher doses may be required for parenteral administration. The applied dose can be adjusted based on the relative bioavailability and potency of the administered compound. Adjusting the dose to achieve maximal efficacy based on the methods described above and other methods as are well-known in the art is well within the capabilities of the ordinarily skilled artisan.

The formulations can be administered in pharmaceutically acceptable solutions, which may routinely contain pharmaceutically acceptable concentrations of salt, buffering agents, preservatives, compatible carriers, adjuvants, and optionally other therapeutic ingredients.

Pharmaceutical compositions comprising the compound of the invention may be manufactured by means of conventional mixing, dissolving, granulating, dragée-making levigating, emulsifying, encapsulating, entrapping or lyophilization processes. The compositions may be formulated in conventional manner using one or more physiologically acceptable carriers, diluents, excipients or auxiliaries which facilitate processing of the compounds into preparations which can be used pharmaceutically.

For use in therapy, an effective amount of the compound of the invention can be administered to a subject by any mode that delivers the compound of the invention to the desired surface. Administering the pharmaceutical composition of the present invention may be accomplished by any means known to the skilled artisan. Routes of administration include but are not limited to oral, buccal, nasal, rectal, vaginal, ocular, topical, intravenous, intramuscular, intraperitoneal, subcutaneous, transdermal, direct injection (for example, into an abscess), mucosal, inhalation, and insufflation.

For oral administration, the compounds (*i.e*., compounds of the invention, and other therapeutic agents) can be formulated readily by combining the active compound(s) with pharmaceutically acceptable carriers well known in the art. Such carriers enable the compounds of the invention to be formulated as tablets, pills, dragées, lozenges, capsules, liquids, gels, syrups, slurries, suspensions and the like, for oral ingestion by a subject to be treated. Pharmaceutical preparations for oral use can be obtained as solid excipient, optionally grinding a resulting mixture, and processing the mixture of granules, after adding suitable auxiliaries, if desired, to obtain tablets or dragée cores. Suitable excipients are, in particular, binding agents, fillers, lubricants, disintegrants, and wetting agents. Suitable fillers include sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate. Optionally the oral formulations may also be formulated in saline or buffers, *e.g.*, EDTA for neutralizing internal acid conditions or may be administered without any carriers.

Also specifically contemplated are oral dosage forms of the above component or components. The component or components may be chemically modified so that oral delivery of the derivative is efficacious. Generally, the chemical modification contemplated is the attachment of at least one moiety to the component molecule itself, where said moiety permits (a) inhibition of acid hydrolysis; and (b) uptake into the blood stream from the stomach or intestine. Also desired is the increase in overall stability of the component or components and increase in circulation time in the body. Examples of such moieties include: polyethylene glycol, copolymers of ethylene glycol and propylene glycol, carboxymethyl cellulose, dextran, polyvinyl alcohol, polyvinyl pyrrolidone and polyproline. Abuchowski and Davis, "Soluble Polymer-Enzyme Adducts", In: Enzymes as Drugs, Hocenberg and Roberts, eds., Wiley-Interscience, New York, N.Y., pp. 367-383 (1981); Newmark et al., J Appl Biochem 4:185-9 (1982). Other polymers that could be used are poly-1,3-dioxolane and poly-1,3,6-tioxocane. Preferred for pharmaceutical usage, as indicated above, are polyethylene glycol moieties.

For the component (or derivative) the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the compound of the invention or by release of the biologically active material beyond the stomach environment, such as in the intestine.

To ensure full gastric resistance a coating impermeable to at least pH 5.0 is essential. Examples of the more common inert ingredients that are used as enteric coatings are cellulose acetate trimellitate (CAT), hydroxypropylmethylcellulose phthalate (HPMCP), HPMCP 50, HPMCP 55, polyvinyl acetate phthalate (PVAP), Eudragit L30D, Aquateric, cellulose acetate phthalate (CAP), Eudragit L, Eudragit S, and shellac. These coatings may be used as mixed films.

A coating or mixture of coatings can also be used on tablets, which are not intended for protection against the stomach. This can include sugar coatings, or coatings which make the tablet easier to swallow. Capsules may consist of a hard shell (such as gelatin) for delivery of dry therapeutic (e.g., powder); for liquid forms, a soft gelatin shell may be used. The shell material of cachets could be thick starch or other edible paper. For pills, lozenges, molded tablets or tablet triturates, moist massing techniques can be used.

The therapeutic can be included in the formulation as fine multi-particulates in the form of granules or pellets of particle size about 1 mm. The formulation of the material for capsule administration could also be as a powder, lightly compressed plugs or even as tablets. The therapeutic could be prepared by compression.

Colorants and flavoring agents may all be included. For example, the compound of the invention may be formulated (such as by liposome or microsphere encapsulation) and then further contained within an edible product, such as a refrigerated beverage containing colorants and flavoring agents.

One may dilute or increase the volume of the therapeutic with an inert material. These diluents could include carbohydrates, especially mannitol, α-lactose, anhydrous lactose, cellulose, sucrose, modified dextrans and starch. Certain inorganic salts may be also be used as fillers including calcium triphosphate, magnesium carbonate and sodium chloride. Some commercially available diluents are Fast-Flo, Emdex, STA-Rx 1500, Emcompress and Avicell.

Disintegrants may be included in the formulation of the therapeutic into a solid dosage form. Materials used as disintegrates include but are not limited to starch, including the commercial disintegrant based on starch, Explotab. Sodium starch glycolate, Amberlite, sodium carboxymethylcellulose, ultramylopectin, sodium alginate, gelatin, orange peel, acid carboxymethyl cellulose, natural sponge and bentonite may all be used. Another form of the disintegrants are the insoluble cationic exchange resins. Powdered gums may be used as disintegrants and as binders and these can include powdered gums such as agar, Karaya or tragacanth. Alginic acid and its sodium salt are also useful as disintegrants.

Binders may be used to hold the therapeutic agent together to form a hard tablet and include materials from natural products such as acacia, tragacanth, starch and gelatin. Others include methyl cellulose (MC), ethyl cellulose (EC) and carboxymethyl cellulose (CMC). Polyvinyl pyrrolidone (PVP) and hydroxypropylmethyl cellulose (HPMC) could both be used in alcoholic solutions to granulate the therapeutic.

An anti-frictional agent may be included in the formulation of the therapeutic to prevent sticking during the formulation process. Lubricants may be used as a layer between the therapeutic and the die wall, and these can include but are not limited to; stearic acid including its magnesium and calcium salts, polytetrafluoroethylene (PTFE), liquid paraffin, vegetable oils and waxes. Soluble lubricants may also be used such as sodium lauryl sulfate, magnesium lauryl sulfate, polyethylene glycol of various molecular weights, Carbowax 4000 and 6000.

Glidants that might improve the flow properties of the drug during formulation and to aid rearrangement during compression might be added. The glidants may include starch, talc, pyrogenic silica and hydrated silicoaluminate.

To aid dissolution of the therapeutic into the aqueous environment a surfactant might be added as a wetting agent. Surfactants may include anionic detergents such as sodium lauryl sulfate, dioctyl sodium sulfosuccinate and dioctyl sodium sulfonate. Cationic detergents which can be used and can include benzalkonium chloride and benzethonium chloride. Potential nonionic detergents that could be included in the formulation as surfactants include lauromacrogol 400, polyoxyl 40 stearate, polyoxyethylene hydrogenated castor oil 10, 50 and 60, glycerol monostearate, polysorbate 40, 60, 65 and 80, sucrose fatty acid ester, methyl cellulose and carboxymethyl cellulose. These surfactants could be present in the formulation of the compound of the invention either alone or as a mixture in different ratios.

Pharmaceutical preparations which can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. Microspheres formulated for oral administration may also be used. Such microspheres have been well defined in the art. All formulations for oral administration should be in dosages suitable for such administration.

Liquid preparations for oral administration may take the form of, for example, elixirs, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g*., lecithin or acacia); non-aqueous vehicles (*e.g*., almond oil, oily esters, ethyl alcohol, or fractionated vegetable oils); and preservatives (*e.g*., methyl or propyl-p-hydroxybenzoates or sorbic acid). The preparations may also contain buffer salts, preservatives, flavoring, coloring and sweetening agents as appropriate.

The pharmaceutical compositions may, if desired, be presented in a pack or dispenser device which may contain one or more unit dosage forms containing the compound(s). The pack may, for example, comprise metal or plastic foil, such as a blister pack. The pack or dispenser device may be accompanied by instructions for administration.

For buccal administration, the compositions may take the form of tablets or lozenges formulated in conventional manner.

For topical administration, the compound may be formulated as solutions, gels, ointments, creams, suspensions, *etc.* as are well-known in the art. Systemic formulations include those designed for administration by injection, *e.g*., subcutaneous, intravenous, intramuscular, intrathecal or intraperitoneal injection, as well as those designed for transdermal, transmucosal, oral or pulmonary administration.

For administration by inhalation, the compounds for use according to the present invention may be conveniently delivered in the form of an aerosol spray presentation from pressurized packs or a nebulizer, with the use of a suitable propellant, *e.g*., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.,* gelatin for use in an inhaler or insufflator may be formulated containing a powder mix of the compound and a suitable powder base such as lactose or starch.

Also contemplated herein is pulmonary delivery of the compounds of the invention. The compound of the invention is delivered to the lungs of a mammal while inhaling and traverses across the lung epithelial lining to the blood stream. Other reports of inhaled molecules include Adjei et al., Pharm Res 7:565-569 (1990); Adjei et al., Int J Pharmaceutics 63:135-144 (1990) (leuprolide acetate); Braquet et al., J Cardiovasc Pharmacol 13(suppl. 5):143-146 (1989) (endothelin-1); Hubbard et al., Annal Int Med 3:206-212 (1989) (α1-antitrypsin); Smith et al., 1989, J Clin Invest 84:1145-1146 (a-1-proteinase); Oswein et al., 1990, "Aerosolization of Proteins", Proceedings of Symposium on Respiratory Drug Delivery II, Keystone, Colorado, March, (recombinant human growth hormone); Debs et al., 1988, J Immunol 140:3482-3488 (interferon-gamma and tumor necrosis factor alpha) and Platz et al., U.S. Pat. No. 5,284,656 (granulocyte colony stimulating factor). A method and composition for pulmonary delivery of drugs for systemic effect is described in U.S. Pat. No. 5,451,569, issued Sep. 19, 1995 to Wong et al.

Contemplated for use in the practice of this invention are a wide range of mechanical devices designed for pulmonary delivery of therapeutic products, including but not limited to nebulizers, metered dose inhalers, and powder inhalers, all of which are familiar to those skilled in the art.

Some specific examples of commercially available devices suitable for the practice of this invention are the Ultravent nebulizer, manufactured by Mallinckrodt, Inc., St. Louis, Mo.; the Acorn II nebulizer, manufactured by Marquest Medical Products, Englewood, Colo.; the Ventolin metered dose inhaler, manufactured by Glaxo Inc., Research Triangle Park, North Carolina; and the Spinhaler powder inhaler, manufactured by Fisons Corp., Bedford, Mass.

All such devices require the use of formulations suitable for the dispensing of compound of the invention. Typically, each formulation is specific to the type of device employed and may involve the use of an appropriate propellant material, in addition to the usual diluents, adjuvants and/or carriers useful in therapy. Also, the use of liposomes, microcapsules or microspheres, inclusion complexes, or other types of carriers is contemplated. Chemically modified compound of the invention may also be prepared in different formulations depending on the type of chemical modification or the type of device employed.

Formulations suitable for use with a nebulizer, either jet or ultrasonic, will typically comprise compound of the invention dissolved in water at a concentration of about 0.1 to 25 mg of biologically active compound of the invention per mL of solution. The formulation may also include a buffer and a simple sugar (*e.g.*, for compound of the invention stabilization and regulation of osmotic pressure). The nebulizer formulation may also contain a surfactant, to reduce or prevent surface induced aggregation of the compound of the invention caused by atomization of the solution in forming the aerosol.

Formulations for use with a metered-dose inhaler device will generally comprise a finely divided powder containing the compound of the invention suspended in a propellant with the aid of a surfactant. The propellant may be any conventional material employed for this purpose, such as a chlorofluorocarbon, a hydrochlorofluorocarbon, a hydrofluorocarbon, or a hydrocarbon, including trichlorofluoromethane, dichlorodifluoromethane, dichlorotetrafluoroethanol, and 1,1,1,2-tetrafluoroethane, or combinations thereof. Suitable surfactants include sorbitan trioleate and soya lecithin. Oleic acid may also be useful as a surfactant.

Formulations for dispensing from a powder inhaler device will comprise a finely divided dry powder containing compound of the invention and may also include a bulking agent, such as lactose, sorbitol, sucrose, or mannitol in amounts which facilitate dispersal of the powder from the device, *e.g.,* 50 to 90% by weight of the formulation. The compound of the invention should advantageously be prepared in particulate form with an average particle size of less than 10 micrometers (µm), most preferably 0.5 to 5 µm, for most effective delivery to the deep lung.

Nasal delivery of a pharmaceutical composition of the present invention is also contemplated. Nasal delivery allows the passage of a pharmaceutical composition of the present invention to the blood stream directly after administering the therapeutic product to the nose, without the necessity for deposition of the product in the lung. Formulations for nasal delivery include those with dextran or cyclodextran.

For nasal administration, a useful device is a small, hard bottle to which a metered dose sprayer is attached. In one embodiment, the metered dose is delivered by drawing the pharmaceutical composition of the present invention solution into a chamber of defined volume, which chamber has an aperture dimensioned to aerosolize and aerosol formulation by forming a spray when a liquid in the chamber is compressed. The chamber is compressed to administer the pharmaceutical composition of the present invention. In a specific embodiment, the chamber is a piston arrangement. Such devices are commercially available.

Alternatively, a plastic squeeze bottle with an aperture or opening dimensioned to aerosolize an aerosol formulation by forming a spray when squeezed is used. The opening is usually found in the top of the bottle, and the top is generally tapered to partially fit in the nasal passages for efficient administration of the aerosol formulation. Preferably, the nasal inhaler will provide a metered amount of the aerosol formulation, for administration of a measured dose of the drug.

The compounds, when it is desirable to deliver them systemically, may be formulated for parenteral administration by injection, *e.g*., by bolus injection or continuous infusion. Formulations for injection may be presented in unit dosage form, *e.g.,* in ampoules or in multidose containers, with an added preservative. The compositions may take such forms as sterile suspensions, solutions, or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethylcellulose, sorbitol, or dextran. Optionally, the suspension may also contain suitable stabilizers or agents which increase the solubility of the compounds to allow for the preparation of highly concentrated solutions.

Alternatively, the active compounds may be in powder form for constitution with a suitable vehicle, *e.g*., sterile pyrogen-free water, buffer, dextrose solution, before use. To this end, the active compound may be dried by any art-known technique, such as lyophilization, and reconstituted prior to use.

The compounds may also be formulated in rectal or vaginal compositions such as suppositories or retention enemas, *e.g*., containing conventional suppository bases such as cocoa butter or other glycerides.

For transmucosal administration, penetrants appropriate to the barrier to be permeated are used in the formulation. Such penetrants are known in the art.

For ocular administration, the compound(s) may be formulated as a solution, emulsion, suspension, *etc.* suitable for administration to the eye. A variety of vehicles suitable for administering compounds to the eye are known in the art.

In addition to the formulations described above, for prolonged delivery, the compounds may also be formulated as a depot preparation for administration by, for example, implantation or intramuscular injection. Such long acting formulations may be formulated with suitable polymeric or hydrophobic materials (for example as an emulsion in an acceptable oil) or ion exchange resins, or as sparingly soluble derivatives, for example, as a sparingly soluble salt. Alternatively, transdermal delivery systems manufactured as an adhesive disc or patch which slowly releases the compound for percutaneous absorption may be used. To this end, permeation enhancers may be used to facilitate transdermal penetration of the compound.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer R, Science 249:1527-33 (1990).

The compounds of the invention and optionally other therapeutics may be administered *per se* (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulfuric, nitric, phosphoric, maleic, acetic, salicylic, *p*-toluenesulfonic, tartaric, citric, methane sulfonic, formic, malonic, succinic, naphthalene-2-sulfonic, and benzenesulfonic. Also, such salts can be prepared as alkaline metal or alkaline earth salts, such as sodium, potassium or calcium salts of the carboxylic acid group. Typically, such salts are more soluble in aqueous solutions than the corresponding free acids and bases, but salts having lower solubility than the corresponding free acids and bases may also be formed.

The compounds may alternatively be formulated in the pharmaceutical composition *per se*, or in the form of a hydrate, solvate, or *N*-oxide.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

Pharmaceutical compositions of the invention contain an effective amount of a compound of the invention and optionally therapeutic agents included in a pharmaceutically acceptable carrier. The term "pharmaceutically acceptable carrier" means one or more compatible solid or liquid filler, diluents or encapsulating substances which are suitable for administration to a human or other vertebrate animal. The term "carrier" denotes an organic or inorganic ingredient, natural or synthetic, with which the active ingredient is combined to facilitate the application. The components of the pharmaceutical compositions also are capable of being commingled with the compounds of the present invention, and with each other, in a manner such that there is no interaction which would substantially impair the desired pharmaceutical efficiency.

The therapeutic agent(s), including specifically but not limited to the compound of the invention, may be provided in particles. Particles as used herein means nanoparticles or microparticles (or in some instances larger particles) which can consist in whole or in part of the compound of the invention or the other therapeutic agent(s) as described herein. The particles may contain the therapeutic agent(s) in a core surrounded by a coating, including, but not limited to, an enteric coating. The therapeutic agent(s) also may be dispersed throughout the particles. The therapeutic agent(s) also may be adsorbed into the particles. The particles may be of any order release kinetics, including zero-order release, first-order release, second-order release, delayed release, sustained release, immediate release, and any combination thereof, *etc.* The particle may include, in addition to the therapeutic agent(s), any of those materials routinely used in the art of pharmacy and medicine, including, but not limited to, erodible, non-erodible, biodegradable, or non-biodegradable material or combinations thereof. The particles may be microcapsules which contain the compound of the invention in a solution or in a semi-solid state. The particles may be of virtually any shape.

Both non-biodegradable and biodegradable polymeric materials can be used in the manufacture of particles for delivering the therapeutic agent(s). Such polymers may be natural or synthetic polymers. The polymer is selected based on the period of time over which release is desired. Bioadhesive polymers of particular interest include bio-erodible hydrogels described in Sawhney H S et al. (1993) Macromolecules 26:581-7, the teachings of which are incorporated herein. These include polyhyaluronic acids, casein, gelatin, glutin, polyanhydrides, polyacrylic acid, alginate, chitosan, poly(methyl methacrylates), poly(ethyl methacrylates), poly(butylmethacrylate), poly(isobutyl methacrylate), poly(hexyl methacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate).

The therapeutic agent(s) may be contained in controlled release systems. The term "controlled release" is intended to refer to any drug-containing formulation in which the manner and profile of drug release from the formulation are controlled. This refers to immediate as well as non-immediate release formulations, with non-immediate release formulations including but not limited to sustained release and delayed release formulations. The term "sustained release" (also referred to as "extended release") is used in its conventional sense to refer to a drug formulation that provides for gradual release of a drug over an extended period of time, and that preferably, although not necessarily, results in substantially constant blood levels of a drug over an extended time period. The term "delayed release" is used in its conventional sense to refer to a drug formulation in which there is a time delay between administration of the formulation and the release of the drug there from. "Delayed release" may or may not involve gradual release of drug over an extended period of time, and thus may or may not be "sustained release."

Use of a long-term sustained release implant may be particularly suitable for treatment of chronic conditions. "Long-term" release, as used herein, means that the implant is constructed and arranged to deliver therapeutic levels of the active ingredient for at least 7 days, and preferably 30-60 days. Long-term sustained release implants are well-known to those of ordinary skill in the art and include some of the release systems described above.

It will be understood by one of ordinary skill in the relevant arts that other suitable modifications and adaptations to the compositions, methods, and uses described herein are readily apparent from the description of the invention contained herein in view of information known to the ordinarily skilled artisan, and may be made without departing from the scope of the invention or any embodiment thereof.

### Methods and Uses

As shown herein, the compounds of the invention are useful for inhibiting the enzymatic and biological activity of ubiquitin specific protease 7.

In another aspect, the invention provides a compound of Formula (Ia) for use in a method for inhibiting USP7 in a cell or a tissue, comprising contacting the cell or the tissue with at least one such compound, or a pharmaceutically acceptable salt, or solvate thereof.

In some aspects, the invention provides use of a compound according to the invention, or a pharmaceutically acceptable salt, or solvate thereof, in the manufacturing of a medicament for the treatment of a disease, disorder, or condition associated with expression of USP7.

Additionally, the invention provides a compound according to the invention, or a pharmaceutically acceptable salt, or solvate thereof, for use in a method for the treatment or prevention of a disease, disorder, or condition associated with aberrant expression or activity of USP7, comprising administering to the subject in need thereof a therapeutically effective amount of at least one compound according to the invention, or a pharmaceutically acceptable salt, or solvate thereof, or a pharmaceutical composition according to the invention.

In certain embodiments, the disease, disorder, or condition is selected from the group consisting of cardiovascular disorders, pulmonary disorders, autoimmune disorders, immune disorders, immunoregulatory disorders, neurodegenerative disorders, metabolic disorders, hemolytic disorders, gastrointestinal disorders, sexual disorders, infections, wound healing disorders, and cancers.

In certain embodiments, the disease, disorder, or condition is a cardiovascular disorder selected from the group consisting of systemic hypertension, pulmonary arterial hypertension (PAH), pulmonary arterial hypertension in high altitude, ischemia reperfusion (IR) injury, myocardial infarction, and atherosclerosis.

In certain embodiments, the cardiovascular disorder is pulmonary arterial hypertension (PAH).

In certain embodiments, the cardiovascular disorder is ischemia reperfusion (IR) injury selected from the group consisting of liver IR, kidney IR, and myocardial IR.

In certain embodiments, the cardiovascular disorder is myocardial infarction or atherosclerosis.

In certain embodiments, the disease, disorder, or condition is a pulmonary disorder selected from the group consisting of chemically-induced lung fibrosis, idiopathic pulmonary fibrosis, cystic fibrosis, chronic obstructive pulmonary disease (COPD), and asthma.

In certain embodiments, the disease, disorder, or condition is an autoimmune disorder selected from the group consisting of encephalomyelitis, multiple sclerosis, anti-phospholipid syndrome 1, autoimmune hemolytic anemia, chronic inflammatory demyelinating polyradiculoneuropathy, psoriasis, dermatitis herpetiformis, dermatomyositis, myasthenia gravis, pemphigus, rheumatoid arthritis, stiff-person syndrome, type 1 diabetes, ankylosing spondylitis, paroxysmal nocturnal hemoglobinuria (PNH), paroxysmal cold hemoglobinuria, severe idiopathic autoimmune hemolytic anemia, and Goodpasture's syndrome.

In certain embodiments, the disease, disorder, or condition is an immune disorder selected from the group consisting of T-cell dysfunction mediated by myeloid-derived suppressor cells (MDSC), human immunodeficiency virus (HIV) infection, autoimmune encephalomyelitis, and AB0 mismatch transfusion reaction.

In certain embodiments, the immune disorder is T-cell dysfunction mediated by myeloid-derived suppressor cells (MDSC).

In certain embodiments, the disease, disorder, or condition is a disease resulting from an immunoregulatory disorder selected from the group consisting of renal disease inflammation, hepatic fibrosis, leishmaniosis, neurodegenerative diseases, wound healing, human immunodeficiency virus (HIV) infection, hepatitis B virus (HBV) infection, *Helicobacter pylori* infection, fibrotic disorders, arthritis, candidiasis, periodontal disease, keloids, adenotonsilar disease, African sleeping sickness, Chagas' disease, and transplant rejection.

In certain embodiments, the disease, disorder, or condition is a neurodegenerative disorder selected from the group consisting of Alzheimer's disease, Parkinson's disease, Huntington's disease, extrapyramidal syndrome, dystonia, akathisia, epilepsy, periodic limb movement, and dementia.

In certain embodiments, the disease, disorder, or condition is a metabolic disorder selected from the group consisting of diabetes, non-alcoholic steatohepatitis (NASH), and non-alcoholic fatty liver disease (NAFLD).

In certain embodiments, the disease, disorder, or condition is a hemolytic disorder selected from the group consisting of sickle-cell disease, thalassemias, hereditary spherocytosis, stomatocytosis, microangiopathic hemolytic anemias, pyruvate kinase deficiency, infection-induced anemia, cardiopulmonary bypass, mechanical heart valve-induced anemia, and chemical-induced anemia.

In certain embodiments, the hemolytic disorder is sickle-cell disease.

In certain embodiments, the disease, disorder, or condition is a gastrointestinal disorder selected from the group consisting of gastrointestinal motility disorders, gastric cancers, inflammatory bowel disease, Crohn's disease, ulcerative colitis, and gastric ulcers.

In certain embodiments, the disease, disorder, or condition is a sexual disorder selected from the group consisting of Peyronie's disease, and erectile dysfunction.

In certain embodiments, the disease, disorder, or condition is a wound healing disorder selected from the group consisting of infected and uninfected wound healing.

In certain embodiments, the disease, disorder, or condition is a cancer selected from the group consisting of oesophagic, gastric, colon, ovary, breast, pancreatic, head-and-neck, bladder, and lung cancers (including squamous and non-small cell lung carcinoma), renal cell carcinoma, prostate carcinoma, multiple myeloma, neuroblastoma, glioblastoma, astrocytoma, mesothelioma and melanoma, B cells, T cells and NK cells lymphomas, acute and chronic, myeloid leukemia, and lymphoid leukemia.

In certain embodiments, the disease, disorder, or condition is a cancer selected from the group consisting of gastric cancer (including, but not limited to, gastric or gastroesophageal junction cancer), colorectal cancer, pancreatic cancer, liver cancer, breast cancer, lung cancers (including, but not limited to, non-small cell lung carcinoma), renal cell carcinoma, prostate carcinoma, multiple myeloma, acute and chronic leukemias, T cell, B cell and NK cell lymphomas, brain tumors (including, but not limited to, neuroblastoma, glioblastoma, astrocytoma), squamous-cell carcinomas of the head and neck, and melanoma.

In certain embodiments, the disease, disorder, or condition is a cancer selected from the group consisting of chronic lymphocytic leukemia, acute lymphoblastic leukemia, chronic myelogenous leukemia, acute myeloid leukemia, adrenocortical carcinoma, anal cancer, appendix cancer, atypical teratoid/rhabdoid tumor, basal cell carcinoma, bile duct cancer, bladder cancer, bone cancer, brain tumor, brain and spinal cord tumor, brain stem glioma, central nervous system atypical teratoid/rhabdoid tumor, central nervous system embryonal tumors, breast cancer, bronchial tumors, Burkitt lymphoma, carcinoid tumor, carcinoma of unknown primary, central nervous system cancer, cervical cancer, childhood cancers, chordoma, chronic myeloproliferative disorders, colon cancer, craniopharyngioma, cutaneous T-cell lymphoma, ductal carcinoma in situ, embryonal tumors, endometrial cancer, ependymoblastoma, ependymoma, esophageal cancer, esthesioneuroblastoma, Ewing sarcoma, extracranial germ cell tumor, extrahepatic bile duct cancer, eye cancer, fibrous histiocytoma of bone, gallbladder cancer, gastric cancer, gastrointestinal carcinoid tumor, gastrointestinal stromal tumors, germ cell tumor, extracranial germ cell tumor, extragonadal germ cell tumor, ovarian germ cell tumor, gestational trophoblastic tumor, glioma, hairy cell leukemia, head and neck cancer, heart cancer, hepatocellular cancer, histiocytosis, Langerhans cell cancer, Hodgkin's lymphoma, hypopharyngeal cancer, intraocular melanoma, islet cell tumors, Kaposi sarcoma, kidney cancer, Langerhans cell histiocytosis, laryngeal cancer, lip and oral cavity cancer, lobular carcinoma in situ, lymphoma, AIDS-related lymphoma, macroglobulinemia, male breast cancer, medulloblastoma, medulloepithelioma, Merkel cell carcinoma, malignant mesothelioma, metastatic squamous neck cancer, midline tract carcinoma involving NUT gene, mouth cancer, multiple endocrine neoplasia syndrome, plasma cell neoplasm, mycosis fungoides, myeloma, chronic myeloproliferative disorder, nasal cavity cancer, paranasal sinus cancer, nasopharyngeal cancer, non-Hodgkin's lymphoma, oral cancer, oral cavity cancer, lip cancer, oropharyngeal cancer, osteosarcoma, ovarian cancer, papillomatosis, paraganglioma, paranasal sinus cancer, nasal cavity cancer, parathyroid cancer, penile cancer, pharyngeal cancer, pheochromocytoma, pineal parenchymal tumors of intermediate differentiation, pineoblastoma, pituitary tumor, plasma cell neoplasm, pleuropulmonary blastoma, primary central nervous system lymphoma, rectal cancer, renal cell cancer, renal pelvis cancer, ureter cancer, transitional cell cancer, retinoblastoma, rhabdomyosarcoma, salivary gland cancer, sarcoma, Sézary syndrome, skin cancer, small cell lung cancer, small intestine cancer, soft tissue sarcoma, squamous cell carcinoma, squamous neck cancer with occult primary, supratentorial primitive neuroectodermal tumors, testicular cancer, throat cancer, thymoma, thymic carcinoma, thyroid cancer, transitional cell cancer of the renal pelvis and ureter, gestational trophoblastic tumor, unknown primary, unusual cancer of childhood, urethral cancer, uterine cancer, uterine sarcoma, Waldenströms macroglobulinemia, or Wilms' tumor.

In certain embodiments, the at least one compound according to the invention is administered simultaneously or sequentially with a therapeutically effective amount of one or more other therapeutic agent(s) selected from the group consisting of anti-viral agents, chemotherapeutic agents (including alkylating antineoplastic agents, antimetabolites, anti-microtubule agents), immunosuppressants, anti-tumor vaccines, antiviral vaccines, cytokine therapy, tyrosine kinase inhibitors, immunotherapeutic agents, including checkpoint inhibitors such as PD-1, PD-L1 or CTLA-4 inhibitors and IDO/TDO inhibitors, adenosine A2A receptor antagonists, ectonucleotidase (CD73 and CD39) inhibitors, agent affecting interleukins, cytokines and chemokines, topoisomerase inhibitors, and cytotoxic antibiotics, or targeted therapies comprising antibodies, antibody drug conjugates, cell-based immunotherapy, nanoparticles, and radiotherapy.

In certain embodiments, the antibodies comprise a therapeutically effective amount of anti-PD-1, anti-PD-L1 or anti-CTLA4 antibodies.

In another aspect, the invention provides use of a compound according to the invention, or a pharmaceutically acceptable salt, or solvate thereof, for protecting an organ during transport.

In certain embodiments, the subject is a mammal selected from the group consisting of human, dog, cat, horse, cow, pig, sheep, goat, and ape.

### EXAMPLES

The present invention is further illustrated by the following examples, which in no way should be construed as limiting the scope of the claimed invention. The compounds having example numbers followed by asterisk (*) are not a part of the present invention, but complement the synthetic schemes of the compounds of the invention.

### Methods of Preparation and Characterization

The compounds of the present disclosure may be prepared by use of known chemical reactions and procedures. Representative methods for synthesizing compounds of the disclosure are presented below. It is understood that the nature of the substituents required for the desired target compound often determines the preferred method of synthesis. All variable groups of these methods are as described in the generic description if they are not specifically defined below. The meaning of the symbols is limited to a particular reaction scheme and is not necessarily the same for all the structural formulas.

Those having skill in the art will recognize that the starting materials and reaction conditions may be varied, the sequence of the reactions altered, and additional steps employed to produce compounds encompassed by the present disclosure, as demonstrated by the following examples. Many general references providing commonly known chemical synthetic schemes and conditions useful for synthesizing the disclosed compounds are available (see, *e.g.,* Smith and March, March's Advanced Organic Chemistry: Reactions, Mechanisms, and Structure, Fifth Edition, Wiley-Interscience, 2001; or Vogel, A Textbook of Practical Organic Chemistry, Including Qualitative Organic Analysis, Fourth Edition, New York: Longman, 1978).

The reactions are performed in a solvent appropriate to the reagents and materials employed and suitable for the transformations being effected. It will be understood by those skilled in the art of organic synthesis that the functionality present on the molecule should be consistent with the transformations proposed. This will sometimes require a judgment to modify the order of the synthetic steps or to select one particular process scheme over another in order to obtain a desired compound of the disclosure.

In some cases, protection of certain reactive functionalities may be necessary to achieve some of the above transformations. In general, the need for such protecting groups as well as the conditions necessary to attach and remove such groups will be apparent to those skilled in the art of organic synthesis. An authoritative account describing the many alternatives to the trained practitioner are in J. F. W. McOmie, "Protective Groups in Organic Chemistry," Plenum Press, London and New York 1973, in T. W. Greene and P. G. M. Wuts, "Protective Groups in Organic Synthesis," Third edition, Wiley, New York 1999, in "The Peptides;" Volume 3 (editors: E. Gross and J. Meienhofer), Academic Press, London and New York 1981, in "Methoden der organischen Chemie," Houben-Weyl, 4th edition, Vol. 15/1, Georg Thieme Verlag, Stuttgart 1974, in H.-D. Jakubke and H. Jescheit, "Aminosauren, Peptide, Proteine," Verlag Chemie, Weinheim, Deerfield Beach, and Basel 1982, and/or in Jochen Lehmann, "Chemie der Kohlenhydrate: Monosaccharide und Derivate," Georg Thieme Verlag, Stuttgart 1974. The protecting groups may be removed at a convenient subsequent stage using methods known from the art.

Starting materials can be obtained from commercial sources or prepared by literature methods.

All solvents, substrates and reagents that were commercially available were used without further purification. TLC analysis was performed using pre-coated glass plates (TLC silica gel 60 F₂₅₄) from Merck. Column chromatography was performed using high-purity grade silica gel (pore size 60 Å, 230-400 mesh particle size, 40-63 µm particle size) from Merck.

Preparative HPLC were performed on LC-20AP Shimadzu with ELSD-LTII detector equipped with Luna 21.2/250 mm, 5 µm C18(2) 100 Å LC column. The target compounds, when subjected to reversed-phase chromatographic purification in the presence of TFA, were usually obtained in the form of TFA salts.

¹H and ¹⁹F NMR spectra were recorded on Bruker AVANCE II PLUS (Ultra Shield) NMR spectrometer at 700 MHz and 250 MHz.

All spectra were recorded in appropriate deuterated solvents (CDCl₃, DMSO-d₆, D₂O, CD₃OD, *etc*.) that were commercially available.

Resonances are given in parts per million relative to tetramethylsilane. Data are reported as follows: chemical shift, multiplicity (s = singlet, d = doublet, t = triplet, m = multiplet, bs = broad singlet), coupling constants (Hz) and integration.

ESI-MS spectra were obtained on a Shimadzu LC-20AD LPG separation module with a SPD-M20A UV detector and LCMS-2020 mass detector equipped with Kinetex 2.1/30 mm, 1.7 µm XB-C18 100 Å LC column eluted with 1 mL/min flow of 10-90% gradient (over 3 min) of acetonitrile in water.

Microwave-assisted reactions were performed using CEM MARS 6^{™} Synthesis system (240/50, Model no. 911105).

Abbreviations used are those conventional in the art or the following: Ac = acetyl, aq = aqueous, Bn = benzyl, Boc = *tert*-butoxycarbonyl, *t*-Bu = *tert*-butyl, °C = degree Celsius, Cod = 1,5-cyclooctadiene, DCE = 1,2-dichloroethane, DCM = dichloromethane, DMF = *N,N-*dimethylformamide, DMSO = dimethyl sulfoxide, dppe = 1,2-bis(diphenylphosphino)ethane, dppf = 1,1'-ferrocenediyl-bis(diphenylphosphine), ELSD = evaporative light scattering detector, EtOAc or AcOEt = ethyl acetate, EtOH = ethanol, ESI+MS = electrospray ionization mass spectrometry (in the positive ion mode), ESI-MS = electrospray ionization mass spectrometry (in the negative ion mode), g = gram, h = hour(s), HMPA = hexamethylphosphoramide, HPLC = high pressure liquid chromatography, K = kelvin, L = liter, LCMS = liquid chromatography and mass spectrometry, MeCN = acetonitrile, MeOH = methanol, min = minutes, mL = milliliter(s), M = molar, MW = microwave irradiation, m/z = mass to charge ratio, nM = nanomolar, NMR = nuclear magnetic resonance, N = normal, RT or rt = room temperature, TEA = triethylamine, TFA = trifluoroacetic acid, THF = tetrahydrofuran, TMSCl = chlorotrimethylsilane, Z-OSu = N-(benzyloxycarbonyloxy)succinimide.

If not otherwise defined, purity of a solid substance is expressed as a ratio of the weight of the component in question to the total weight, multiplied by 100 (weight %); purity of a liquid is expressed as a ratio of the volume of the component in question to the total volume, multiplied by 100 (volume %); concentration of a solution is expressed as a ratio of the weight of the solute (in grams) to the total volume (in mL) of the solution, multiplied by 100 (w/v %). Yield of a reaction is expressed as a ratio of the weight of the product in question to the theoretical yield of this product, multiplied by 100 (%). Composition of a mixed solvent is expressed as a proportion of volume parts of the component solvents (*e.g.*, 3:1).

### Biological Assays

### Enzymatic assay to determine IC50 of USP7 inhibitors

The IC50 is the concentration of an inhibitor where the measured enzyme activity is reduced by half. In the case of USP7 deubiquitinase, the IC50 of an USP7 inhibitor is the molar concentration of the compound that inhibits 50% of the activity observed in a USP7-mediated-ubiquitin-rhodamine cleavage assay. For the inhibitors disclosed herein, their potency was measured using the following method.

A 45 µl reaction volume containing full-length USP7 (0.5 nM) in 50 mM HEPES pH 7.5, 150 mM NaCl, 1 mM DTT, 1 mg/ml BSA and 0.05% Tween20 was assembled in wells of 96 well half area black flat bottom plates. Test compounds were first dissolved to 25 mM stocks in DMSO and subsequently introduced to enzyme 15 min after USP7 incubation with DTT at room temperature. The enzymatic reaction was started by adding 0.5 µM Ubiquitin-Rhodamine 110 (final concentration) and allowed to proceed for 45 min at 37 °C, 250 RPM shaking before Rhodamine fluorescence (485 nm excitation/520 nm emission) was measured using Tecan Spark M10 plate reader. The IC50 values were determined by data fitting to variable slope model four-parameter dose-response curve using GraphPad 7.05.

### B-catenin reporter assay

### Cellular assay to determine EC50 of USP7 inhibitors

The EC₅₀ refers to the concentration of the inhibitor that in a cellular context inhibits activation of a target protein-dependent pathway by half. For the described here compounds, EC₅₀ was measured as the potency to inhibit the activation of the β-catenin-dependent WNT signaling pathway measured using TOPFlash and FOPFlash wnt/b-catenin activity assays (Sigma Aldrich, #17-285).

Briefly, colon adenocarcinoma derived SW480 cells were seeded at the density of 15 000 cells/well in a full medium containing DMEM-029, 10% FBS, 1% penicillin-streptomycin in a 96-well transparent plate. 48 hours later cells were transfected either with TOPFlash (Cat.# 21-170, Sigma Aldrich) firefly luciferase plasmid or FOPFlash (Cat.# 21-169, Sigma Aldrich) firefly luciferase plasmid. Additionally, cells at each well were transfected with Renilla luciferase plasmid (pGL4.75, Promega, #E693A) as the internal control. 24 h after transfection cells were treated with DMSO (0.3%) or described compounds at 10 different concentrations performed at 3-fold serial dilutions starting from 33,3 µM concentration. After 24 h luminescence signal of firefly (from TOP and FOP) and Renilla luciferase were evaluated using DualGlo^{®} Luciferase Assay System (E2920). Luminescence signal was measured using Tecan Spark M10 plate reader. The EC₅₀ values were determined by data fitting to variable slope model (four parameters) inhibitor-response curve using GraphPad 7.05.

### Immunomodulatory effect of Example 77 on isolated CD4+ T-cells from C56BL/6 mice

Splenocytes from three C57BL/6 mice donors were isolated separately. Next, specific CD4⁺ cells were extracted from all splenocytes with EasySep^{™} Mouse CD4⁺ T cell Isolation Kit by negative selection (StemCell; Cat. #19852). The purity of splenic CD4⁺ T cells exceeded 95%, as confirmed by flow cytometric analysis following staining with anti-mouse CD3 and CD4 antibodies. Pure CD4⁺cells were subsequently seeded in RPMI full medium (50 000 cells/well) on 96-well U-bottom plate covered with 1 µg/mL antiCD3 and 1 µg/mL antiCD28 (for stimulation and activation). Then, OAT compounds were added in 6 concentrations (0.3, 0.6, 1.25, 2.5, 5 and 10 µM) in three technical replicates. The final concentration of DMSO in wells was 0.03%. After 72 h, lymphocyte activation was evaluated by counting, testing viability (LDH Assay, CellTiter Glo Assay) and cytokine. Statistical significance of observed effects was determined based on mean values calculated from technical replicates for each mouse (n=9; experiment was performed three times).

Readouts:
1. ATP level in cell lysates (CellTiterGlo Viability Assay, Promega;)
2. LDH level in cell culture medium
3. Cell counting (Orange-Acridine staining, Luna automated cell counter, Logos Biosystems)
4. mTNF-α in supernatants (ELISA)
5. mIFN-γ in supernatants (ELISA)
6. Magpix Luminex (T-Cells Panel: GM-CSF, IFN-γ, IL-10-TNF-α)

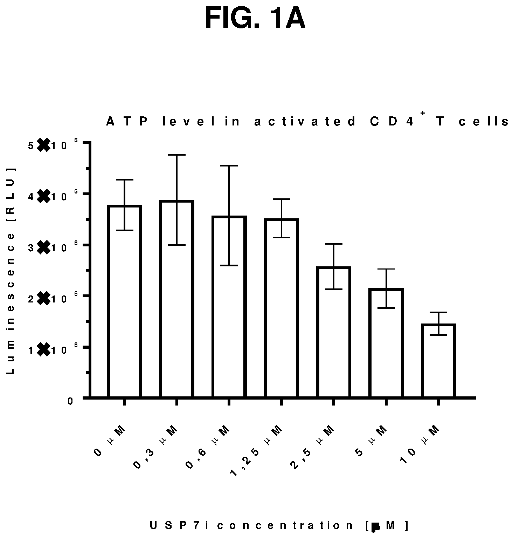

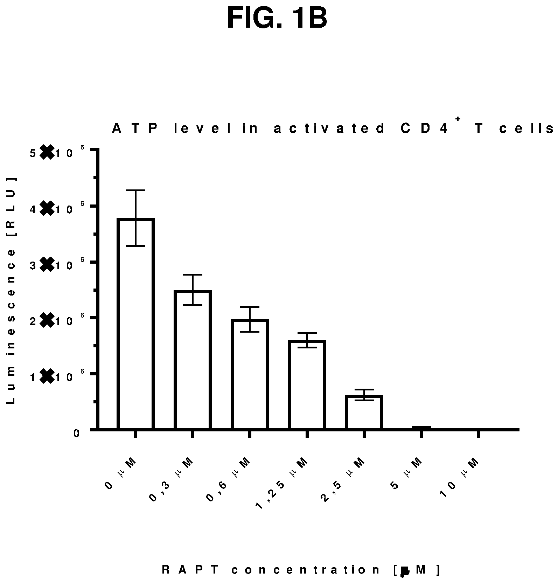

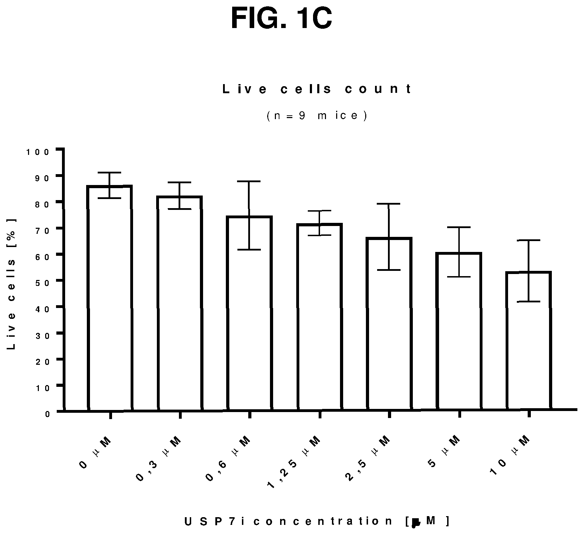

The cell viability was measured "head to head" for the compound Example 77 (denoted also USP7i in the Figures) and reference compound RAPT Therapeutics with CellTiter Glo Assay (Promega). Example 77 was 10-time less cytotoxic (IC50 ~ 9uM) than the reference RAPT compound. Cells were also counted after 72 h of incubation with Example 77 with the Luna cell counter, after trypan blue staining (1:1). The viability was comparable to that obtained with CellTiter Glo. The assay results are presented in FIGS. 1A-1C.

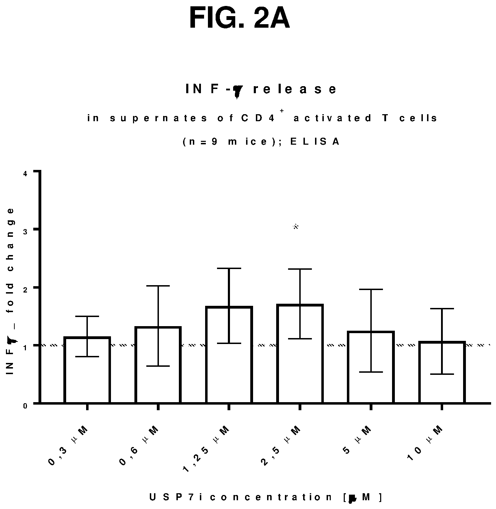

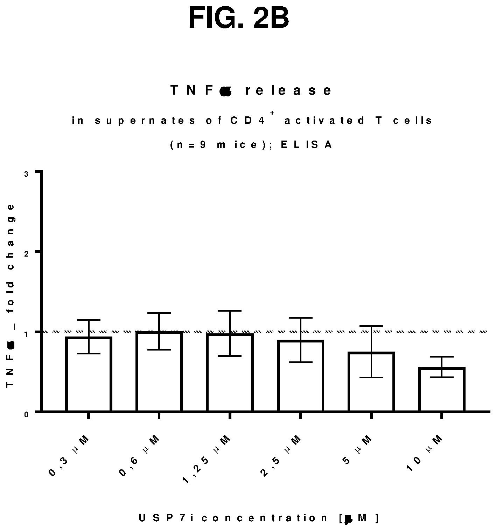

Results for TNF-α and IFN-γ level in supernatants obtained from three independent sets of experiments by ELISA are combined on bar graphs below (n=9 mice). Data were confirmed and complemented with Magpix Luminex instrument where 6 different cytokines (IFN-γ, TNF-α, GM-CSF, IL-10) were measured in supernatants from activated CD4⁺ T cells treated with Example 77 (samples from 3 independent experiments, n=9 mice). See, FIGS. 2A and 2B.

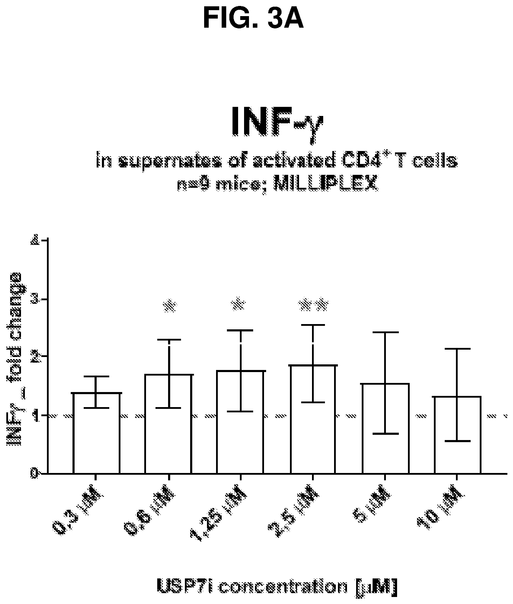

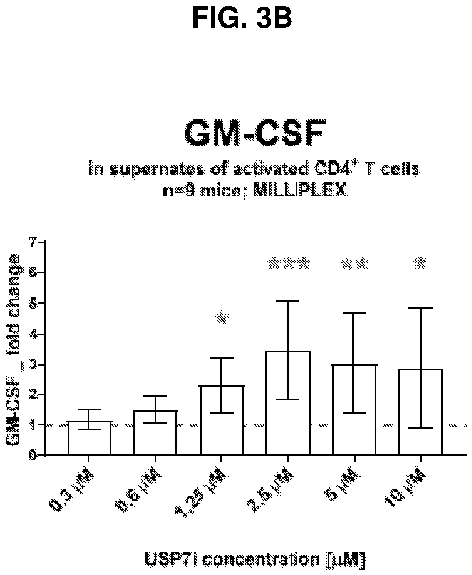

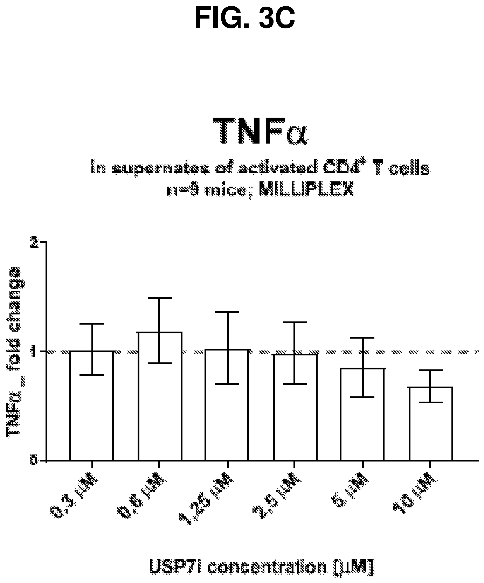

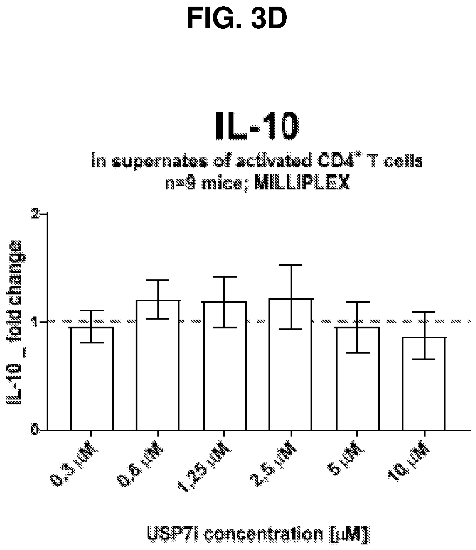

Example 77 added at low concentration (0,5-2,5 µM) does not disturb CD4⁺T cells activation and pro-inflammatory cytokine release. Statistically significant increase of IFNg and GM-CSF was observed. The level of TNFa and IL-10 was not affected. See, FIGS. 3A-3D.

### In vivo and ex vivo immunomodulatory effect of Example 77 in BALB/c mice

BALB/c mice (10-week-old females, n = 3) were administered with Example 77 at 200 mg/kg (BID) by oral gavage for 3 consecutive days. Untreated mice were used as the control group (n = 3). Animals were sacrificed 16 hours following the last dosing. The spleens were collected and total CD4⁺ lymphocytes were isolated by bead-based negative separation according to the manufacturers manual (EasySep^{™} Mouse CD4+ T cell Isolation Kit, StemCell; Cat. #19852). The purity of splenic CD4⁺ T cells exceeded 95%, as confirmed by flow cytometric analysis following staining with anti-mouse CD3 and CD4 antibodies. Isolated CD4⁺ T cells were seeded in triplicates (50 000 cells/well in RPMI full medium) on 96-well U-bottom plate covered with 1 µg/mL anti-CD3 and 1 µg/mL anti-CD28 antibodies (for stimulation and activation of cells). Following one hour preincubation in 37°C degree, CD4⁺ T cells were treated with 2 µM of Example 77, the final concentration of DMSO in wells was 0.03%. After 72 h lymphocytes activation was evaluated by testing viability, cytotoxicity and cytokine release: mTNF-α and mIFN-γ.

Readouts:
1. ATP level in cell lysates (CellTiterGlo Viability Assay, Promega)
2. Lactate dehydrogenase (LDH) level in cell culture medium (LDH-Glo Cytotoxicity Assay, Promega)
3. Cell counting (Fluorescence staining, Luna automated cell counter, Logos Biosystems)
4. mTNF-α in supernatants by ELISA
5. mIFN-γ in supernatants by ELISA

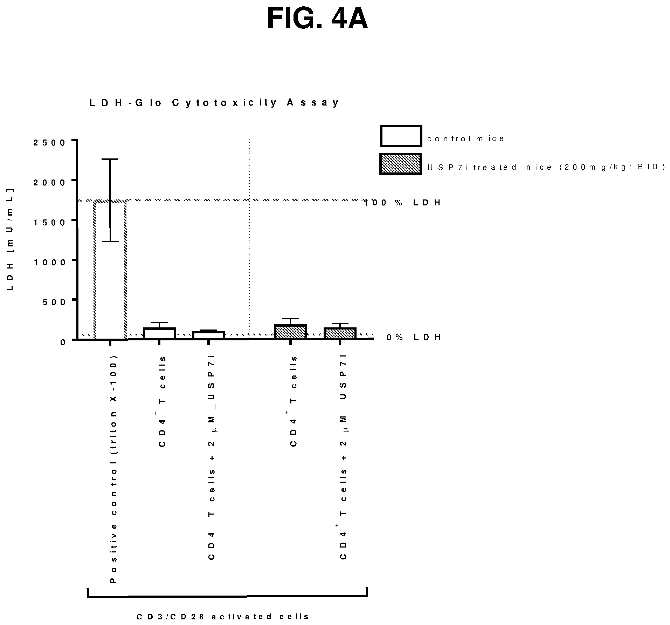

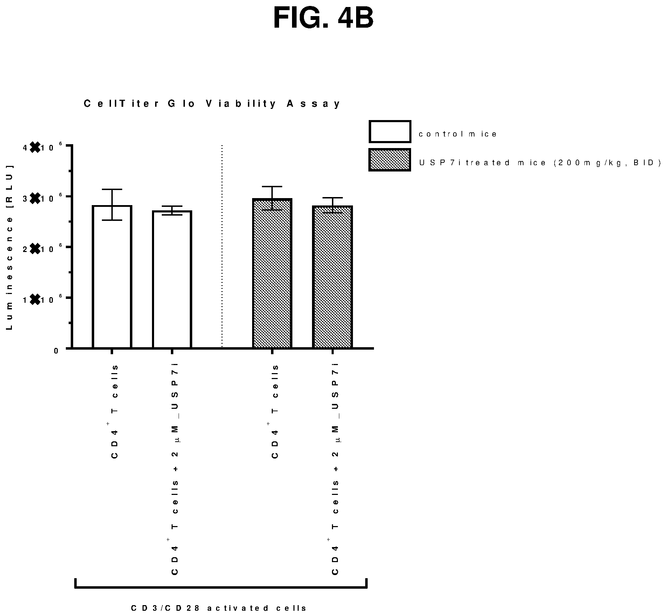

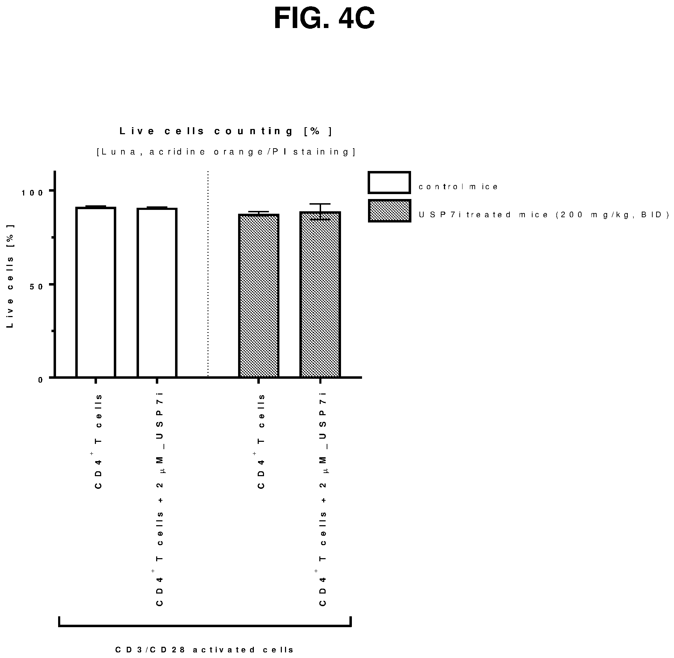

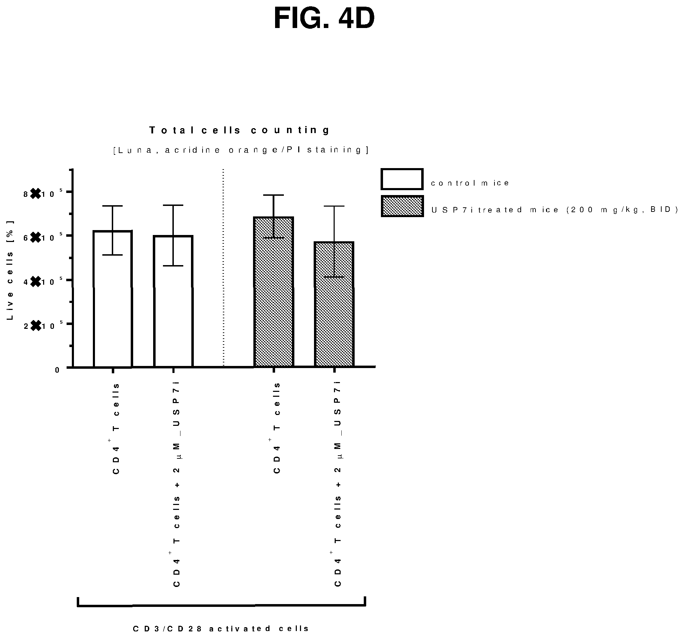

Results of the assay are presented in FIGS. 4A-4D.

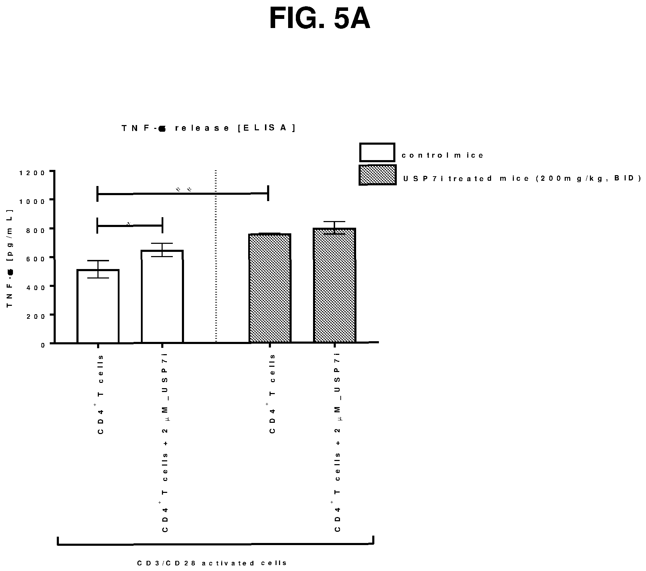

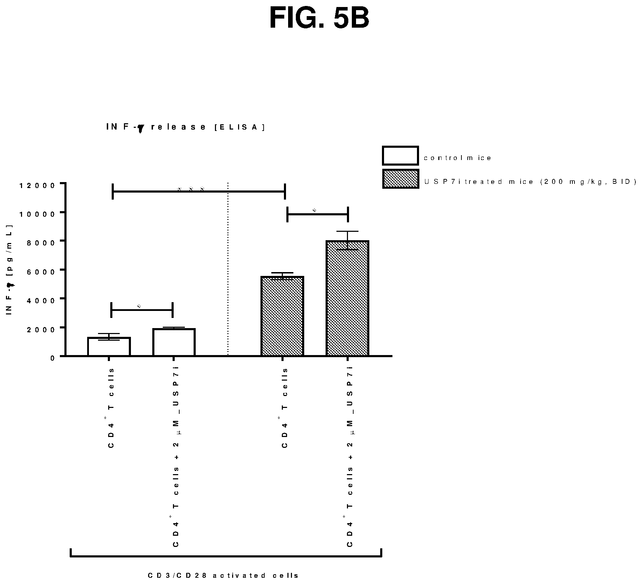

Example 77 does not affect T cells viability. At tested 2 µM concentration, Example 77 was not cytotoxic for CD4⁺ T-cells, what was confirmed by LDH-Glo cytotoxicity assay, where LDH concentration in the culture medium didn't exceed 5% vs. positive control. Viability of cells was additionally conformed by CellTiter Glo Assay where compound didn't affect total ATP level. Results of the assay are presented in FIGS. 5A and 5B.

Example 77 does not affect CD4⁺ T cells activation and pro-inflammatory cytokines release: TNF-α and IFN-γ. Activated CD4⁺ T cells isolated from Example 77 treated mice release *ex vivo* much more pro-inflammatory cytokines than CD4⁺ T cells from control mice. Furthermore, *ex vivo* treatment of isolated T-cells with Example 77 additionally increases pro-inflammatory cytokines release.

To evaluate the antitumor efficacy of Example 77 the syngeneic mouse model of colon cancer was used. The 7-8-week old BALB/c females (BALB/cAnNCrl, Charles River Laboratories) were injected subcutaneously in the right flank with 5 × 10⁵ CT26 cells. USP7 inhibitors were dosed by oral gavage twice per day at indicated doses starting from 2-5 days after the tumor implantation. Anti-PD-1 antibody (InVivoPlus anti-mouse PD-1, clone RMP1-14, BioXCell) or isotype antibody (GoInVivo Purified rat IgG2b anti-KLH, clone LTF-2) were injected intraperitoneally at dose 2.5 mg/kg (Example 77) or 5 mg/kg (Example 124, US11084829B2). In the T cell depletion experiments, an anti-CD8 antibody (InVivoPlus anti-mouse CD8, clone YTS169.4, BioXCell) was injected intraperitoneally at dose of 100 µg on days: 1, 7, and 14. Tumor volume was estimated by a formula: width × length × depth × π/6. Differences at a p-value less than 0.05 were considered statistically significant. TGI (tumor growth inhibition) index was calculated by the formula: (1 - A / B) × 100%, where A is the tumor volume in the treated group and B is the tumor volume in the control group.

To investigate the killing capability of T cells the splenocytes were isolated from tumor-bearing mice treated and untreated with different concentrations of Example 77. CT26 cells were seeded and stained with Green Cell Tracker. Stained CT26 cells were co-cultured with splenocytes from experimental animals in two ratios 1:10 and 1:50. After 36 h cells were harvested and analyzed on the flow cytometer.

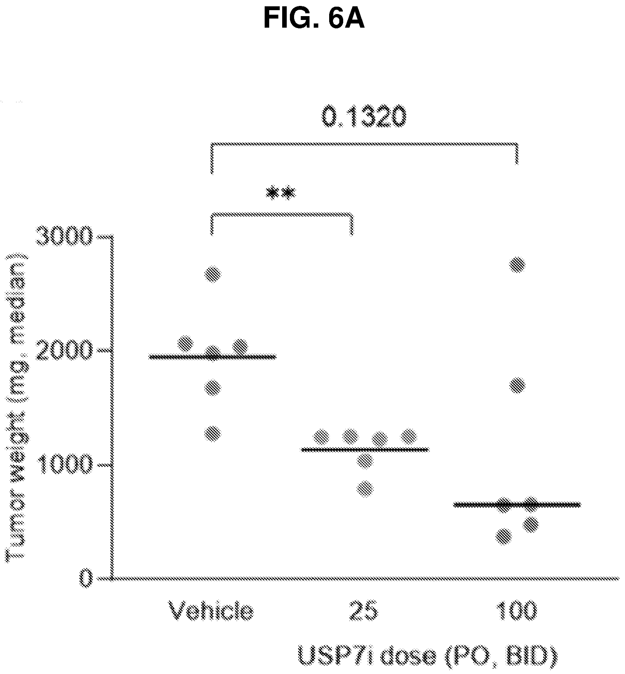

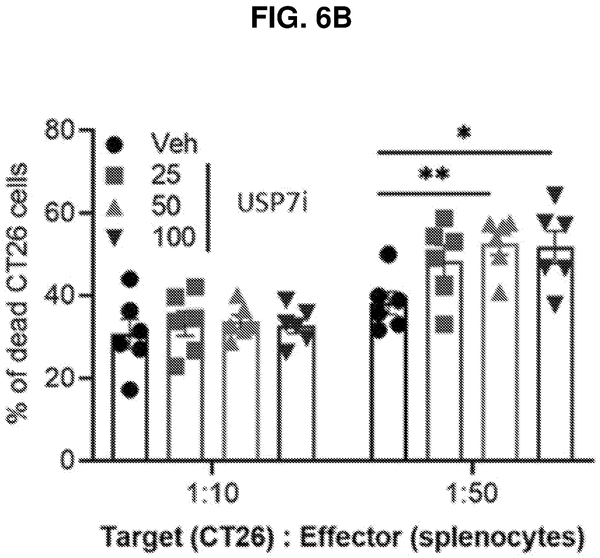

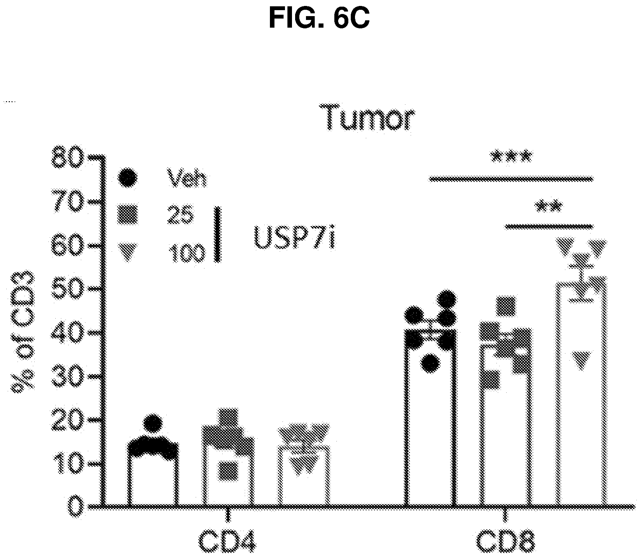

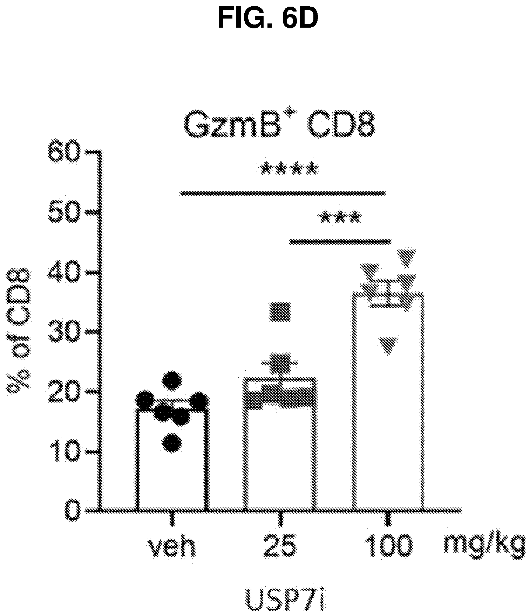

Example 77 exerted a dose-dependent antitumor effect of up to 67% tumor growth inhibition (TGI, p=0.048, FIG. 6A) at day 20 post-inoculation. Notably, splenocytes isolated from CT26-bearing mice treated with different concentrations of Example 77 showed enhanced killing capability in comparison to splenocytes isolated from control mice (FIG. 6B). Additionally, the obtained results indicated an increase in the percent of CD8+ T cells (FIG. 6C) that produce enhanced amounts of both interferon-γ (data not shown) and granzyme B in mice treated with 100 mg/kg of Example 77 (FIG. 6D).

FIGS. 6A-6D show the results of an evaluation of the antitumor efficacy in the subcutaneous CT26 model of colon carcinoma (6A) and killing assay presenting splenocytes cytotoxicity to tumor cells (6B), cytometric analysis of % of CD4 and CD8 T lymphocytes in tumor (6C) and granzyme B production in CD8+ T cells also measured via flow cytometry. Mann-Whitney test was used for statistical analysis.

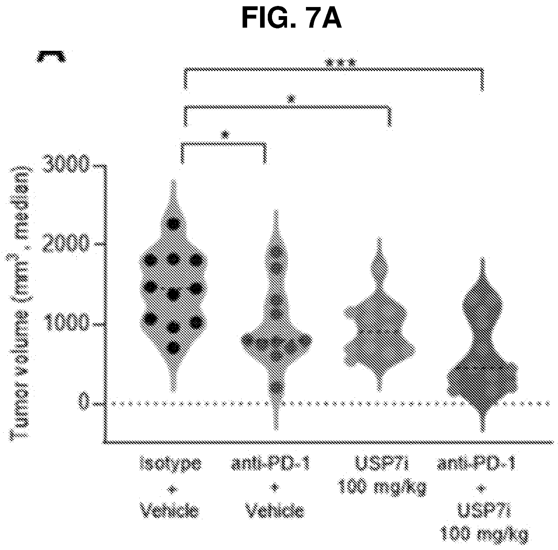

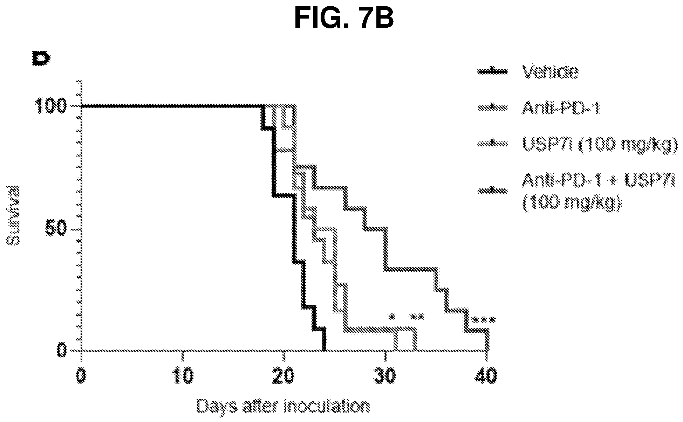

Moreover, Example 77 potentiated the therapeutic effect of anti-PD-1 antibody (immune checkpoint inhibitor), (FIG. 7A). Treatment with 100 mg/kg (PO, BID) of Example 77 resulted in the TGI of 69%, while monotherapy with anti-PD-1 antibody reached the TGI of 45%. Survival analysis, performed for the experiment, proved a statistically significant increase in survival time (by 26%, 29 days vs. 25 days) upon the treatment with Example 77 (100 mg/kg, PO, BID) vs. anti-PD-1 monotherapy group (FIG. 7B).

FIGS. 7A and 7B show the results of an evaluation of the antitumor efficacy of Example 77 in combination with anti-PD-1 antibodies in the subcutaneous CT26 model of colon carcinoma. Man-Whitney test (7A), and log-rank test (7B) were used for statistical significance analysis.

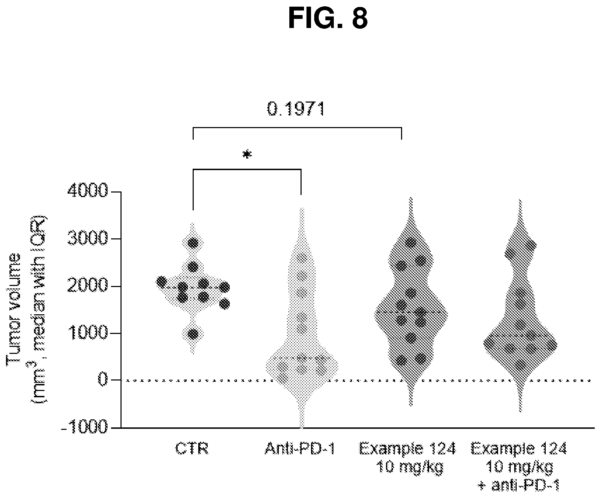

Importantly, the compound described as a USP7 inhibitor in U.S. Patent No. 11,084,829B2 patent (Example 124) did not exert significant antitumor activity as well as diminished the efficacy of anti-PD-1 therapy in the CT26 model. FIG. 8 shows the antitumor activity of Example 124 from U.S. Patent No. 11,084,829B2) as assessed in the CT26 model. Example 124 was administered at a dose of 10 mg/kg. The dose was selected based on pharmacokinetic data and potential toxicity.

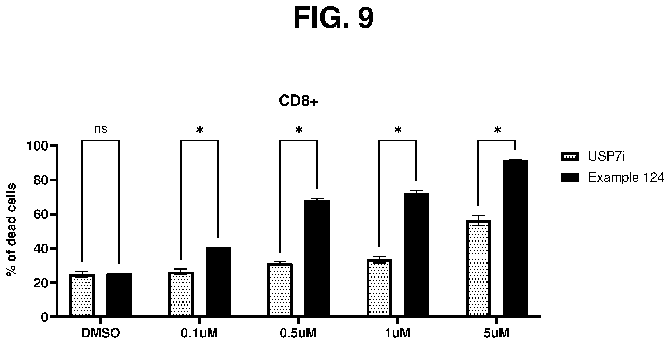

Example 124 of US11084829B2 exerted significant cytotoxicity to CD8 T cells while the toxicity of Example 77 was shown to be limited. In these experiments, the mouse splenocytes were seeded into in 24-well plate and after 24 hours of incubation with USP7 inhibitors the T cells viability was analyzed with flow cytometry. FIG. 9 compares the cytotoxic effect of Example 77 and Example 124 (US Patent No. 11,084,829B2) to mouse CD8+ T cells, Mann-Whitney test.

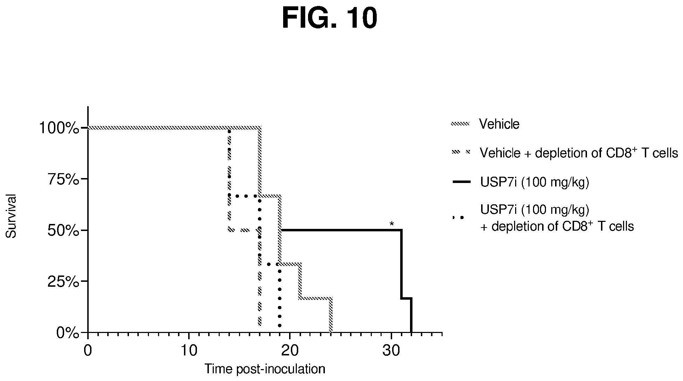

Next, the role of CD8+ T cells in the antitumor activity of Example 77 was evaluated. Tumor-bearing mice were injected with anti-CD8 antibodies that completely abrogated the antitumor effect of Example 77. FIG. 10 shows the rate of survival after treatment with Example 77 and CD8-depleting antibodies.

The results indicate that Example 77 requires CD8+ T cells for its antitumor activity, which is exerted in the syngeneic model and increases the efficiency of other immunotherapy - anti-PD-1 antibodies. Notably, the reference compound Example 124 of US11084829B2 is not efficient in the CT26 model, it does not potentiate the anti-PD-1 treatment as well as presents toxicity to CD8 T cells.

The compounds disclosed in Table 1 below for which IC₅₀ values towards USP7 have been calculated as described above are characterized as falling into the following groups:
A: < 0.5 µM;
B: 0.5-1 µM;
C: 1-10 µM;
D: > 10 µM.

If the IC₅₀ value has not been determined yet, a percent value of inhibition of USP7 activity at 1 µM of the test compound is specified. The following percentage ranges correspond to the corresponding letters with an asterisk; the compounds that lack IC₅₀ values are characterized as falling into the following groups:
A*: > 70% inhibition;
B*: 50 - 70% inhibition;
C*: 20 - 49% inhibition;
D*: < 20% inhibition.

**Table 1**

| Ex. No. | Structure | USP7 IC₅₀ |
|---|---|---|
| 1. | 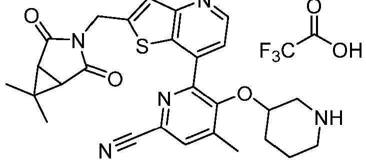 | A |
| 2. | 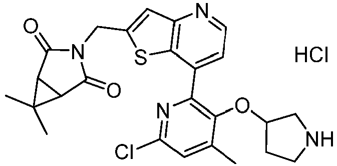 | A |
| 3. | 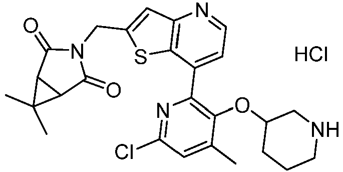 Racemate | A |
| 4. | 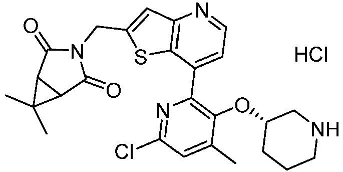 Single enantiomer of Ex. 3 | A |
| 18. | 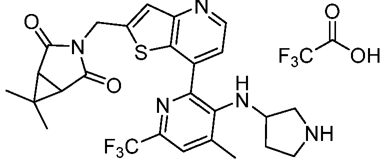 | A |
| 20. | 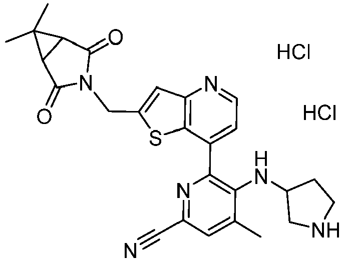 | A |
| 24. | 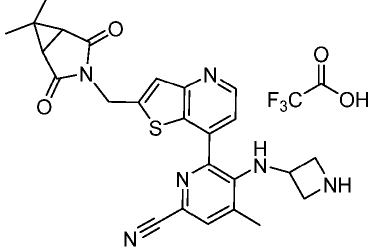 | A |
| 28. | 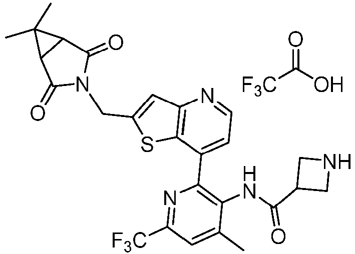 | A |
| 35. | 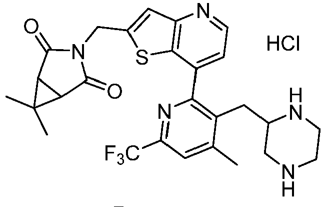 Racemate | A |
| 37. | 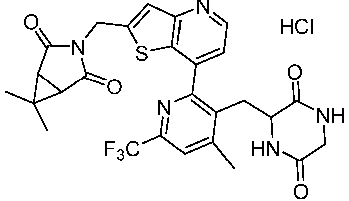 | D* |
| 38. | 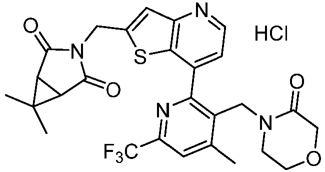 | D* |
| 39. | 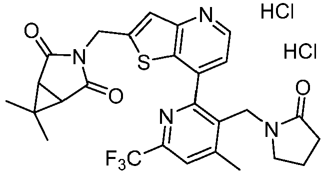 | C* |
| 40. | 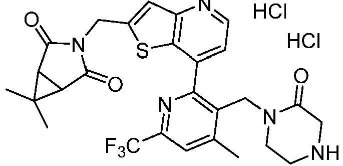 | C* |
| 41. | 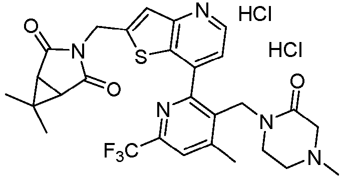 | D* |
| 43. | 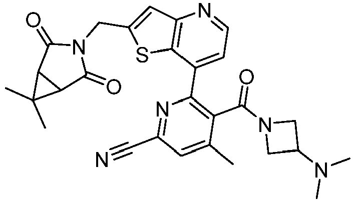 | B* |
| 44. | 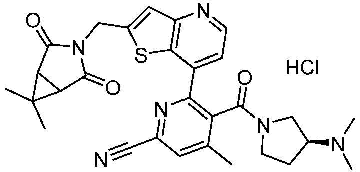 | C |
| 45. | 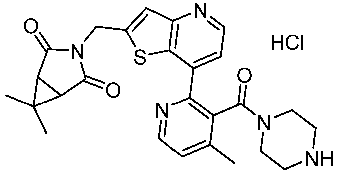 | D* |
| 46. | 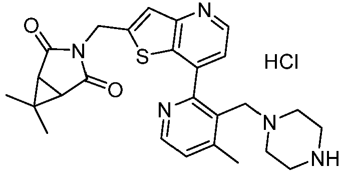 | D* |
| 47. | 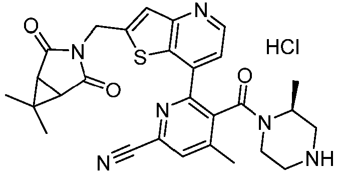 | A |
| 48. | 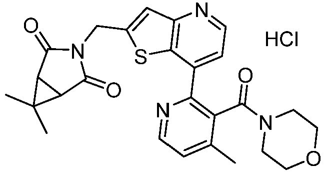 | D* |
| 49. | 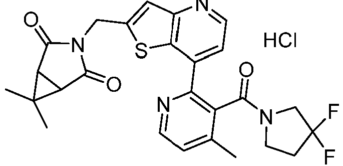 | D* |
| 50. | 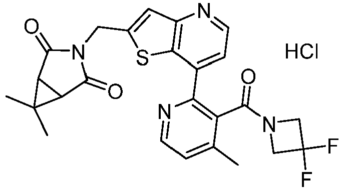 | D* |
| 51. | 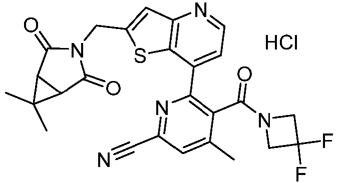 | A |
| 52. | 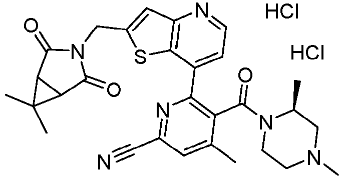 | A |
| 53. | 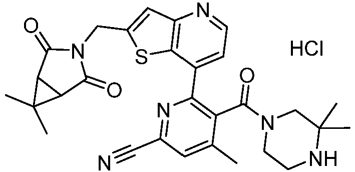 | C |
| 54. | 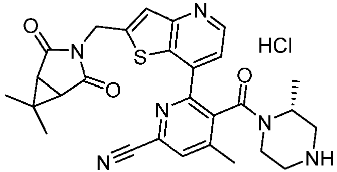 | B |
| 55. | 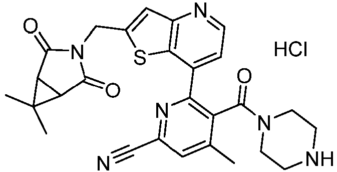 | A |
| 58. | 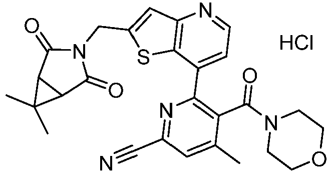 | B |
| 59. | 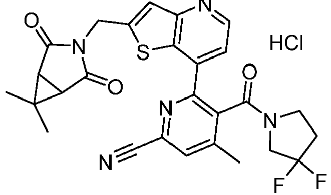 | A |
| 73. | 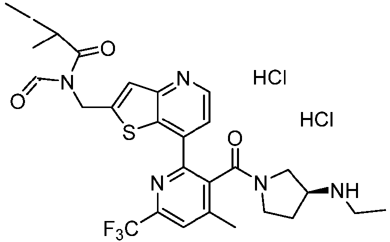 | A |
| 74. | 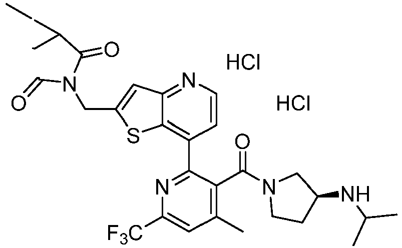 | A |
| 76. | 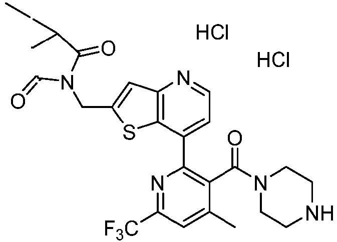 | A |
| 77. | 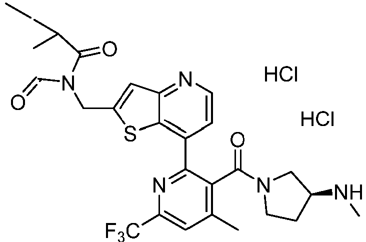 | A |
| 78. | 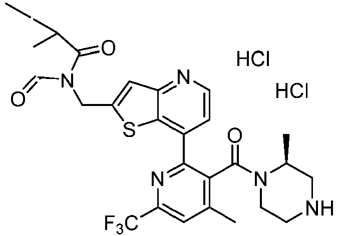 | A |
| 79. | 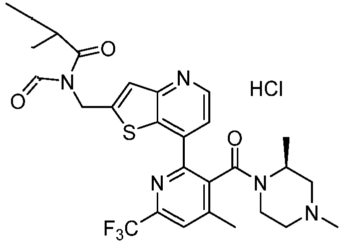 | A |
| 80. | 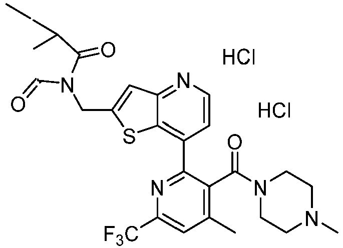 | A |
| 81. | 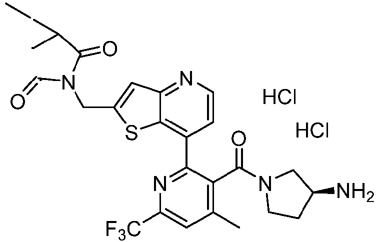 | A |
| 82. | 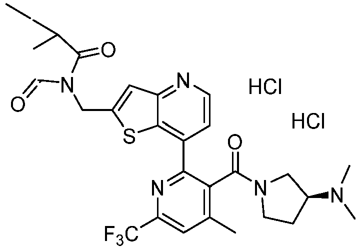 | A |
| 83. | 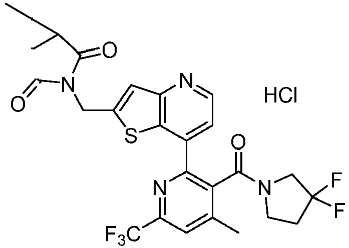 | A |
| 84. | 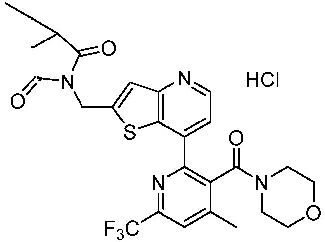 | B |
| 85. | 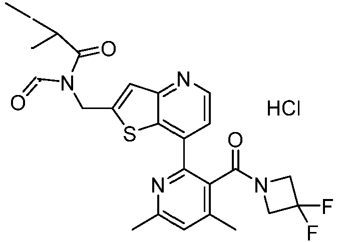 | C |
| 92. | 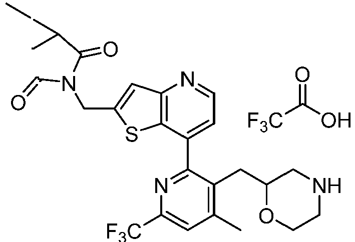 Racemate | A |
| 94. | 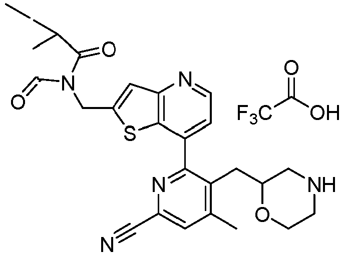 Racemate | A |
| 95. | 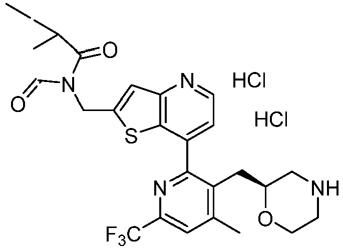 | A |
| 96. | 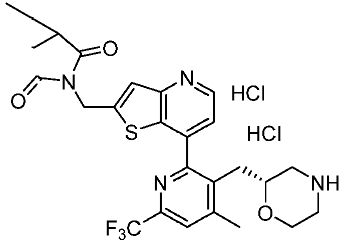 | A |
| 97. | 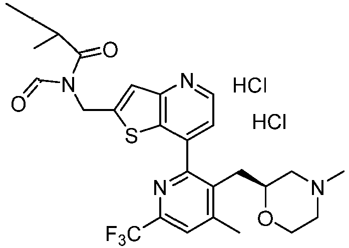 | C |
| 98. | 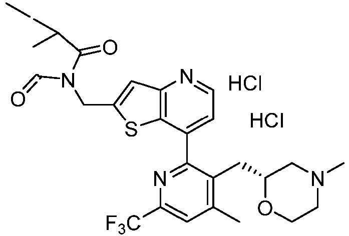 | A |
| 104. | 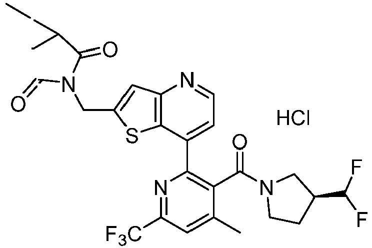 | A |
| 105. | 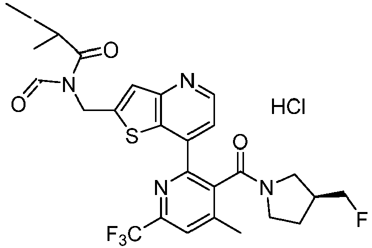 | A |
| 106. | 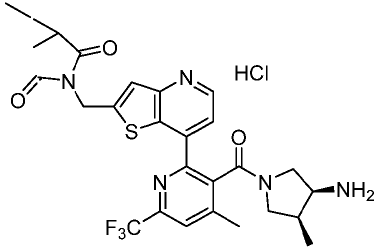 | A |
| 107. | 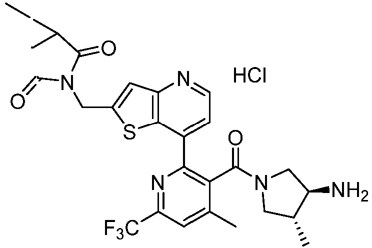 | A |
| 109. | 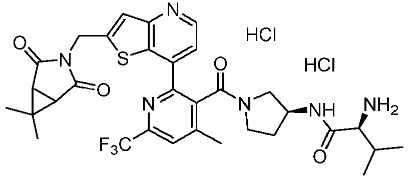 | A |
| 110. | 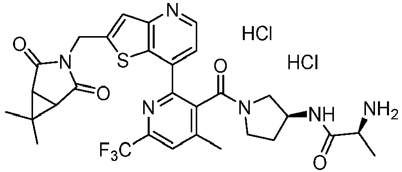 | B |
| 112. | 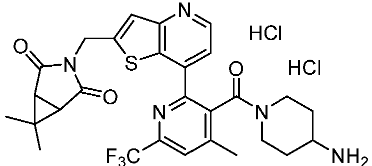 | A |
| 113. | 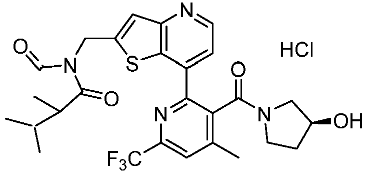 | A |
| 114. | 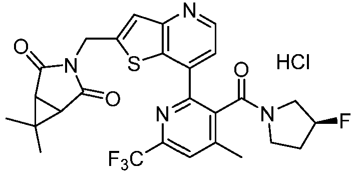 | A |
| 115. | 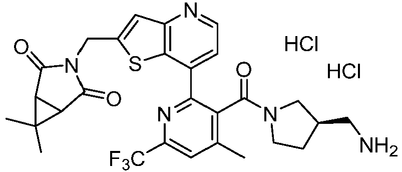 | B |
| 117. | 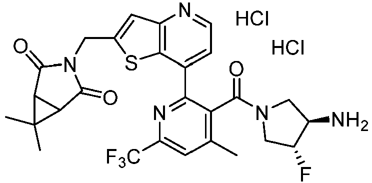 | A |
| 121. | 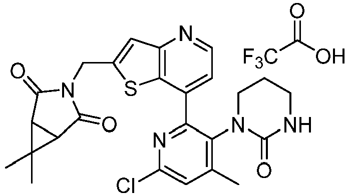 | B |
| 122. | 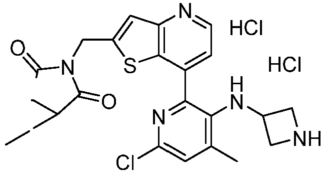 | A |
| 124. | 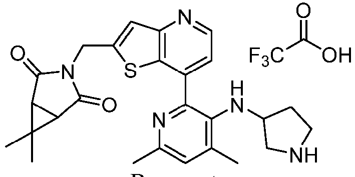 Racemate | A |
| 126. | 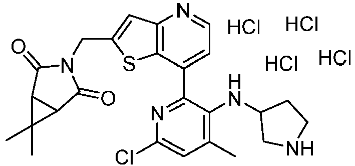 Racemate | A |
| 127. | 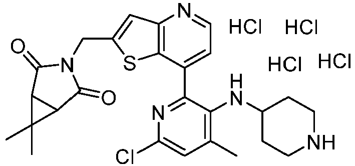 | A |
| 128. | 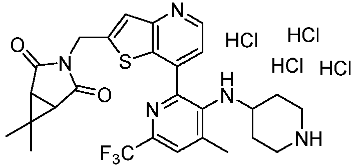 | A |
| 129. | 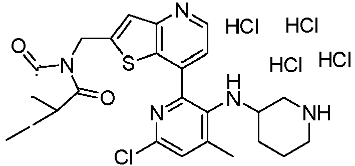 Racemate | A |
| 130. | 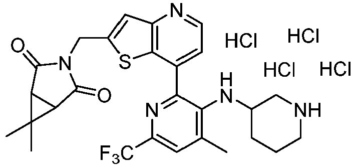 Racemate | A |
| 133. | 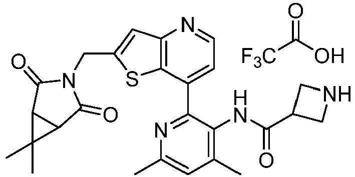 | C |
| 134. | 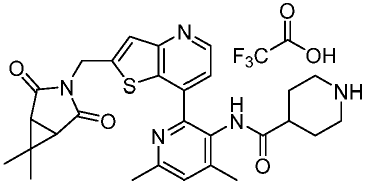 | D* |
| 135. | 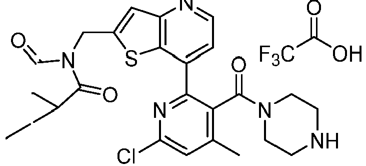 | D* |
| 136. | 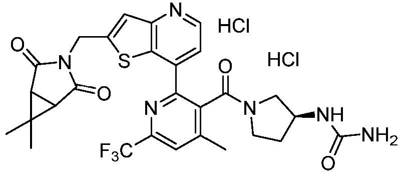 | B |
| 137. | 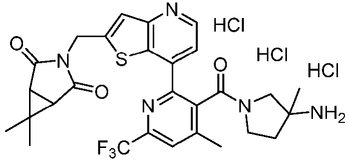 Racemate | B |
| 138. | 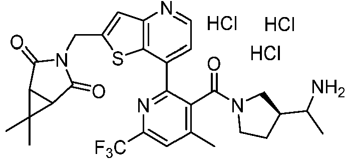 One diastereoisomer | B* |
| 139. | 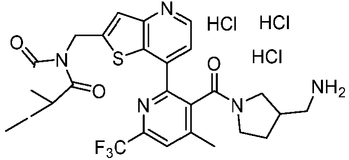 Racemate | A |
| 140. | 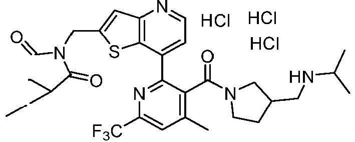 Racemate | A |
| 141. | 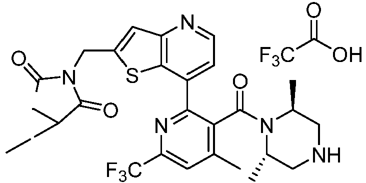 | A |
| 142. | 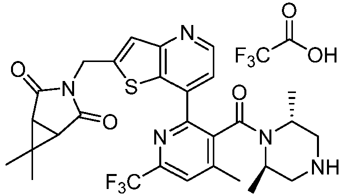 | C |
| 143. | 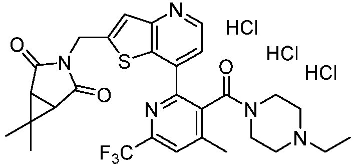 | A |
| 144. | 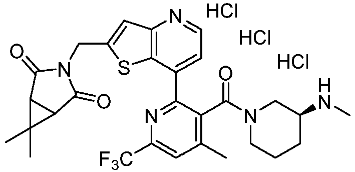 | C |
| 160. | 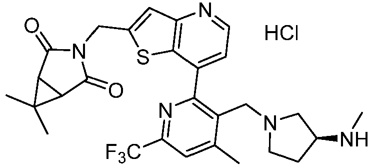 | A |
| 165. | 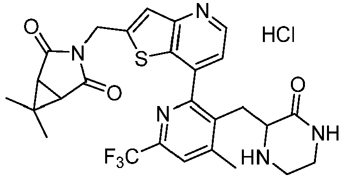 | C* |
| 168. | 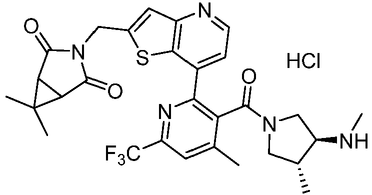 | A |
| 169. | 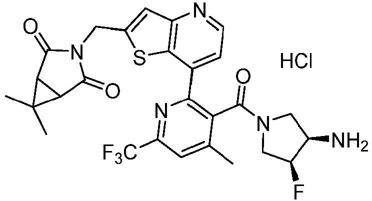 | A |
| 170. | | A |
| 171. | | B* |
| 172. | | A* |

### General Synthetic Procedures

### General Procedure I

Reaction of appropriate carboxylic acid with an appropriate amine or alcohols.

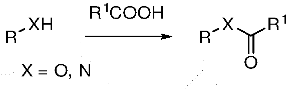

To the solution of carboxylic acid (1 equivalent) and diisopropylethylamine (DIPEA; 3 equivalents) or triethylamine (Et₃N; 3 equivalents) in dichloromethane (6 mL/mmol) or in DMF (5 mL/mmol) appropriate alcohol (1 - 1.2 equivalent) or amine (1 equivalent) was added. Then EDCI hydrochloride (1.1 - 1.5 equivalent) or HATU (1.1 equivalent) and DMAP (0.05 - 0.1 equivalent, when it was necessary) were added sequentially and the reaction mixture was stirred overnight at room temperature. After this time LC-MS control showed complete consumption of the starting materials and the reaction mixture was taken into DCM/water. An organic layer was washed with water and brine, dried over anhydrous MgSO₄, filtered and concentrated *in vacuo* and the crude product was further purified by flash column chromatography on silica.

### General Procedure II

Acylation of appropriate amine with acid chloride.

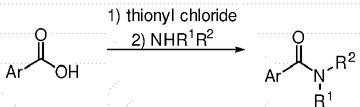

To the solution of carboxylic acid (1 - 1.5 equivalent) and triethylamine (4 equivalents) or pyridine (2.5 equivalents) in DCM or DMF (6 mL/mmol) thionyl chloride (1.7 equivalents) was added dropwise at 0 °C and the reaction mixture was stirred at this temperature for the time necessary for the complete consumption of the starting material (usually 1 to 3 hours) as judged by TLC or LC-MS. After this time thionyl chloride was concentrated *in vacuo.* Then to the residue a mixture of DCM (4 mL/mmol), triethylamine (2 - 7 equivalents), an appropriate amine (1 - 1.5 equivalents) and DMAP (0.1 equivalent) was added dropwise at 0 °C and the reaction mixture was stirred at 0 °C for 1 hour and then in room temperature and then heated at 40 °C. After consumption of the starting material (confirmed by TLC or LC-MS analysis), to this mixture 1 M K₂CO₃ or 1 M NaOH was added. The layers were separated and the aqueous one was additionally extracted with DCM (3 x). The combined organic extracts were dried over anhydrous Na₂SO₄, filtered and evaporated *in vacuo.* The crude product was purified by silica-gel chromatography or by preparative reversed-phase column chromatography.

### General Procedure III

Protection of amine group by di-*tert*-butyl dicarbonate (Boc₂O).

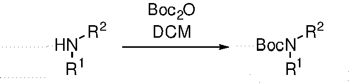

To the solution of appropriate amine (1 equivalent) in DCM (5 mL/mmol) Boc₂O (1.2 equivalents) was added and then stirred at room temperature overnight. The reaction progress was monitored by TLC and LC-MS. When analyses indicated completion of the reaction, DCM was removed *in vacuo* and the crude product was purified by silica-gel or flash column chromatography on silica.

### General Procedure IVa

Removal of the *tert*-butoxycarbonyl (Boc-) group from amine with HCl.

The *N*-Boc protected amine was treated with a 4 M solution of HCl (5 mL/mmol of starting material) in an appropriate organic solvent (*e.g*., AcOEt, 1,4-dioxane, MeOH, DCM) for the time necessary for complete consumption of the starting material (typically 30 minutes - 2 hours). The volatiles were then removed *in vacuo* providing de-protected amine in the form of its hydrochloride salt. The crude product was usually purified by preparative reversed-phase column chromatography to give the corresponding product.

### General Procedure IVb

Removal of the *tert*-butoxycarbonyl (Boc-) group from amine with TFA. The N-Boc protected amine was treated with solution of TFA (6 equivalents) in DCM for the time necessary for complete consumption of the starting material (typically 30 minutes - 2 hours). The volatiles were then removed *in vacuo* providing de-protected amine in the form of its TFA salt. The crude product was usually purified by preparative reversed-phase column chromatography to give the corresponding product.

### General Procedure Va

The Suzuki coupling.

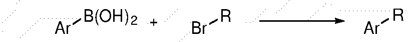

A palladium source (Pd(PPh₃)₄ or Pd(PPh₃)₂Cl₂ (0.05 - 0.1 equivalent)/PPh₃ (0.1 - 0.2 equivalent) or Pd(dppf)Cl₂ (0.05 - 0.1 equivalent) was dissolved in a combination of solvents such as dioxane (1 - 1.5 mL/mmol)/water (0.3 mL/mmol) and the mixture was degassed by bubbling with Ar. An appropriate halogen compound (1 equivalent) and K₂CO₃ (2 - 3 equivalents) were added and stirred for 5 minutes. Then to this mixture an organoboronic compound (1.1 - 2.5 equivalents) was added and the mixture was stirred vigorously at 90 - 110°C (1 - 24 hours) under Ar. The reaction progress was monitored by TLC and LC-MS. After analytical control indicated completion of the reaction, the reaction was diluted with AcOEt (60 mL/mmol) and filtered through a Pad of Celite, evaporated thoroughly and redissolved in AcOEt/H₂O. The layers were separated and the aqueous one was extracted with AcOEt (3 x). The combined organic layers were washed with brine, dried over anhydrous Na₂SO₄, filtered and concentrated *in vacuo.* The crude product was purified by flash column chromatography on silica.

### General Procedure Vb

The Suzuki coupling - optional cyclisation reaction of succinimide ring in case when succinimide ring opening product was observed in significant amount.

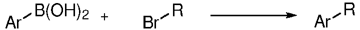

A palladium source (Pd(PPh₃)₄ or Pd(PPh₃)₂Cl₂ (0.05 - 0.1 equivalent)/PPh₃ (0.1 - 0.2 equivalent) or Pd(dppf)Cl₂ (0.05 - 0.1 equivalent) was dissolved in a combination of solvents such as dioxane (1 - 1.5 mL/mmol)/water (0.3 mL/mmol) and the mixture was degassed by bubbling with Ar. An appropriate halogen compound (1 equivalent) and K₂CO₃ (2 - 3 equivalents) were added and stirred for 5 minutes. Then to this mixture an organoboronic compound (1.1 - 2.5 equivalents) was added and the mixture was stirred vigorously at 90 - 110°C (1 - 24 hours) under Ar. The reaction progress was monitored by TLC and LC-MS. The reaction mixture was concentrated *in vacuo* and dried under high vacuum. Then an acetic anhydride (5 ml/mmol) and sodium carbonate (2 equivalents) were added and the resulting suspension was stirred at 50 °C for 1 hour. The progress of cyclisation reaction was monitored by LC-MS. Then the mixture was concentrated *in vacuo* and the residue was purified by flash column chromatography on silica.

### General Procedure VIa

Reductive amination of the cyclic ketone with appropriate amine.

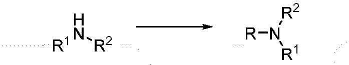

An appropriate amine or an amine hydrochloride (1 equivalent) was dissolved in DCE and acetic acid (AcOH, 2 equivalents) and appropriate ketone (2 equivalents) were added and the mixture was heated at 50 °C for 2 days. The reaction mixture was cooled to room temperature and sodium triacetoxyborohydride (NaBH(OAc)₃) (4 equivalents) was then added in one portion and the mixture was stirred overnight at room temperature. After this time a 5% aqueous solution of sodium bicarbonate (NaHCO₃) and dichloromethane (DCM) were added and the mixture was stirred for 30 minutes. The layers were separated and the aqueous layer was additionally extracted with dichloromethane. The organic extracts were combined, washed with brine, dried over anhydrous MgSO₄, filtered and evaporated to dryness. The crude product was purified by flash column chromatography on silica.

### General Procedure VIb

Alternative procedure for reductive amination of the cyclic ketone with appropriate amine.

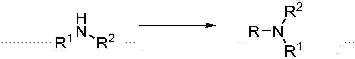

To the solution of an appropriate amine or an amine hydrochloride (1 equivalent) in dimethylformamide (DMF, 2 mL/mmol) under Ar, trimethylsilyl chloride (TMSCl, 2.5 equivalents) was added and the mixture was cooled to 0 °C and sodium borohydride (NaBH₄, 1 equivalent) was added. The reaction mixture was slowly warmed to room temperature and stirred overnight. After this time the mixture was taken into AcOEt/H₂O. The layers were separated and the aqueous layer was additionally extracted with AcOEt. The organic extracts were combined, washed with brine, dried over anhydrous MgSO₄, filtered and evaporated to dryness. The crude product was purified by flash column chromatography on silica.

### General Procedure VII

Reduction of morpholin-3-one to morpholine or amide to amine.

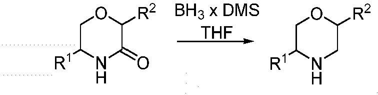

To the solution of either morpholin-3-one or 2-piperazinone or amide in THF (6 mL/mmol) borane-dimethylsulfide complex (BH₃ x DMS; 5 equivalents) was added and the reaction mixture was refluxed overnight, after which time the TLC or LC-MS control indicated completed consumption of the starting material. Reaction mixture was cooled to room temperature and 2 M or 6 M HCl was cautiously added (6 equivalents with respect to the starting material). The resulting reaction mixture was refluxed for 2 hours and cooled back to room temperature. The pH of the solution was then adjusted to strongly alkaline (~10) by a dropwise addition of 6 M NaOH. The organic layer was separated and the aqueous layer was additionally extracted with diethyl ether or AcOEt. The combined organic extracts were then dried over anhydrous MgSO₄, filtered and the solvents were evaporated. The crude product obtained was, in most cases, sufficiently pure to be used to the next step without any additional purification.

### General Procedure VIII

Installation of the appropriate R-group on the primary or secondary amine or alcohol

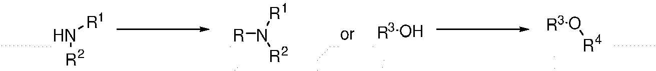

To a solution of the primary or secondary amine or alcohol in DCM (10 mL/mmol) or THF (4 mL/mmol), Et₃N (2 - 4 equivalents) or DIPEA (2 equivalents) followed by DMAP (0.1 equivalent, when it was necessary) were added and then appropriate carbamoyl chloride (2.5 equivalents) or appropriate isocyanate (1.1 - 2.5 equivalents) or acid chloride (1 - 2 equivalents) or appropriate anhydride (1.1 - 2 equivalents) was added at room temperature. The reaction progress was monitored by TLC and LC-MS. When analyses indicated completion of the reaction, the mixture was quenched by addition of 4 M NaOH or 5% NaHCO₃ (for tosylation). Product was extracted with DCM (2 times). The combined organic solutions were dried over anhydrous Na₂SO₄, filtered and concentrated *in vacuo* and the crude product was purified by preparative reversed-phase column chromatography or by silica-gel column chromatography.

### Exemplary Synthetic Procedures

### General procedure A

Preparation of (2-((6,6-dimethyl-2,4-dioxo-3-azabicyclo[3.1.0]hexan-3-yl)methyl)thieno[3,2-*b*]pyridin-7-yl)boronic acid (**I**).

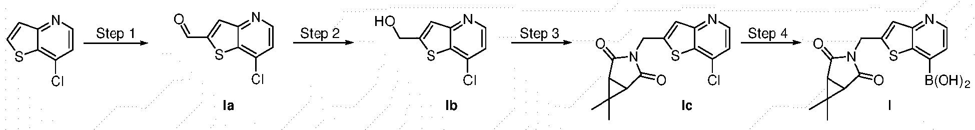

### Step 1.

Synthesis of 7-chlorothieno[3,2-*b*]pyridine-2-carbaldehyde (**Ia**).

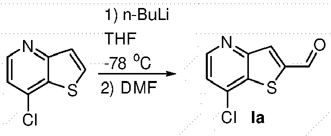

To a solution of 7-chlorothieno[3.2-*b*]pyridine (10 g; 58.96 mmol) in anhydrous THF (120 mL) n-BuLi (2.5 M in hexane; 26 mL; 64.86 mmol) was added dropwise over a period of 30 minutes at -78 °C. The resulting yellow suspension was stirred for additional 30 minutes. Then an anhydrous *N*,*N-* dimethylformamide (45.4 mL; 589.60 mmol) was added dropwise over period of 30 minutes at -78 °C and the reaction was stirred at this temperature for 2 hours. The reaction progress was monitored by LC-MS. When analysis indicated completion of the reaction, MeOH (20 mL) was added dropwise at -78 °C and the reaction mixture was warmed to room temperature. Next 1 M HCl_{aq} (100 mL) was slowly added and then the pH was adjusted to ~7 with 1 M HCl_{aq}. The resulting suspension was extracted with AcOEt (4 x 200 mL). The combined organic solutions were washed with water (1 x 100 mL) and brine (2 x 150 mL), dried over anhydrous Na₂SO₄, filtered and concentrated *in vacuo.* The crude product was triturated with cold MTBE, the solid was filtered, washed with hexane and dried. The crude product was used to the next step without additional purification. Compound **Ia** was obtained as a beige solid in 89% yield (10.34 g; 52.50 mmol).
ESI-MS m/z for C₈H₅ClNOS found 198.0/200.0 [M+H]⁺ , Rₜ= 1.09 min; ¹H NMR (700 MHz, CDCl₃) δ 10.21 (s, 1H), 8.72 (d, *J =* 5.0 Hz, 1H), 8.25 (s, 1H), 7.44 (d, *J =* 5.0 Hz, 1H).

### Step 2.

Synthesis of (7-chlorothieno[3,2-b]pyridin-2-yl)methanol (**Ib**).

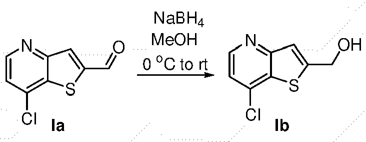

To a cooled to 0 °C suspension of aldehyde **Ia** (10.34 g; 52.50 mmol) in MeOH (120 mL), NaBH₄ (2.98 g; 78.75 mmol) was added portionwise. The resulting mixture was warmed to room temperature and stirred for 1 hour. Then the reaction mixture was quenched with water (100 mL) and MeOH was evaporated. The residue was extracted with AcOEt (3 x 100 mL). The combined organic solutions were dried over anhydrous Na₂SO₄, filtered and concentrated *in vacuo*. The crude product was used to the next step without additional purification. Compound **Ib** was obtained as a beige solid in 98% yield (10.28 g; 51.66 mmol).
ESI-MS m/z for C₈H₇ClNOS found 200.0/202.0 [M+H]⁺; Rₜ= 0.82 min; ¹H NMR (700 MHz, CDCl₃) δ 8.55 (d, *J =* 5.1 Hz, 1H), 7.44 (t, *J =* 1.1 Hz, 1H), 7.26 (d, *J* = 5.1 Hz, 1H), 5.01 (s, 2H).

### Step 3.

Synthesis of 3-((7-chlorothieno[3,2-*b*]pyridin-2-yl)methyl)-6,6-dimethyl-3-azabicyclo[3.1.0]hexane-2,4-dione (**Ic**).

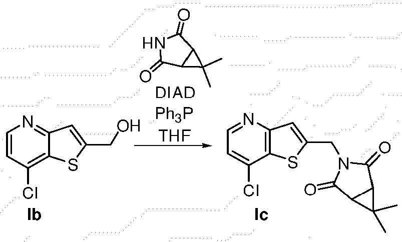

To a cooled to -10 °C solution of **Ib** (4.5 g; 22.5 mmol), 6,6-dimethyl-3-azabicyclo[3.1.0]hexane-2,4-dione (3.45 g; 24.8 mmol) and Ph₃P (7.1 g; 27.0 mmol) in THF (100 mL) DIAD (5.9 g; 5.7 mL; 29.3 mmol) was slowly added. The resulting mixture was stirred at room temperature overnight. The formed solid was filtered and washed with hexane (3 x). The title compound **Ic** was obtained as a white solid in 72% yield (5.2 g; 16.2 mmol) (95% purity, P(O)Ph₃ contaminated product).
ESI-MS m/z for C₁₅H₁₄ClN₂O₂S found 320.9/322.9 [M+H]⁺; Rₜ = 1.27 min

### Step 4.

Synthesis of (2-((6,6-dimethyl-2,4-dioxo-3-azabicyclo[3.1.0]hexan-3-yl)methyl)thieno[3,2-*b*]pyridin-7-yl)boronic acid (**I**).

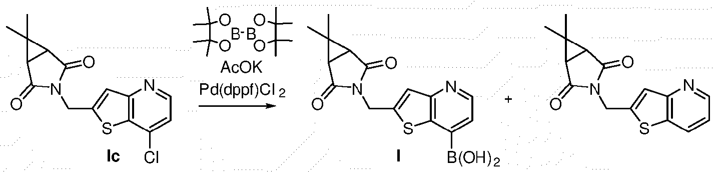

To the solution of **Ic** (5.62 g; 17.52 mmol) in dry dioxane (88.0 mL) bis(pinacolato)diboron (8.90 g; 35.05 mmol) and AcOK (5.16 g; 52.56 mmol) were added. The reaction mixture was intensively flushed with Ar. Then to this mixture Pd(dppf)Cl₂ (0.71 g; 0.876 mmol) was added in one portion and the reaction mixture was flushed with Ar. The mixture was stirred overnight at 100 °C in a sealed tube. The reaction progress was monitored by LC-MS. When analysis indicated completion of the reaction, the reaction mixture was filtered off and 1 M HCL_{aq} (200 mL) was added to the filtrate and it was washed with Et₂O (2 x 300 mL). To the aqueous fraction the solid NaHCO₃ was carefully added portionwise to pH 8 and it was extracted with AcOEt (3 x). The combined organic layers were dried over anhydrous MgSO₄, filtered and concentrated *in vacuo.* The solid residue was triturated with Et₂O and filtered off. The title compound **I** was obtained as a grey solid in 93% yield (5.40 g; 16.36 mmol). The crude product (included approx c.a 5-7% of dehalogenated substrate) was used to the next step without additional purification.
ESI-MS m/z for C₁₅H₁₆BN₂O₄S found 330.8 [M+H]⁺; Rₜ = 0.84 min

### General procedure B

Preparation of 2-chloro-4-methyl-6-(trifluoromethyl)nicotinic acid (**II**).

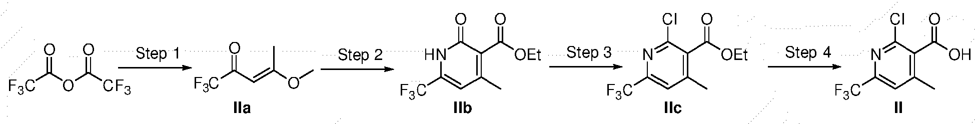

### Step 1.

Synthesis of (*E*)-1,1,1-trifluoro-4-methoxypent-3-en-2-one (**IIa**).

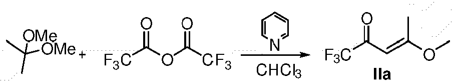

To a cooled to 0°C solution of 2,2-dimethoxypropane (20 g; 192 mmol), pyridine (31 mL, 398 mmol) in chloroform (50 mL) a solution of 2,2,2-trifluoroacetic anhydride (53 mL; 381 mmol) in chloroform (50 mL) was added dropwise. The reaction mixture was stirred at room temperature overnight. Then the reaction mixture was cooled in ice-bath to 0 °C and quenched with 5% NaHCO₃. Next the reaction mixture was extracted with DCM (2 x). The combined organic layers were washed again with 5% NaHCO₃, cold 1 M HCl, brine and dried over anhydrous Na₂SO₄, filtered and concentrated *in vacuo.* The crude product was distilled under reduced pressure (25 mmHg/45 °C). The title compound **IIa** was obtained as a yellow oil in 48% yield (15.7 g; 93.38 mmol).
ESI-MS m/z for C₆H₈F₃O₂ found 169.1 [M+H]⁺; Rₜ = 1.14 min; ¹H NMR (700 MHz, CDCl₃) δ 5.70 (s, 1H), 3.81 (s, 3H), 2.43 (s, 1H).

### Step 2.

Synthesis of ethyl 4-methyl-2-oxo-6-(trifluoromethyl)-1,2-dihydropyridine-3-carboxylate (**IIb**).

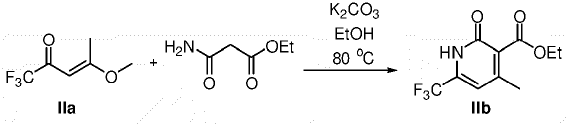

To a solution of ethyl-3-amino-3-oxopropanoate (0.78 g; 5.95 mmol) in EtOH (20 mL) compound **IIa** (1 g; 5.95 mmol) and K₂CO₃ (2.4 g; 17.85 mmol) were added. The reaction mixture was stirred at 80 °C for 3 hours. The reaction mixture was quenched with 2 M HCl. An aqueous residue was extracted with AcOEt (2 x). The combined organic layers were washed with brine, dried over anhydrous Na₂SO₄, filtered and concentrated *in vacuo* and the crude product was used to the next step without additional purification. Compound **IIb** was obtained as an orange oil in 72% yield (1.07 g; 4.29 mmol).
ESI-MS m/z for C₁₀H₁₁F₃NO₃ found 250.0 [M+H]⁺; Rₜ = 1.08 min; ¹H NMR (700 MHz, CDCl₃) δ 7.06 (s, 1H), 4.55 - 4.49 (m, 2H), 2.62 (s, 3H), 1.47 (t, *J =* 7.2 Hz, 3H).

### Step 3.

Synthesis of ethyl 2-chloro-4-methyl-6-(trifluoromethyl)nicotinate (**IIc**).

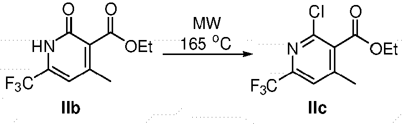

A mixture of **IIb** (1 g; 4.0 mmol) and phenyl dichlorophosphate (2.1 mL; 14.04 mmol) was heated under microwave irradiation using described method - 1500W, 165 °C (ramp temp. 10 minutes + hold temp. 20 minutes). The reaction mixture was poured into ice, stirred and diluted with AcOEt. Then the solution was adjusted to pH 8 by addition of 5% NaHCO₃. The layers were separated and an organic one was washed with brine, dried over anhydrous Na₂SO₄, filtered and concentrated *in vacuo.* The crude product was purified by flash column chromatography on silica (hexane/AcOEt, 100:0 to 80:20, 15 minutes, v/v). Compound **IIc** was obtained in 33% yield (0.33 g; 1.23 mmol).
¹H NMR (700 MHz, CDCl₃) δ 7.52 (s, 1H), 4.52 - 4.49 (m, 2H), 2.46 (s, 3H), 1.45 (t, *J =* 7.2 Hz, 3H).

### Step 4.

Synthesis of 2-chloro-4-methyl-6-(trifluoromethyl)nicotinic acid (**II**).

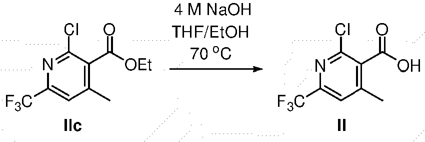

To the solution of **IIc** (6.9 g; 25.78 mmol) in THF/EtOH (30 mL/30 mL) a solution of 4 M NaOH (30 mL) was added. The reaction mixture was heated at 70 °C for 4 hours. Next solvents were evaporated *in vacuo* and an aqueous residue was acidified with 6 M HCl to pH 5 and then extracted with AcOEt (5 x) and concentrated to small volume and again extracted with AcOEt (3 x). The combined organic layers were dried over anhydrous Na₂SO₄, filtered and concentrated *in vacuo* and the crude product was used to the next step without additional purification. Compound **II** was obtained as a beige solid in 99% yield (6.10 g; 25.52 mmol). ESI-MS m/z for C₈H₆ClF₃NO₂ found 237.7 [M-H]⁺; Rₜ = 0.53 min; ¹H NMR (700 MHz, Methanol-*d₄*) δ 7.78 (s, 1H), 2.51 (s, 3H).

### General procedure C

Preparation of *tert*-butyl (*S*)-ethyl(pyrrolidin-3-yl)carbamate (**III**).

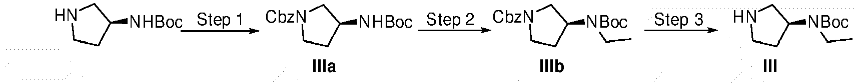

### Step 1.

Synthesis of benzyl (*S*)-3-((*tert*-butoxycarbonyl)amino)pyrrolidine-1-carboxylate (**IIIa**).

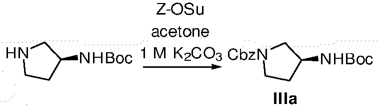

To the solution of *tert*-butyl (*S*)-pyrrolidin-3-ylcarbamate (0.26 g; 1.40 mmol) in a mixture of acetone (6.5 mL) and 1 M K₂CO₃ (5.2 mL) cooled to 0 °C a solution of Z-OSu (0.35 g; 1.40 mmol) in acetone (1 mL) was added dropwise. The reaction mixture was stirred at room temperature overnight. Then acetone was evaporated *in vacuo.* To the aqueous residue DCM was added and the product was extracted with DCM. Then an organic layer was washed with 1 M HCl, 1 M NaOH and brine. The organic layer was dried over anhydrous Na₂SO₄, filtered and concentrated *in vacuo.* The crude product was used to the next step without additional purification. Compound **IIIa** was obtained as a white solid in 95% yield (0.43 g; 1.34 mmol). ESI-MS m/z for C₁₇H₂₄N₂O₄Na found 343.1 [M+Na]⁺; Rₜ= 1.5 min; ¹H NMR (700 MHz, CDCl₃) δ 7.40 - 7.36 (m, 4H), 7.33 (ddd, *J* = 12.3, 8.3, 2.4 Hz, 1H), 5.16 (s, 2H), 4.74 - 4.56 (m, 1H), 4.23 (s, 1H), 3.76 - 3.63 (m, 2H), 3.52 (dd, *J =* 13.0, 6.7 Hz, 2H), 3.40 - 3.26 (m, 1H), 2.21 - 2.11 (m, 1H), 1.86 (dd, *J =* 43.9, 16.8 Hz, 1H), 1.47 (s, 9H).

### Step 2.

Synthesis of benzyl (*S*)-3-((*tert*-butoxycarbonyl)(ethyl)amino)pyrrolidine-1-carboxylate (**IIIb**).

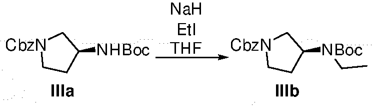

To the solution of **IIIa** (0.21 g; 0.66 mmol) in THF (3.5 mL) under argon atmosphere NaH (60% in mineral oil; 40 mg; 1.65 mmol) was added. The reaction mixture was then stirred at room temperature for 20 minutes. Next EtI (0.18 mL; 2.64 mmol) was added and whole was stirred at room temperature for 1 hour. The reaction mixture was diluted with AcOEt and washed with 1 M HCl, 1 M NaOH and brine. An organic layer was dried over anhydrous Na₂SO₄, filtered and concentrated *in vacuo* and the crude product was purified by flash column chromatography on silica (hexane/AcOEt, 100:0 to 0:100, v/v). Compound **IIIb** was obtained in 48% yield (0.11 g; 0.32 mmol).
ESI-MS m/z for C₁₉H₂₈N₂O₄Na found 371.1 [M+Na]⁺; Rₜ= 1.8 min; ¹H NMR (700 MHz, CDCl₃) δ 7.42 - 7.37 (m, 4H), 7.35 - 7.32 (m, 1H), 5.16 (q, *J =* 12.8 Hz, 2H), 4.58 (s, 1H), 3.66 (dd, *J =* 30.7, 21.9 Hz, 2H), 3.30 (dd, *J =* 36.6, 26.4 Hz, 2H), 3.22 (d, *J =* 9.8 Hz,2H), 2.04 (dd, *J* = 31.9, 8.2 Hz, 2H), 1.48 (s, 9H), 1.13 (t, *J =* 6.9 Hz, 3H).

### Step 3.

Synthesis of *tert*-butyl (*S*)-ethyl(pyrrolidin-3-yl)carbamate (**III**).

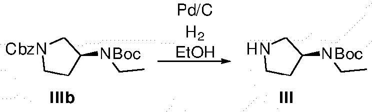

To the solution of **IIIb** (0.1 g; 0.29 mmol) in EtOH (3 mL) under argon atmosphere Pd/C (10 mol%; cat.) was added. Then argon was replaced by hydrogen and the reaction mixture was conducted under hydrogen atmosphere at room temperature for 2 hours. The reaction progress was monitored by LC-MS. When analysis indicated completion of the reaction, Pd/C was filtered off through a Celite pad and the solvent was stripped *in vacuo.* The crude product was used to the next step without additional purification. Compound **III** was obtained as a transparent oil in 98% yield (60 mg; 0.28 mmol).
ESI-MS m/z for C₁₁H₂₃N₂O₂ found 215.10 [M+H]⁺; Rₜ= 0.65 min; ¹H NMR (700 MHz, Methanol-*d*₄) δ 4.32 - 4.06 (m, 1H), 3.31 - 3.23 (m, 3H), 3.02 (ddd, *J =* 28.6, 19.8, 9.9 Hz, 3H), 2.17 - 1.88 (m, 2H), 1.49 (d, *J =* 4.1 Hz, 9H), 1.15 (q, *J =* 7.4 Hz, 3H).

### Example 1.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile 2,2,2-trifluoroacetate (**1**).

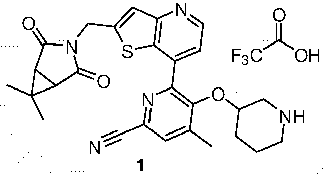

### Step 1.

Synthesis of 6-bromo-5-hydroxy-4-methylpicolinonitrile (**1a**)**.**

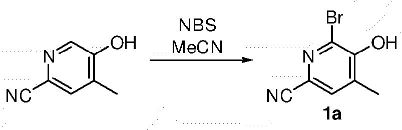

To the solution of 5-hydroxy-4-methylpicolinonitrile (136 mg; 1.01 mmol) in MeCN (3 mL) NBS (180 mg; 1.01 mmol) was added and then the reaction mixture was stirred at room temperature overnight. The reaction progress was monitored by LC-MS. When analysis indicated completion of the reaction, to this mixture 10% Na₂S₂O₃ was added and then extracted with AcOEt. The organic layer was washed with brine, dried over anhydrous MgSO₄, filtered and concentrated *in vacuo* and the crude product was used to the next step without additional purification. Compound **1a** was obtained in 99% yield (214 mg; 1.01 mmol).
ESI-MS m/z for C₇H₄BrN₂O found 211.0/213.0 [M-H]⁺, Rₜ = 0.86 min

### Step 2.

Synthesis of *tert*-butyl 3-((2-bromo-6-cyano-4-methylpyridin-3-yl)oxy)piperidine-1-carboxylate (**1b**).

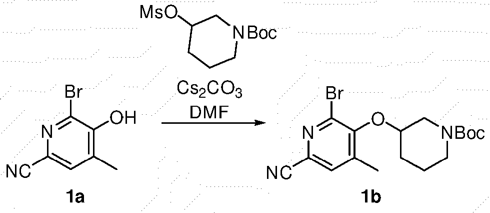

To the solution of **1a** (84 mg; 0.39 mmol) in DMF (1.5 mL) *tert*-butyl 3-((methylsulfonyl)oxy)piperidine-1-carboxylate (132 mg; 0.47 mmol) and Cs₂CO₃ (257 mg; 0.79 mmol) were added and then the reaction mixture was heated at 70 °C overnight. The reaction progress was monitored by TLC and LC-MS. Next another portion of *tert*-butyl 3-((methylsulfonyl)oxy)piperidine-1-carboxylate (132 mg; 0.47 mmol) was added and stirred at 70 °C overnight. When analyses indicated completion of the reaction, this mixture was taken into AcOEt/water. An organic layer was washed with brine, dried over anhydrous MgSO₄, filtered and concentrated *in vacuo* and the crude product was purified by flash column chromatography on silica (hexane/AcOEt, 100:0 to 80:20, *v*/*v*, 15 minutes). Compound **1b** was obtained in 10% yield (15 mg; 0.04 mmol).
ESI-MS m/z for C₁₃H₁₅BrN₃O₃ found 340.0/342.0 [M+H-*t*Bu]⁺; Rₜ= 1.67 min

### Step 3.

Synthesis of tert-butyl 3-((6-cyano-2-(2-((6,6-dimethyl-2,4-dioxo-3-azabicyclo[3.1.0]hexan-3-yl)methyl)thieno[3,2-*b*]pyridin-7-yl)-4-methylpyridin-3-yl)oxy)piperidine-1-carboxylate (**1c**).

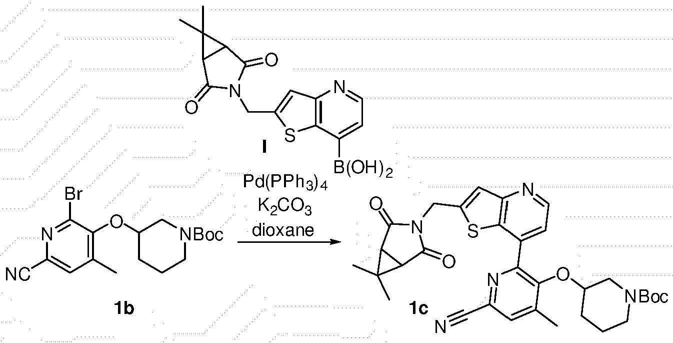

The title compound (**1c**) was obtained from **1b** (15 mg; 0.040 mmol) and from boronic acid **I** (15 mg; 0.045 mmol) according to the General Procedure **Va** and after standard work-up all the crude product mixture was taken for the next step.
ESI-MS m/z for C₃₂H₃₆N₅O₅S found 602.3 [M+H]⁺; Rₜ= 1.63 min

### Step 4.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile 2,2,2-trifluoroacetate (**1**).

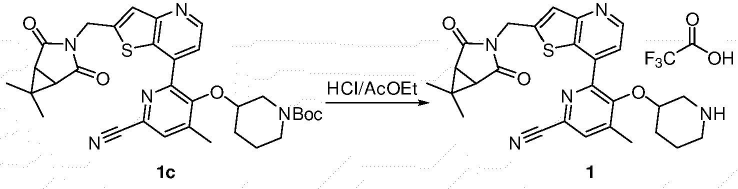

The title compound (**1**) was obtained as a racemate as a TFA salt from **1c** (crude reaction mixture) according to the General Procedure **IVa** in 15% yield (per two steps) (4 mg; 0.006 mmol). The crude product was purified by preparative reversed-phase column chromatography (column: Cosmosil Cholester 20 x 250 mm, water/MeCN + 1‰ TFA, 99:1 to 40:60, 30 min, 20 mL/min).
ESI-MS m/z for C₂₇H₂₈N₅O₃S found 502.3 [M+H]⁺; Rₜ= 0.93 min; ¹H NMR (700 MHz, D₂O) δ 8.82 - 8.78 (m, 1H), 8.05 - 8.01 (m, 1H), 7.98 - 7.95 (m, 1H), 7.67 - 7.64 (m, 1H), 4.99 - 4.90 (m, 2H), 4.07 - 3.95 (m, 1H), 3.27 - 3.17 (m, 1H), 3.17 - 3.09 (m, 1H), 2.99 - 2.89 (m, 2H), 2.61 (s, 2H), 2.46 (s, 3H), 1.56 - 1.44 (m, 2H), 1.33 - 1.22 (m, 2H), 1.18 (s, 3H), 1.01 (s, 3H).

### Example 2.

Synthesis of 3-((7-(6-chloro-4-methyl-3-(pyrrolidin-3-yloxy)pyridin-2-yl)thieno[3,2-*b*]pyridin-2-yl)methyl)-6,6-dimethyl-3-azabicyclo[3.1.0]hexane-2,4-dione hydrochloride (**2**).

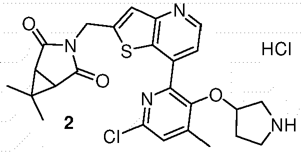

The title compound (**2**) was obtained as a hydrochloride salt as a racemate in 5% overall yield in a similar way to Example 1 with the exception that, in the first step of the synthesis, 6-chloro-4-methylpyridin-3-ol was used instead of 5-hydroxy-4-methylpicolinonitrile, in the second step of the synthesis, *tert*-butyl 3-((methylsulfonyl)oxy)pyrrolidine-1-carboxylate was used instead of *tert*-butyl 3-((methylsulfonyl)oxy)piperidine-1-carboxylate and in the last step of the synthesis the crude product was purified by preparative reversed-phase column chromatography (C-18, water + 0.3‰ HCl (36%)/MeCN, 99:1 to 30:70, 30 min, 20 mL/min). ESI-MS m/z for C₂₅H₂₆ClN₄O₃S found 497.3/499.3 [M+H]⁺; Rₜ= 0.96 min; ¹H NMR (700 MHz, D₂O) δ 8.75 - 8.72 (m, 1H), 8.03 - 7.98 (m, 1H), 7.62 - 7.58 (m, 1H), 7.49 - 7.46 (m, 1H), 4.94 - 4.84 (m, 2H), 4.50 - 4.37 (m, 1H), 3.46 - 3.36 (m, 1H), 3.30 - 3.17 (m, 2H), 3.07 - 2.94 (m, 1H), 2.61 (s, 2H), 2.38 (s, 3H), 1.79 - 1.69 (m, 2H), 1.18 (s, 3H), 1.02 (s, 3H).

### Example 3.

Synthesis of 3-((7-(6-chloro-4-methyl-3-(piperidin-3-yloxy)pyridin-2-yl)thieno[3,2-*b*]pyridin-2-yl)methyl)-6,6-dimethyl-3-azabicyclo[3.1.0]hexane-2,4-dione hydrochloride (**3**).

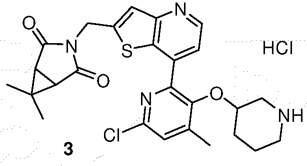

The title compound (**3**) was obtained as a hydrochloride salt as a racemate in 14% overall yield in a similar way to Example 1 with the exception that, in the first step of the synthesis, 6-chloro-4-methylpyridin-3-ol was used instead of 5-hydroxy-4-methylpicolinonitrile and in the last step of the synthesis the crude product was purified by preparative reversed-phase column chromatography (column: Cosmosil Cholester 20 x 250 mm, water + 0.3‰ HCl (36%)/MeCN, 99:1 to 30:70, 30 min, 20 mL/min).
ESI-MS m/z for C₂₆H₂₈ClN₄O₃S found 511.3/513.3 [M+H]⁺; Rₜ= 0.99 min; ¹H NMR (700 MHz, D₂O) δ 8.81 - 8.76 (m, 1H), 8.11 - 8.08 (m, 1H), 7.66 - 7.63 (m, 1H), 7.52 - 7.49 (m, 1H), 4.98 - 4.86 (m, 2H), 3.96 - 3.88 (m, 1H), 3.27 - 3.20 (m, 1H), 3.15 - 3.07 (m, 1H), 3.04 - 2.97 (m, 1H), 2.94 - 2.86 (m, 1H), 2.61 (s, 2H), 2.39 (s, 3H), 1.57 - 1.46 (m, 2H), 1.33 - 1.25 (m, 1H), 1.25 - 1.21 (m, 1H), 1.19 (s, 3H), 1.02 (s, 3H).

### Example 4.

Synthesis of 3-((7-(6-chloro-4-methyl-3-(((*S*)-piperidin-3-yl)oxy)pyridin-2-yl)thieno[3,2-*b*]pyridin-2-yl)methyl)-6,6-dimethyl-3-azabicyclo[3.1.0]hexane-2,4-dione hydrochloride (**4**).

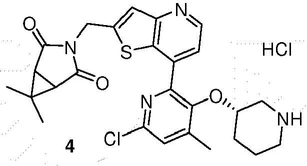

The title compound (**4**) was obtained as a hydrochloride salt as a single enantiomer of compound 3 in 1% overall yield in a similar way to Example 1 with the exception that, in the first step of the synthesis, 6-chloro-4-methylpyridin-3-ol was used instead of 5-hydroxy-4-methylpicolinonitrile, in the second step of the synthesis, *tert*-butyl (*R*)-3-((methylsulfonyl)oxy)piperidine-1-carboxylate was used instead of *tert*-butyl 3-((methylsulfonyl)oxy)piperidine-1-carboxylate and in the last step of the synthesis the crude product was purified by preparative reversed-phase column chromatography (C-18, water + 0.3‰ HCl (36%)/MeCN, 99:1 to 30:70, 30 min, 20 mL/min).
ESI-MS m/z for C₂₆H₂₈ClN₄O₃S found 511.4/513.4 [M+H]⁺; Rₜ= 0.99 min; ¹H NMR (700 MHz, D₂O) δ 8.80 - 8.78 (m, 1H), 8.11 - 8.07 (m, 1H), 7.68 - 7.64 (m, 1H), 7.55 - 7.52 (m, 1H), 5.00 - 4.88 (m, 2H), 3.96 - 3.88 (m, 1H), 3.28 - 3.20 (m, 1H), 3.14 - 3.07 (m, 1H), 3.05 - 2.97 (m, 1H), 2.96 - 2.84 (m, 1H), 2.61 (s, 2H), 2.40 (s, 3H), 1.57 - 1.44 (m, 2H), 1.34 - 1.25 (m, 1H), 1.24 - 1.19 (m, 1H), 1.19 (s, 3H), 1.02 (s, 3H).

### Example 18.

Synthesis of 6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione 2,2,2-trifluoroacetate (**18**).

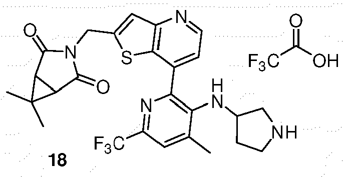

### Step 1.

Synthesis of *tert*-butyl 3-((4-methyl-6-(trifluoromethyl)pyridin-3-yl)amino)pyrrolidine-1-carboxylate (**18a**).

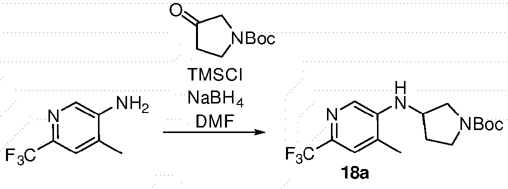

The title compound (**18a**) was obtained from 4-methyl-6-(trifluoromethyl)pyridin-3-amine (0.20 g; 1.16 mmol) and from *tert*-butyl 3-oxopyrrolidine-1-carboxylate (0.32 g; 1.74 mmol) according to the General Procedure **VIb** in 50% yield (0.20 g; 0.58 mmol).
ESI-MS m/z for C₁₆H₂₃F₃N₃O₂ found 346.0 [M+H]⁺; Rₜ= 1.51 min

### Step 2.

Synthesis of *tert*-butyl 3-((2-bromo-4-methyl-6-(trifluoromethyl)pyridin-3-yl)amino)pyrrolidine-1-carboxylate (**18b**).

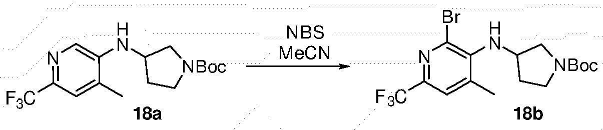

To the solution of **18a** (0.2 g; 0.58 mmol) in MeCN (5 mL) NBS (103 mg; 0.58 mmol) was added and then the reaction mixture was stirred at room temperature overnight. The reaction progress was monitored by TLC and LC-MS. When analyses indicated completion of the reaction, to this mixture 10% Na₂S₂O₃ was added and then extracted with AcOEt. The organic layer was washed with brine, dried over anhydrous MgSO₄, filtered and concentrated *in vacuo* and the crude product was used to the next step without additional purification. Compound **18b** was obtained in 98% yield (240 mg; 0.57 mmol).
ESI-MS m/z for C₁₂H₁₄BrF₃N₃O₂ found 368.0/370.0 [M+H-*t*Bu]⁺; Rₜ= 1.70 min

### Step 3.

Synthesis of *tert*-butyl 3-((2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)amino)pyrrolidine-1-carboxylate (**18c**).

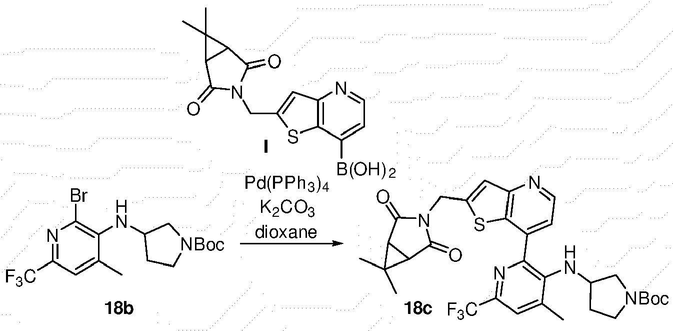

The title compound (**18c**) was obtained from **18b** (120 mg; 0.28 mmol) and from boronic acid **I** (93 mg; 0.28 mmol) according to the General Procedure **Va** and after standard work-up all the crude product mixture was taken to the next step.
ESI-MS m/z for C₃₁H₃₅F₃N₅O₄S found 630.1 [M+H]⁺; Rₜ= 1.62 min

### Step 4.

Synthesis of 6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione 2,2,2-trifluoroacetate (**18**).

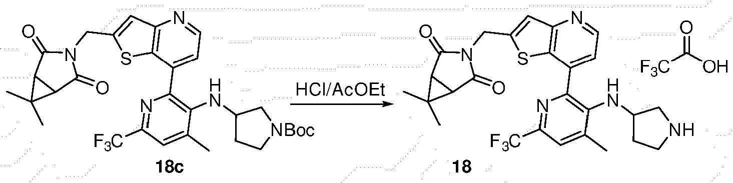

The title compound (**18**) was obtained as a TFA salt from **18c** (the crude reaction mixture) according to the General Procedure **IVa** in 6% yield (per two steps) (10 mg; 0.016 mmol). The crude product was purified three times by preparative reversed-phase column chromatography (first: C-18, water/MeCN, 99:1 to 45:55, 30 min, 20 mL/min; second: column: Cosmosil Cholester 20 x 250 mm, water/MeCN, 99:1 to 50:50, 40 min, 20 mL/min; third: column: Cosmosil Cholester 20 x 250 mm, water/MeCN + 1‰ TFA, 95:5 to 45:55, 30 min, 20 mL/min).
ESI-MS m/z for C₂₆H₂₇F₃N₅O₂S found 530.7 [M+H]⁺; Rₜ= 1.03 min; ¹H NMR (700 MHz, D₂O, 300 K) δ 8.85 - 8.80 (m, 1H), 8.15 - 8.11 (m, 1H), 7.81 (s, 1H), 7.72 - 7.68 (m, 1H), 4.96 (s, 2H), 3.52 - 3.40 (m, 1H), 3.35 - 3.23 (m, 1H), 3.05 - 2.92 (m, 3H), 2.63 (s, 2H), 2.45 (s, 3H), 1.90 - 1.79 (m, 1H), 1.79 - 1.68 (m, 1H), 1.20 (s, 3H), 1.02 (s, 3H).

### Example 20.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-(pyrrolidin-3-ylamino)picolinonitrile dihydrochloride (**20**).

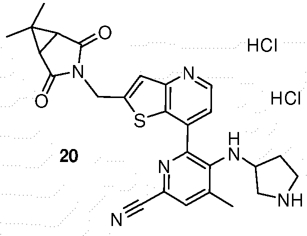

The title compound (**20**) was obtained as a dihydrochloride salt in 6% overall yield in a similar way to Example 18 with the exception that, in the first step of the synthesis, 5-amino-4-methylpicolinonitrile was used instead of 4-methyl-6-(trifluoromethyl)pyridin-3-amine and in the last step of the synthesis the crude product was purified three times by preparative reversed-phase column chromatography (first: C-18, water/MeCN, 99:1 to 45:55, 30 min, 20 mL/min; second: C-18, water/MeCN, 99:1 to 50:50, 40 min, 20 mL/min; third: column: Cosmosil Cholester 20 x 250 mm, water/MeCN + HCl, 99:1 to 45:55, 30 min, 20 mL/min).
ESI-MS m/z for C₂₆H₂₇N₆O₂S found 487.5 [M+H]⁺; Rₜ= 0.93 min; ¹H NMR (700 MHz, D₂O, 300 K) δ 8.79 - 8.74 (m, 1H), 7.88 - 7.85 (m, 1H), 7.71 (s, 1H), 7.64 - 7.60 (m, 1H), 4.91 (s, 2H), 3.46 - 3.38 (m, 1H), 3.32 - 3.25 (m, 1H), 3.02 - 2.86 (m, 3H), 2.61 (s, 2H), 2.38 (s, 3H), 1.79 - 1.69 (m, 2H), 1.20 (s, 3H), 1.04 (s, 3H).

### Example 24.

Synthesis of 5-(azetidin-3-ylamino)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile 2,2,2-trifluoroacetate (**24**).

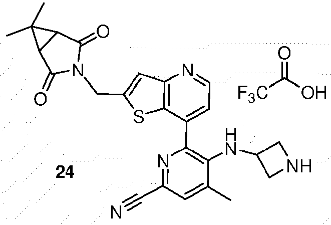

The title compound (**24**) was obtained as a TFA salt in 3% overall yield in a similar way to Example 18 with the exception that, in the first step of the synthesis, 5-amino-4-methylpicolinonitrile was used instead of 4-methyl-6-(trifluoromethyl)pyridin-3-amine and *tert-*butyl 3-oxoazetidine-1-carboxylate was used instead of *tert*-butyl 3-oxopyrrolidine-1-carboxylate and in the last step of the synthesis the crude product was purified by preparative reversed-phase column chromatography (C-18, water/MeCN + 1‰ TFA, 99:1 to 45:55, 30 min, 20 mL/min).
ESI-MS m/z for C₂₅H₂₅N₆O₂S found 473.6 [M+H]⁺; Rₜ= 0.88 min; ¹H NMR (700 MHz, D₂O, 300 K) δ 8.82 - 8.79 (m, 1H), 7.89 - 7.86 (m, 1H), 7.71 - 7.67 (m, 2H), 4.94 (s, 2H), 3.95 - 3.89 (m, 2H), 3.86 - 3.79 (m, 1H), 3.65 - 3.56 (m, 2H), 2.63 (s, 2H), 2.38 (s, 3H), 1.21 (s, 3H), 1.07 (s, 3H).

### Example 27.*(this compound is not a part of the present invention)

Synthesis of *N*-(6-cyano-4-(2-((6,6-dimethyl-2,4-dioxo-3-azabicyclo[3.1.0]hexan-3-yl)methyl)thieno[3,2-*b*]pyridin-7-yl)-2-methylpyridin-3-yl)azetidine-2-carboxamide 2,2,2-trifluoroacetate (**27**).

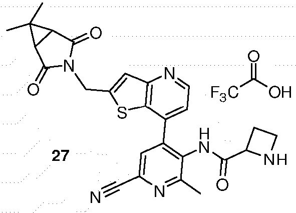

### Step 1.

Synthesis of 5-amino-4-bromo-6-methylpicolinonitrile (**27a**).

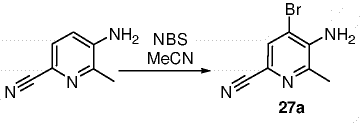

To the solution of 5-amino-6-methylpicolinonitrile (0.25 g; 1.88 mmol) in MeCN (10 mL) NBS (368 mg; 2.06 mmol) was added portionwise at 0 °C and then the reaction mixture was stirred at this temperature for 1 hour, then at room temperature for 1 hour. The reaction progress was monitored by TLC and LC-MS. When analyses indicated completion of the reaction, the reaction mixture was diluted with MeCN and filtered through a pad of Celite. The filtrate was concentrated *in vacuo* and the residue was dissolved in water/AcOEt. The layers were separated and the aqueous one was extracted with AcOEt (3 x). The combined organic solutions were washed with 10% Na₂S₂O₃ and brine, dried over anhydrous Na₂SO₄, filtered and concentrated *in vacuo* and the crude product was purified by flash column chromatography on silica (hexane 100%, 2 minutes and then hexane/AcOEt 100:0 to 0:100, *v*/*v,* 20 minutes). Compound **27a** was obtained in 68% yield (271 mg; 1.28 mmol).
ESI-MS m/z for C₇H₇BrN₃ found 212.0/214.0 [M+H]⁺; Rt= 0.79 min

### Step 2.

Synthesis of *tert*-butyl 2-((4-bromo-6-cyano-2-methylpyridin-3-yl)carbamoyl)azetidine-1-carboxylate (**27b**).

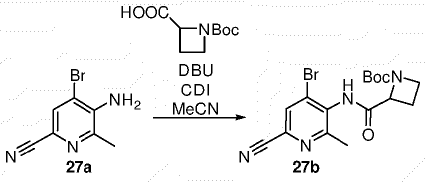

To the solution of **27a** (79 mg; 0.37 mmol) in MeCN (0.8 mL) DBU (61 µL; 0.41 mmol) was added and stirred at room temperature for 1 hour. To the solution of 1-(*tert-*butoxycarbonyl)azetidine-2-carboxylic acid (75 mg; 0.37 mmol) in MeCN (0.8 mL) CDI (67 mg; 0.41 mmol) was added and stirred at room temperature for 1 hour, then added to the solution of substrate with DBU and stirred at 45 °C overnight. The reaction progress was monitored by LC-MS. When analysis indicated completion of the reaction, MeCN was evaporated to 30% vol. The residue was taken into AcOEt/water. An organic layer was washed with water and brine, dried over anhydrous MgSO₄, filtered and concentrated *in vacuo* and the crude product was purified by flash column chromatography on silica (hexane/AcOEt, 100:0 to 80:20, *v*/*v*, 15 minutes). Compound **27b** was obtained in 76% yield (110 mg; 0.28 mmol).
ESI-MS m/z for C₁₆H₂₀BrN₄O₃ found 395.0/397.0 [M+H]⁺; Rt= 1.21 min

### Step 3.

Synthesis of *tert*-butyl 2-((6-cyano-4-(2-((6,6-dimethyl-2,4-dioxo-3-azabicyclo[3.1.0]hexan-3-yl)methyl)thieno[3,2-b]pyridin-7-yl)-2-methylpyridin-3-yl)carbamoyl)azetidine-1-carboxylate (**27c**).

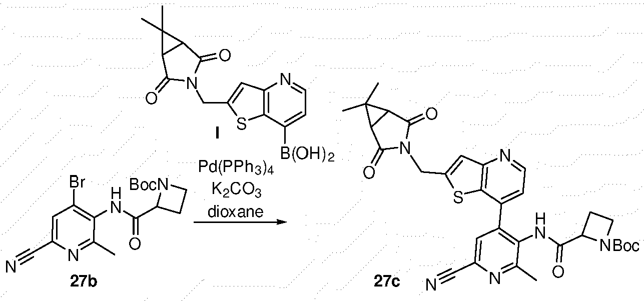

The title compound (**27c**) was obtained from **27b** (55 mg; 0.14 mmol) and from boronic acid **I** (60 mg; 0.18 mmol) according to the General Procedure **Va** and after standard work-up all the crude product mixture was taken to the next step.
ESI-MS m/z for C₃₁H₃₃N₆O₅S found 601.1 [M+H]⁺; Rt= 1.36 min

### Step 4.

Synthesis of *N*-(6-cyano-4-(2-((6,6-dimethyl-2,4-dioxo-3-azabicyclo[3.1.0]hexan-3-yl)methyl)thieno[3,2-*b*]pyridin-7-yl)-2-methylpyridin-3-yl)azetidine-2-carboxamide 2,2,2-trifluoroacetate (**27**).

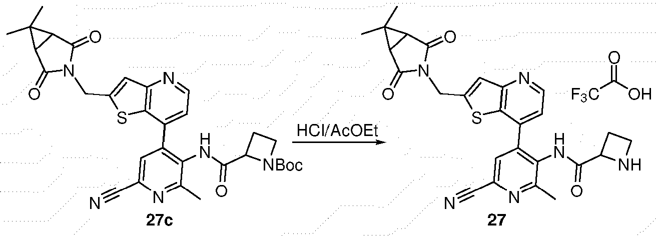

The title compound (**27**) was obtained as a TFA salt from **27c** (the crude reaction mixture) according to the General Procedure **IVa** in 11% yield (per two steps) (9 mg; 0.015 mmol). The crude product was purified by preparative reversed-phase column chromatography (C-18, water/MeCN + 1‰ TFA, 99:1 to 45:55, 30 min, 20 mL/min).
ESI-MS m/z for C₂₆H₂₅N₆O₃S found 501.7 [M+H]⁺; Rₜ = 0.81 min; ¹H NMR (700 MHz, D₂O, 333 K) δ 9.17 - 9.13 (m, 1H), 8.39 - 8.35 (m, 1H), 8.00 - 7.97 (m, 1H), 7.85 - 7.82 (m, 1H), 5.44 - 5.38 (m, 1H), 5.27 (s, 2H), 4.43 - 4.32 (m, 1H), 4.08 - 4.00 (m, 1H), 2.98 (s, 2H), 2.95 (s, 4H), 2.01 - 1.90 (m, 1H), 1.57 (s, 3H), 1.40 (s, 3H).

### Example 28.

Synthesis of *N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide 2,2,2-trifluoroacetate (**28**).

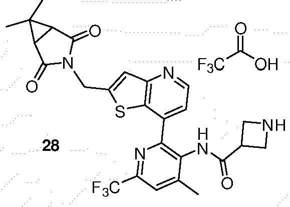

### Step 1.

Synthesis of 2-bromo-4-methyl-6-(trifluoromethyl)pyridin-3-amine (**28a**).

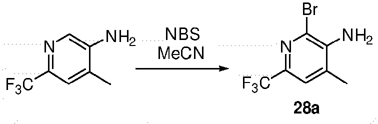

To the solution of 4-methyl-5-nitro-2-(trifluoromethyl)pyridine (96 mg; 0.55 mmol) in MeCN (2 mL) NBS (97 mg; 0.55 mmol) was added at 0 °C and then the reaction mixture was stirred at this temperature for 2 hours. The reaction progress was monitored by TLC and LC-MS. When analyses indicated completion of the reaction, to this mixture 10% Na₂S₂O₃ was added followed by AcOEt. The layers were separated and an organic layer was washed with 10% Na₂S₂O₃, brine, dried over anhydrous Na₂SO₄, filtered and concentrated *in vacuo* and the crude product was used to the next step without additional purification. Compound **28a** was obtained in 84% yield (117 mg; 0.46 mmol).
ESI-MS m/z for C₇H₇BrF₃N₂ found 255.0/257.0 [M+H]⁺; Rt= 1.23 min

### Step 2.

Synthesis of *tert*-butyl 3-((2-bromo-4-methyl-6-(trifluoromethyl)pyridin-3-yl)carbamoyl)azetidine-1-carboxylate (**28b**).

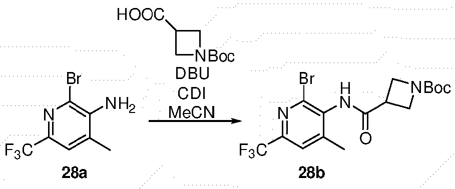

To the solution of **28a** (74 mg; 0.29 mmol) in MeCN (1 mL) DBU (48 µL; 0.32 mmol) was added and stirred at room temperature for 1 hour. To the solution of 1-(*tert-*butoxycarbonyl)azetidine-3-carboxylic acid (59 mg; 0.29 mmol) in MeCN (1 mL) CDI (52 mg; 0.32 mmol) was added and stirred at room temperature for 1 hour, then added to the solution of substrate with DBU and stirred at 45 °C overnight. The reaction progress was monitored by LC-MS. When analysis indicated completion of the reaction, MeCN was evaporated to 30% vol. The residue was taken into AcOEt/water. An organic layer was washed with water and brine, dried over anhydrous MgSO₄, filtered and concentrated *in vacuo* and the crude product was purified by flash column chromatography on silica (hexane/AcOEt, 100:0 to 80:20, *v*/*v,* 15 minutes). Compound **28b** was obtained in 24% yield (31 mg; 0.07 mmol).
ESI-MS m/z for C₁₆H₂₀BrF₃N₃O₃ found 438.0/440.0 [M+H]⁺; Rt= 1.40 min

### Step 3.

Synthesis of *tert*-butyl 3-((2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)carbamoyl)azetidine-1-carboxylate (**28c**).

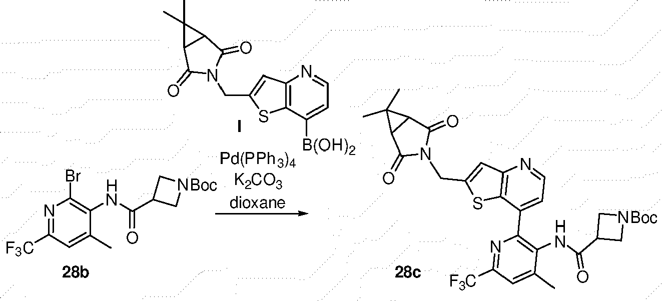

The title compound (**28c**) was obtained from **28b** (31 mg; 0.07 mmol) and from boronic acid **I** (30 mg; 0.09 mmol) according to the General Procedure **Va** and after standard work-up all the crude product mixture was taken to the next step.
ESI-MS m/z for C₃₁H₃₂F₃N₅O₅S found 644.1 [M+H]⁺; Rt= 1.47 min

### Step 5.

Synthesis of *N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide 2,2,2-trifluoroacetate (**28**).

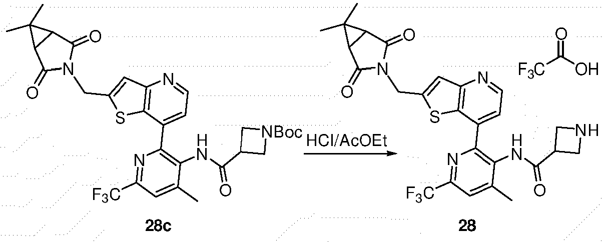

The title compound (**28**) was obtained as a TFA salt from **28c** (the crude reaction mixture) according to the General Procedure **IVa** in 11% yield (per two steps) (5 mg; 0.008 mmol). The crude product was purified by preparative reversed-phase column chromatography (C-18, water/MeCN + 1‰ TFA, 99:1 to 45:55, 30 min, 20 mL/min).
ESI-MS m/z for C₂₆H₂₅F₃N₅O₃S found 544.6 [M+H]⁺; Rt= 0.99 min; ¹H NMR (700 MHz, D₂O, 333 K) δ 9.18 - 9.13 (m, 1H), 8.45 - 8.40 (m, 1H), 8.17 - 8.14 (m, 1H), 8.08 - 8.04 (m, 1H), 5.36 - 5.27 (m, 2H), 4.57 - 4.49 (m, 2H), 4.26 - 4.13 (m, 3H), 3.02 - 2.95 (m, 2H), 2.81 (s, 3H), 1.57 (s, 3H), 1.40 (s, 3H).

Synthesis of 6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione hydrochloride (**35**).

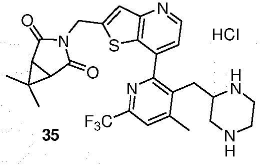

### Step 1.

Synthesis of (2-chloro-4-methyl-6-(trifluoromethyl)pyridin-3-yl)methanol (**35a**).

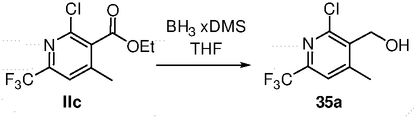

To the solution of **IIc** (5.1 g; 19.03 mmol) in THF (100 mL) BH₃ x DMS (3.6 mL; 38.06 mmol) was added dropwise and then the reaction mixture was allowed to 60 °C and stirred at this temperature overnight. The reaction progress was monitored by LC-MS. When analysis indicated completion of the reaction, to this mixture 2 M HCl was added and all was extracted with Et₂O. The organic phase was washed with 1 M NaOH and brine and then dried over anhydrous MgSO₄, filtered and concentrated *in vacuo* and the crude product was crystallized from Et₂O/hexane. Compound **35a** was obtained in 58% yield (2.5 g; 11.11 mmol).
ESI-MS m/z for C₈H₈ClF₃NO found 226.0/228.0 [M+H]⁺; Rt = 0.99 min

### Step 2.

Synthesis of 3-(bromomethyl)-2-chloro-4-methyl-6-(trifluoromethyl)pyridine (**35b**).

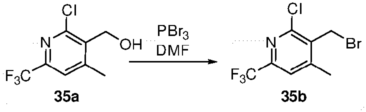

To a cooled to 0 °C solution of **35a** (2.4 g; 10.64 mmol) in DMF (50 mL) PBr₃ (2 mL; 21.27 mmol) was added portionwise and then the reaction mixture was allowed to room temperature. The reaction progress was monitored by LC-MS. When analysis indicated completion of the reaction, the obtained solid precipitate was removed by filtration, washed with Et₂O. The filtrate was poured into saturated solution of NH₄Cl. The phases were separated and an organic one was dried over anhydrous MgSO₄, filtered and concentrated *in vacuo* and the crude product was purified by flash column chromatography on silica (hexane/AcOEt, 100:0 to 90:10, *v*/*v*, 10 minutes). Compound **35b** was obtained in 70% yield (2.1 g; 7.28 mmol).
ESI-MS m/z for C₈H₇BrClF₃N found 287.7/289.7 [M+H]⁺; Rt = 1.55 min; ¹H NMR (700 MHz, CDCl₃) δ 7.48 (s, 1H), 4.65 (s, 2H), 2.61 - 2.49 (m, 3H).

### Step 3.

Synthesis of ethyl 2-amino-3-(2-chloro-4-methyl-6-(trifluoromethyl)pyridin-3-yl)propanoate (**35c**).

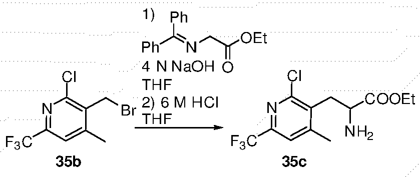

To a solution of **35b** (2 g; 6.93 mmol) and ethyl 2-((diphenylmethylene)amino)acetate (1.85 g; 6.93 mmol) in THF (100 mL) NaOH (4 N; 8.7 mL; 34.66 mmol) was added and then the reaction mixture was stirred at room temperature for 3 days. The reaction progress was monitored by LC-MS. When analysis indicated completion of the reaction, the mixture was diluted with AcOEt and washed with water and brine. An organic layer was dried over anhydrous MgSO₄, filtered and concentrated *in vacuo* and the crude product (2.7 g; 5.68 mmol) was dissolved in THF (28.5 mL), then whole was cooled to 0 °C and 6 M HCl (1.9 mL; 11.36 mmol) was added and the mixture was stirred at 0 °C for 1 hour. When analysis indicated completion of the reaction, to this mixture 1 M HCl (100 mL) was added and whole was washed with Et₂O (2 x). To the aqueous layer a solid K₂CO₃ was carefully added to pH 8-9 and extracted with AcOEt (2 x). The combined organic solutions were dried over anhydrous MgSO₄, filtered and concentrated *in vacuo* and the crude product was used to the next step without additional purification. Compound **35c** was obtained as a colorless oil in 68% yield (1.19 g; 3.84 mmol). ESI-MS m/z for C₁₂H₁₅ClF₃N₂O₂ found 310.9/312.8 [M+H]⁺; Rt = 0.57 min; ¹H NMR (700 MHz, CDCl₃) δ 7.46 (s, 1H), 4.24 - 4.16 (m, 2H), 3.89 - 3.84 (m, 1H), 3.32 - 3.25 (m, 1H), 3.15 - 3.05 (m, 1H), 2.54 (s, 3H), 1.24 (t, *J =* 7.2 Hz, 3H); ¹⁹F NMR (235 MHz, CDCl₃) δ -65.24 - - 72.83 (m).

### Step 4.

Synthesis of ethyl 2-(2-((*tert*-butoxycarbonyl)amino)acetamido)-3-(2-chloro-4-methyl-6-(trifluoromethyl)pyridin-3-yl)propanoate (**35d**).

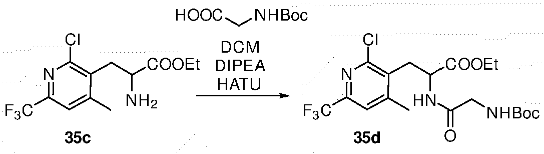

The title compound (**35d**) was obtained from **35c** (500 mg; 1.62 mmol) and from (*tert-*butoxycarbonyl)glycine (283 mg; 1.62 mmol) according to the General Procedure **I** in 96% yield (730 mg; 1.56 mmol).
ESI-MS m/z for C₁₉H₂₅ClF₃N₃O₅Na found 490.0/492.0 [M+Na]⁺; Rt = 1.51 min; ¹H NMR (700 MHz, CDCl₃) δ 7.46 (s, 1H), 6.92 - 6.88 (m, 1H), 5.10 - 5.01 (m, 1H), 4.86 - 4.78 (m, 1H), 4.23 - 4.09 (m, 2H), 3.83 - 3.75 (m, 2H), 3.41 - 3.36 (m, 1H), 3.35 - 3.28 (m, 1H), 2.61 (s, 3H), 1.49 (s, 9H), 1.17 (t, *J =* 7.2 Hz, 3H).

### Step 5.

Synthesis of ethyl 2-(2-aminoacetamido)-3-(2-chloro-4-methyl-6-(trifluoromethyl)pyridin-3-yl)propanoate hydrochloride (**35e**).

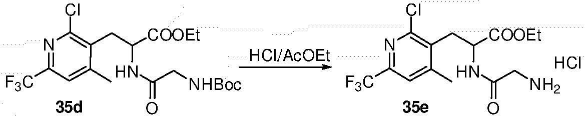

The title compound (**35e**) was obtained from **35d** (730 mg; 1.56 mmol) according to the General Procedure **IVa** in 99% yield (730 mg; 1.56 mmol).
ESI-MS m/z for C₁₄H₁₈ClF₃N₃O₃ found 367.9/369.9 [M+H]⁺; Rₜ = 0.81 min

### Step 6.

Synthesis of 3-((2-chloro-4-methyl-6-(trifluoromethyl)pyridin-3-yl)methyl)piperazine-2,5-dione (**35f**).

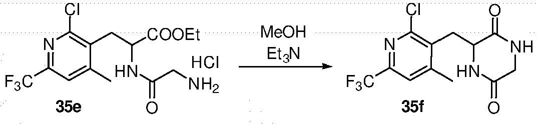

To a solution of **35e** (730 mg; 1.56 mmol) in MeOH (20 mL) Et₃N (0.91 mL; 6.53 mmol) was added and then the reaction mixture was stirred at room temperature overnight. The reaction progress was monitored by LC-MS. When analysis indicated completion of the reaction, the solvent was removed *in vacuo* and the residue was suspended in 5% NaHCO₃ and extracted with DCM (+ 10% of MeOH). The combined organic solutions were dried over anhydrous MgSO₄, filtered and concentrated *in vacuo* and the crude product was purified by preparative reversed-phase column chromatography (C-18, water/MeCN, 100:0 to 0:100, 30 min, 20 mL/min). Compound **35f** was obtained as a white solid in 90% yield (0.45 g; 1.40 mmol). ESI-MS m/z for C₁₂H₁₂ClF₃N₃O₂ found 321.9/323.9 [M+H]⁺; Rt = 0.83 min

### Step 7.

Synthesis of 2-((2-chloro-4-methyl-6-(trifluoromethyl)pyridin-3-yl)methyl)piperazine (**35g**).

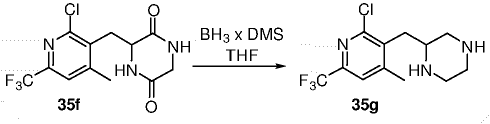

The title compound (**35g**) was obtained as a light yellow oil from **35f** (430 mg; 1.33 mmol) according to the General Procedure **VII** in 67% yield (260 mg; 0.89 mmol). ESI-MS m/z for C₁₂H₁₆ClF₃N₃ found 293.9/295.8 [M+H]⁺; Rt = 0.30 min

### Step 8.

Synthesis of di-*tert*-butyl 2-((2-chloro-4-methyl-6-(trifluoromethyl)pyridin-3-yl)methyl)piperazine-1,4-dicarboxylate (**35h**).

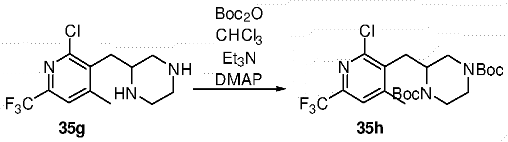

The title compound (**35h**) was obtained from **35g** (260 mg; 0.89 mmol) according to the General Procedure **VIII** in 22% yield (100 mg; 0.20 mmol).
ESI-MS m/z for C₁₄H₁₆ClF₃N₃O₄ found 382.0/384.0 [M+H-*t*Bu-*t*Bu]⁺; Rₜ = 1.97 min

### Step 9.

Synthesis of di-*tert*-butyl 2-((2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)methyl)piperazine-1,4-dicarboxylate (**35i**).

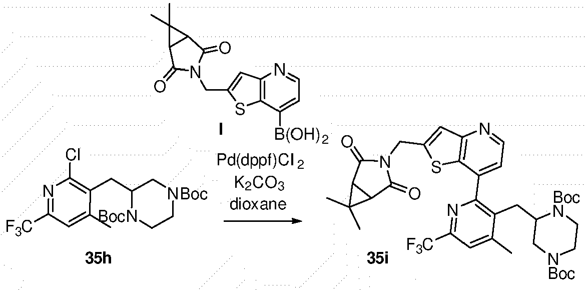

The title compound (**35i**) was obtained from **35h** (50 mg; 0.10 mmol) and from **I** (34 mg; 0.10 mmol) according to the General Procedure **Va** and after standard work-up all the crude product mixture was taken to the next step.
ESI-MS m/z for C₃₇H₄₅F₃N₅O₆S found 744.1 [M+H]⁺; Rt = 1.84 min

### Step 10.

Synthesis of 6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione hydrochloride (**35**).

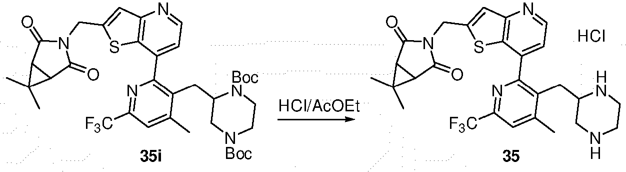

The title compound (**35**) was obtained as a racemate as a hydrochloride salt from **35i** (the crude reaction mixture) according to the General Procedure **IVa** in 66% yield (per two steps)(38 mg; 0.066 mmol). The crude product was purified by preparative reversed-phase column chromatography (C-18, water + 0.3‰ HCl (36%)/MeCN, 90:10 to 90:10, 30 min, 20 mL/min).
ESI-MS m/z for C₂₇H₂₉F₃N₅O₂S found 544.3 [M+H]⁺; Rt = 0.93 min; ¹H NMR (700 MHz, DMSO-*d*₆ + D₂O, 348 K) δ 8.84 - 8.80 (m, 1H), 7.96 - 7.92 (m, 1H), 7.58 - 7.55 (m, 1H), 7.55 - 7.52 (m, 1H), 4.85 - 4.72 (m, 2H), 3.64 - 3.57 (m, 1H), 3.39 - 3.35 (m, 2H), 3.29 - 3.24 (m, 1H), 3.19 - 3.13 (m, 1H), 3.12 - 3.07 (m, 1H), 3.06 - 3.01 (m, 2H), 2.76 - 2.69 (m, 1H), 2.64 (s, 3H), 2.53 - 2.52 (m, 2H), 1.19 (s, 3H), 1.05 (s, 3H); ¹⁹F NMR (659 MHz, DMSO-*d*₆) δ -66.36 (s).

### Example 37.

Synthesis of 3-((7-(3-((3,6-dioxopiperazin-2-yl)methyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**37**).

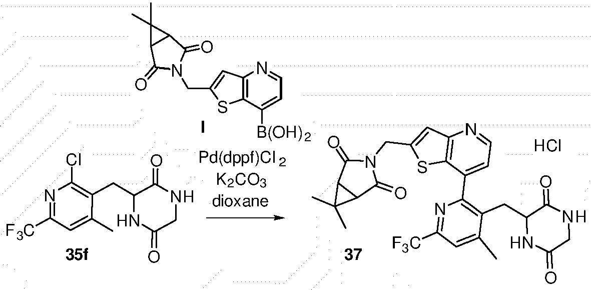

The title compound (**37**) was obtained as a hydrochloride salt from **35f** (30 mg; 0.09 mmol) and from **I** (33 mg; 0.1 mmol) according to the General Procedure **Va** in 89% yield (48 mg; 0.08 mmol). The crude product was purified by flash column chromatography on silica (DCM/MeOH, 100:0 to 70:30, *v*/*v,* 30 minutes) and then by preparative reversed-phase column chromatography (C-18, water + 0.5‰ HCl/MeCN, 90:10 to 10:90, 30 min, 20 mL/min). ESI-MS m/z for C₂₇H₂₅F₃N₅O₄S found 572.2 [M+H]⁺; Rt = 1.15 min; ¹H NMR (700 MHz, DMSO-*d*₆ + D₂O, 348 K) δ 8.75 - 8.72 (m, 1H), 7.84 (s, 1H), 7.53 - 7.51 (m, 1H), 7.47 - 7.43 (m, 1H), 4.80 - 4.72 (m, 2H), 3.68 - 3.60 (m, 1H), 3.48 - 3.42 (m, 1H), 3.34 - 3.27 (m, 1H), 3.16 - 3.09 (m, 1H), 3.09 - 3.01 (m, 1H), 2.55 (s, 3H), 2.50 (s, 2H), 1.16 (s, 3H), 0.99 (s, 3H); ¹⁹F NMR (659 MHz, DMSO-*d*₆) δ -66.29 (s).

### Example 38.

Synthesis of 6,6-dimethyl-3-((7-(4-methyl-3-((3-oxomorpholino)methyl)-6-(trifluoromethyl)pyridin-2-yl)thieno[3,2-*b*]pyridin-2-yl)methyl)-3-azabicyclo[3.1.0]hexane-2,4-dione hydrochloride (**38**).

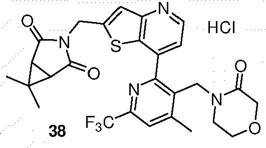

### Step 1.

Synthesis of 4-((2-chloro-4-methyl-6-(trifluoromethyl)pyridin-3-yl)methyl)morpholin-3-one (**38a**).

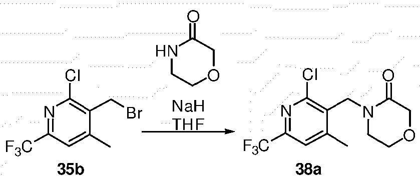

The solution of **35b** (70 mg; 0.24 mmol) and morpholin-3-one (27 mg; 0.27 mmol) in THF (2 mL) was cooled to 0 °C and then to this mixture NaH (50%; 15 mg; 0.32 mmol) was added in one portion and then the cooling bath was removed and the mixture was stirred at room temperature for 1 hour. The reaction progress was monitored by LC-MS. When analysis indicated completion of the reaction, the mixture was diluted with AcOEt and washed carefully with water. The organic layer was dried over anhydrous MgSO₄, filtered and concentrated *in vacuo* and the crude product was used to the next step without additional purification. Compound **38a** was obtained as a light yellow oil in 99% yield (74 mg; 0.24 mmol).
ESI-MS m/z for C₁₂H₁₃ClF₃N₄₂O₂ found 309.0/311.0 [M+H]⁺; Rt = 1.19 min

### Step 2.

Synthesis of 6,6-dimethyl-3-((7-(4-methyl-3-((3-oxomorpholino)methyl)-6-(trifluoromethyl)pyridin-2-yl)thieno[3,2-*b*]pyridin-2-yl)methyl)-3-azabicyclo[3.1.0]hexane-2,4-dione hydrochloride (**38**).

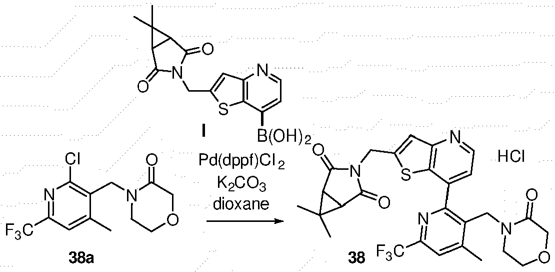

The title compound (**38**) was obtained as a hydrochloride salt from **38a** (74 mg; 0.24 mmol) and from **I** (79 mg; 0.24 mmol) according to the General Procedure **Va** in 83% yield (121 mg; 0.20 mmol). The crude product was purified by flash column chromatography on silica (DCM/MeOH, 100:0 to 70:30, *v*/*v,* 30 minutes) and then by preparative reversed-phase column chromatography (C-18, water + 0.5‰ HCl/MeCN, 99:1 to 40:60, 30 min, 20 mL/min). ESI-MS m/z for C₂₇H₂₆F₃N₄O₄S found 559.4 [M+H]⁺; Rt = 1.29 min; ¹H NMR (700 MHz, DMSO-*d*₆ + D₂O, 348 K) δ 8.74 - 8.69 (m, 1H), 7.88 (s, 1H), 7.50 (s, 1H), 7.42 - 7.36 (m, 1H), 4.80 - 4.75 (m, 2H), 4.63 (s, 2H), 3.67 (s, 2H), 3.41 - 3.40 (m, 2H), 2.76 - 2.69 (m, 2H), 2.54 (s, 3H), 1.17 (s, 3H), 1.03 (s, 3H); ¹⁹F NMR (235 MHz, DMSO-*d*₆) δ -66.55 - -66.90 (m).

### Example 39.

Synthesis of 6,6-dimethyl-3-((7-(4-methyl-3-((2-oxopyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**39**).

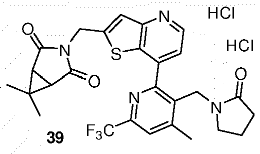

The title compound (**39**) was obtained as a dihydrochloride salt in 27% overall yield in a similar way to Example 38 with the exception that, in the first step of the synthesis pyrrolidin-2-one was used instead of morpholin-3-one and in the last step of the synthesis the crude product was purified by preparative reversed-phase column chromatography (C-18, water + 0.5‰ HCl (6 M)/MeCN, 98:2 to 30:70, 30 min, 20 mL/min).
ESI-MS m/z for C₂₇H₂₆F₃N₄O₃S found 543.2 [M+H]⁺; Rt = 1.33 min; ¹H NMR (700 MHz, DMSO-*d*₆ + D₂O, 348 K) δ 8.77 - 8.71 (m, 1H), 7.89 (s, 1H), 7.56 - 7.52 (m, 1H), 7.48 - 7.41 (m, 1H), 4.81 - 4.75 (m, 2H), 4.44 (s, 2H), 2.78 - 2.70 (m, 2H), 2.54 - 2.52 (m, 2H), 2.52 (s, 3H), 1.93 - 1.84 (m, 2H), 1.62 - 1.52 (m, 2H), 1.17 (s, 3H), 1.02 (s, 3H); ¹⁹F NMR (235 MHz, DMSO-*d*₆) δ -61.37 - -75.73 (m).

### Example 40.

Synthesis of 6,6-dimethyl-3-((7-(4-methyl-3-((2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**40**).

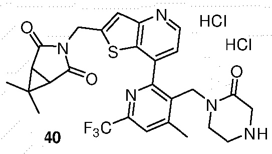

### Step 1.

Synthesis of *tert*-butyl 4-((2-chloro-4-methyl-6-(trifluoromethyl)pyridin-3-yl)methyl)-3-oxopiperazine-1-carboxylate (**40a**).

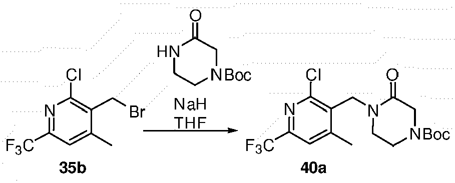

The solution of **35b** (50 mg; 0.17 mmol) and *tert*-butyl 3-oxopiperazine-1-carboxylate (38 mg; 0.19 mmol) in THF (2 mL) was cooled to 0 °C and then to this mixture NaH (50%; 10 mg; 0.22 mmol) was added in one portion and then the cooling bath was removed and the mixture was stirred at room temperature for 30 minutes. The reaction progress was monitored by LC-MS. When analysis indicated completion of the reaction, to this mixture water was added and the product was extracted with AcOEt (2 x). The combined organic solutions was dried over anhydrous MgSO₄, filtered and concentrated *in vacuo* and the crude product was purified by flash column chromatography on silica (hexane/AcOEt, 100:0 to 50:50, *v*/*v,* 30 minutes). Compound **40a** was obtained in 82% yield (55 mg; 0.14 mmol).
ESI-MS m/z for C₁₇H₂₂ClF₃N₃O₃ found 407.5/409.5 [M+H]⁺; Rt = 1.47 min

### Step 2.

Synthesis of *tert*-butyl 4-((2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)methyl)-3-oxopiperazine-1-carboxylate (**40b**).

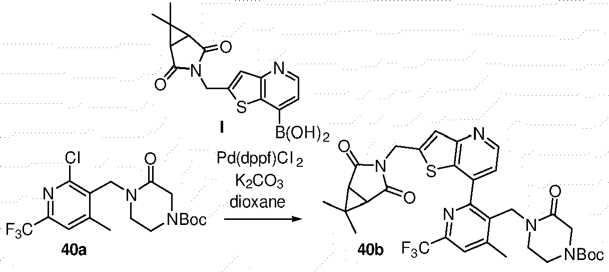

The title compound (**40b**) was obtained from **40a** (55 mg; 0.14 mmol) and from **I** (53 mg; 0.16 mmol) according to the General Procedure **Va** and after standard work-up all the crude product mixture was taken to the next step.
ESI-MS m/z for C₃₂H₃₅F₃N₅O₅S found 658.1 [M+H]⁺; Rt = 1.55 min

### Step 3.

Synthesis of 6,6-dimethyl-3-((7-(4-methyl-3-((2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**40**).

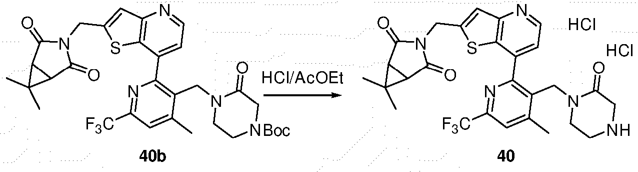

The title compound (**40**) was obtained as a dihydrochloride salt from **40b** (the crude reaction mixture) according to the General Procedure **IVa** in 57% yield (per two steps)(52 mg; 0.08 mmol). The crude product was purified by flash column chromatography on silica (DCM/MeOH, 100:0 to 70:30, *v*/*v,* 30 minutes) and then by preparative reversed-phase column chromatography (C-18, water + 0.5‰ HCl (36%)/MeCN, 98:2 to 30:70, 30 min, 20 mL/min). ESI-MS m/z for C₂₇H₂₇F₃N₅O₃S found 558.2 [M+H]⁺; Rt = 0.97 min; ¹H NMR (700 MHz, DMSO-*d*₆ + D₂O, 348 K) δ 8.77 - 8.74 (m, 1H), 7.91 (s, 1H), 7.55 - 7.51 (m, 1H), 7.48 - 7.44 (m, 1H), 4.84 - 4.74 (m, 2H), 4.64 (s, 2H), 3.54 (s, 2H), 3.16 - 3.07 (m, 2H), 3.05 - 2.95 (m, 2H), 2.53 (s, 3H), 2.52 (s, 2H), 1.18 (s, 3H), 1.03 (s, 3H); ¹⁹F NMR (235 MHz, DMSO-*d*₆) δ -66.78 - - 67.11 (m).

### Example 41.

Synthesis of 6,6-dimethyl-3-((7-(4-methyl-3-((4-methyl-2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**41**).

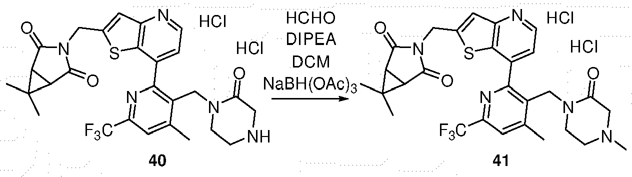

To the solution of **40** (35 mg; 0.06 mmol) in DCM (1 mL) DIPEA (0.03 mL; 0.19 mmol) and formalin (37% aqueous solution; 0.01 mL; 0.31 mmol) were added and this mixture was stirred at room temperature for 30 minutes. Then to this mixture NaBH(OAc)₃ (21 mg; 0.09 mmol) was added and whole was stirred at room temperature. The reaction progress was monitored by LC-MS. When analysis indicated completion of the reaction, the solvent was removed *in vacuo* and the residue was purified by preparative reversed-phase column chromatography (C-18, water + 0.5‰ HCl (36%)/MeCN, 98:2 to 30:70, 30 min, 20 mL/min). ESI-MS m/z for C₂₈H₂₉F₃N₅O₃S found 572.1 [M+H]⁺; Rt = 0.99 min; ¹H NMR (700 MHz, DMSO-*d*₆ + D₂O, 348 K) δ 8.79 - 8.75 (m, 1H), 7.92 - 7.87 (m, 1H), 7.55 - 7.51 (m, 1H), 7.50 - 7.47 (m, 1H), 4.84 - 4.74 (m, 2H), 4.64 (s, 2H), 3.68 (s, 2H), 3.24 (s, 2H), 3.11 - 3.02 (m, 2H), 2.77 (s, 3H), 2.54 - 2.53 (m, 3H), 2.52 (s, 2H), 1.18 (s, 3H), 1.03 (s, 3H); ¹⁹F NMR (235 MHz, DMSO-*d*₆) δ -66.78 - -67.06 (m).

### Example 42.*(this compound is not a part of the present invention)

Synthesis of 6-cyano-2-(2-((6,6-dimethyl-2,4-dioxo-3-azabicyclo[3.1.0]hexan-3-yl)methyl)thieno[3,2-*b*]pyridin-7-yl)-*N*-(2-(dimethylamino)ethyl)-*N*,4-dimethylnicotinamide (**42**).

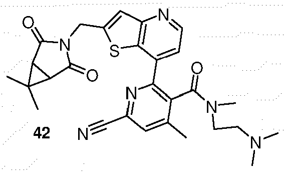

### Step 1.

Synthesis of *tert*-butyl 2-chloro-4-methylnicotinate (**42a**).

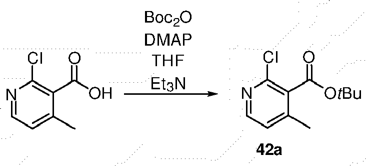

To the suspension of 2-chloro-4-methylnicotinic acid (4.14 g; 24.21 mmol) in THF (160 mL) Boc₂O (7.93 g; 36.31 mmol), DMAP (1.48 g; 12.10 mmol) and Et₃N (5 mL) were added and then the reaction mixture was stirred at room temperature overnight. Then DCM (30 mL) and another portion of Boc₂O (4 g; 15.00 mmol) were added and the reaction mixture was stirred at room temperature overnight. The reaction progress was monitored by LC-MS. When analysis indicated completion of the reaction, the solvent was evaporated *in vacuo* and the residue was purified by flash column chromatography on silica (hexane/AcOEt, 100:0 to 50:50, *v*/*v,* 25 minutes). Compound **42a** was obtained in 76% yield (4.2 g; 18.50 mmol).
ESI-MS m/z for C₁₁H₁₅ClNO₂ found 228.0/230.0 [M+H]⁺; Rt = 1.44 min

### Step 2.

Synthesis of 3-(*tert*-butoxycarbonyl)-2-chloro-4-methylpyridine 1-oxide (**42b**).

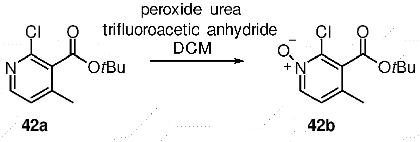

To the solution of **42a** (3 g; 13.17 mmol) and peroxide urea (6.2 g; 65.84 mmol) in DCM (100 mL) trifluoroacetic anhydride (7.3 mL; 52.68 mmol) was added dropwise and this mixture was stirred at room temperature for 1 hour. The reaction progress was monitored by LC-MS. When analysis indicated completion of the reaction, to this mixture a mixture of 1 M K₂CO₃/5% NaHCO_{3aq} (1:1, *v*/*v*) was carefully added to pH 8 and the product was extracted with DCM (3 x). The combined organic solutions were dried over anhydrous MgSO₄, filtered and concentrated *in vacuo* and the crude product was used to the next step without additional purification. Compound **42b** was obtained in 94% yield (3 g; 12.34 mmol).
ESI-MS m/z for C₁₁H₁₅ClNO₃ found 244.0/246.0 [M+H]⁺; Rₜ = 1.01 min

### Step 3.

Synthesis of *tert*-butyl 2-chloro-6-cyano-4-methylnicotinate (**42c**).

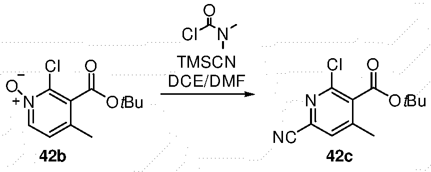

To the solution of **42b** (3 g; 12.34 mmol) and dimethylcarbamic chloride (2.26 mL; 24.62 mmol) in DCE/DMF (36 mL/12 mL; 3:1, *v*/*v*) trimethylsilyl cyanate (4.95 mL; 36.93 mmol) was added dropwise and this mixture was stirred at 50 °C for 5 hours and then at room temperature for 3 days. The reaction progress was monitored by LC-MS. When analysis indicated completion of the reaction, the mixture was diluted with DCM and washed with 5% NaHCO_{3aq}. The organic layer was dried over anhydrous MgSO₄, filtered and concentrated *in vacuo* and the crude product was purified by flash column chromatography on silica (hexane/AcOEt, 100:0 to 80:20, *v*/*v,* 30 minutes). Compound **42c** was obtained in 61% yield (1.9 g; 7.54 mmol).
ESI-MS m/z for C₁₄H₁₇ClN₃O₂ found 294.0/296.0 [M+H+MeCN]⁺; Rt = 1.60 min

### Step 4.

Synthesis of *tert*-butyl 6-cyano-2-(2-((6,6-dimethyl-2,4-dioxo-3-azabicyclo[3.1.0]hexan-3-yl)methyl)thieno[3,2-*b*]pyridin-7-yl)-4-methylnicotinate (**42d**).

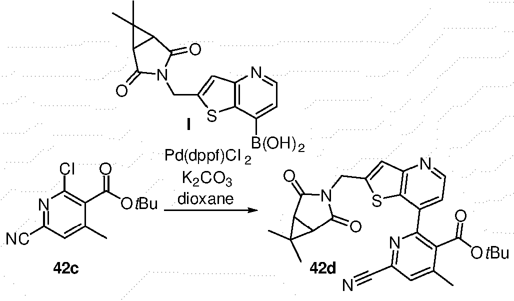

The title compound (**42d**) was obtained from **42c** (300 mg; 1.98 mmol) and from boronic acid **I** (718 mg; 2.17 mmol) according to the General Procedure **Va** in 51% yield (500 mg; 1.00 mmol).
ESI-MS m/z for C₂₇H₂₇N₄O₄S found 503.4 [M+H]⁺; Rt = 1.60 min

### Step 5.

Synthesis of 6-cyano-2-(2-((6,6-dimethyl-2,4-dioxo-3-azabicyclo[3.1.0]hexan-3-yl)methyl)thieno[3,2-*b*]pyridin-7-yl)-4-methylnicotinic acid (**42e**).

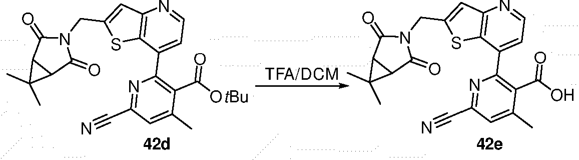

To the solution of **42d** (0.6 g; 1.19 mmol) in DCM (12 mL) TFA (9 mL; 121 mmol) was added and this mixture was stirred at 45 °C for 4 hours. Then the solvent was evaporated *in vacuo* and the residue was purified by flash column chromatography on silica (DCM/MeOH, 100:0 to 50:50, *v*/*v,* 30 minutes). Compound **42e** was obtained in 91% yield (480 mg; 1.08 mmol).
ESI-MS m/z for C₂₃H₁₉N₄O₄S found 447.2 [M+H]⁺; Rt = 1.10 min

### Step 6.

Synthesis of 6-cyano-2-(2-((6,6-dimethyl-2,4-dioxo-3-azabicyclo[3.1.0]hexan-3-yl)methyl)thieno[3,2-*b*]pyridin-7-yl)-*N*-(2-(dimethylamino)ethyl)-*N*,4-dimethylnicotinamide (**42**).

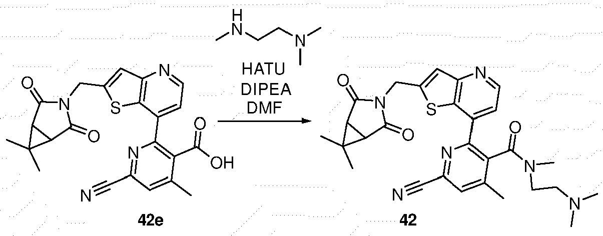

The title compound (**42**) was obtained from **42e** (55 mg; 0.12 mmol) and from *N*¹,*N*¹,*N*²-trimethylethane-1,2-diamine (0.02 mL; 0.15 mmol) according to the General Procedure **I** in 19% yield (12 mg; 0.023 mmol). The crude product was purified by preparative reversed-phase column chromatography (C-18, water/MeCN, 99:1 to 10:90, 30 min, 20 mL/min).
ESI-MS m/z for C₂₈H₃₀N₆O₃S found 531.3 [M+H]⁺; Rt = 0.88 min; ¹H NMR (700 MHz, D₂O) δ 8.71 - 8.66 (m, 1H), 8.05 - 8.02 (m, 1H), 7.58 - 7.54 (m, 1H), 7.40 - 7.36 (m, 1H), 4.91 - 4.81 (m, 2H), 3.76 - 3.70 (m, 1H), 3.58 - 3.50 (m, 1H), 2.99 - 2.92 (m, 1H), 2.92 - 2.85 (m, 1H), 2.78 (s, 6H), 2.58 (s, 2H), 2.55 (s, 3H), 2.41 (s, 3H), 1.17 (s, 3H), 0.95 (s, 3H).

### Example 43.

Synthesis of 6-(2-((6,6-dimethyl-2,4-dioxo-3-azabicyclo[3.1.0]hexan-3-yl)methyl)thieno[3,2-*b*]pyridin-7-yl)-5-(3-(dimethylamino)azetidine-1-carbonyl)-4-methylpicolinonitrile (**43**).

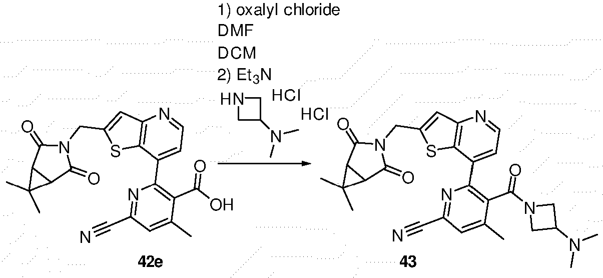

To the suspension of **42e** (40 mg; 0.090 mmol) in DCM (1 mL) DMF (5 drops) was added and then an oxalyl chloride (0.008 mL; 0.098 mmol) was added dropwise at room temperature under an argon atmosphere. Then this mixture was stirred at room temperature for 1.5 hour. The reaction progress was monitored by LC-MS. Next to this mixture Et₃N (0.062 mL; 0.358 mmol) and *N*,*N*-dimethylazetidin-3-amine dihydrochloride (19 mg; 0.107 mmol) were added and whole was stirred at room temperature for 2 hours. When analysis indicated completion of the reaction, the mixture was concentrated *in vacuo* and the crude product was purified by preparative reversed-phase column chromatography (column: Cosmosil Cholester 20 x 250 mm, water/MeCN, 99:1 to 20:80, 30 min, 20 mL/min). The final compound (**43**) was obtained in 20% yield (10 mg; 0.018 mmol).
ESI-MS m/z for C₂₈H₂₉N₆O₃S found 529.3 [M+H]⁺; Rt = 0.87 min; ¹H NMR (700 MHz, D₂O) δ 9.08 - 7.58 (m, 4H), 5.04 - 4.87 (m, 2H), 4.69 - 3.38 (m, 5H), 2.88 - 2.42 (m, 9H), 1.30 - 0.92 (m, 6H).

### Example 44.

Synthesis of 6-(2-((6,6-dimethyl-2,4-dioxo-3-azabicyclo[3.1.0]hexan-3-yl)methyl)thieno[3,2-*b*]pyridin-7-yl)-5-((*S*)-3-(dimethylamino)pyrrolidine-1-carbonyl)-4-methylpicolinonitrile hydrochloride (**44**).

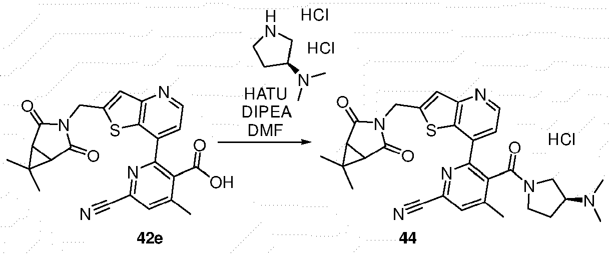

The title compound (**44**) was obtained as a hydrochloride salt from **42e** (55 mg; 0.12 mmol) and from (*S*)-*N*,*N*-dimethylpyrrolidin-3-amine dihydrochloride (28 mg; 0.15 mmol) according to the General Procedure **I** in 25% yield (16 mg; 0.03 mmol). The crude product was purified by preparative reversed-phase column chromatography (C-18, water + 0.5‰ HCl (36%)//MeCN, 98:2 to 40:60, 30 min, 20 mL/min).
ESI-MS m/z for C₂₉H₃₁N₆O₃S found 543.4 [M+H]⁺; Rt = 0.89 min; ¹H NMR (700 MHz, DMSO-*d*₆ + D₂O, 348 K) δ 8.78 - 8.72 (m, 1H), 8.15 - 8.08 (m, 1H), 7.53 - 7.37 (m, 2H), 4.87 - 4.76 (m, 2H), 4.07 - 3.41 (m, 3H), 3.24 - 3.07 (m, 1H), 2.86 - 2.73 (m, 2H), 2.70 - 2.52 (m, 6H), 2.49 - 2.39 (m, 4H), 2.38 - 1.65 (m, 2H), 1.22 - 1.17 (m, 3H), 1.12 - 1.03 (m, 3H).

### Example 45.

Synthesis of 6,6-dimethyl-3-((7-(4-methyl-3-(piperazine-1-carbonyl)pyridin-2-yl)thieno[3,2-*b*]pyridin-2-yl)methyl)-3-azabicyclo[3.1.0]hexane-2,4-dione hydrochloride (**45**).

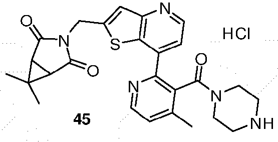

### Step 1.

Synthesis of *tert*-butyl 4-(2-chloro-4-methylnicotinoyl)piperazine-1-carboxylate (**45a**).

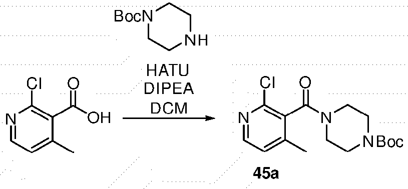

The title compound (**45a**) was obtained as a white solid from 2-chloro-4-methylnicotinic acid (200 mg; 1.17 mmol) and from *tert*-butyl piperazine-1-carboxylate (261 mg; 1.40 mmol) according to the General Procedure **I** in 88% yield (350 mg; 1.03 mmol). ESI-MS m/z for C₁₆H₂₃ClN₃O₃ found 340.1/342.1 [M+H]⁺; Rt = 1.19 min

### Step 2.

Synthesis of *tert*-butyl 4-(2-(2-((6,6-dimethyl-2,4-dioxo-3-azabicyclo[3.1.0]hexan-3-yl)methyl)thieno[3,2-*b*]pyridin-7-yl)-4-methylnicotinoyl)piperazine-1-carboxylate (**45b**).

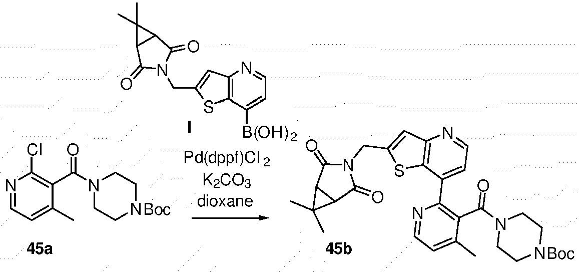

The title compound (**45b**) was obtained from **45a** (76 mg; 0.23 mmol) and from **I** (60 mg; 0.18 mmol) according to the General Procedure **Va** and after standard work-up all the crude product mixture was taken to the next step.
ESI-MS m/z for C₃₁H₃₆N₅O₅S found 590.6 [M+H]⁺; Rₜ = 1.39 min

### Step 3.

Synthesis of 6,6-dimethyl-3-((7-(4-methyl-3-(piperazine-1-carbonyl)pyridin-2-yl)thieno[3,2-*b*]pyridin-2-yl)methyl)-3-azabicyclo[3.1.0]hexane-2,4-dione hydrochloride (**45**).

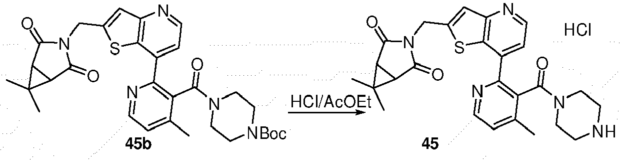

The title compound (**45**) was obtained as a hydrochloride salt from **45b** (the crude reaction mixture) according to the General Procedure **IVa** in 26% yield (per two steps) (32 mg; 0.06 mmol). The crude product was purified by preparative reversed-phase column chromatography (C-18, water/MeCN, 98:2 to 20:80, 30 min, 20 mL/min). The obtained product was redissolved in water and to this solution 2 M HCl (3 drops) was added and then obtained hydrochloride salt was lyophilized.
ESI-MS m/z for C₂₆H₂₈N₅O₃S found 490.4 [M+H]⁺; Rt = 0.82 min; ¹H NMR (700 MHz, DMSO-*d*₆ + D₂O, 348 K) δ 8.75 - 8.73 (m, 1H), 8.73 - 8.70 (m, 1H), 7.55 - 7.51 (m, 1H), 7.51 - 7.47 (m, 1H), 7.46 - 7.43 (m, 1H), 4.84 - 4.72 (m, 2H), 3.95 - 3.89 (m, 1H), 3.62 - 3.55 (m, 1H), 3.27 - 3.20 (m, 1H), 3.17 - 3.10 (m, 1H), 2.94 - 2.88 (m, 1H), 2.84 - 2.76 (m, 2H), 2.53 - 2.52 (m, 2H), 2.36 (s, 3H), 2.29 - 2.22 (m, 1H), 1.19 (s, 3H), 1.03 (s, 3H).

### Example 46.

Synthesis of 6,6-dimethyl-3-((7-(4-methyl-3-(piperazin-1-ylmethyl)pyridin-2-yl)thieno[3,2-*b*]pyridin-2-yl)methyl)-3-azabicyclo[3.1.0]hexane-2,4-dione hydrochloride (**46**).

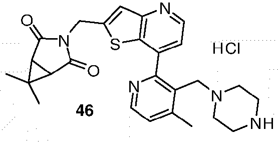

### Step 1.

Synthesis of *tert*-butyl 4-((2-chloro-4-methylpyridin-3-yl)methyl)piperazine-1-carboxylate (**46a**).

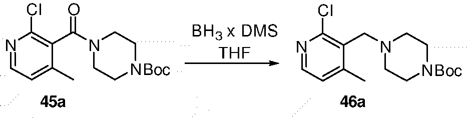

The title compound (**46a**) was obtained as a white solid from **45a** (274 mg; 0.80 mmol) according to the General Procedure **VII** in 14% yield (35 mg; 0.11 mmol).
ESI-MS m/z for C₁₆H₂₅ClN₃O₂ found 326.1/328.1 [M+H]⁺, Rt = 0.85 min

### Step 2.

Synthesis of *tert*-butyl 4-((2-(2-((6,6-dimethyl-2,4-dioxo-3-azabicyclo[3.1.0]hexan-3-yl)methyl)thieno[3,2-*b*]pyridin-7-yl)-4-methylpyridin-3-yl)methyl)piperazine-1-carboxylate (**46b**).

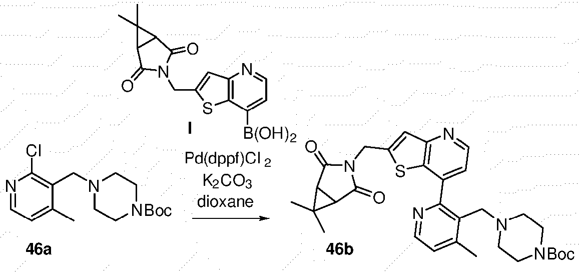

The title compound (**46b**) was obtained from **46a** (35 mg; 0.11 mmol) and from **I** (39 mg; 0.12 mmol) according to the General Procedure **Va** and after standard work-up all the crude product mixture was taken to the next step.
ESI-MS m/z for C₃₁H₃₈N₅O₄S found 576.6 [M+H]⁺, Rt = 1.32 min

### Step 3.

Synthesis of 6,6-dimethyl-3-((7-(4-methyl-3-(piperazin-1-ylmethyl)pyridin-2-yl)thieno[3,2-*b*]pyridin-2-yl)methyl)-3-azabicyclo[3.1.0]hexane-2,4-dione hydrochloride (**46**).

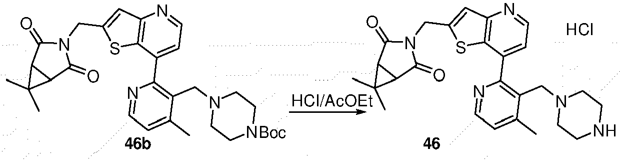

The title compound (**46**) was obtained as a hydrochloride salt from **46b** (the crude reaction mixture) according to the General Procedure **IVa** in 36% yield (per two steps) (23 mg; 0.04 mmol). The crude product was purified by preparative reversed-phase column chromatography (C-18, water + 0.5‰ HCl (36%)/MeCN, 99:1 to 30:70, 30 min, 20 mL/min). ESI-MS m/z for C₂₆H₃₀N₅O₂S found 476.5 [M+H]⁺; Rt= 0.73 min; ¹H NMR (700 MHz, DMSO-*d*₆ + D₂O, 348 K) δ 8.82 - 8.79 (m, 1H), 8.56 - 8.53 (m, 1H), 7.66 - 7.63 (m, 1H), 7.61 - 7.56 (m, 1H), 7.53 - 7.50 (m, 1H), 4.84 - 4.76 (m, 2H), 3.57 (s, 2H), 2.82 - 2.73 (m, 4H), 2.55 (s, 3H), 2.52 (s, 2H), 2.38 - 2.30 (m, 4H), 1.19 (s, 3H), 1.04 (s, 3H).

### Example 47.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-((*S*)-2-methylpiperazine-1-carbonyl)picolinonitrile hydrochloride (**47**).

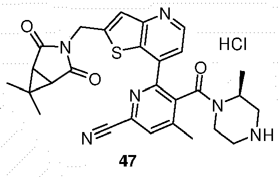

### Step 1.

Synthesis of *tert*-butyl (3*S*)-4-(6-cyano-2-(2-((6,6-dimethyl-2,4-dioxo-3-azabicyclo[3.1.0]hexan-3-yl)methyl)thieno[3,2-*b*]pyridin-7-yl)-4-methylnicotinoyl)-3-methylpiperazine-1-carboxylate (**47a**).

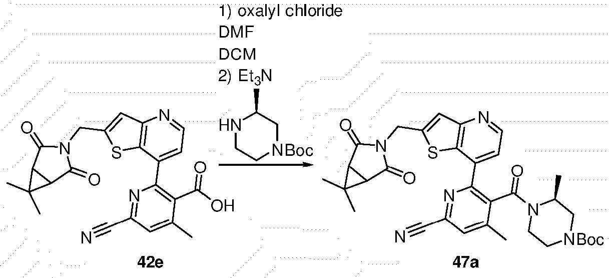

To the suspension of **42e** (50 mg; 0.11 mmol) in DCM (1 mL) DMF (4 drops) was added and then an oxalyl chloride (0.011 mL; 0.12 mmol) was added dropwise at room temperature under an argon atmosphere. Then this mixture was stirred at room temperature for 1 hour. The reaction progress was monitored by LC-MS. Next to this mixture Et₃N (0.05 mL; 0.28 mmol) and *tert*-butyl (S)-3-methylpiperazine-1-carboxylate (25 mg; 0.12 mmol) were added and whole was stirred at room temperature for 2 hours. When analysis indicated completion of the reaction, the mixture was concentrated *in vacuo* and all the crude product mixture was taken to the next step.
ESI-MS m/z for C₃₃H₃₇N₆O₅S found 629.3 [M+H]⁺; Rₜ = 1.39 min

### Step 2.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-((*S*)-2-methylpiperazine-1-carbonyl)picolinonitrile hydrochloride (**47**).

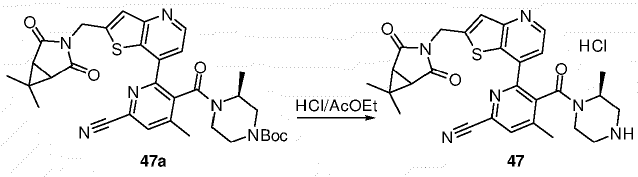

The title compound (**47**) was obtained as a hydrochloride salt from **47a** (the crude reaction mixture) according to the General Procedure **IVa** in 55% yield (per two steps) (36 mg; 0.06 mmol). The crude product was purified by preparative reversed-phase column chromatography (C-18, water + 0.3‰ HCl (36%)/MeCN, 98:2 to 30:70, 30 min, 20 mL/min). ESI-MS m/z for C₂₈H₂₉N₆O₃S found 529.3 [M+H]⁺; Rt = 0.93 min; ¹H NMR (700 MHz, DMSO-*d*₆ + D₂O, 348 K) δ 8.83 - 8.48 (m, 1H), 8.17 - 8.07 (m, 1H), 7.57 - 7.34 (m, 2H), 4.94 - 4.73 (m, 3H), 3.44 - 3.33 (m, 1H), 3.25 - 3.05 (m, 2H), 2.98 - 2.57 (m, 2H), 2.55 - 2.52 (m, 3H), 2.47 - 2.34 (m, 3H), 1.39 - 0.84 (m, 9H).

### Example 48.

Synthesis of 6,6-dimethyl-3-((7-(4-methyl-3-(morpholine-4-carbonyl)pyridin-2-yl)thieno[3,2-*b*]pyridin-2-yl)methyl)-3-azabicyclo[3.1.0]hexane-2,4-dione hydrochloride (**48**).

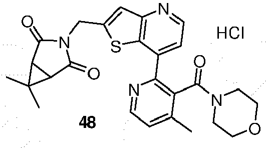

### Step 1.

Synthesis of (2-chloro-4-methylpyridin-3-yl)(morpholino)methanone (**48a**).

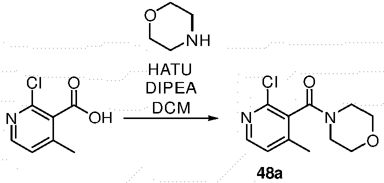

The title compound (**48a**) was obtained as a white solid from 2-chloro-4-methylnicotinic acid (200 mg; 1.17 mmol) and from morpholine (152 mg; 1.75 mmol) according to the General Procedure **I** in 59% yield (166 mg; 0.69 mmol).
ESI-MS m/z for C₁₁H₁₄ClN₂O₂ found 241.1/243.1 [M+H]⁺; Rt = 0.57 min

### Step 2.

Synthesis of 6,6-dimethyl-3-((7-(4-methyl-3-(morpholine-4-carbonyl)pyridin-2-yl)thieno[3,2-*b*]pyridin-2-yl)methyl)-3-azabicyclo[3.1.0]hexane-2,4-dione hydrochloride (**48**).

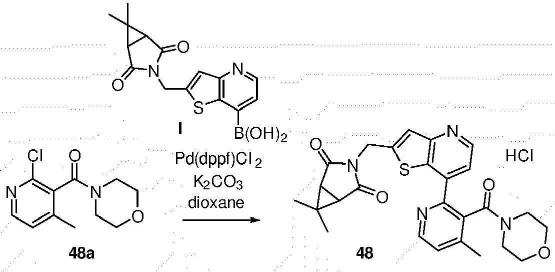

The title compound (**48**) was obtained from **48a** (55 mg; 0.23 mmol) and from **I** (60 mg; 0.18 mmol) according to the General Procedure **Va** in 17% yield (18 mg; 0.03 mmol). The crude product was purified by preparative reversed-phase column chromatography (C-18, water + 0.3‰ HCl (36%)/MeCN, 90:10 to 20:80, 30 min, 20 mL/min).
ESI-MS m/z for C₂₆H₂₇N₄O₄S found 491.2 [M+H]⁺; Rt = 1.07 min; ¹H NMR (700 MHz, DMSO-*d*₆ + D₂O, 348 K) δ 8.70 - 8.68 (m, 1H), 8.69 - 8.65 (m, 1H), 7.49 - 7.44 (m, 3H), 4.81 - 4.70 (m, 2H), 3.61 - 3.45 (m, 3H), 3.24 - 3.10 (m, 2H), 3.02 - 2.90 (m, 1H), 2.63 - 2.52 (m, 4H), 2.33 (s, 3H), 1.17 (s, 3H), 1.00 (s, 3H).

### Example 49.

Synthesis of 3-((7-(3-(3,3-difluoropyrrolidine-1-carbonyl)-4-methylpyridin-2-yl)thieno[3,2-*b*]pyridin-2-yl)methyl)-6,6-dimethyl-3-azabicyclo[3.1.0]hexane-2,4-dione hydrochloride (**49**).

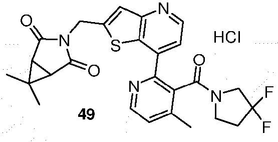

The title compound (**49**) was obtained as a hydrochloride salt in 27% overall yield in a similar way to Example 48 with the exception that, in the first step of the synthesis 3,3-difluoropyrrolidine hydrochloride was used instead of morpholine and in the last step of the synthesis the crude product was purified by preparative reversed-phase column chromatography (C-18, water + 0.3‰ HCl (36%)/MeCN, 90:10 to 20:80, 30 min, 20 mL/min).
ESI-MS m/z for C₂₆H₂₅F₂N₄O₃S found 511.2 [M+H]⁺; Rt = 1.23 min; ¹H NMR (700 MHz, DMSO-*d*₆ + D₂O, 348 K) δ 8.72 - 8.70 (m, 1H), 8.70 - 8.67 (m, 1H), 7.52 - 7.49 (m, 1H), 7.48 - 7.43 (m, 2H), 4.86 - 4.72 (m, 2H), 3.92 - 3.67 (m, 1H), 3.51 - 3.33 (m, 2H), 3.23 - 3.11 (m, 1H), 2.92 - 2.66 (m, 1H), 2.50 - 1.88 (m, 6H), 1.20 - 1.13 (m, 3H), 1.02 - 0.91 (m, 3H).

### Example 50.

Synthesis of 3-((7-(3-(3,3-difluoroazetidine-1-carbonyl)-4-methylpyridin-2-yl)thieno[3,2-*b*]pyridin-2-yl)methyl)-6,6-dimethyl-3-azabicyclo[3.1.0]hexane-2,4-dione hydrochloride (**50**).

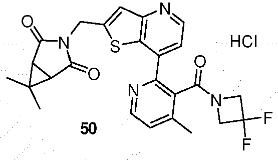

The title compound (**50**) was obtained as a hydrochloride salt in 2% overall yield in a similar way to Example 48 with the exception that, in the first step of the synthesis 3,3-difluoroazetidine hydrochloride was used instead of morpholine and in the last step of the synthesis the crude product was purified by preparative reversed-phase column chromatography (C-18, water + 0.3‰ HCl (36%)/MeCN, 90:10 to 20:80, 30 min, 20 mL/min).
ESI-MS m/z for C₂₆H₂₃F₂N₄O₃S found 497.2 [M+H]⁺; Rₜ = 1.93 min; ¹H NMR (700 MHz, DMSO-*d*₆ + D₂O) δ 8.76 - 8.72 (m, 2H), 7.57 - 7.54 (m, 1H), 7.52 - 7.49 (m, 1H), 7.49 - 7.45 (m, 1H), 4.84 - 4.71 (m, 2H), 4.55 - 4.42 (m, 1H), 4.26 - 4.15 (m, 2H), 3.38 - 3.28 (m, 1H), 2.55 (s, 2H), 2.38 (s, 3H), 1.14 (s, 3H), 0.91 (s, 3H); ¹⁹F NMR (235 MHz, DMSO-*d*₆) δ -100.99 - -101.33 (m).

### Example 51.

Synthesis of 5-(3,3-difluoroazetidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile hydrochloride (**51**).

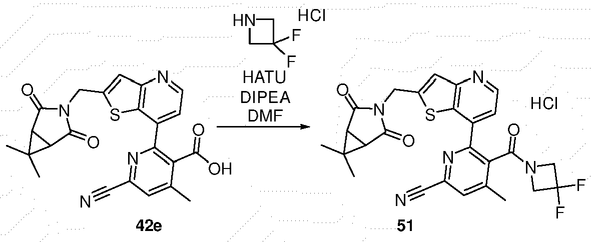

The title compound (**51**) was obtained as a hydrochloride salt from **42e** (50 mg; 0.11 mmol) and from 3,3-difluoroazetidine hydrochloride (20 mg; 0.13 mmol) according to the General Procedure **I** in 27% yield (16 mg; 0.03 mmol). The crude product was purified twice by preparative reversed-phase column chromatography (first: C-18, water + 0.3‰ HCl (36%)/MeCN, 98:2 to 30:70, 30 min, 20 mL/min; second: C-18, water + 0.3‰ HCl (36%)/MeCN, 90:10 to 10:90, 30 min, 20 mL/min).
ESI-MS m/z for C₂₆H₂₂F₂N₅O₃S found 522.3 [M+H]⁺; Rₜ = 1.35 min; ¹H NMR (700 MHz, DMSO-*d*₆ + D₂O) δ 8.78 - 8.75 (m, 1H), 8.19 - 8.15 (m, 1H), 7.55 - 7.51 (m, 1H), 7.47 - 7.43 (m, 1H), 4.89 - 4.75 (m, 2H), 4.54 - 4.43 (m, 1H), 4.38 - 4.19 (m, 2H), 3.44 - 3.33 (m, 1H), 2.58 - 2.54 (m, 2H), 2.45 (s, 3H), 1.14 (s, 3H), 0.93 (s, 3H).

### Example 52.

Synthesis of 6-(2-((6,6-dimethyl-2,4-dioxo-3-azabicyclo[3.1.0]hexan-3-yl)methyl)thieno[3,2-*b*]pyridin-7-yl)-5-((*S*)-2,4-dimethylpiperazine-1-carbonyl)-4-methylpicolinonitrile dihydrochloride (**52**).

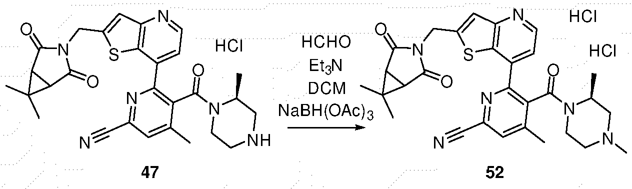

To the solution of **47** (100 mg; 0.19 mmol) in DCM (5 mL) Et₃N (0.13 mL; 0.76 mmol) and formalin (36% aqueous solution; 0.3 mL) were added and this mixture was stirred at room temperature for 5 minutes. Then to this mixture NaBH(OAc)₃ (62 mg; 0.287 mmol) was added and whole was stirred at room temperature for 2 hours. The reaction progress was monitored by LC-MS. Then another portion of formalin (36% aqueous solution; 0.1 mL) and NaBH(OAc)₃ (62 mg; 0.287 mmol) was added and stirred at room temperature for 1 hour. When analysis indicated completion of the reaction, the mixture was diluted with DCM and washed with 5% NaHCO₃. The organic layer was dried over anhydrous MgSO₄, filtered and concentrated *in vacuo* and the residue was purified by preparative reversed-phase column chromatography (C-18, water + 0.3‰ HCl (36%)/MeCN, 98:2 to 30:70, 30 min, 20 mL/min). The title compound (**52**) was obtained as a dihydrochloride salt as a white solid in 47% yield (56 mg; 0.09 mmol).
ESI-MS m/z for C₂₉H₃₁N₆O₃S found 543.6 [M+H]⁺; Rt = 0.94 min; ¹H NMR (700 MHz, DMSO-*d*₆ + D₂O, 348 K) δ 8.85 - 8.69 (m, 1H), 8.16 - 8.08 (m, 1H), 7.58 - 7.31 (m, 2H), 4.96 - 4.70 (m, 3H), 3.61 - 3.38 (m, 1H), 3.23 - 2.83 (m, 3H), 2.77 - 2.60 (m, 4H), 2.54 - 2.53 (m, 2H), 2.47 - 2.34 (m, 4H), 1.39 - 0.17 (m, 9H).

### Example 53.

Synthesis of 6-(2-((6,6-dimethyl-2,4-dioxo-3-azabicyclo[3.1.0]hexan-3-yl)methyl)thieno[3,2-*b*]pyridin-7-yl)-5-(3,3-dimethylpiperazine-1-carbonyl)-4-methylpicolinonitrile hydrochloride (**53**).

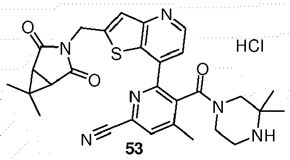

### Step 1.

Synthesis of *tert*-butyl 4-(6-cyano-2-(2-((6,6-dimethyl-2,4-dioxo-3-azabicyclo[3.1.0]hexan-3-yl)methyl)thieno[3,2-*b*]pyridin-7-yl)-4-methylnicotinoyl)-2,2-dimethylpiperazine-1-carboxylate (**53a**).

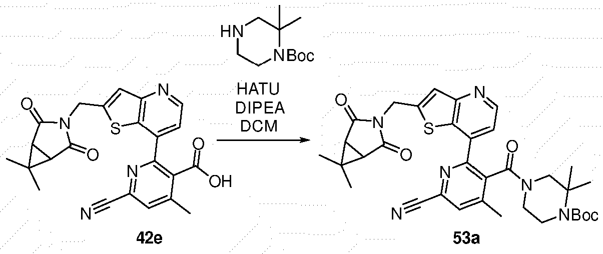

The title compound (**53a**) was obtained as a white solid from **42e** (50 mg; 0.11 mmol) and from *tert*-butyl 2,2-dimethylpiperazine-1-carboxylate (29 mg; 0.13 mmol) according to the General Procedure **I** and after standard work-up all the crude product mixture was taken to the next step.
ESI-MS m/z for C₃₄H₃₉N₆O₅S found 643.6 [M+H]⁺; Rₜ = 1.57 min

### Step 2.

Synthesis of 6-(2-((6,6-dimethyl-2,4-dioxo-3-azabicyclo[3.1.0]hexan-3-yl)methyl)thieno[3,2-*b*]pyridin-7-yl)-5-(3,3-dimethylpiperazine-1-carbonyl)-4-methylpicolinonitrile hydrochloride (**53**).

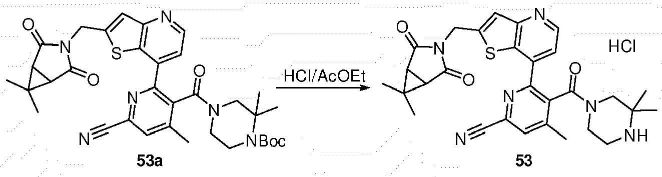

The title compound (**53**) was obtained as a hydrochloride salt as a white solid from **53a** (the crude reaction mixture) according to the General Procedure **IVa** in 22% yield (per two steps) (14 mg; 0.024 mmol). The crude product was purified by preparative reversed-phase column chromatography (C-18, water/MeCN, 98:2 to 30:70, 30 min, 20 mL/min). The obtained product was redissolved in water and to this solution 2 M HCl (3 drops) was added and then obtained hydrochloride salt was lyophilized.
ESI-MS m/z for C₂₀H₃₁N₆O₃S found 543.4 [M+H]⁺; Rt = 0.97 min; ¹H NMR (700 MHz, DMSO-*d*₆ + D₂O, 348 K) δ 8.76 - 8.71 (m, 1H), 8.15 - 8.08 (m, 1H), 7.54 - 7.36 (m, 2H), 4.86 - 4.76 (m, 2H), 4.05 - 3.61 (m, 1H), 3.28 - 3.15 (m, 1H), 3.03 - 2.73 (m, 2H), 2.55 - 2.52 (m, 2H), 2.49 - 2.37 (m, 5H), 1.34 - 1.19 (m, 5H), 1.09 - 0.94 (m, 6H), 0.62 - 0.45 (m, 1H).

### Example 54.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-((*R*)-2-methylpiperazine-1-carbonyl)picolinonitrile hydrochloride (**54**).

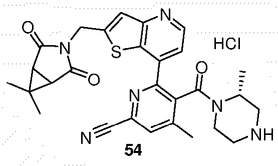

The title compound (**54**) was obtained as a hydrochloride salt in 6% overall yield in a similar way to Example 53 with the exception that, in the first step of the synthesis *tert*-butyl (*R*)-3-methylpiperazine-1-carboxylate was used instead of *tert*-butyl 2,2-dimethylpiperazine-1-carboxylate and in the last step of the synthesis the crude product was purified by preparative reversed-phase column chromatography (C-18, water/MeCN, 98:2 to 30:70, 30 min, 20 mL/min). The obtained product was redissolved in water and to this solution 2 M HCl (3 drops) was added and then obtained hydrochloride salt was lyophilized.
ESI-MS m/z for C₂₈H₂₉N₆O₃S found 529.3 [M+H]⁺; Rt = 0.96 min; ¹H NMR (700 MHz, DMSO-*d*₆ + D₂O, 348 K) δ 8.82 - 8.68 (m, 1H), 8.16 - 8.06 (m, 1H), 7.60 - 7.33 (m, 2H), 4.93 - 4.71 (m, 3H), 3.49 - 3.33 (m, 1H), 3.24 - 2.57 (m, 5H), 2.55 - 2.52 (m, 2H), 2.49 - 2.35 (m, 3H), 1.53 - 0.16 (m, 9H).

### Example 55.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-(piperazine-1-carbonyl)picolinonitrile hydrochloride (**55**).

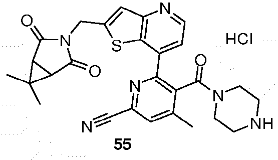

The title compound (**55**) was obtained as a hydrochloride salt in 45% overall yield in a similar way to Example 53 with the exception that, in the first step of the synthesis *tert*-butyl piperazine-1-carboxylate was used instead of *tert*-butyl 2,2-dimethylpiperazine-1-carboxylate and in the last step of the synthesis the crude product was purified by preparative reversed-phase column chromatography (C-18, water/MeCN, 98:2 to 30:70, 30 min, 20 mL/min). The obtained product was redissolved in water and to this solution 2 M HCl (3 drops) was added and then obtained hydrochloride salt was lyophilized.
ESI-MS m/z for C₂₇H₂₇N₆O₃S found 515.4 [M+H]⁺; Rt = 0.94 min; ¹H NMR (700 MHz, DMSO-*d*₆ + D₂O, 348 K) δ 8.78 - 8.75 (m, 1H), 8.12 - 8.09 (m, 1H), 7.53 - 7.49 (m, 1H), 7.45 - 7.40 (m, 1H), 4.87 - 4.77 (m, 2H), 4.00 - 3.90 (m, 1H), 3.57 - 3.45 (m, 1H), 3.30 - 3.22 (m, 1H), 3.17 - 3.11 (m, 1H), 2.97 - 2.90 (m, 1H), 2.84 - 2.76 (m, 2H), 2.55 - 2.52 (m, 2H), 2.42 (s, 3H), 2.35 - 2.28 (m, 1H), 1.19 (s, 3H), 1.06 (s, 3H).

### Example 58.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-(morpholine-4-carbonyl)picolinonitrile hydrochloride (**58**).

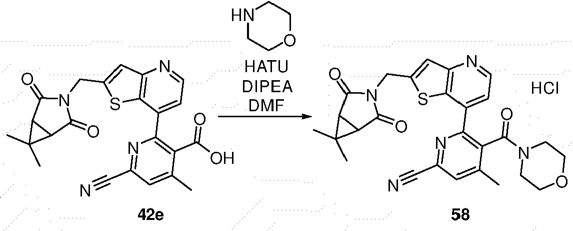

The title compound (**58**) was obtained as a hydrochloride salt from **42e** (50 mg; 0.110 mmol) and from morpholine (16 mg; 0.180 mmol) according to the General Procedure **I** in 40% yield (24 mg; 0.044 mmol). The crude product was purified by preparative reversed-phase column chromatography (C-18, water + 0.5‰ HCl (6 M)/MeCN, 98:2 to 30:70, 30 min, 20 mL/min).
ESI-MS m/z for C₂₇H₂₅N₅O₄S found 516.3 [M+H]⁺; Rt = 1.23 min; ¹H NMR (700 MHz, DMSO-*d*₆ + D₂O) δ 8.76 - 8.72 (m, 1H), 8.11 - 8.04 (m, 1H), 7.51 - 7.46 (m, 2H), 4.83 - 4.76 (m, 2H), 3.58 - 3.54 (m, 1H), 3.51 - 3.47 (m, 2H), 3.26 - 3.19 (m, 2H), 3.05 - 2.98 (m, 1H), 2.64 - 2.56 (m, 2H), 2.55 - 2.52 (m, 1H), 2.50 - 2.47 (m, 1H), 2.40 (s, 3H), 1.18 (s, 3H), 1.03 (s, 3H).

### Example 59.

Synthesis of 5-(3,3-difluoropyrrolidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile hydrochloride (**59**).

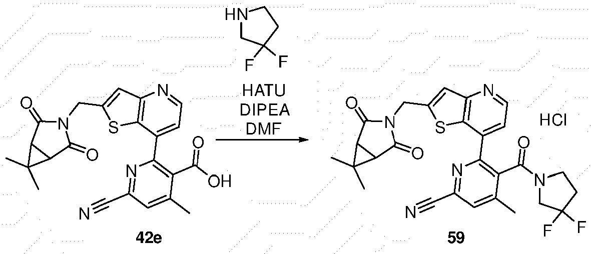

The title compound (**59**) was obtained as a hydrochloride salt from **42e** (50 mg; 0.110 mmol) and from 3,3-difluoropyrrolidine (20 mg; 0.130 mmol) according to the General Procedure **I** in 57% yield (36 mg; 0.063 mmol). The crude product was purified by preparative reversed-phase column chromatography (C-18, water/MeCN, 98:2 to 30:70, 30 min, 20 mL/min). The obtained product was redissolved in water and to this solution 2 M HCl (3 drops) was added and then obtained hydrochloride salt was lyophilized.
ESI-MS m/z for C₂₇H₂₄F₂N₅O₃S found 536.5 [M+H]⁺; Rt = 1.38 min; ¹H NMR (700 MHz, DMSO-*d*₆ + D₂O) δ 8.73 - 8.69 (m, 1H), 8.12 - 8.04 (m, 1H), 7.50 - 7.47 (m, 1H), 7.46 - 7.40 (m, 1H), 4.89 - 4.71 (m, 2H), 3.94 - 3.68 (m, 2H), 3.54 - 3.41 (m, 1H), 3.27 - 3.22 (m, 1H), 2.99 - 2.69 (m, 1H), 2.53 - 2.52 (m, 1H), 2.44 - 1.90 (m, 5H), 1.23 - 1.14 (m, 3H), 1.07 - 0.95 (m, 3H).

### Example 70.*(this compound is not a part of the present invention)

Synthesis of *N*-(azetidin-3-yl)-2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinamide dihydrochloride (**70**).

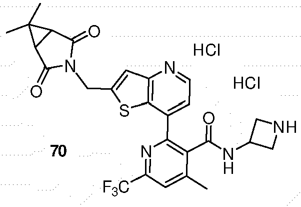

### Step 1.

Synthesis of *tert*-butyl 2-chloro-4-methyl-6-(trifluoromethyl)nicotinate (**70a**).

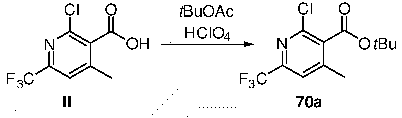

To the solution of acid **II** (2.9 g; 12.1 mmol) in tBuOAc (60 mL) HClO₄ (70%; 2.19 mL; 36.3 mmol) was added dropwise at 0 °C and then the reaction mixture was warmed to at room temperature and stirred for 72 hours. The reaction progress was monitored by TLC. When analysis indicated completion of the reaction, the reaction mixture was cooled to 0 °C and 5% NaHCO₃ was added to this mixture followed by Et₂O. The layers were separated and the aqueous one was extracted with Et₂O (3 x). The combined organic solutions were washed with brine, dried over anhydrous Na₂SO₄, filtered and concentrated *in vacuo* and the crude product was used to the next step without additional purification. Compound **70a** was obtained in 9% yield (322 mg; 1.09 mmol).
ESI-MS m/z for C₁₂H₁₄ClF₃NO₂ found 296.0/298.0 [M+H]⁺; Rt= 1.86 min

### Step 2.

Synthesis of *tert*-butyl 2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinate (**70b**).

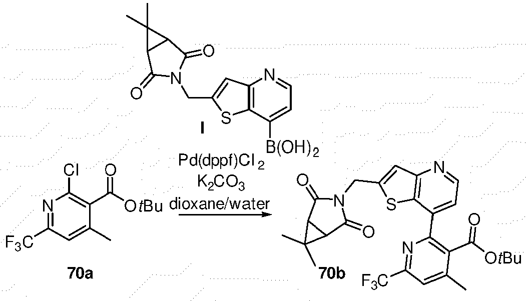

The title compound (**70b**) was obtained from **70a** (311 mg; 1.05 mmol) and from boronic acid **I** (453 mg; 1.37 mmol) according to the General Procedure **Va** in 80% yield (458 mg; 0.84 mmol).
ESI-MS m/z for C₂₇H₂₇F₃N₃O₄S found 546.4 [M+H]⁺; Rt = 1.75 min

### Step 3.

Synthesis of 2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinic acid (**70c**).

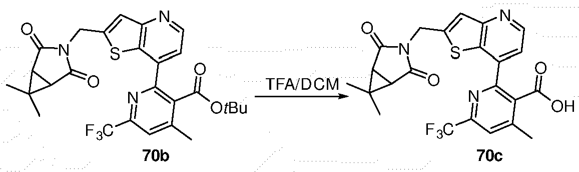

To the solution of **70b** (458 mg; 0.84 mmol) in DCM (10 mL) TFA (8 mL; 108 mmol) was added and this mixture was stirred at 40 °C for 3 hours. Then the solvent was evaporated *in vacuo* and the crude product was used to the next step without additional purification. Compound **70c** was obtained in 99% yield (406 mg; 0.83 mmol).
ESI-MS m/z for C₂₃H₁₉F₃N₃O₄S found 490.2 [M+H]⁺; Rt = 1.28 min

### Step 4.

Synthesis of *tert*-butyl 3-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinamido)azetidine-1-carboxylate (**70d**).

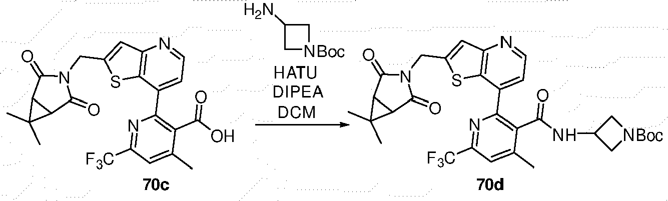

The title compound (**70d**) was obtained from **70c** (50 mg; 0.08 mmol) and from *tert-*butyl 3-aminoazetidine-1-carboxylate (16 mg; 0.09 mmol) according to the General Procedure **I** in 99% yield (51 mg; 0.08 mmol). The crude product was purified by flash column chromatography on silica (DCM/MeOH, 8:2, *v*/*v,* 30 min).
ESI-MS m/z for C₃₁H₃₃F₃N₅O₅S found 644.3 [M+H]⁺; Rt = 1.54 min

### Step 5.

Synthesis of *N*-(azetidin-3-yl)-2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinamide dihydrochloride (**70**).

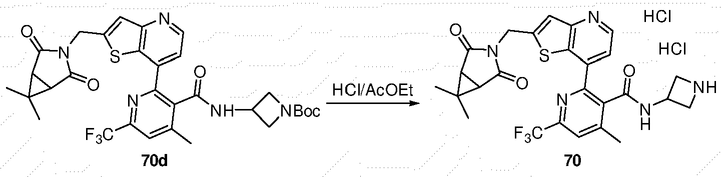

The title compound (**70**) was obtained as a dihydrochloride salt from **70d** (51 mg; 0.08 mmol) according to the General Procedure **IVa** in 19% yield (10 mg; 0.016 mmol). The crude product was purified by preparative reversed-phase column chromatography (column: Cosmosil Cholester 20 x 250 mm, water + 0.5‰ HCl (36%)/MeCN, 99:1 to 40:60, 30 min, 20 mL/min). ESI-MS m/z for C₂₆H₂₅F₃N₅O₃S found 544.4 [M+H]⁺; Rt = 1.63 min; ¹H NMR (700 MHz, Methanol-*d*₄) δ 9.04 (d, *J =* 6.0 Hz, 1H), 8.20 (d, *J =* 6.0 Hz, 1H), 8.12 (d, *J =* 0.8 Hz, 1H), 7.80 (s, 1H), 5.03 (d, *J =* 0.9 Hz, 2H), 4.78 (tt, *J =* 8.3, 7.0 Hz, 1H), 4.41 - 4.31 (m, 2H), 4.24 - 4.13 (m, 2H), 2.66 (s, 3H), 2.56 (s, 2H), 1.29 (s, 3H), 1.18 (s, 3H).

### Example 73.

Synthesis of 3-((7-(3-((*S*)-3-(ethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**73**).

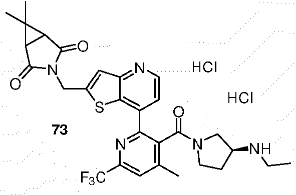

The title compound (**73**) was obtained as a dihydrochloride salt in 24% overall yield in a similar way to Example 45 with the exception that, in the first step of the synthesis the compound **III** was used instead of *tert*-butyl piperazine-1-carboxylate and acid **II** was used instead of 2-chloro-4-methylnicotinic acid and in the last step of the synthesis, the crude product was purified by preparative reversed-phase column chromatography (C-18, water + 0.5‰ HCl (36%)/MeCN, 98:2 to 30:70, 30 min, 20 mL/min).
ESI-MS m/z for C₂₉H₃₁F₃N₅O₃S found 586.1 [M+H]⁺; Rt = 1.04 min; ¹H NMR (700 MHz, DMSO-*d*₆+ D₂O) δ 8.80 - 8.68 (m, 1H), 8.02 - 7.99 (m, 1H), 7.62 - 7.45 (m, 2H), 4.85 - 4.74 (m, 2H), 4.18 - 3.59 (m, 2H), 3.47 - 3.04 (m, 2H), 3.03 - 2.92 (m, 1H), 2.90 - 2.55 (m, 3H), 2.49 - 2.39 (m, 3H), 2.37 - 1.55 (m, 3H), 1.26 - 1.15 (m, 5H), 1.11 - 0.92 (m, 4H).

### Example 74.

Synthesis of 3-((7-(3-((*S*)-3-(isopropylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**74**).

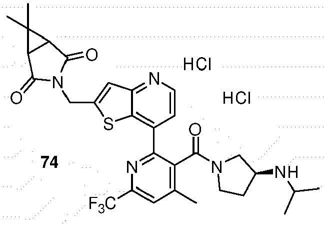

### Step 1.

Synthesis of *tert*-butyl (*S*)-(1-(2-chloro-4-methyl-6-(trifluoromethyl)nicotinoyl)pyrrolidin-3-yl)carbamate (**74a**).

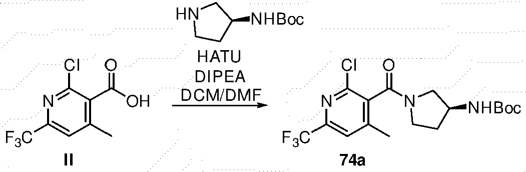

The title compound (**74a**) was obtained from **II** (100 mg; 0.42 mmol) and from *tert-*butyl (*S*)-pyrrolidin-3-ylcarbamate (86 mg; 0.46 mmol) according to the General Procedure **I** in 61% yield (104 mg; 0.26 mmol).
ESI-MS m/z for C₁₃H₁₄ClF₃N₃O₃ found 352.0/354.0 [M+H-*t*Bu]⁺; Rₜ = 1.37 min

### Step 2.

Synthesis of (*S*)-(3-aminopyrrolidin-1-yl)(2-chloro-4-methyl-6-(trifluoromethyl)pyridin-3-yl)methanone hydrochloride (**74b**).

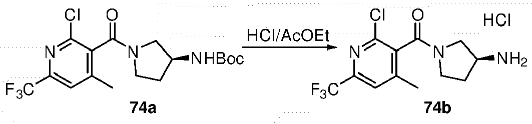

The title compound (**74b**) was obtained as a hydrochloride salt from **74a** (31 mg; 0.076 mmol) according to the General Procedure **IVa** and after standard work-up all the crude product mixture was taken for the next step.
ESI-MS m/z for C₁₂H₁₄ClF₃N₃O found 308.0/310.0 [M+H]⁺; Rt= 0.50 min

### Step 3.

Synthesis of (*S*)-(2-chloro-4-methyl-6-(trifluoromethyl)pyridin-3-yl)(3-(isopropylamino)pyrrolidin-1-yl)methanone (**74c**).

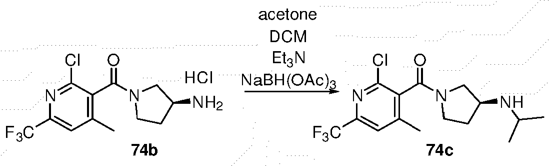

The title compound (**74c**) was obtained from **74b** (crude reaction mixture) and from acetone (0.01 mL; 0.091 mmol) according to the General Procedure **VIa** in 83% yield (per two steps)(22 mg; 0.063 mmol) with the exception that this reaction was carried on in DCM and Et₃N was added instead of acetic acid. The crude product was purified flash column chromatography on silica (DCM/MeOH, 95:5 to 50:50, *v*/*v,* 30 min).
ESI-MS m/z for C₁₅H₂₀ClF₃N₃O found 350.0/352.0 [M+H]⁺; Rₜ = 0.70 min

### Step 4.

Synthesis of 3-((7-(3-((*S*)-3-(isopropylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**74**).

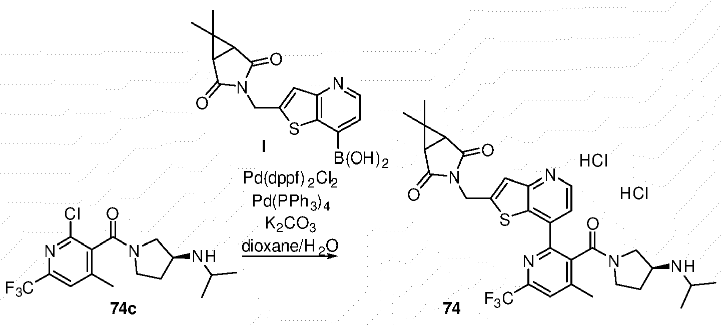

The title compound (**74**) was obtained as a dihydrochloride salt from **74c** (20 mg; 0.057 mmol) and from boronic acid **I** (30 mg; 0.091 mmol) according to the General Procedure **Va** in 33% yield (13 mg; 0.019 mmol). The crude product was purified by preparative reversed-phase column chromatography (C-18, water + 0.5‰ HCl (36%)/MeCN, 98:2 to 30:70, 30 min, 20 mL/min).
ESI-MS m/z for C₃₀H₃₃F₃N₅O₃S found 600.0 [M+H]⁺; Rt = 1.08 min; ¹H NMR (700 MHz, DMSO-*d*₆) δ 8.94 - 8.76 (m, 1H), 8.18 - 8.07 (m, 1H), 7.78 - 7.47 (m, 2H), 4.91 - 4.73 (m, 2H), 4.19 - 3.56 (m, 2H), 3.42 - 2.82 (m, 2H), 2.68 - 2.58 (m, 2H), 2.57 - 2.52 (m, 3H), 2.49 - 2.33 (m, 2H), 2.25 - 1.70 (m, 2H), 1.39 - 1.14 (m, 6H), 1.14 - 0.97 (m, 6H).

### Example 76.

Synthesis of 6,6-dimethyl-3-((7-(4-methyl-3-(piperazine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride (**76**).

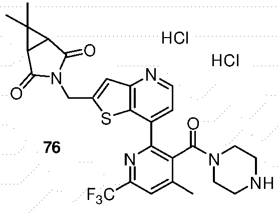

The title compound (**76**) was obtained as a dihydrochloride salt in 30% overall yield in a similar way to Example 45 with the exception that, in the first step of the synthesis *tert*-butyl piperazine-1-carboxylate was used instead of *tert*-butyl piperazine-1-carboxylate and acid **II** was used instead of 2-chloro-4-methylnicotinic acid and in the last step of the synthesis, the crude product was dissolved in water and to this solution 2 M HCl (3 drops) was added and then obtained dihydrochloride salt was lyophilized.
ESI-MS m/z for C₂₇H₂₇F₃N₅O₃S found 558.2 [M+H]⁺; Rt = 1.01 min; ¹H NMR (700 MHz, DMSO-*d*₆+ D₂O) δ 8.76 (d, *J =* 4.9 Hz, 1H), 8.06 (d, *J =* 0.7 Hz, 1H), 7.52 (d, *J =* 0.9 Hz, 1H), 7.44 (d, *J =* 4.9 Hz, 1H), 4.83 - 4.75 (m, 2H), 3.52 - 3.42 (m, 1H), 3.23 (ddd, *J =* 14.1, 6.3, 3.4 Hz, 1H), 3.10 (td, *J =* 8.8, 4.2 Hz, 1H), 2.97 (dt, *J =* 13.4, 4.6 Hz, 1H), 2.84 - 2.76 (m, 1H), 2.76 - 2.67 (m, 1H), 2.58 - 2.52 (m, 3H), 2.46 - 2.42 (m, 3H), 2.32 - 2.27 (m, 1H), 1.15 (s, 3H), 0.98 (s, 3H).

### Example 77.

Synthesis of 6,6-dimethyl-3-((7-(4-methyl-3-((*S*)-3-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride (**77**).

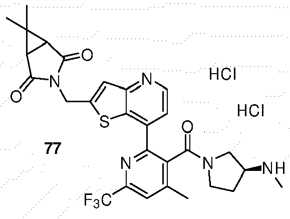

The title compound (**77**) was obtained as a dihydrochloride salt as a mixture of rotamers in 4% overall yield in a similar way to Example 45 with the exception that, in the first step of the synthesis the *tert*-butyl (*S*)-methyl(pyrrolidin-3-yl)carbamate was used instead of *tert-*butyl piperazine-1-carboxylate and acid **II** was used instead of 2-chloro-4-methylnicotinic acid and in the last step of the synthesis, the crude product was purified by preparative reversed-phase column chromatography (C-18, water + 0.5‰ HCl (36%)/MeCN, 98:2 to 30:70, 30 min, 20 mL/min).
ESI-MS m/z for C₂₈H₂₉F₃N₅O₃S found 572.2 [M+H]⁺; Rt = 1.00 min; ¹H NMR (700 MHz, DMSO-*d*₆+ D₂O) δ 8.77 - 8.69 (m, 1H), 8.10 - 7.94 (m, 1H), 7.60 - 7.44 (m, 2H), 4.83 - 4.74 (m, 2H), 4.12 - 3.58 (m, 2H), 3.42 - 3.05 (m, 2H), 2.93 - 2.80 (m, 3H), 2.68 - 2.56 (m, 2H), 2.49 - 2.29 (m, 3H), 2.23 - 1.78 (m, 2H), 1.63 - 1.55 (m, 1H), 1.21 - 1.15 (m, 3H), 1.10 - 0.99 (m, 3H).

### Example 78.

Synthesis of 6,6-dimethyl-3-((7-(4-methyl-3-((*S*)-2-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride (**78**).

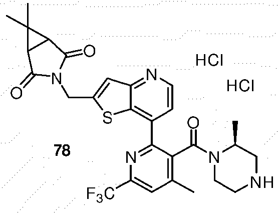

The title compound (**78**) was obtained as a dihydrochloride salt in 12% overall yield in a similar way to Example 53 with the exception that, in the first step of the synthesis *tert*-butyl (*S*)-3-methylpiperazine-1-carboxylate was used instead of *tert*-butyl 2,2-dimethylpiperazine-1-carboxylate and an acid **70c** was used instead of an acid **42e** and in the last step of the synthesis the crude product was purified by preparative reversed-phase column chromatography (column: Cosmosil Cholester 20 x 250 mm, water + 0.5‰ HCl (36%)/MeCN, 99:1 to 40:60, 30 min, 20 mL/min).
ESI-MS m/z for C₂₈H₂₉F₃N₅O₃S found 572.5 [M+H]⁺; Rt = 1.02 min; ¹H NMR (700 MHz, DMSO-*d*₆) δ 8.86 - 8.65 (m, 1H), 8.06 - 7.95 (m, 1H), 7.67 - 7.33 (m, 2H), 5.00 - 4.53 (m, 3H), 3.55 - 3.36 (m, 1H), 3.25 - 3.03 (m, 2H), 3.00 - 2.89 (m, 1H), 2.90 - 2.73 (m, 1H), 2.72 - 2.56 (m, 1H), 2.50 - 2.38 (m, 5H), 1.51 - 1.21 (m, 1H), 1.21 - 1.07 (m, 3H), 1.08 - 0.99 (m, 3H), 0.96 - 0.14 (m, 2H).

### Example 79.

Synthesis of 3-((7-(3-((*S*)-2,4-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**79**).

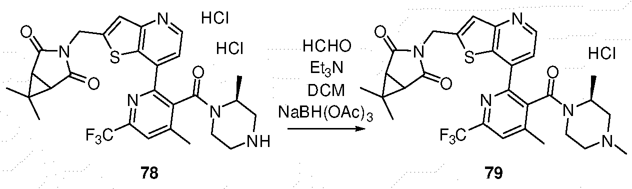

To the solution of **78** (9 mg; 0.014 mmol) in DCM (0.7 mL) Et₃N (0.01 mL; 0.058 mmol) and formalin (36% aqueous solution; 0.05 mL) were added and this mixture was stirred at room temperature for 1 hour. Then to this mixture NaBH(OAc)₃ (15 mg; 0.069 mmol) was added and whole was stirred at room temperature for 1 hour. The reaction progress was monitored by LC-MS. When analysis indicated completion of the reaction, the mixture was diluted with DCM and washed with 5% NaHCO₃. The organic layer was dried over anhydrous MgSO₄, filtered and concentrated *in vacuo* and the residue was purified by preparative reversed-phase column chromatography (C-18, water + 0.5‰ HCl (36%)/MeCN, 98:2 to 30:70, 30 min, 20 mL/min). The title compound (**79**) was obtained as a hydrochloride salt as a mixture of rotamers in 71% yield (6 mg; 0.010 mmol).
ESI-MS m/z for C₂₉H₃₁F₃N₅O₃S found 586.6 [M+H]⁺; Rₜ = 1.34 min; ¹H NMR (700 MHz, D₂O) δ 9.22 - 9.11 (m, 1H), 8.46 - 8.35 (m, 1H), 8.18 - 7.95 (m, 2H), 5.39 - 4.87 (m, 3H), 4.06 - 3.71 (m, 2H), 3.68 - 3.40 (m, 2H), 3.34 - 3.15 (m, 3H), 2.95 (q, *J =* 3.6 Hz, 3H), 2.87 - 2.48 (m, 3H), 1.75 - 1.60 (m, 1H), 1.56 - 1.42 (m, 3H), 1.40 - 1.30 (m, 3H), 1.25 - 0.52 (m, 2H).

### Example 80.

Synthesis of 6,6-dimethyl-3-((7-(4-methyl-3-(4-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione dihydrochloride (**80**).

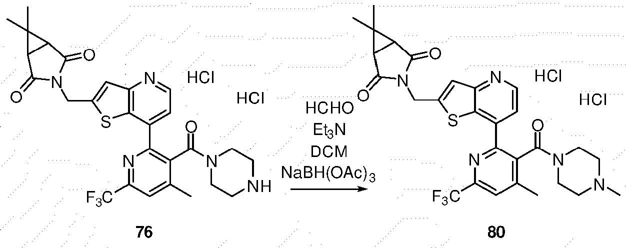

To the solution of **76** (9 mg; 0.014 mmol) in DCM (1 mL) Et₃N (0.008 mL; 0.057 mmol) and formalin (36% aqueous solution; 0.05 mL) were added and this mixture was stirred at room temperature for 1 hour. Then to this mixture NaBH(OAc)₃ (6 mg; 0.028 mmol) was added and whole was stirred at room temperature for 1 hour. The reaction progress was monitored by LC-MS. When analysis indicated completion of the reaction, the mixture was diluted with DCM and washed with 5% NaHCO₃. The organic layer was dried over anhydrous MgSO₄, filtered and concentrated *in vacuo* and the residue was purified by preparative reversed-phase column chromatography (C-18, water + 0.5‰ HCl (36%)/MeCN, 98:2 to 30:70, 30 min, 20 mL/min). The title compound (**80**) was obtained as a dihydrochloride salt as a white solid in 71% yield (7 mg; 0.010 mmol).
ESI-MS m/z for C₂₈H₂₉F₃N₅O₃S found 572.1 [M+H]⁺; Rt = 1.01 min; ¹H NMR (700 MHz, DMSO-*d*₆+ D₂O) δ 8.76 (d, *J =* 5.0 Hz, 1H), 8.02 (s, 1H), 7.50 (t, *J =* 0.9 Hz, 1H), 7.42 (d, *J =* 4.9 Hz, 1H), 4.84 - 4.73 (m, 2H), 3.34 - 3.15 (m, 3H), 2.98 - 2.91 (m, 1H), 2.70 (s, 3H), 2.63 - 3.15 (m, 1H), 2.56 - 2.53 (m, 4H), 2.47 (s, 3H), 2.41 (p, *J* = 1.9 Hz, 1H), 1.18 (s, 3H), 1.03 (s, 3H).

### Example 81.

Synthesis of 3-((7-(3-((*S*)-3-aminopyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**81**).

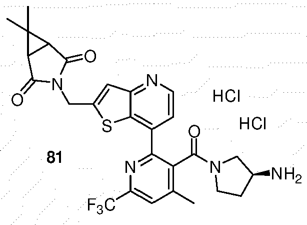

The title compound (**81**) was obtained as a dihydrochloride salt as a mixture of rotamers in 27% overall yield in a similar way to Example 45 with the exception that, in the first step of the synthesis *tert*-butyl (*S*)-pyrrolidin-3-ylcarbamate was used instead of *tert*-butyl piperazine-1-carboxylate and acid **II** was used instead of 2-chloro-4-methylnicotinic acid and in the last step of the synthesis, the crude product was dissolved in a solution of HCl in AcOEt and stirred at room temperature for 5 minutes. Then Et₂O was added to this mixture and the obtained white solid was filtered off, then washed with Et₂O. The solid was dissolved in water and lyophilized.
ESI-MS m/z for C₂₇H₂₇F₃N₅O₃S found 558.4 [M+H]⁺; Rt = 0.97 min; ¹H NMR (700 MHz, D₂O) δ 9.20 - 9.07 (m, 1H), 8.51 - 8.30 (m, 1H), 8.14 - 7.89 (m, 2H), 5.32 - 5.20 (m, 2H), 4.57 - 4.06 (m, 2H), 4.03 - 3.77 (m, 1H), 3.69 - 3.13 (m, 2H), 3.04 - 2.92 (m, 3H), 2.91 - 2.78 (m, 2H), 2.76 - 2.35 (m, 1H), 2.27 - 1.86 (m, 1H), 1.53 (d, *J =* 9.7 Hz, 3H), 1.42 - 1.26 (m, 3H).

### Example 82.

Synthesis of 3-((7-(3-((*S*)-3-(dimethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**82**).

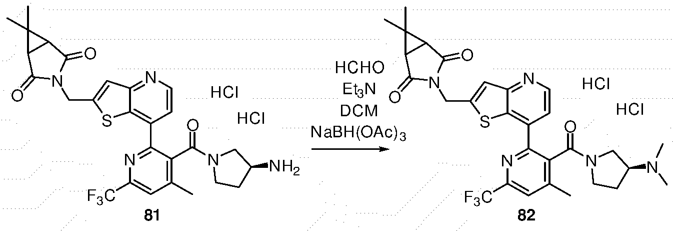

To the solution of **81** (20 mg; 0.032 mmol) in DCM (0.7 mL) Et₃N (0.018 mL; 0.120 mmol) and formalin (36% aqueous solution; 0.05 mL) were added and this mixture was stirred at room temperature for 1 hour. Then to this mixture NaBH(OAc)₃ (13 mg; 0.063 mmol) was added and whole was stirred at room temperature for 1 hour. The reaction progress was monitored by LC-MS. When analysis indicated completion of the reaction, the mixture was diluted with DCM and washed with 5% NaHCO₃. The organic layer was dried over anhydrous MgSO₄, filtered and concentrated *in vacuo* and the residue was purified by preparative reversed-phase column chromatography (C-18, water + 0.5‰ HCl (36%)/MeCN, 98:2 to 30:70, 30 min, 20 mL/min). The title compound (**82**) was obtained as a dihydrochloride salt as a white solid in 44% yield (8 mg; 0.014 mmol).
ESI-MS m/z for C₂₉H₃₁F₃N₅O₃S found 586.2 [M+H]⁺; Rt = 1.06 min; ¹H NMR (700 MHz, DMSO-*d*₆+ D₂O) δ 8.81 - 8.61 (m, 1H), 8.15 - 7.96 (m, 1H), 7.54 - 7.44 (m, 2H), 4.86 - 4.73 (m, 2H), 4.11 - 3.78 (m, 1H), 3.77 - 3.60 (m, 1H), 3.49 - 3.32 (m, 1H), 3.28 - 3.05 (m, 1H), 2.77 (s, 2H), 2.63 (s, 3H), 2.58 - 2.52 (m, 4H), 2.49 - 2.43 (m, 3H), 2.39 - 1.59 (m, 3H), 1.21 - 1.15 (m, 2H), 1.09 - 0.98 (m, 3H).

### Example 83.

Synthesis of 3-((7-(3-(3,3-difluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**83**).

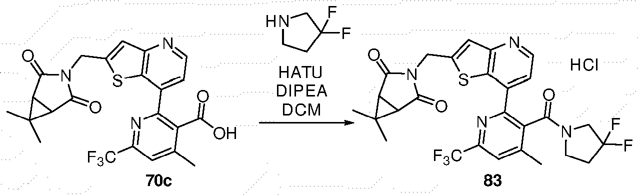

The title compound (**83**) was obtained as a hydrochloride salt from **70c** (40 mg; 0.066 mmol) and from 3,3-difluoropyrrolidine (10 mg; 0.073 mmol) according to the General Procedure **I** in 59% yield (24 mg; 0.039 mmol). The crude product was purified by flash column chromatography on silica (hexane/AcOEt, 2:8, *v*/*v,* 30 min; then AcOEt, 100%) and then dissolved in a solution of HCl in AcOEt and stirred at room temperature for 5 minutes. Then Et₂O was added to this mixture and the obtained white solid was filtered off, then washed with Et₂O. The solid was dissolved in water and lyophilized.
ESI-MS m/z for C₂₇H₂₄F₅N₄O₃S found 579.3 [M+H]⁺; Rt = 1.45 min; ¹H NMR (700 MHz, DMSO-*d*₆) δ 8.81 - 8.73 (m, 1H), 8.16 - 8.04 (m, 1H), 7.56 - 7.47 (m, 2H), 4.89 - 4.76 (m, 2H), 4.01 - 3.75 (m, 2H), 3.61 - 3.25 (m, 1H), 3.03 - 2.72 (m, 1H), 2.59 - 2.52 (m, 3H), 2.48 - 2.24 (m, 3H), 2.08 - 1.97 (m, 1H), 1.22 - 1.19 (m, 3H), 1.06 - 1.03 (m, 3H).

### Example 84.

Synthesis of 6,6-dimethyl-3-((7-(4-methyl-3-(morpholine-4-carbonyl)-6-(trifluoromethyl)pyridin-2-yl)thieno[3,2-*b*]pyridin-2-yl)methyl)-3-azabicyclo[3.1.0]hexane-2,4-dione hydrochloride (**84**).

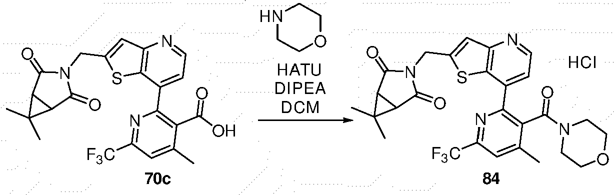

The title compound (**84**) was obtained as a hydrochloride salt from **70c** (50 mg; 0.082 mmol) and from morpholine (9 mg; 0.090 mmol) according to the General Procedure **I** in 45% yield (22 mg; 0.037 mmol). The crude product was purified by flash column chromatography on silica (hexane/AcOEt, 2:8, *v*/*v,* 30 min; then AcOEt, 100%) and then dissolved in MeCN/water with addition of 2 N HCl (few drops), frozen and lyophilized.
ESI-MS m/z for C₂₇H₂₆F₃N₄O₄S found 559.2 [M+H]⁺; Rt = 1.36 min; ¹H NMR (700 MHz, DMSO-*d*₆) δ 8.78 (d, *J =* 4.9 Hz, 1H), 8.09 - 7.94 (m, 1H), 7.56 (d, *J =* 4.9 Hz, 1H), 7.51 (s, 1H), 4.81 (s, 2H), 3.69 - 3.46 (m, 3H), 3.37 - 3.30 (m, 2H), 3.06 - 3.01 (m, 1H), 2.66 (dddt, *J =* 16.5, 13.3, 6.1, 3.1 Hz, 2H), 2.57 - 2.52 (m, 2H), 2.49 (s, 3H), 1.21 (s, 3H), 1.06 (s, 3H).

### Example 85.

Synthesis of 3-((7-(3-(3,3-difluoroazetidine-1-carbonyl)-4,6-dimethylpyridin-2-yl)thieno[3,2-*b*]pyridin-2-yl)methyl)-6,6-dimethyl-3-azabicyclo[3.1.0]hexane-2,4-dione hydrochloride (**85**).

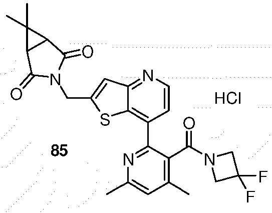

The title compound (**85**) was obtained as a hydrochloride salt in 2% overall yield in a similar way to Example 48 with the exception that, in the first step of the synthesis 3,3-difluoroazetidine hydrochloride was used instead of morpholine and 2-chloro-4,6-dimethylnicotinic acid was used instead of 2-chloro-4-methylnicotinic acid and in the last step of the synthesis the crude product was purified by preparative reversed-phase column chromatography (C-18, water + 0.5‰ HCl (36%)/MeCN, 98:2 to 40:60, 30 min, 20 mL/min). ESI-MS m/z for C₂₆H₂₄F₂N₄O₃S found 511.7 [M+H]⁺; Rt = 1.23 min; ¹H NMR (700 MHz, Methanol-*d*₄) δ 9.02 (d, *J =* 6.1 Hz, 1H), 8.13 (d, *J =* 6.0 Hz, 1H), 7.80 (s, 1H), 7.57 (s, 1H), 5.02 (d, *J =* 0.8 Hz, 2H), 4.73 - 4.44 (m, 2H), 4.23 (d, *J =* 12.0 Hz, 1H), 3.76 (d, *J =* 11.8 Hz, 1H), 2.74 (s, 3H), 2.56 (s, 2H), 2.49 (d, *J =* 0.6 Hz, 3H), 1.28 (s, 3H), 1.15 (s, 3H).

### Example 91.*(this compound is not a part of the present invention)

Synthesis of 6,6-dimethyl-3-((7-(2-methyl-3-(morpholin-2-ylmethyl)-6-(trifluoromethyl)pyridin-4-yl)thieno[3,2-*b*]pyridin-2-yl)methyl)-3-azabicyclo[3.1.0]hexane-2,4-dione 2,2,2-trifluoroacetate (**91**).

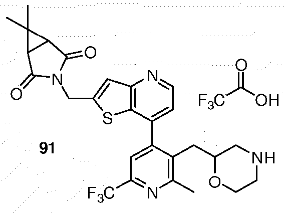

### Step 1.

Synthesis of *tert*-butyl 2-((4,4,5,5-tetramethyl-1,3,2-dioxaborolan-2-yl)methyl)morpholine-4-carboxylate (91a).

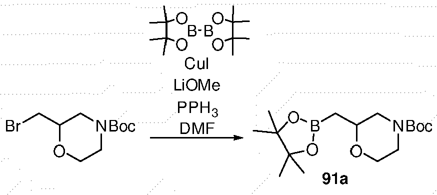

A suspension of *tert*-butyl 2-(bromomethyl)morpholine-4-carboxylate (0.6 g; 2.14 mmol), CuI (41 mg; 0.21 mmol), LiOMe (162 mg; 4.28 mmol), PPh₃ (112 mg; 0.43 mmol) and bis(pinacolato)diboron (815 mg; 3.21 mmol) in DMF (12 mL) was purged with Ar and then stirred at 35 °C in a sealed capsule for 2 hours. The reaction completion was confirmed by LC-MS. Then DMF was evaporated *in vacuo* and the residue was purified by flash column chromatography on silica (hexane/AcOEt, 7:3, *v*/*v,* 20 minutes). The title compound **91a** was obtained in 23% yield (160 mg; 0.49 mmol).
ESI-MS m/z for C₁₁H₂₃BNO₃ found 228.0 [M+H-Boc]⁺; Rₜ = 1.52 min

### Step 2.

Synthesis of *tert*-butyl 2-((4-(2-((6,6-dimethyl-2,4-dioxo-3-azabicyclo[3.1.0]hexan-3-yl)methyl)thieno[3,2-*b*]pyridin-7-yl)-2-methyl-6-(trifluoromethyl)pyridin-3-yl)methyl)morpholine-4-carboxylate (**91b**).

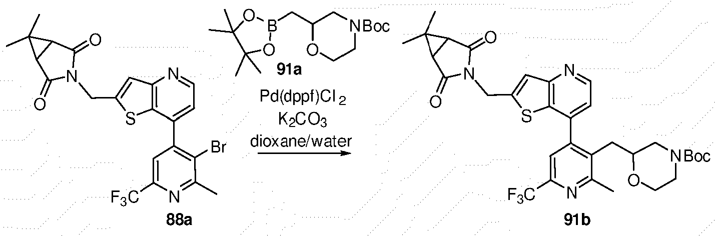

The title compound (**91b**) was obtained from **88a** (60 mg; 0.114 mmol) and from **91a** (45 mg; 0.137 mmol) according to the General Procedure **Va** and after standard work-up the crude product was taken to the next step.
ESI-MS m/z for C₃₂H₃₆F₃N₄O₅S found 645.5 [M+H]⁺; Rt= 1.74 min

### Step 3.

Synthesis of 6,6-dimethyl-3-((7-(2-methyl-3-(morpholin-2-ylmethyl)-6-(trifluoromethyl)pyridin-4-yl)thieno[3,2-*b*]pyridin-2-yl)methyl)-3-azabicyclo[3.1.0]hexane-2,4-dione 2,2,2-trifluoroacetate (**91**).

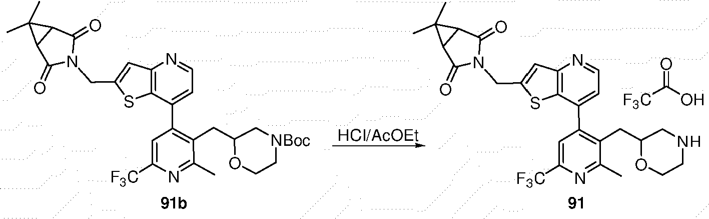

The title compound (**91**) was obtained as a TFA salt from **91b** (the crude reaction mixture) according to the General Procedure **IVa** in 1% yield (per two steps)(1 mg; 0.001 mmol). The crude product was purified by preparative reversed-phase column chromatography (C-18, water/MeCN + 1‰ TFA, 99:1 to 35:65, 30 min, 20 mL/min).
ESI-MS m/z for C₂₇H₂₈F₃N₄O₃S found 545.5 [M+H]⁺; Rt= 1.05 min; ¹H NMR (700 MHz, D₂O) δ 8.93 (m, 1H), 7.85-7.72 (m, 3H), 5.02 (s, 2H), 3.97-3.79 (m, 1H), 3.78-3.56 (m, 1H), 3.55-3.49 (m, 1H), 3.34 - 3.13 (m, 3H), 3.08-2.96 (m, 1H), 2.87 - 2.72 (m, 2H), 2.79 (s, 3H), 2.70 (s, 2H), 1.29 (s, 3H), 1.11 (d, *J* = 9.5 Hz, 3H).

### Example 92.

Synthesis of 6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione 2,2,2-trifluoroacetate (**92**).

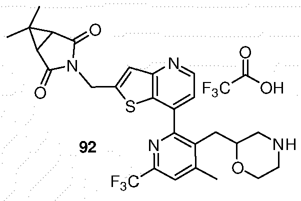

### Step 1.

Synthesis of 3-((7-(3-amino-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione (**92a**).

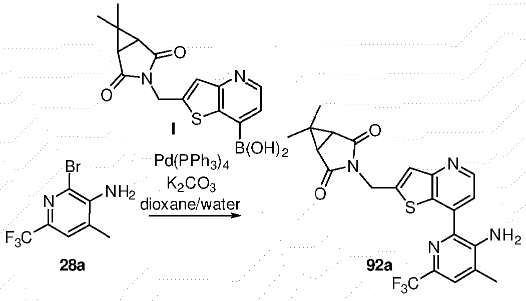

The title compound (**92a**) was obtained from **28a** (380 mg; 1.49 mmol) and from boronic acid **I** (541 mg; 1.64 mmol) according to the General Procedure **Va** in 38% yield (262 mg; 0.57 mmol). The crude product was purified by flash column chromatography on silica (AcOEt, 100%, 10 minutes, then AcOEt/MeOH, 9:1, *v*/*v,* 10 minutes).
ESI-MS m/z for C₂₂H₂₀F₃N₄O₂S found 461.2 [M+H]⁺; Rt= 1.45 min; ¹H NMR (700 MHz, CDCl₃) δ 8.75 (s, 1H), 7.55 (s, 1H), 7.54 - 7.52 (m, 1H), 7.48 - 7.46 (m, 1H), 4.84 (s, 2H), 4.24 (s, 2H), 2.36 (s, 2H), 2.34 (s, 3H), 1.23 (s, 3H), 1.14 (s, 3H).

### Step 2.

Synthesis of 3-((7-(3-bromo-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione (**92b**).

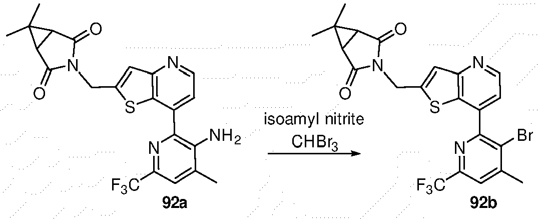

To the solution of **92a** (228 mg; 0.49 mmol) in CHBr₃ (5 mL) isoamyl nitrite (0.13 mL; 0.99 mmol) was added and the reaction mixture was stirred at 100 °C overnight. The reaction progress was monitored by LC-MS. When analysis indicated completion of the reaction, to this mixture brine was added and the product was extracted with AcOEt. An organic layer was dried over anhydrous Na₂SO₄, filtered and concentrated *in vacuo.* The crude product was purified by flash column chromatography on silica (hexane/AcOEt, 2:8, *v*/*v*, 20 minutes). The title compound **92b** was obtained in 8% yield (21 mg; 0.04 mmol).
ESI-MS m/z for C₂₂H₁₈BrF₃N₃O₂S found 523.7/525.7 [M+H]⁺; Rt = 1.56 min

### Step 3.

Synthesis of *tert*-butyl 2-((2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)methyl)morpholine-4-carboxylate (**92c**).

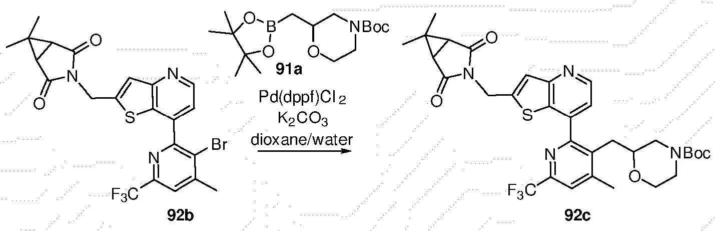

The title compound (**92c**) was obtained from **92b** (25 mg; 0.048 mmol) and from **91a** (39 mg; 0.120 mmol) according to the General Procedure **Va** and after standard work-up the crude product was taken to the next step.
ESI-MS m/z for C₃₂H₃₆F₃N₄O₅S found 645.7 [M+H]⁺; Rt= 1.74 min

### Step 4.

Synthesis of 6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione 2,2,2-trifluoroacetate (**92**).

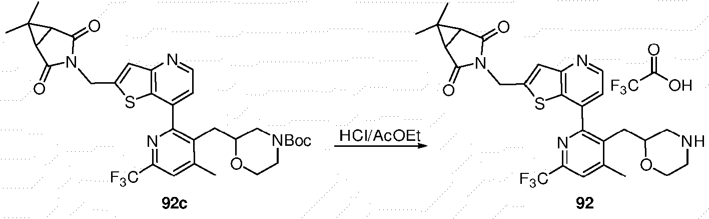

The title compound (**92**) was obtained as a TFA salt from **92c** (the crude product) according to the General Procedure **IVa** in 7% yield (per two steps)(2 mg; 0.003 mmol). The crude product was purified by preparative reversed-phase column chromatography (C-18, water/MeCN + 1‰ TFA, 99:1 to 35:65, 30 min, 20 mL/min).
ESI-MS m/z for C₂₇H₂₈F₃N₄O₃S found 545.7 [M+H]⁺; Rt= 1.04 min; ¹H NMR (700 MHz, D₂O) δ 8.91 (d, *J =* 5.6 Hz, 1H), 8.01 (s, 1H), 7.82 (d, *J =* 6.3 Hz, 1H), 7.78 (s, 1H), 5.01 (s, 2H), 3.79 - 3.65 (m, 2H), 3.46 (t, *J =* 12.7 Hz, 1H), 3.26 (d, *J =* 13.0 Hz, 1H), 3.19 (d, *J =* 13.5 Hz, 1H), 3.14-3.09 (m, 1H), 3.07-2.98 (m, 1H), 2.97 - 2.88 (m, 1H), 2.77 (t, *J =* 12.0 Hz, 1H), 2.69 (s, 2H), 2.65 (s, 3H), 1.29 (s, 3H), 1.10 (s, 3H).

### Example 94.

Synthesis of 6-(2-((6,6-dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitrile 2,2,2-trifluoroacetate (**94**).

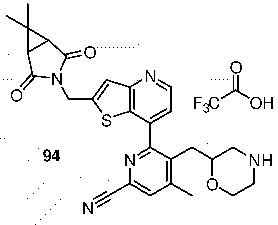

The title compound (**94**) was obtained as a TFA salt in 1% overall yield in a similar way to Example 92 with the exception that, in the first step of the synthesis, 5-amino-4-bromo-6-methylpicolinonitrile (**27a**) was used instead of compound **28a** and in the last step of the synthesis, the crude product was purified by preparative reversed-phase column chromatography (C-18, water/MeCN + 1‰ TFA, 99:1 to 40:60, 30 min, 20 mL/min).
ESI-MS m/z for C₂₇H₂₈N₅O₃S found 502.4 [M+H]⁺; Rₜ = 0.90 min; ¹H NMR (700 MHz, D₂O) δ 8.93 - 8.91 (m, 1H), 8.05 (s, 1H), 7.82-7.80 (m, 1H), 7.79 (s, 1H), 5.03 (s, 2H), 3.75 - 3.68 (m, 2H), 3.46 (t, J = 12.9 Hz, 1H), 3.26 (d, J = 13.0 Hz, 1H), 3.19 (d, J = 12.8 Hz, 1H), 3.11 (d, J = 14.9 Hz, 1H), 3.03 - 2.90 (m, 2H), 2.76 (t, J = 12.0 Hz, 1H), 2.71 (s, 2H), 2.63 (s, 3H), 1.29 (s, 3H), 1.13 (s, 3H).

### Example 95.

Synthesis of 6,6-dimethyl-3-((7-(4-methyl-3-(((*S*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**95**).

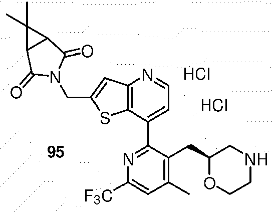

The title compound (**95**) was obtained as a dihydrochloride salt in 6% overall yield in a similar way to Example 91 with the exception that, in the first step of the synthesis, *tert*-butyl (*R*)-2-(bromomethyl)morpholine-4-carboxylate was used instead of *tert*-butyl 2-(bromomethyl)morpholine-4-carboxylate, in the second step of the synthesis, compound **92b** was used instead of compound **88a** and in the last step of the synthesis, the crude product was purified by preparative reversed-phase column chromatography (C-18, water + 0.3‰ HCl (12 M)/MeCN, 99:1 to 40:60, 30 min, 20 mL/min).
ESI-MS m/z for C₂₇H₂₈F₃N₄O₃S found 545.5 [M+H]⁺; Rt= 1.03 min;
¹H NMR (700 MHz, D₂O) δ 8.98 - 8.95 (m, 1H), 8.05 - 8.03 (m, 1H), 7.98 - 7.96 (m, 1H), 7.85 - 7.82 (m, 1H), 5.05 (s, 2H), 3.75 (t, *J =* 10.4 Hz, 1H), 3.71 - 3.67 (m, 1H), 3.50 - 3.44 (m, 1H), 3.32 (d, *J =* 12.7 Hz, 1H), 3.22 - 3.14 (m, 2H), 3.02 (dd, *J =* 15.0, 9.9 Hz, 1H), 2.94 (td, *J =* 12.5, 3.8 Hz, 1H), 2.79 (t, *J =* 12.1 Hz, 1H), 2.71 (s, 2H), 2.66 (s, 3H), 1.29 (s, 3H), 1.12 (s, 3H).

### Example 96.

Synthesis of 6,6-dimethyl-3-((7-(4-methyl-3-(((*R*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**96**).

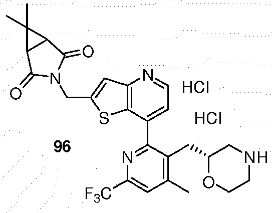

The title compound (**96**) was obtained as a dihydrochloride salt in 3% overall yield in a similar way to Example 91 with the exception that, in the first step of the synthesis, *tert*-butyl (S)-2-(bromomethyl)morpholine-4-carboxylate was used instead of *tert*-butyl 2-(bromomethyl)morpholine-4-carboxylate, in the second step of the synthesis, compound **92b** was used instead of compound **88a** and in the last step of the synthesis, the crude product was purified by preparative reversed-phase column chromatography (C-18, water + 0.3‰ HCl (12 M)/MeCN, 99:1 to 40:60, 30 min, 20 mL/min).
ESI-MS m/z for C₂₇H₂₈F₃N₄O₃S found 545.4 [M+H]⁺; Rt= 1.04 min; ¹H NMR (700 MHz, D₂O) δ 8.98 - 8.95 (m, 1H), 8.03 (s, 1H), 7.98 - 7.93 (m, 1H), 7.84 - 7.81 (m, 1H), 5.04 (s, 2H), 3.77 - 3.67 (m, 2H), 3.47 (td, *J =* 12.9, 2.1 Hz, 1H), 3.31 (d, *J =* 12.8 Hz, 1H), 3.22 - 3.13 (m, 2H), 3.06 - 2.99 (m, 1H), 2.97- 2.91 (m, 1H), 2.81 - 2.76 (m, 1H), 2.70 (s, 2H), 2.66 (s, 3H), 1.29 (s, 3H), 1.11 (s, 3H).

### Example 97.

Synthesis of 6,6-dimethyl-3-((7-(4-methyl-3-(((*S*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**97**).

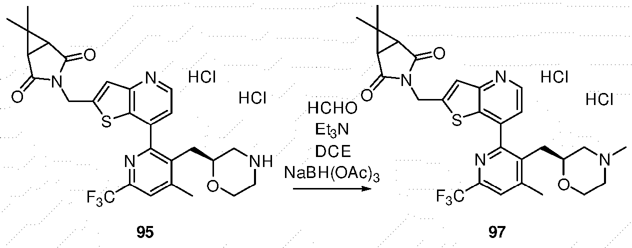

To the solution of **95** (12 mg; 0.019 mmol) in DCE (0.5 mL) Et₃N (0.008 mL; 0.057 mmol) and formalin (36% aqueous solution; 0.003 mL; 0.038 mmol) were added and this mixture was stirred at room temperature for 1 hour. Then to this mixture NaBH(OAc)₃ (8 mg; 0.038 mmol) was added and whole was stirred at room temperature for 1 hour. The reaction progress was monitored by LC-MS. When analysis indicated completion of the reaction, the mixture was diluted with DCM and washed with 5% NaHCO₃. The organic layer was dried over anhydrous MgSO₄, filtered and concentrated *in vacuo* and the residue was purified by preparative reversed-phase column chromatography (C-18, water + 0.3‰ HCl (36%)/MeCN, 99:1 to 40:60, 30 min, 20 mL/min). The title compound (**97**) was obtained as a dihydrochloride salt in 31% yield (4 mg; 0.006 mmol).
ESI-MS m/z for C₂₈H₃₀F₃N₄O₃S found 559.3 [M+H]⁺; Rt= 1.02 min; ¹H NMR (700 MHz, D₂O) δ 8.98 (d, *J =* 5.7 Hz, 1H), 8.03 (s, 1H), 8.01 (d, *J =* 5.6 Hz, 1H), 7.85 (s, 1H), 5.04 (s, 2H), 3.74 - 3.68 (m, 2H), 3.67 - 3.47 (m, 2H), 3.32 (d, *J =* 12.7 Hz, 1H), 3.16 (dd, *J =* 15.1, 3.2 Hz, 1H), 3.04 - 2.97 (m, 1H), 2.90 - 2.83 (m, 1H), 2.82 (s, 3H), 2.75 - 2.69 (m, 1H), 2.69 (s, 2H), 2.64 (s, 3H), 1.27 (s, 3H), 1.10 (s, 3H).

### Example 98.

Synthesis of 6,6-dimethyl-3-((7-(4-methyl-3-(((*R*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione dihydrochloride (**98**).

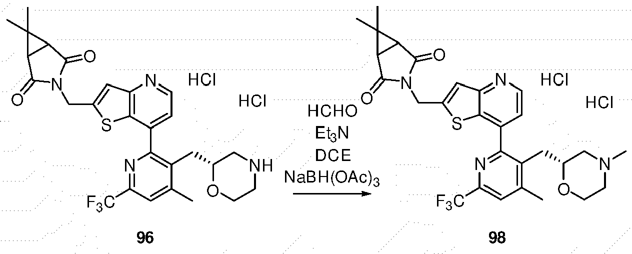

To the solution of **96** (23 mg; 0.037 mmol) in DCE (1 mL) Et₃N (0.016 mL; 0.112 mmol) and formalin (36% aqueous solution; 0.006 mL; 0.074 mmol) were added and this mixture was stirred at room temperature for 1 hour. Then to this mixture NaBH(OAc)₃ (16 mg; 0.076 mmol) was added and whole was stirred at room temperature for 1 hour. The reaction progress was monitored by LC-MS. When analysis indicated completion of the reaction, the mixture was diluted with DCM and washed with 5% NaHCO₃. The organic layer was dried over anhydrous MgSO₄, filtered and concentrated *in vacuo* and the residue was purified by preparative reversed-phase column chromatography (C-18, water + 0.3‰ HCl (36%)/MeCN, 99:1 to 40:60, 30 min, 20 mL/min). The title compound (**98**) was obtained as a dihydrochloride salt in 72% yield (17 mg; 0.027 mmol).
ESI-MS m/z for C₂₈H₃₀F₃N₄O₃S found 559.2 [M+H]⁺; Rt= 1.03 min; ¹H NMR (700 MHz, D₂O) δ 8.92 (d, *J =* 5.5 Hz, 1H), 8.01 (s, 1H), 7.86 (d, *J =* 5.5 Hz, 1H), 7.79 (s, 1H), 5.02 - 5.00 (m, 2H), 3.77 - 3.73 (m, 1H), 3.71 - 3.65 (m, 1H), 3.47 - 3.40 (m, 1H), 3.40 - 3.36 (m, 1H), 3.33 (d, *J* = 12.8 Hz, 1H), 3.13 (dd, *J =* 15.0, 3.6 Hz, 1H), 3.05 - 3.00 (m, 1H), 2.88 (td, *J =* 12.4, 3.8 Hz, 1H), 2.81 (s, 3H), 2.73-2.68 (m, 1H), 2.69 (s, 2H), 2.64 (s, 3H), 1.28 (s, 3H), 1.10 (s, 3H).

### Example 104.

Synthesis of 3-((7-(3-((*S*)-3-(difluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**104**).

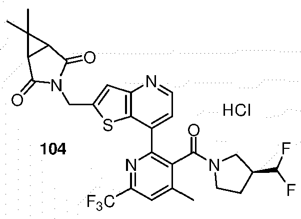

The title compound (**104**) was obtained as a hydrochloride salt in 11% overall yield in a similar way to Example 48 with the exception that, in the first step of the synthesis (*S*)-3-(difluoromethyl)pyrrolidine hydrochloride was used instead of a morpholine and an acid **II** was used instead of 2-chloro-4-methylnicotinic acid and in the last step of the synthesis the crude product was purified by flash column chromatography on silica (hexane, 100%, 15 min) and then by preparative reversed-phase column chromatography (C-18, water + 0.3‰ HCl (36%)/MeCN, 99:1 to 20:80, 30 min, 20 mL/min).
ESI-MS m/z for C₂₈H₂₆F₅N₄O₃S found 593.2 [M+H]⁺; Rt = 1.45 min; ¹H NMR (700 MHz, Methanol-*d*₄) δ 8.76-8.72 (m, 1H), 8.00 - 7.97 (m, 1H), 7.72-7.68 (m, 1H), 7.58-7.55(m, 1H), 6.03-5.17 (m, 1H), 4.91-4.89 (m, 2H), 3.80-3.63 (m, 1H), 3.58-3.41 (m, 1H), 3.16-3.00 (m, 1H), 2.74-2.55 (m, 1H), 2.54-2.51 (m, 3H), 2.50-2.47 (m, 2H), 2.09-1.82 (m, 1H), 1.76-1.43 (m, 1H), 1.33-1.27 (m, 1H), 1.24-1.21 (m, 3H), 1.10-1.04 (m, 3H).

### Example 105.

Synthesis of 3-((7-(3-((*S*)-3-(fluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**105**).

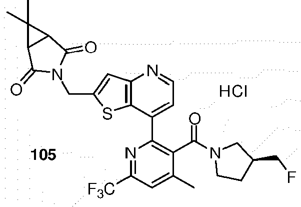

The title compound (**105**) was obtained as a hydrochloride salt in 6% overall yield in a similar way to Example 48 with the exception that, in the first step of the synthesis (*S*)-3-(fluoromethyl)pyrrolidine hydrochloride was used instead of a morpholine and an acid **II** was used instead of 2-chloro-4-methylnicotinic acid and in the last step of the synthesis the crude product was purified by preparative reversed-phase column chromatography (C-18, water + 0.3‰ HCl (36%)/MeCN, 99:1 to 20:80, 30 min, 20 mL/min).
ESI-MS m/z for C₂₈H₂₇F₄N₄O₃S found 575.2 [M+H]⁺, Rₜ = 1.47 min; ¹H NMR (700 MHz, Methanol-*d*₄) δ 8.81-8.76 (m, 1H), 8.02-7.99 (m, 1H), 7.80-7.74 (m, 1H), 7.61-7.56 (m, 1H), 4.92-4.89 (m, 2H), 4.47-4.28 (m, 1H), 4.26-4.02 (m, 1H), 4.00-3.75 (m, 1H), 3.66-3.50 (m, 2H), 3.16-2.98 (m, 1H), 2.91-2.70 (m, 1H), 2.55-2.51 (m, 3H), 2.50-2.46 (m, 2H), 2.04-1.85 (m, 1H), 1.77-1.50 (m, 1H), 1.25-1.21 (m, 3H), 1.11-1.04 (m, 3H).

### Example 106.

Synthesis of 3-((7-(3-((3*S*,4*S*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**106**).

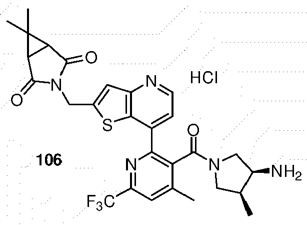

The title compound (**106**) was obtained as a hydrochloride salt in 8% overall yield in a similar way to Example 45 with the exception that, in the first step of the synthesis an acid **II** and *tert*-butyl ((3*S*,4*S*)-4-methylpyrrolidin-3-yl)carbamate were used instead of 2-chloro-4-methylnicotinic acid and *tert*-butyl piperazine-1-carboxylate and in the last step of the synthesis the crude product was purified by preparative reversed-phase column chromatography (C-18, water + 0.3‰ HCl (36%)/MeCN, 98:2 to 30:70, 30 min, 20 mL/min).
ESI-MS m/z for C₂₈H₂₉F₃N₅O₃S found 572.3 [M+H]⁺, Rt= 1.11 min; ¹H NMR (700 MHz, Methanol-*d*₄) δ 8.98 - 8.83 (m, 1H), 8.15 - 8.07 (m, 1H), 8.05 - 7.85 (m, 1H), 7.74 - 7.67 (m, 1H), 5.03 - 4.95 (m, 2H), 4.20 - 3.93 (m, 1H), 3.89 - 3.78 (m, 1H), 3.73 - 3.60 (m, 1H), 3.53 - 3.45 (m, 1H), 3.16 - 2.94 (m, 1H), 2.66 - 2.60 (m, 2H), 2.58 - 2.52 (m, 3H), 2.52 - 2.28 (m, 1H), 1.39 - 1.23 (m, 3H), 1.23 - 1.11 (m, 3H), 1.08 - 0.42 (m, 3H).

### Example 107.

Synthesis of 3-((7-(3-((3*S*,4*R*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**107**).

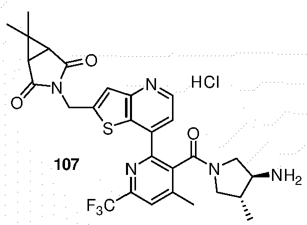

The title compound (**107**) was obtained as a hydrochloride salt in 9% overall yield in a similar way to Example 45 with the exception that, in the first step of the synthesis an acid **II** and *tert*-butyl ((3*S*,4*R*)-4-methylpyrrolidin-3-yl)carbamate hydrochloride were used instead of 2-chloro-4-methylnicotinic acid and *tert*-butyl piperazine-1-carboxylate and in the last step of the synthesis the crude product was purified by preparative reversed-phase column chromatography (C-18, water + 0.3‰ HCl (36%)/MeCN, 98:2 to 30:70, 30 min, 20 mL/min).
ESI-MS m/z for C₂₈H₂₉F₃N₅O₃S found 572.1 [M+H]⁺, Rₜ = 1.12 min; ¹H NMR (700 MHz, Methanol-*d*₄) δ 8.96 - 8.75 (m, 1H), 8.10 - 8.05 (m, 1H), 7.95 - 7.80 (m, 1H), 7.68 - 7.61 (m, 1H), 4.98 - 4.92 (m, 2H), 4.30 - 3.92 (m, 1H), 3.69 - 3.46 (m, 1H), 3.38 - 3.36 (m, 1H), 3.30-3.01 (m, 1H), 2.64 - 2.56 (m, 3H), 2.56 - 2.50 (m, 2H), 2.40 - 2.04 (m, 2H), 1.31 - 1.26 (m, 3H), 1.20 - 1.13 (m, 3H), 1.04 - 0.45 (m, 3H).

### Example 109.

Synthesis of (2S)-2-amino-N-((3S)-1-(2-(2-((6,6-dimethyl-2,4-dioxo-3-azabicyclo[3.1.0]hexan-3-yl)methyl)thieno[3,2-b]pyridin-7-yl)-4-methyl-6-(trifluoromethyl)nicotinoyl)pyrrolidin-3-yl)-3-methylbutanamide dihydrochloride (109).

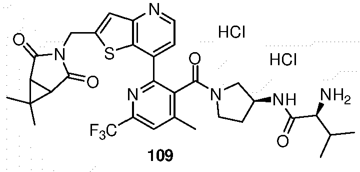

### Step 1.

Synthesis of tert-butyl ((2S)-1-(((3S)-1-(2-(2-((6,6-dimethyl-2,4-dioxo-3-azabicyclo[3.1.0]hexan-3-yl)methyl)thieno[3,2-b]pyridin-7-yl)-4-methyl-6-(trifluoromethyl)nicotinoyl)pyrrolidin-3-yl)amino)-3-methyl-1-oxobutan-2-yl)carbamate (109a).

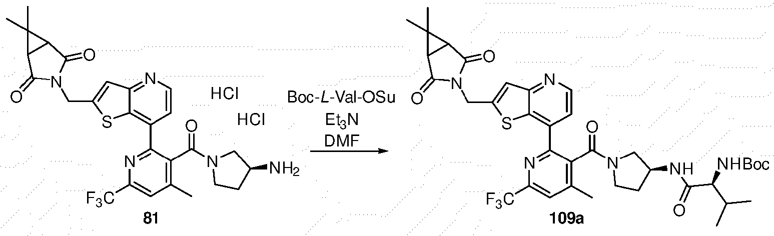

To a stirred solution of **81** (25 mg; 0.04 mmol) in DMF (0.5 mL), Boc-*L*-Val-OSu (14 mg; 0.04 mmol) and Et₃N (28 µL;0.20 mmol) were added and the resulting mixture was stirred at room temperature overnight. The reaction progress was monitored by LC-MS. When analysis indicated completion of the reaction, DMF was evaporated under reduced pressure. The residue was dissolved in AcOEt (20 mL) and washed with 5% NaHCO₃ (1 x 10 mL), 1 M KHSO₄ (1 x 10 mL) and water (1 x 10 mL). The organic phase was dried over anhydrous NaSO₄, filtered and concentrated *in vacuo.* The crude product was used to the next step without additional purification. The title compound (**109a**) was obtained as a colorless oil in 99% yield (29 mg; 0.04 mmol).
ESI-MS m/z for C₃₇H₄₄F₃N₆O₆S found 757.1 [M+H]⁺; Rt= 1.57 min

### Step 2.

Synthesis of (2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)-3-methylbutanamide dihydrochloride (**109**).

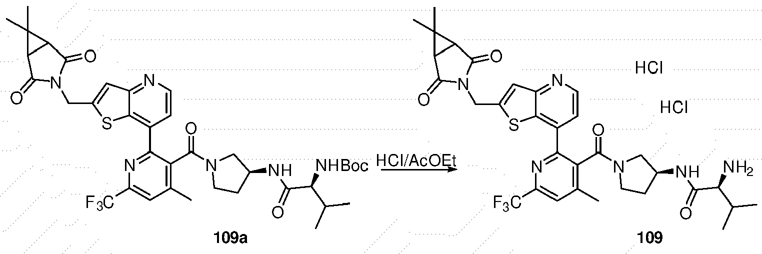

The title compound (**109**) was obtained as a white solid as a dihydrochloride salt from **109a** (29 mg; 0.040 mmol) according to the General Procedure **IVa** in in 85% yield (25 mg; 0.034 mmol). The crude product was purified by preparative reversed-phase column chromatography (C-18, water + 0.3‰ HCl (36%)/MeCN, 98:2 to 30:70, 30 min, 20 mL/min). ESI-MS m/z for C₃₂H₃₆F₃N₆O₄S found 657.1 [M+H]⁺, Rₜ= 1.09 min; ¹H NMR (700 MHz, D₂O) δ 8.92 - 8.82 (m, 1H), 8.18 - 8.12 (m, 1H), 7.88 - 7.69 (m, 2H), 5.06 - 4.99 (m, 2H), 4.39 - 3.90 (m, 1H), 3.83 - 3.67 (m, 1H), 3.66 - 3.51 (m, 1H), 3.47 - 3.20 (m, 1H), 2.96 - 2.76 (m, 1H), 2.72 - 2.69 (m, 2H), 2.58 - 2.54 (m, 3H), 2.32 - 2.20 (m, 1H), 2.18 - 2.02 (m, 1H), 1.99 - 1.86 (m, 1H), 1.83 - 1.60 (m, 1H), 1.30 - 1.25 (m, 3H), 1.15 - 1.04 (m, 4H), 0.96 - 0.87 (m, 3H), 0.57 - 0.51 (m, 2H).

### Example 110.

Synthesis of (2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)propanamide dihydrochloride (**110**).

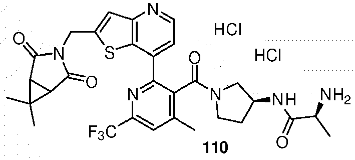

The title compound (**110**) was obtained as a dihydrochloride salt in 81% overall yield in a similar way to Example 109 with the exception that, in the first step of the synthesis Boc-*L-*Ala-OSu was used instead of Boc-*L*-Val-OSu and in the last step of the synthesis the crude product was purified by preparative reversed-phase column chromatography (C-18, water + 0.3‰ HCl (36%)/MeCN, 98:2 to 30:70, 30 min, 20 mL/min).
ESI-MS m/z for C₃₀H₃₂F₃N₆O₄S found 629.1 [M+H]⁺, Rₜ= 1.05 min; ¹H NMR (700 MHz, D₂O) δ 8.89 - 8.80 (m, 1H), 8.17 - 8.11 (m, 1H), 7.78 - 7.66 (m, 2H), 5.05 - 4.95 (m, 2H), 4.33 - 3.76 (m, 2H), 3.69 - 3.55 (m, 1H), 3.45 - 3.19 (m, 2H), 3.03 - 2.73 (m, 1H), 2.72 - 2.67 (m, 2H), 2.60 - 2.52 (m, 3H), 2.31 - 1.97 (m, 2H), 1.87 - 1.78 (m, 1H), 1.58 - 1.45 (m, 2H), 1.37 - 1.24 (m, 3H), 1.14 - 0.94 (m, 3H).

### Example 112.

Synthesis of 3-((7-(3-(4-aminopiperidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**112**).

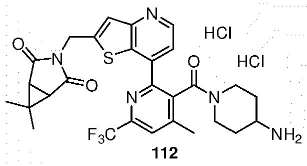

The title compound (**112**) was obtained as a dihydrochloride salt in 35% overall yield in a similar way to Example 45 with the exception that, in the first step of the synthesis an acid **II** and *tert-*butyl piperidin-4-ylcarbamate were used instead of 2-chloro-4-methylnicotinic acid and *tert-*butyl piperazine-1-carboxylate and in the last step of the synthesis the crude product was purified by preparative reversed-phase column chromatography (C-18, water + 0.3‰ HCl (36%)/MeCN, 99:1 to 30:70, 30 min, 20 mL/min).
ESI-MS m/z for C₂₈H₂₉F₃N₅O₃S found 572.1 [M+H]⁺, Rₜ = 1.12 min; ¹H NMR (700 MHz, D₂O) δ 8.79 - 8.75 (m, 1H), 8.06 - 8.00 (m, 1H), 7.74 - 7.57 (m, 2H), 4.96 - 4.87 (m, 2H), 4.65 - 4.59 (m, 1H), 3.35 - 3.12 (m, 2H), 2.98 - 2.73 (m, 1H), 2.61 (d, *J =* 2.8 Hz, 2H), 2.57 - 2.44 (m, 4H), 2.19 - 2.06 (m, 1H), 1.83 - 1.64 (m, 1H), 1.57 - 1.45 (m, 1H), 1.20 (s, 3H), 1.15 (ddd, *J* = 16.9, 12.7, 4.6 Hz, 1H), 1.01 (d, *J* = 14.5 Hz, 3H).

### Example 113.

Synthesis of 3-((7-(3-((*S*)-3-hydroxypyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**113**).

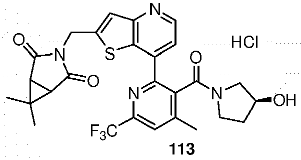

The title compound (**113**) was obtained as a hydrochloride salt in 10% overall yield in a similar way to Example 48 with the exception that, in the first step of the synthesis (S)-3-hydroxypyrrolidine was used instead of a morpholine and an acid **II** was used instead of 2-chloro-4-methylnicotinic acid and in the last step of the synthesis the crude product was purified by preparative reversed-phase column chromatography (C-18, water + 0.3‰ HCl (36%)/MeCN, 99:1 to 25:75, 30 min, 18 mL/min).
ESI-MS m/z for C₂₇H₂₆F₃N₄O₄S found 559.3 [M+H]⁺, Rt= 1.25 min; ¹H NMR (700 MHz, DMSO-*d*₆) δ 8.81 - 8.69 (m, 1H), 8.14 - 8.09 (m, 1H), 7.72 - 7.50 (m, 2H), 4.82 (s, 2H), 4.28 - 4.12 (m, 1H), 3.96 - 3.61 (m, 1H), 3.22 - 2.97 (m, 1H), 2.71 - 2.61 (m, 1H), 2.60 - 2.58 (m, 2H), 2.48 - 2.45 (m, 3H), 2.00 - 1.57 (m, 2H), 1.53 - 1.26 (m, 1H), 1.21 - 1.14 (m, 3H), 1.02 (dd, *J* = 19.4, 14.4 Hz, 3H).

### Example 114.

Synthesis of 3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**114**).

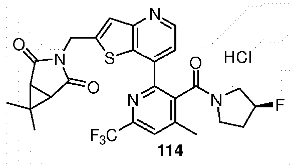

### Step 1.

Synthesis of 7-chloro-2-(((tetrahydro-2*H*-pyran-2-yl)oxy)methyl)thieno[3,2-b]pyridine (**114a**).

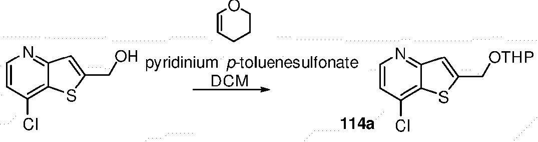

To a solution of (7-chlorothieno[3,2-*b*]pyridin-2-yl)methanol (0.6 g; 3.01 mmol) in anhydrous DCM (17 mL) 3,4-dihydro-2*H*-pyran (0.41 mL; 4.51 mmol) and pyridinium p-toluenesulfonate (0.76 g; 3.01 mmol) were added and the reaction mixture was stirred at room temperature overnight. Then another part of 3,4-dihydro-2*H*-pyran (0.27 mL; 3.01 mmol) was added and the reaction mixture was stirred at room temperature additional day. The reaction progress was monitored by LC-MS. When analysis indicated completion of the reaction, it was washed with an aqueous saturated solution of Na₂CO₃ (1 x 15 mL). The organic phase was dried over anhydrous Na₂SO₄, filtered and concentrated *in vacuo* and the crude product was purified by flash column chromatography on silica (hexane/AcOEt, 100:0 to 0:100, *v*/*v*). The title compound (**114a**) was obtained as a colorless oil in 96% yield (0.82 g; 2.88 mmol).
ESI-MS m/z for C₁₃H₁₅ClNO₂S found 284.0/286.0 [M+H]⁺; Rₜ = 1.55 min; ¹H NMR (700 MHz, CDCl₃) δ 8.55 (d, *J* = 5.1 Hz, 1H), 7.45 (t, *J =* 1.1 Hz, 1H), 7.24 (d, *J* = 5.1 Hz, 1H), 5.03 (dd, *J* = 13.4, 1.2 Hz, 1H), 4.86 (dd, *J =* 13.4, 1.0 Hz, 1H), 4.80 (t, *J =* 3.4 Hz, 1H), 3.96 - 3.89 (m, 1H), 3.61 - 3.56 (m, 1H), 1.92 - 1.84 (m, 1H), 1.80 - 1.74 (m, 1H), 1.73 - 1.68 (m, 1H), 1.65 - 1.61 (m, 1H), 1.59 - 1.54 (m, 2H).

### Step 2.

Synthesis of (2-(((tetrahydro-2*H*-pyran-2-yl)oxy)methyl)thieno[3,2-*b*]pyridin-7-yl)boronic acid (**114b**).

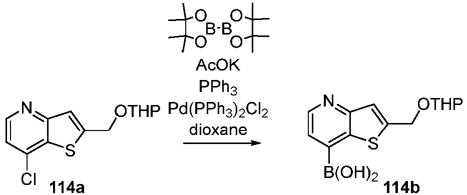

In a Schlenk flask was placed the compound **114a** (100 mg; 0.35 mmol), potassium acetate (104 mg; 0.42 mmol), Pd(PPh₃)₂Cl₂ (25 mg; 0.035 mmol), PPh₃ (19 mg; 0.07 mmol) and anhydrous, degassed dioxane (2 mL). The flask was backfilled with an argon and stirred for 10 minutes. Then bis(pinacolo)diboron (25 mg; 0.035 mmol) was added and the resulting mixture was stirred at 90 °C overnight. The reaction progress was monitored by LC-MS. When analysis indicated completion of the reaction, the mixture was cooled to room temperature and the product was not isolated. It was used to the next step without purification.
ESI-MS m/z for C₁₃H₁₇BNO₄S found 293.9 [M+H]⁺, Rₜ = 1.04 min

### Step 3.

Synthesis of (*S*)-(2-chloro-4-methyl-6-(trifluoromethyl)pyridin-3-yl)(3-fluoropyrrolidin-1-yl)methanone (**114c**).

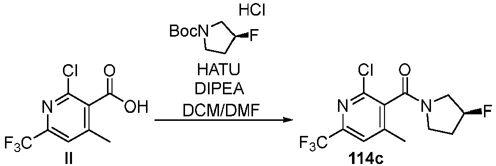

The title compound (**114c**) was obtained from an acid **II** (100 mg; 0.42 mmol) and from *tert-*butyl (*S*)-3-fluoropyrrolidine-1-carboxylate hydrochloride (58 mg; 0.46 mmol) according to the General Procedure **I** as a colorless oil in 99% yield (128 mg; 0.41 mmol).
ESI-MS m/z for C₁₂H₁₂ClF₄N₂O found 310.9 [M+H]⁺; Rₜ = 1.21 min; ¹H NMR (700 MHz, Methanol-*d*₄) δ 7.86 - 7.78 (m, 1H), 5.49 - 5.22 (m, 1H), 4.10 - 3.90 (m, 1H), 3.85 - 3.68 (m, 1H), 3.63 - 3.37 (m, 2H), 2.47 - 2.42 (m, 3H), 2.41 - 2.15 (m, 2H).

### Step 4.

Synthesis of ((*S*)-3-fluoropyrrolidin-1-yl)(4-methyl-2-(2-(((tetrahydro-2*H*-pyran-2-yl)oxy)methyl)thieno[3,2-*b*]pyridin-7-yl)-6-(trifluoromethyl)pyridin-3-yl)methanone (**114d**).

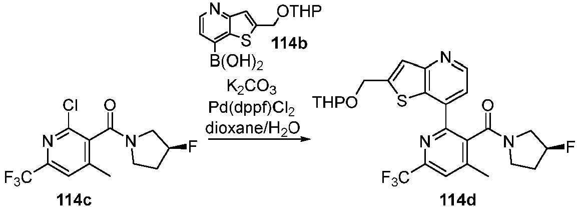

The title compound (**114d**) was obtained from **114b** (the crude product) and from **114c** (109 mg; 0.35 mmol) according to the General Procedure **Va** as a yellow oil in 70% yield (130 mg; 0.25 mmol).
ESI-MS m/z for C₂₅H₂₆F₄N₃O₃S found 524.2 [M+H]⁺; Rt= 1.61 min

### Step 5.

Synthesis of (*S*)-(3-fluoropyrrolidin-1-yl)(2-(2-(hydroxymethyl)thieno[3,2-b]pyridin-7-yl)-4-methyl-6-(trifluoromethyl)pyridin-3-yl)methanone (**114e**).

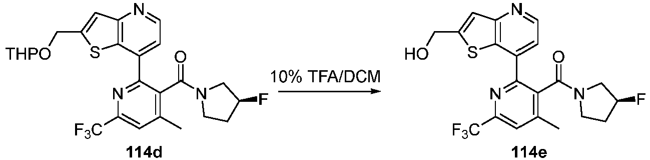

The solution of **114d** (100 mg; 0.19 mmol) in DCM (9 mL) and TFA (1 mL) was stirred at room temperature for 3 h. The reaction progress was monitored by LC-MS. When analysis indicated completion of the reaction, the mixture of TFA/DCM was removed *in vacuo* and the residue was redissolved in AcOEt (20 mL) and washed with 5% NaHCO₃ (1 x 20 mL). The organic phase was dried over anhydrous Na₂SO₄, filtered and concentrated *in vacuo.* The crude product was purified by flash column chromatography on silica (hexane/AcOEt, 100:0 to 0:100, v/v, then: AcOEt/MeOH, 90:10, *v*/*v*). The title compound (**114e**) was obtained as a colorless oil in 71% yield (60 mg; 0.14 mmol).
ESI-MS m/z for C₂₀H₁₈F₄N₃O₂S found 440.0 [M+H]⁺; Rₜ = 1.17 min

### Step 6.

Synthesis of (*S*)-(7-(3-(3-fluoropyrrolidine-1-carbonyl)-4-methyl-6-(trifluoromethyl)pyridin-2-yl)thieno[3,2-*b*]pyridin-2-yl)methyl methanesulfonate (**114f**).

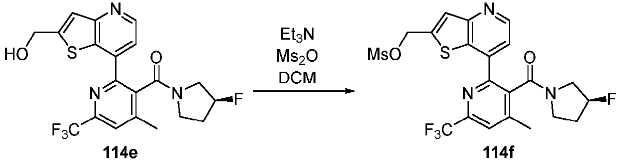

The title compound (**114f**) was obtained as a brown oil from **114e** (50 mg; 0.11 mmol) according to the General Procedure **VIII** in 98% yield (58 mg; 0.11 mmol).
ESI-MS m/z for C₂₁H₂₀F₄N₃O₄S₂ found 518.0 [M+H]⁺; Rₜ = 1.38 min

### Step 7.

Synthesis of 3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**114**).

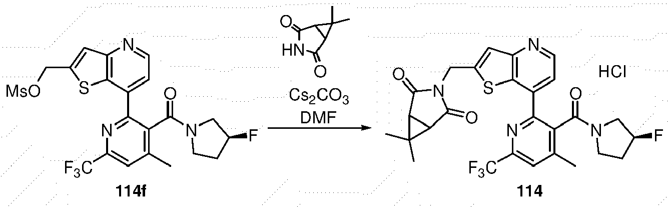

To a solution of **114f** (55 mg; 0.11 mmol) in anhydrous DMF (3 mL), 6,6-dimethy-3-aza-bicyclo[3.1.0]hexane 2,4-dione (16 mg; 0.12 mmol) and Cs₂CO₃ (69 mg; 0.21 mmol) were added. The resulting mixture was stirred at room temperature overnight. The reaction progress was monitored by LC-MS. When analysis indicated completion of the reaction, DMF was removed *in vacuo* and the residue was dissolved in AcOEt (20 mL) and washed with water (1 x 15 mL). The organic phase was dried over anhydrous Na₂SO₄, filtered and concentrated *in vacuo.* The crude product was purified by preparative reversed-phase column chromatography (C-18, water + 0.3 HCl (36%)/MeCN, 98:2 to 20:80, 30 min, 20 mL/min). The title compound

(**114**) was obtained as a hydrochloride salt as a white solid in 33% yield (21 mg; 0.035 mmol). ESI-MS m/z for C₂₇H₂₅F₄N₄O₃S found 561.3 [M+H]⁺; Rₜ = 1.48 min; ¹H NMR (700 MHz, Methanol-*d*₄) δ8.90 - 8.81 (m, 1H), 8.10 - 8.06 (m, 1H), 7.93 - 7.86 (m, 1H), 7.69 - 7.63 (m, 1H), 5.33 - 5.03 (m, 1H), 5.00 - 4.96 (m, 2H), 4.12 - 3.54 (m, 2H), 3.44 - 3.16 (m, 1H), 2.98 - 2.63 (m, 1H), 2.60 - 2.56 (m, 3H), 2.56 - 2.52 (m, 2H), 2.32 - 1.87 (m, 2H), 1.28 (dd, *J =* 21.7, 14.5 Hz, 3H), 1.13 (dd, *J =* 21.7, 14.5 Hz, 3H).

### Example 115.

Synthesis of 3-((7-(3-((*R*)-3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**115**).

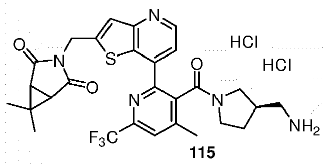

### Step 1.

Synthesis of (*S*)-*tert*-butyl 3-(((methylsulfonyl)oxy)methyl)pyrrolidine-1-carboxylate (**115a**).

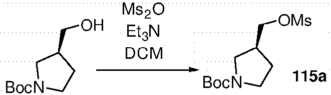

The title compound (**115a**) was obtained as a yellow oil from *tert*-butyl (*S*)-3-(hydroxymethyl)pyrrolidine-1-carboxylate (0.30 g; 1.49 mmol) according to the General Procedure **VIII** in 99% yield (0.41 mg; 1.49 mmol).
ESI-MS m/z for C₁₁H₂₁NO₅SNa found 302.0 [M+Na]⁺; Rt= 1.21 min; ¹H NMR (700 MHz, CDCl₃) δ 4.25 - 4.11 (m, 2H), 3.63 - 3.28 (m, 3H), 3.20 - 3.09 (m, 1H), 3.03 (s, 3H), 2.68 - 2.58 (m, 1H), 2.09 - 2.01 (m, 1H), 1.82 - 1.65 (m, 1H), 1.47 (s, 9H).

### Step 2.

Synthesis of (*S*)-*tert*-butyl 3-(azidomethyl)pyrrolidine-1-carboxylate (**115b**).

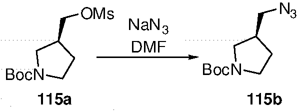

A compound **115a** (0.42 g; 1.49 mmol) and sodium azide (0.19 g; 2.98 mmol) in DMF (5 mL) were stirred at 70 °C overnight. The reaction progress was monitored by LC-MS. When analysis indicated completion of the reaction, the mixture was poured into a saturated solution of NaCl (20 mL) and extracted with Et₂O (3 x 20 mL). The combined organic layers were dried over anhydrous NaSO₄, filtered and concentrated *in vacuo.* The crude product was purified by flash column chromatography on silica (hexane/AcOEt, 100:0 to 0:100, *v*/*v*). The title compound (**115b**) was obtained as a colorless oil in 94% yield (0.31 mg; 1.4 mmol).
ESI-MS m/z for C₆H₁₁N₄O₂ found 171.2 [M+H-*t*Bu]⁺; Rₜ = 1.44 min; ¹H NMR (700 MHz, CDCl₃) δ 3.62 - 3.37 (m, 2H), 3.39 - 3.22 (m, 3H), 3.13 - 2.99 (m, 1H), 2.48 - 2.37 (m, 1H), 2.08 - 1.96 (m, 1H), 1.73 - 1.63 (m, 1H), 1.46 (s, 9H).

### Step 3.

Synthesis of (S)-3-(azidomethyl)pyrrolidine hydrochloride (115c).

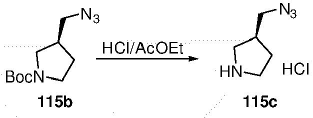

The title compounds (**115c**) was obtained as a hydrochloride salt as a colorless oil from **115b** (0.11 g; 0.49 mmol) according to the General Procedure **IVa** in 99% yield (79 mg; 0.48 mmol).
ESI-MS m/z for C₅H₁₁N₄ found 127.3 [M+H]⁺; Rt= 0.18 min; ¹H NMR (700 MHz, Methanol-*d*₄) δ 3.52 (ddd, *J* = 46.1, 12.4, 6.4 Hz, 2H), 3.43 (dd, *J =* 11.9, 8.1 Hz, 1H), 3.38 (ddd, *J =* 11.7, 8.4, 5.1 Hz, 1H), 3.30 - 3.24 (m, 1H), 3.03 (dd, *J =* 11.9, 7.8 Hz, 1H), 2.66 - 2.58 (m, 1H), 2.20 (dtd, *J =* 13.0, 7.8, 5.1 Hz, 1H), 1.80 (dq, *J =* 13.3, 8.2 Hz, 1H).

### Step 4.

Synthesis of (*S*)-(3-(azidomethyl)pyrrolidin-1-yl)(2-chloro-4-methyl-6-(trifluoromethyl)pyridin-3-yl)methanone (**115d**).

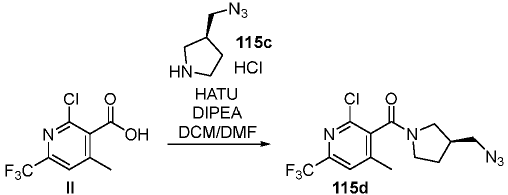

The title compound (**115d**) was obtained from an acid **II** (110 mg; 0.44 mmol) and from the compound **115c** (79 mg; 0.49 mmol) according to the General Procedure **I** as a colorless oil in 44% yield (68 mg; 0.2 mmol).
ESI-MS m/z for C₁₃H₁₄ClF₃N₅O found 348.0/350.0 [M+H]⁺; Rₜ = 1.42 min; ¹H NMR (700 MHz, CDCl₃) δ 7.53 - 7.49 (m, 1H), 3.90 - 3.82 (m, 1H), 3.72 - 3.63 (m, 1H), 3.45 - 3.39 (m, 2H), 3.32 - 3.27 (m, 1H), 3.14 - 3.07 (m, 1H), 2.63 - 2.56 (m, 1H), 2.43 - 2.40 (m, 3H), 2.21 - 2.13 (m, 1H), 1.87 - 1.80 (m, 1H).

### Step 5.

Synthesis of 3-((7-(3-((*S*)-3-(azidomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione (**115e**).

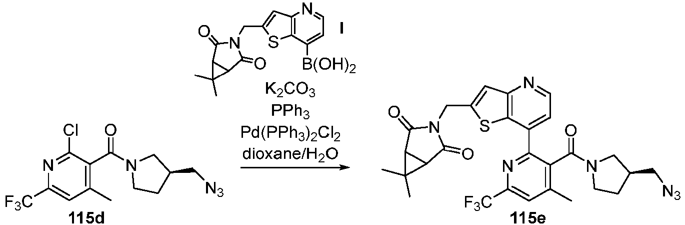

The title compound (**115e**) was obtained from **115d** (67 mg; 0.19 mmol) and from the boronic acid **I** (76 mg; 0.23 mmol) according to the General Procedure **Va.** The crude product was used to the next step without purification.
ESI-MS m/z for C₂₈H₂₇F₃N₇O₃S found 598.1 [M+H]⁺; Rt= 1.34 min

### Step 6.

Synthesis of 3-((7-(3-((*R*)-3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**115**).

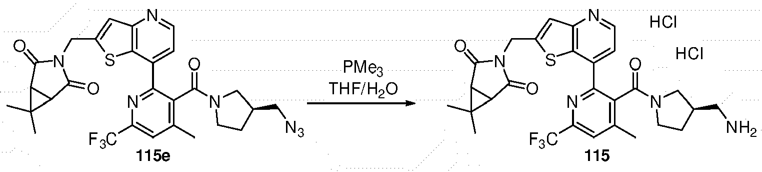

To the solution of **115e** (the crude product) in THF (1.8 mL) water (0.2 mL) and PMe₃ (1 M solution in THF; 0.58 mL; 0.58 mmol) were added and the mixture was stirred at room temperature overnight. The reaction progress was monitored by LC-MS. When analysis indicated completion of the reaction, the mixture was concentrated *in vacuo* and the residue was purified by preparative reversed-phase column chromatography (C-18, water + 0.3‰ HCl (36%)/MeCN, 98:2 to 30:70, 30 min, 20 mL/min). The title compound (115) was obtained as a dihydrochloride salt as a white solid in 6% yield (per two steps)(8 mg; 0.01 mmol).
ESI-MS m/z for C₂₈H₂₉F₃N₅O₃S found 572.3 [M+H]⁺; Rₜ = 1.10 min; ¹H NMR (700 MHz, D₂O) δ 8.83 - 8.73 (m, 1H), 8.09 - 8.02 (m, 1H), 7.76 - 7.59 (m, 2H), 4.97 - 4.87 (m, 2H), 3.93 - 3.60 (m, 1H), 3.55 - 3.23 (m, 1H), 3.13 - 2.84 (m, 2H), 2.78 - 2.64 (m, 1H), 2.63 - 2.58 (m, 2H), 2.56 - 2.37 (m, 4H), 2.30 - 2.09 (m, 1H), 1.95 - 1.70 (m, 1H), 1.59 - 1.40 (m, 1H), 1.22 - 1.16 (m, 3H), 1.04 - 0.92 (m, 3H).

### Example 117.

Synthesis of 3-((7-(3-((3*R*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione dihydrochloride (**117**).

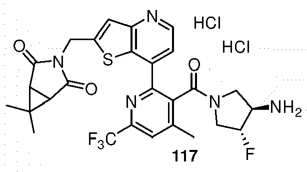

The title compound (**117**) was obtained as a dihydrochloride salt in 3% overall yield in a similar way to Example 45 with the exception that, in the first step of the synthesis an acid **II** and *tert*-butyl ((3*R*,4*R*)-4-fluoropyrrolidin-3-yl)carbamate hydrochloride were used instead of 2-chloro-4-methylnicotinic acid and *tert-*butyl piperazine-1-carboxylate and in the last step of the synthesis the crude product was purified by preparative reversed-phase column chromatography (C-18, water + 0.3‰ HCl (36%)/MeCN, 99:1 to 20:80, 30 min, 20 mL/min).
ESI-MS m/z for C₂₇H₂₆F₄N₅O₃S found 576.2 [M+H]⁺; Rₜ = 1.15 min; ¹H NMR (700 MHz, Methanol-*d*₄) δ 9.00 - 8.91 (m, 1H), 8.18 - 7.93 (m, 2H), 7.73 (dd, *J* = 9.5, 7.0 Hz, 1H), 5.40 - 5.14 (m, 1H), 5.04 - 4.94 (m, 2H), 4.49 - 3.14 (m, 5H), 3.10 - 2.65 (m, 2H), 2.60 - 2.49 (m, 3H), 1.32 - 1.23 (m, 3H), 1.21 - 1.08 (m, 3H).

### Example 121.

Synthesis of 3-((7-(6-chloro-4-methyl-3-(2-oxotetrahydropyrimidin-1(2*H*)-yl)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**121**).

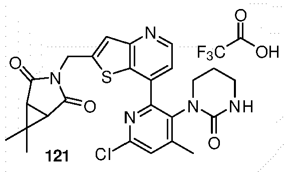

### Step 1.

Synthesis of 2-bromo-6-chloro-4-methylpyridin-3-amine (**121a**).

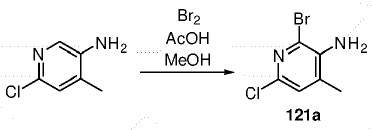

To the solution of 6-chloro-4-methylpyridin-3-amine (1.00 g; 7.01 mmol) in MeOH (10 mL) AcOH (0.80 mL; 14.02 mmol) was added and then the reaction mixture was cooled to 0 °C and then to this mixture a bromine (0.36 mL; 7.01 mmol) was added dropwise and the resulting mixture was stirred at room temperature for 1.5 hours. The reaction progress was monitored by TLC and LC-MS. When analyses indicated almost completion of the reaction, to this mixture 10% Na₂S₂O₃ was added and then a whole was stirred for a time necessary to obtain a precipitate. The precipitate was filtered off and dried in oven overnight. The title compound (**121a**) was obtained in 93% yield (1.43 g; 6.50 mmol).
ESI-MS m/z for C₆H₇BrClN₂ found 220.9/222.9 [M+H]⁺; Rₜ = 0.98 min; ¹H NMR (700 MHz, CDCl₃) δ 7.02 - 6.96 (m, 1H), 4.09 (s, 2H), 2.23 (s, 3H).

### Step 2.

Synthesis of *tert*-butyl (3-((2-bromo-6-chloro-4-methylpyridin-3-yl)amino)propyl)carbamate (**121b**).

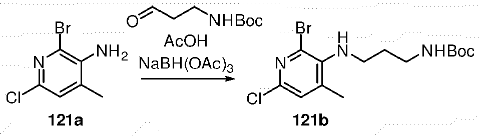

The title compound (**121b**) was obtained from **121a** (154 mg, 0.70 mmol) and from *tert-*butyl (3-oxopropyl)carbamate (145 mg; 0.84 mmol) according to the General Procedure **VIa** in 14% yield (37 mg; 0.10 mmol) with the exception that this reaction was carried on only in AcOH (2 mL) without addition of DCE. The crude product was purified by silica-gel column chromatography (hexane/AcOEt, 2:1, v/v) and then by preparative reversed-phase column chromatography (C-18, water/MeCN, 70:30 to 10:90, 30 min, 20 mL/min).
ESI-MS m/z for C₁₄H₂₂BrClN₃O₂ found 377.9/379.9 [M+H]⁺; Rt= 1.57 min

### Step 3.

Synthesis of *tert-*butyl (3-((6-chloro-2-(2-((6,6-dimethyl-2,4-dioxo-3-azabicyclo[3.1.0]hexan-3-yl)methyl)thieno[3,2-*b*]pyridin-7-yl)-4-methylpyridin-3-yl)amino)propyl)carbamate (**121c**).

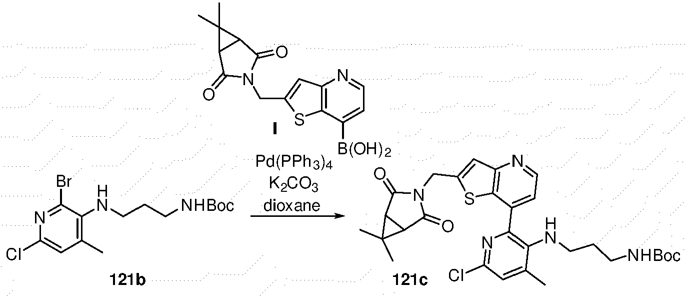

The title compound (**121c**) was obtained from **121b** (33 mg; 0.086 mmol) and from boronic acid **I** (34 mg; 0.103 mmol) according to the General Procedure **Va** in 53% yield (27 mg; 0.046 mmol). The crude product was purified by preparative reversed-phase column chromatography (C-18, water/MeCN, 50:50 to 10:90, 30 min, 20 mL/min).
ESI-MS m/z for C₂₉H₃₅ClN₅O₄S found 584.3/586.3 [M+H]⁺; Rₜ = 1.54 min

### Step 4.

Synthesis of 3-((7-(3-((3-aminopropyl)amino)-6-chloro-4-methylpyridin-2-yl)thieno[3,2-*b*]pyridin-2-yl)methyl)-6,6-dimethyl-3-azabicyclo[3.1.0]hexane-2,4-dione tetrahydrochloride (**121d**).

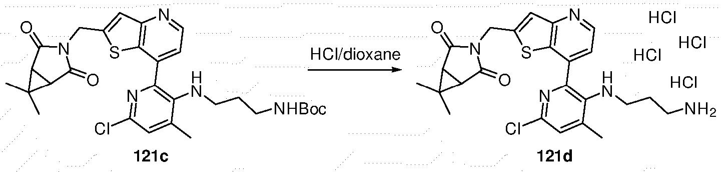

The title compound (**121d**) was obtained as a tetrahydrochloride salt from **121c** (27 mg; 0.046 mmol) according to the General Procedure **IVa** in 89% yield (26 mg; 0.041 mmol). ESI-MS m/z for C₂₄H₂₇ClN₅O₂S found 484.3/486.3 [M+H]⁺; Rₜ = 0.96 min

### Step 5.

Synthesis of 3-((7-(6-chloro-4-methyl-3-(2-oxotetrahydropyrimidin-1(2*H*)-yl)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**121**).

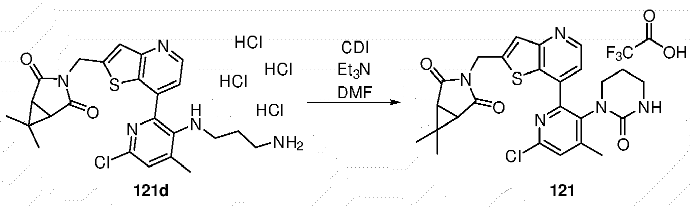

A solution of **121d** (26 mg; 0.041 mmol), CDI (10 mg; 0.062 mmol) and Et₃N (114 µL; 0.820 mmol) in dry DMF (0.4 mL) was stirred at 85 °C in a sealed vial for 3 hours. The reaction progress was monitored by LC-MS. When analysis indicated completion of the reaction, this mixture was concentrated *in vacuo* and the crude product was purified by preparative reversed-phase column chromatography (C-18, water + 0.4‰ TFA/MeCN + 0.4‰ TFA, 75:25 to 45:55, 30 min, 20 mL/min). The title compound (**121**) was obtained as a TFA salt in 13% yield (5 mg; 0.008 mmol).
ESI-MS m/z for C₂₅H₂₅ClN₅O₃S found 510.3/512.3 [M+H]⁺; Rt= 1.52 min; ¹H NMR (700 MHz, Methanol-*d*₄) δ 8.82 - 8.79 (m, 1H), 7.71 - 7.68 (m, 1H), 7.65 - 7.62 (m, 1H), 7.62 - 7.60 (m, 1H), 4.96 - 4.89 (m, 2H), 3.38 - 3.34 (m, 2H), 3.25 - 3.19 (m, 1H), 2.89 - 2.84 (m, 1H), 2.52 (s, 2H), 2.41 (s, 3H), 1.93 - 1.82 (m, 1H), 1.66 - 1.56 (m, 1H), 1.26 (s, 3H), 1.12 (s, 3H).

### Example 122.

Synthesis of 3-((7-(3-(azetidin-3-ylamino)-6-chloro-4-methylpyridin-2-yl)thieno[3,2-*b*]pyridin-2-yl)methyl)-6,6-dimethyl-3-azabicyclo[3.1.0]hexane-2,4-dione tetrahydrochloride (**122**).

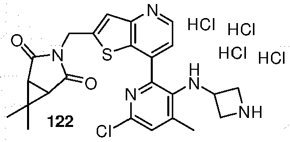

### Step 1.

Synthesis of *tert-*butyl 3-((6-chloro-4-methylpyridin-3-yl)amino)azetidine-1-carboxylate (**122a**).

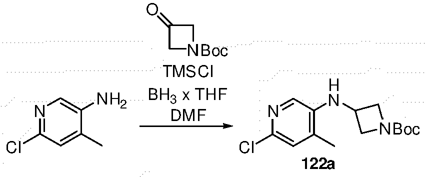

To the solution of 6-chloro-4-methylpyridin-3-amine (212 mg; 1.49 mmol) and *tert-*butyl 3-oxoazetidine-1-carboxylate (200 mg; 1.17 mmol) in DMF (3.5 mL) under an argon atmosphere TMSCl (0.46 mL; 3.65 mmol) was added and then the reaction mixture was cooled to 0 °C and then to this mixture BH₃ x THF (1.17 mL; 1.17 mmol) was added dropwise and the resulting mixture was stirred at 0 °C for 1 hour and then at room temperature for 30 minutes. The reaction progress was monitored by TLC and LC-MS. When analyses indicated completion of the reaction, the reaction mixture was cooled to 0 °C and quenched with water and stirred to obtain room temperature. Then this mixture was diluted with water and to this mixture a saturated solution of NaHCO₃ was added and then a whole was extracted with AcOEt (3 x). The combined organic solutions were dried over anhydrous Na₂SO₄, filtered and concentrated *in vacuo.* The crude product was purified by preparative reversed-phase column chromatography (C-18, water/MeCN, 75:25 to 15:85, 30 min, 20 mL/min). The title compound (**122a**) was obtained in 64% yield (224 mg; 0.75 mmol).
ESI-MS m/z for C₁₄H₂₁ClN₃O₂ found 298.2/300.2 [M+H]⁺; Rₜ = 1.35 min; ¹H NMR (700 MHz, CDCl₃) δ 7.44 (s, 1H), 7.08 - 7.04 (m, 1H), 4.39 - 4.34 (m, 2H), 4.29 - 4.23 (m, 1H), 3.79 - 3.75 (m, 3H), 2.19 (s, 3H), 1.47 (s, 9H).

### Step 2.

Synthesis of *tert*-butyl 3-((2-bromo-6-chloro-4-methylpyridin-3-yl)amino)azetidine-1-carboxylate (**122b**).

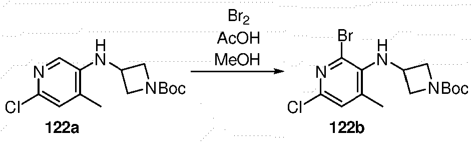

To the solution of **122a** (224 mg; 0.75 mmol) in MeOH (2.25 mL) AcOH (86 µL; 1.50 mmol) was added and then the reaction mixture was cooled to 0 °C and then to this mixture a bromine (38 µL; 0.75 mmol) was added dropwise and the resulting mixture was stirred at room temperature overnight. The reaction progress was monitored by TLC and LC-MS. When analyses indicated almost completion of the reaction, to this mixture 10% Na₂S₂O₃ was added and then a whole was stirred for a several minutes. Then this mixture was alkalized by addition of 1 M K₂CO₃. Then MeOH was evaporated *in vacuo* and the residue was extracted with AcOEt (3 x). The combined organic solutions were dried over anhydrous Na₂SO₄, filtered and concentrated *in vacuo.* The crude product was purified by silica-gel column chromatography (hexane/AcOEt, 100:0 to 70:30, *v*/*v*). The title compound (**122b**) was obtained in 29% yield (82 mg; 0.22 mmol).
ESI-MS m/z for C₁₄H₂₀BrClN₃O₂ found 375.9/377.9 [M+H]⁺; Rt= 1.61 min; ¹H NMR (700 MHz, CDCl₃) δ 7.08 - 7.06 (m, 1H), 4.27 - 4.22 (m, 2H), 4.10 - 4.05 (m, 2H), 3.90 - 3.85 (m, 2H), 2.30 (s, 3H), 1.47 (s, 9H).

### Step 3.

Synthesis of *tert-*butyl 3-((6-chloro-2-(2-((6,6-dimethyl-2,4-dioxo-3-azabicyclo[3.1.0]hexan-3-yl)methyl)thieno[3,2-*b*]pyridin-7-yl)-4-methylpyridin-3-yl)amino)azetidine-1-carboxylate (**122c**).

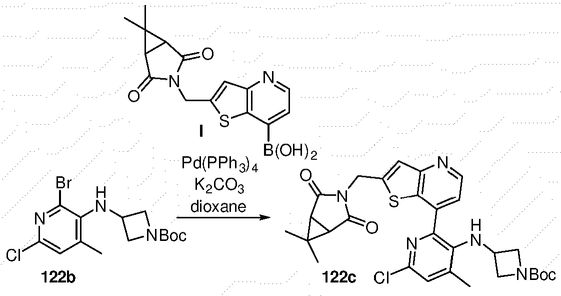

The title compound (**122c**) was obtained from **122b** (82 mg; 0.22 mmol) and from boronic acid **I** (86 mg; 0.26 mmol) according to the General Procedure **Va** in 27% yield (36 mg; 0.06 mmol). The crude product was purified by preparative reversed-phase column chromatography (C-18, water/MeCN, 70:30 to 10:90, 30 min, 20 mL/min).
ESI-MS m/z for C₂₉H₃₃ClN₅O₄S found 582.5/584.5 [M+H]⁺; Rₜ = 1.57 min

### Step 4.

Synthesis of 3-((7-(3-(azetidin-3-ylamino)-6-chloro-4-methylpyridin-2-yl)thieno[3,2-b]pyridin-2-yl)methyl)-6,6-dimethyl-3-azabicyclo[3.1.0]hexane-2,4-dione tetrahydrochloride (**122**).

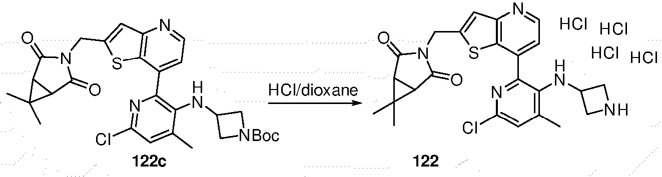

The title compound (**122**) was obtained as a tetrahydrochloride salt from **122c** (36 mg; 0.060 mmol) according to the General Procedure **IVa** in 18% yield (7 mg; 0.011 mmol). The crude product was purified twice by preparative reversed-phase column chromatography (first: C-18, water/MeCN, 80:20 to 50:50, 30 min, 20 mL/min; second: C-18, water/MeCN, 83:17 to 63:37, 60 min, 20 mL/min).
ESI-MS m/z for C₂₄H₂₅ClN₅O₂S found 482.3/484.3 [M+H]⁺; Rt= 0.96 min; ¹H NMR (700 MHz, Methanol-*d*₄) δ 8.77 - 8.74 (m, 1H), 7.73 - 7.69 (m, 1H), 7.57 - 7.54 (m, 1H), 7.37 (d, *J* = 0.8 Hz, 1H), 4.92 - 4.86 (m, 2H), 3.91 - 3.85 (m, 2H), 3.82 - 3.74 (m, 1H), 3.62 - 3.52 (m, 2H), 2.52 (s, 2H), 2.42 (d, *J* = 0.8 Hz, 3H), 1.27 (s, 3H), 1.15 (s, 3H).

### Example 124.

Synthesis of 3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**124**).

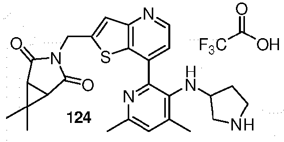

### Step 1.

Synthesis of *tert-*butyl 3-((2-bromo-4,6-dimethylpyridin-3-yl)amino)pyrrolidine-1-carboxylate (**124a**).

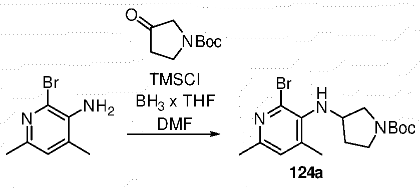

To the solution of 2-bromo-4,6-dimethylpyridin-3-amine (107 mg; 0.53 mmol) and *tert-*butyl 3-oxopyrrolidine-1-carboxylate (78 mg; 0.42 mmol) in DMF (1.5 mL) under an argon atmosphere TMSCl (0.17 mL; 1.33 mmol) was added and then the reaction mixture was cooled to 0 °C and then to this mixture BH₃ x THF (0.42 mL; 0.42 mmol) was added dropwise over 10 minutes and the resulting mixture was stirred at 0 °C for 40 minutes. The reaction progress was monitored by TLC and LC-MS. When analyses indicated completion of the reaction, the reaction mixture was quenched with water and stirred to obtain room temperature. Then the reaction mixture was poured into water ( 11 mL) and alkalized with a saturated solution of NaHCO₃ and then extracted with AcOEt (3 x). The combined organic solutions were dried over anhydrous Na₂SO₄, filtered and concentrated *in vacuo.* The crude product was purified by preparative reversed-phase column chromatography (C-18, water/MeCN, 60:40 to 10:90, 30 min, 20 mL/min). The title compound (**124a**) was obtained in 29% yield (46 mg; 0.12 mmol).
ESI-MS m/z for C₁₆H₂₅BrN₃O₂ found 369.9/371.9 [M+H]⁺; Rₜ = 1.47 min

### Step 2.

Synthesis of *tert-*butyl 3-((2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)amino)pyrrolidine-1-carboxylate (**124b**).

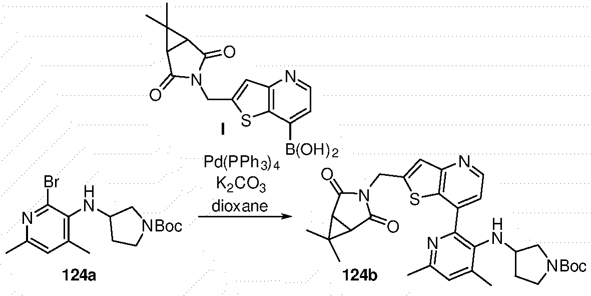

The title compound (**124b**) was obtained from **124a** (46 mg; 0.120 mmol) and from boronic acid **I** (47 mg; 0.144 mmol) according to the General Procedure **Va** in 43% yield (30 mg; 0.052 mmol). The crude product was purified by planar liquid chromatography (AcOEt, 100%).
ESI-MS m/z for C₃₁H₃₈N₅O₄S found 576.8 [M+H]⁺; Rₜ = 1.29 min

### Step 3.

Synthesis of 3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**124**).

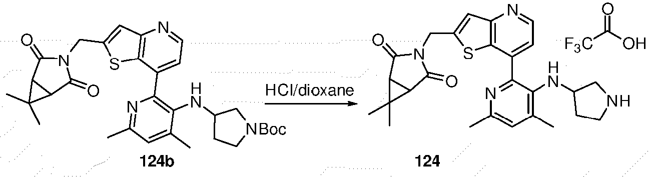

The title compound (**124**) was obtained as a TFA salt as a racemate from **124b** (30 mg; 0.052 mmol) according to the General Procedure **IVa** in 79% yield (24 mg; 0.041 mmol). The crude product was purified twice by preparative reversed-phase column chromatography (C-18, water + 0.4‰ TFA/MeCN + 0.4‰ TFA, 85:15 to 60:40, 30 min, 20 mL/min).
ESI-MS m/z for C₂₆H₃₀N₅O₂S found 476.5 [M+H]⁺; Rₜ = 1.21 min; ¹H NMR (700 MHz, Methanol-*d*₄) δ 8.90 - 8.86 (m, 1H), 8.03 - 7.98 (m, 1H), 7.64 - 7.60 (m, 1H), 7.54 (s, 1H), 4.95 - 4.89 (m, 2H), 3.47 - 3.41 (m, 1H), 3.31 - 3.26 (m, 1H), 3.09 - 3.03 (m, 1H), 2.97 - 2.92 (m, 2H), 2.62 (s, 3H), 2.54 (s, 3H), 2.52 (s, 2H), 1.89 - 1.82 (m, 1H), 1.78 - 1.70 (m, 1H), 1.26 (s, 3H), 1.13 (s, 3H).

### Example 126.

Synthesis of 3-((7-(6-chloro-4-methyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione tetrahydrochloride (**126**).

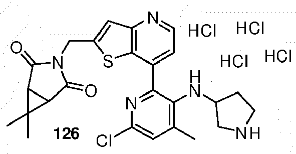

The title compound (**126**) was obtained as a tetrahydrochloride salt as a racemate in 12% overall yield in a similar way to Example 124 with the exception that, in the first step of the synthesis 2-bromo-6-chloro-4-methylpyridin-3-amine (**121a**) was used instead of 2-bromo-4,6-dimethylpyridin-3-amine and in the last step of the synthesis, the crude product was purified by preparative reversed-phase column chromatography (C-18, water/MeCN, 99:1 to 20:80, 30 min, 20 mL/min).
ESI-MS m/z for C₂₅H₂₇ClN₅O₂S found 496.4/498.2 [M+H]⁺; Rt= 0.93 min; ¹H NMR (700 MHz, Methanol-*d*₄) δ 8.79 - 8.74 (m, 1H), 7.95 - 7.87 (m, 1H), 7.57 - 7.49 (m, 1H), 7.41 - 7.34 (m, 1H), 4.91 - 4.88 (m, 2H), 3.48 - 3.42 (m, 1H), 3.31 - 3.25 (m, 1H), 3.07 - 2.98 (m, 2H), 2.97 - 2.91 (m, 1H), 2.52 (s, 2H), 2.46 (s, 3H), 1.95 - 1.86 (m, 1H), 1.80 - 1.72 (m, 1H), 1.26 (s, 3H), 1.14 (s, 3H).

### Example 127.

Synthesis of 3-((7-(6-chloro-4-methyl-3-(piperidin-4-ylamino)pyridin-2-yl)thieno[3,2-*b*]pyridin-2-yl)methyl)-6,6-dimethyl-3-azabicyclo[3.1.0]hexane-2,4-dione tetrahydrochloride (**127**).

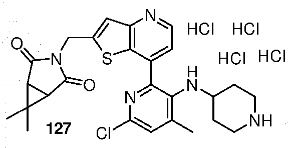

The title compound (**127**) was obtained as a tetrahydrochloride salt in 15% overall yield in a similar way to Example 124 with the exception that, in the first step of the synthesis 2-bromo-6-chloro-4-methylpyridin-3-amine (**121a**) and *tert*-butyl 4-oxopiperidine-1-carboxylate were used instead of 2-bromo-4,6-dimethylpyridin-3-amine and *tert-*butyl 3-oxopyrrolidine-1-carboxylate, and in the last step of the synthesis, the crude product was purified by preparative reversed-phase column chromatography (C-18, water/MeCN, 80:20 to 40:60, 20 min, 20 mL/min).
ESI-MS m/z for C₂₆H₂₉ClN₅O₂S found 510.4/512.4 [M+H]⁺; Rt= 1.00 min; ¹H NMR (700 MHz, Methanol-*d*₄) δ 8.73 - 8.70 (m, 1H), 7.96 - 7.93 (m, 1H), 7.53 - 7.49 (m, 1H), 7.41 - 7.37 (m, 1H), 4.89 - 4.88 (m, 2H), 3.24 - 3.17 (m, 2H), 2.75 - 2.68 (m, 1H), 2.63 - 2.58 (m, 2H), 2.52 (s, 2H), 2.45 (s, 3H), 1.81 - 1.75 (m, 2H), 1.54 - 1.45 (m, 2H), 1.26 (s, 3H), 1.14 (s, 3H).

### Example 128.

Synthesis of 6,6-dimethyl-3-((7-(4-methyl-3-(piperidin-4-ylamino)-6-(trifluoromethyl)pyridin-2-yl)thieno[3,2-*b*]pyridin-2-yl)methyl)-3-azabicyclo[3.1.0]hexane-2,4-dione tetrahydrochloride (**128**).

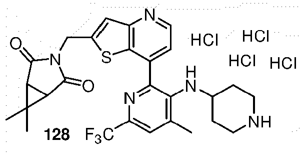

The title compound (**128**) was obtained as a tetrahydrochloride salt in 10% overall yield in a similar way to Example 122 with the exception that, in the first step of the synthesis 4-methyl-6-(trifluoromethyl)pyridin-3-amine and *tert-*butyl 4-oxopiperidine-1-carboxylate were used instead of 6-chloro-4-methylpyridin-3-amine and *tert-*butyl 3-oxoazetidine-1-carboxylate, and in the last step of the synthesis, the crude product was purified by preparative reversed-phase column chromatography (C-18, water + 0.3‰ HCl (36%)/MeCN + 0.3‰ HCl (36%), 90:10 to 40:60, 30 min, 20 mL/min).
ESI-MS m/z for C₂₇H₂₉F₃N₅O₂S found 544.4 [M+H]⁺; Rt= 1.02 min; ¹H NMR (700 MHz, Methanol-*d*₄) δ 8.92 - 8.89 (m, 1H), 8.61 - 8.57 (m, 1H), 7.82 - 7.79 (m, 1H), 7.72 - 7.69 (m, 1H), 5.02 - 4.96 (m, 2H), 3.30 - 3.24 (m, 2H), 3.04 - 2.93 (m, 1H), 2.79 - 2.66 (m, 2H), 2.57 - 2.52 (m, 5H), 1.98 - 1.90 (m, 2H), 1.75 - 1.66 (m, 2H), 1.28 (s, 3H), 1.18 (s, 3H); ¹⁹F NMR (235 MHz, Methanol-*d*₄) δ -63.68.

### Example 129.

Synthesis of 3-((7-(6-chloro-4-methyl-3-(piperidin-3-ylamino)pyridin-2-yl)thieno[3,2-*b*]pyridin-2-yl)methyl)-6,6-dimethyl-3-azabicyclo[3.1.0]hexane-2,4-dione tetrahydrochloride (**129**).

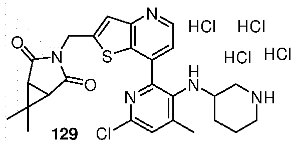

The title compound (**129**) was obtained as a tetrahydrochloride salt in 12% overall yield in a similar way to Example 124 with the exception that, in the first step of the synthesis 2-bromo-6-chloro-4-methylpyridin-3-amine (**121a**) and *tert-*butyl 3-oxopiperidine-1-carboxylate were used instead of 2-bromo-4,6-dimethylpyridin-3-amine and *tert-*butyl 3-oxopyrrolidine-1-carboxylate, and in the last step of the synthesis, the crude product was purified by preparative reversed-phase column chromatography (C-18, water/MeCN, 90:10 to 30:70, 30 min, 20 mL/min).
ESI-MS m/z for C₂₆H₂₉ClN₅O₂S found 510.1/512.1 [M+H]⁺; Rt= 1.05 min; ¹H NMR (700 MHz, Methanol-*d*₄) δ 8.76 - 8.73 (m, 1H), 8.03 - 7.98 (m, 1H), 7.55 - 7.50 (m, 1H), 7.43 - 7.38 (m, 1H), 4.89 (d, *J* = 0.9 Hz, 2H), 3.16 - 3.10 (m, 1H), 3.09 - 3.04 (m, 1H), 2.84 - 2.78 (m, 1H), 2.78 - 2.70 (m, 2H), 2.52 (s, 2H), 2.44 (s, 3H), 1.83 - 1.77 (m, 2H), 1.42 - 1.33 (m, 1H), 1.32 - 1.24 (m, 4H), 1.16 (s, 3H).

### Example 130.

Synthesis of 6,6-dimethyl-3-((7-(4-methyl-3-(piperidin-3-ylamino)-6-(trifluoromethyl)pyridin-2-yl)thieno[3,2-*b*]pyridin-2-yl)methyl)-3-azabicyclo[3.1.0]hexane-2,4-dione tetrahydrochloride (**130**).

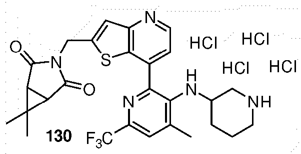

The title compound (**130**) was obtained as a tetrahydrochloride salt in 1% overall yield in a similar way to Example 122 with the exception that, in the first step of the synthesis 4-methyl-6-(trifluoromethyl)pyridin-3-amine and *tert-*butyl 3-oxopiperidine-1-carboxylate were used instead of 6-chloro-4-methylpyridin-3-amine and *tert-*butyl 3-oxoazetidine-1-carboxylate, and in the last step of the synthesis, the crude product was purified by preparative reversed-phase column chromatography (C-18, water + 0.3‰ HCl (36%)/MeCN + 0.3‰ HCl (36%), 75:25 to 35:65, 30 min, 20 mL/min).
ESI-MS m/z for C₂₇H₂₉F₃N₅O₂S found 544.5 [M+H]⁺; Rₜ = 1.07 min; ¹H NMR (700 MHz, Methanol-*d*₄) δ 8.92 - 8.83 (m, 1H), 8.40 - 8.32 (m, 1H), 7.82 - 7.75 (m, 1H), 7.68 - 7.63 (m, 1H), 4.95 (d, *J =* 0.8 Hz, 2H), 3.22 - 3.12 (m, 2H), 3.08 - 2.99 (m, 1H), 2.89 - 2.82 (m, 1H), 2.80 - 2.73 (m, 1H), 2.57 - 2.52 (m, 5H), 1.91 - 1.86 (m, 1H), 1.86 - 1.79 (m, 1H), 1.54 - 1.45 (m, 1H), 1.39 - 1.34 (m, 1H), 1.28 (s, 3H), 1.17 (s, 3H).

### Example 133.

Synthesis of *N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)azetidine-3-carboxamide 2,2,2-trifluoroacetate (**133**).

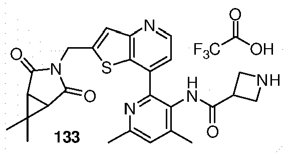

### Step 1.

Synthesis of 3-((7-(3-amino-4,6-dimethylpyridin-2-yl)thieno[3,2-*b*]pyridin-2-yl)methyl)-6,6-dimethyl-3-azabicyclo[3.1.0]hexane-2,4-dione (**133a**).

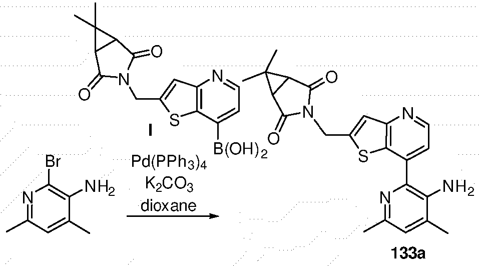

The title compound (**133a**) was obtained from 2-bromo-4,6-dimethylpyridin-3-amine (194 mg; 0.960 mmol) and from boronic acid **I (150** mg; 0.450 mmol) according to the General Procedure **Va** in 23% yield (43 mg; 0.106 mmol). The crude product was purified by silica-gel column chromatography (hexane/AcOEt, 70:30 to 0:100, *v*/*v*, then: AcOEt/MeOH, 99:1 to 90:10, *v*/*v*).
ESI-MS m/z for C₂₂H₂₃N₄O₂S found 407.8 [M+H]⁺; Rₜ = 0.81 min

### Step 2.

Synthesis of *tert-*butyl 3-((2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)carbamoyl)azetidine-1-carboxylate (**133b**).

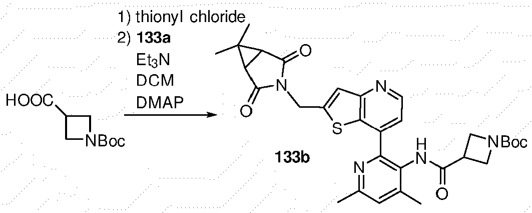

The title compound (**133b**) was obtained from 1-(*tert*-butoxycarbonyl)azetidine-3-carboxylic acid (66 mg; 0.330 mmol) and from the amine **133a** (43 mg; 0.106 mmol) according to the General Procedure **II** in 90% yield (56 mg; 0.095 mmol).
ESI-MS m/z for C₃₁H₃₆N₅O₅S found 590.6 [M+H]⁺; Rₜ = 1.28 min

### Step 3.

Synthesis of *N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)azetidine-3-carboxamide 2,2,2-trifluoroacetate (**133**).

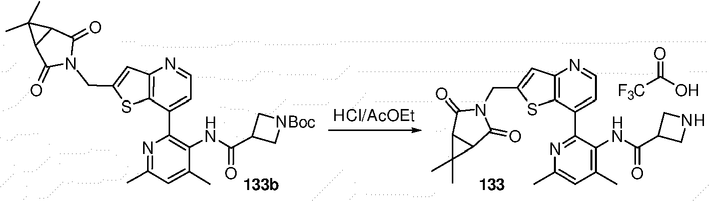

The title compound (**133**) was obtained as a TFA salt from **133b** (56 mg; 0.095 mmol) according to the General Procedure **IVa** in 2% yield (1 mg; 0.002 mmol). The crude product was purified by preparative reversed-phase column chromatography (column: Cosmosil Cholester 20 x 250 mm, water + 0.4‰ TFA/MeCN + 0.4‰ TFA, 80:20 to 60:40, 30 min, 40 mL/min). ESI-MS m/z for C₂₆H₂₈N₅O₃S found 490.6 [M+H]⁺; Rt= 0.78 min; ¹H NMR (700 MHz, D₂O) δ 8.83 - 8.79 (m, 1H), 7.74 - 7.69 (m, 2H), 7.63 (s, 1H), 4.98 - 4.93 (m, 2H), 4.12 - 4.05 (m, 2H), 3.82 - 3.68 (m, 3H), 2.65 - 2.55 (m, 5H), 2.36 (s, 3H), 1.20 (s, 3H), 1.04 (s, 3H).

### Example 134.

Synthesis of *N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)piperidine-4-carboxamide 2,2,2-trifluoroacetate (**134**).

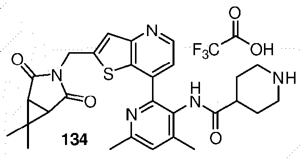

### Step 1.

Synthesis of benzyl 4-((2-bromo-4,6-dimethylpyridin-3-yl)carbamoyl)piperidine-1-carboxylate (**134a**).

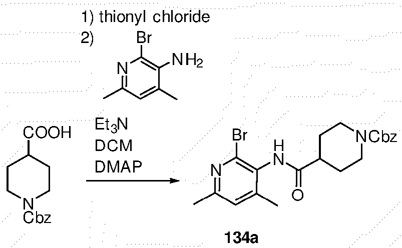

The title compound (**134a**) was obtained from 1-((benzyloxy)carbonyl)piperidine-4-carboxylic acid (671 mg; 2.55 mmol) and from 2-bromo-4,6-dimethylpyridin-3-amine (170 mg; 0.85 mmol) according to the General Procedure **II** in 18% yield (65 mg; 0.15 mmol). The crude product was purified by silica-gel column chromatography (hexane/AcOEt, 70:30 to 10:90, *v*/*v*). ESI-MS m/z for C₂₁H₂₅BrN₃O₃ found 445.9/447.9 [M+H]⁺; Rₜ = 1.30 min; ¹H NMR (700 MHz, CDCl₃) δ 7.42 - 7.26 (m, 5H), 6.97 (s, 1H), 5.17 (s, 2H), 4.37 - 4.20 (m, 2H), 3.06 - 2.92 (m, 2H), 2.64 - 2.54 (m, 1H), 2.51 (s, 3H), 2.25 (s, 3H), 2.09 - 2.00 (m, 2H), 1.89 - 1.81 (m, 2H).

### Step 2.

Synthesis of benzyl 4-((2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)carbamoyl)piperidine-1-carboxylate (**134b**).

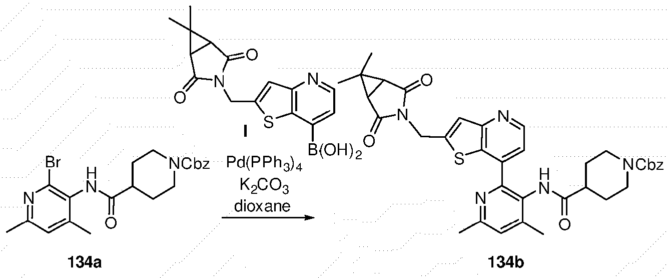

The title compound (**134b**) was obtained from **134a** (65 mg; 0.15 mmol) and from boronic acid **I** (59 mg; 0.18 mmol) according to the General Procedure **Va** in 40% yield (40 mg; 0.06 mmol). The crude product was purified by silica-gel column chromatography (AcOEt/MeOH, 100:0 to 92.5:7.5, *v*/*v*).
ESI-MS m/z for C₃₆H₃₈N₅O₅S found 652.9 [M+H]⁺; Rₜ = 1.35 min

### Step 3.

Synthesis of *N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)piperidine-4-carboxamide 2,2,2-trifluoroacetate (**134**).

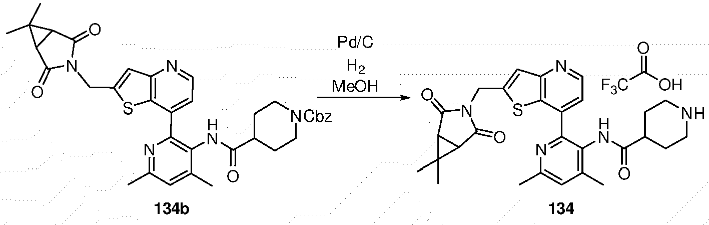

To the solution of **134b** (40 mg; 0.06 mmol) in MeOH (0.25 mL) under argon atmosphere Pd/C (10 mol%; cat.) was added. Then argon was replaced by hydrogen and the reaction mixture was conducted under hydrogen atmosphere at room temperature overnight. The reaction progress was monitored by LC-MS. When analysis indicated completion of the reaction, Pd/C was filtered off through a Celite pad and the solvent was evaporated *in vacuo.* The crude product was purified by preparative reversed-phase column chromatography (C-18, water + 0.4‰ TFA/MeCN + 0.4‰ TFA, 85:15 to 55:45, 30 min, 20 mL/min). The title compound (**134**) was obtained as a TFA salt in 50% yield (19 mg; 0.03 mmol).
ESI-MS m/z for C₂₈H₃₂N₅O₃S found 518.4 [M+H]⁺; Rt= 0.75 min; ¹H NMR (700 MHz, Methanol-*d*₄) δ 8.75 - 8.71 (m, 1H), 7.59 - 7.53 (m, 2H), 7.48 - 7.45 (m, 1H), 4.91 - 4.88 (m, 2H), 3.56 - 3.49 (m, 1H), 3.38 - 3.34 (m, 1H), 3.02 - 2.95 (m, 2H), 2.67 - 2.59 (m, 4H), 2.54 - 2.50 (m, 2H), 2.37 (s, 2H), 1.91 - 1.82 (m, 2H), 1.72 - 1.63 (m, 2H), 1.26 (s, 3H), 1.13 (s, 3H).

### Example 135.

Synthesis of 3-((7-(6-chloro-4-methyl-3-(piperazine-1-carbonyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**135**).

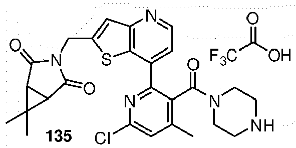

### Step 1.

Synthesis of 2-bromo-3-(ethoxycarbonyl)-4-methylpyridine 1-oxide (**135a**).

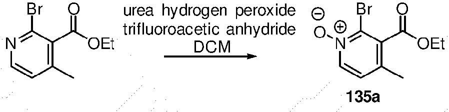

To the solution of ethyl 2-bromo-4-methylnicotinate (5.00 g; 20.48 mmol) in DCM (150 mL) urea hydrogen peroxide (9.63 g; 102.40 mmol) was added at room temperature. Then the reaction mixture was cooled to 0 °C and to this mixture trifluoroacetic anhydride (11.40 mL; 81.92 mmol) was added dropwise and the whole was stirred at room temperature for 1 hour. The reaction progress was monitored by TLC. When analysis indicated completion of the reaction, 5% NaHCO₃ (150 mL) was carefully added and this mixture was stirred for a while. The layers were separated and an aqueous one was carefully alkalized by solid K₂CO₃ and then extracted with DCM (3 x). The combined organic solutions were washed with brine and dried over anhydrous Na₂SO₄, filtered and concentrated *in vacuo.* The title compound (**135a**) was obtained in 99% yield (5.25 g; 20.27 mmol).
ESI-MS m/z for C₉H₁₁BrNO₃ found 259.9/261.9 [M+H]⁺; Rt= 0.68 min; ¹H NMR (700 MHz, CDCl₃) δ 8.36 - 8.31 (m, 1H), 7.14 - 7.10 (m, 1H), 4.50 (q, *J =* 7.2 Hz, 2H), 2.36 (s, 3H), 1.45 (t, *J =* 7.2 Hz, 3H).

### Step 2.

Synthesis of ethyl 2-bromo-6-chloro-4-methylnicotinate (**135b**).

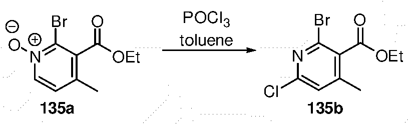

To the cooled to 0 °C solution of **135a** (1.85 g; 7.14 mmol) in anhydrous toluene (50 mL) POCl₃ (13 mL; 139.50 mmol) was added dropwise and the resulting mixture was allowed to warm to room temperature and then it was stirred at 120 °C for 3 hours. The reaction progress was monitored by TLC. When analysis indicated completion of the reaction, the reaction mixture was cooled to room temperature and to this mixture an ice was added. When an ice had melt, the mixture was alkalized to pH 8 by addition of solid K₂CO₃ and then extracted with AcOEt (3 x). The combined organic solutions were dried over anhydrous Na₂SO₄, filtered and concentrated *in vacuo.* The crude product was purified by silica-gel column chromatography (hexane/AcOEt, 100:0 to 92:8, *v*/*v*). The title compound (**135b**) was obtained in 41% yield (0.82 g; 2.96 mmol). ESI-MS m/z for C₉H₁₀B₁ClNO₂ found 277.8/279.8 [M+H]⁺; Rₜ = 1.46 min; ¹H NMR (700 MHz, CDCl₃) δ 8.51 (s, 1H), 4.49 (q, *J* = 7.1 Hz, 2H), 2.42 (s, 3H), 1.44 (t, *J =* 7.1 Hz, 3H).

### Step 3.

Synthesis of 2-bromo-6-chloro-4-methylnicotinic acid (135c).

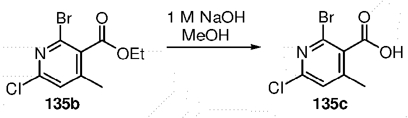

To the solution of **135b** (648 mg; 2.33 mmol) in MeOH (6 mL) 1 M NaOH (9.32 mL; 9.32 mmol) was added and the resulting mixture was stirred at room temperature overnight. Then another portions of 1 M NaOH (3 x 2 mL) were added and stirred for 2 days. The reaction progress was monitored by LC-MS. When analysis indicated almost completion of the reaction, MeOH was evaporated and the residue was neutralized to pH 7 and this solution was cooled in acetone/dry ice bath and lyophilized. Then the product was extracted with 2-propanol (3 x) from the precipitate. The combined organic solutions were concentrated *in vacuo.* The crude product was purified by silica-gel column chromatography (AcOEt/MeOH, 70:30 to 50:50, *v*/*v*). The title compound (**135c**) was obtained in 61% yield (357 mg; 1.43 mmol).
ESI-MS m/z for C₇H₆BrClNO₂ found 249.9/251.9 [M+H]⁺; Rₜ = 0.56 min

### Step 4.

Synthesis of *tert-*butyl 4-(2-bromo-6-chloro-4-methylnicotinoyl)piperazine-1-carboxylate (**135d**).

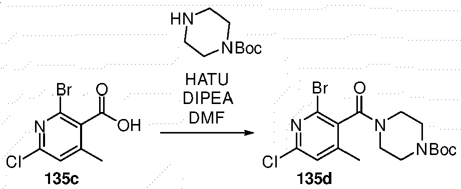

The title compound (**135d**) was obtained from **135c** (197 mg; 0.79 mmol) and from *tert-*butyl piperazine-1-carboxylate (531 mg; 2.85 mmol) according to the General Procedure **I** in 66% yield (217 mg; 0.52 mmol).
ESI-MS m/z for C₁₂H₁₄BrClN₃O₃ found 361.8/363.8 [M+H-*t*Bu]⁺; Rₜ = 1.58 min

### Step 5.

Synthesis of *tert*-butyl 4-(6-chloro-2-(2-((6,6-dimethyl-2,4-dioxo-3-azabicyclo[3.1.0]hexan-3-yl)methyl)thieno[3,2-*b*]pyridin-7-yl)-4-methylnicotinoyl)piperazine-1-carboxylate (135e).

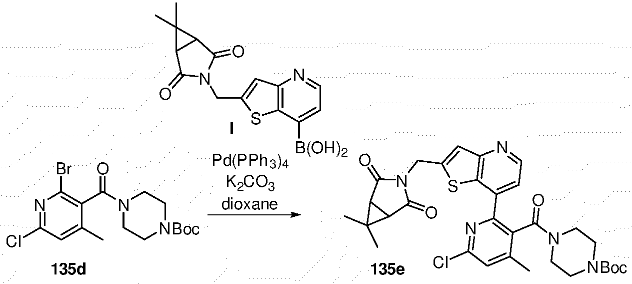

The title compound (**135e**) was obtained from **135d** (217 mg; 0.52 mmol) and from boronic acid **I** (172 mg; 0.52 mmol) according to the General Procedure **Va** in 10% yield (30 mg; 0.05 mmol). The crude product was purified by silica-gel column chromatography (hexane/AcOEt, 100:0 to 40:60, v/v) and then by preparative reversed-phase column chromatography (C-18, water/MeCN, 70:30 to 20:80, 30 min, 20 mL/min).
ESI-MS m/z for C₃₁H₃₅ClN₅O₅S found 624.4/626.4 [M+H]⁺; Rₜ = 1.72 min

### Step 6.

Synthesis of 3-((7-(6-chloro-4-methyl-3-(piperazine-1-carbonyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**135**).

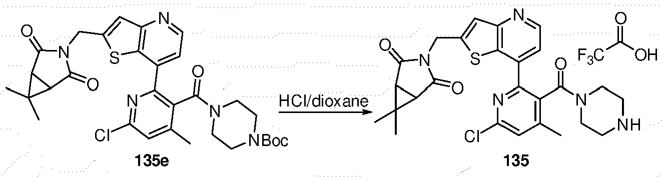

The title compound (**135**) was obtained as a TFA salt from **135e** (32 mg; 0.051 mmol) according to the General Procedure **IVa** in 4% yield (1 mg; 0.002 mmol). The crude product was purified by preparative reversed-phase column chromatography (Cosmosil Cholester 20 x 250 mm, water + 0.4‰ TFA/MeCN + 0.4‰ TFA, 80:20 to 50:50, 30 min, 20 mL/min).
ESI-MS m/z for C₂₆H₂₇ClN₅O₃S found 524.2/526.2 [M+H]⁺; Rₜ = 1.21 min

### Example 136.

Synthesis of 1-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)urea dihydrochloride (**136**).

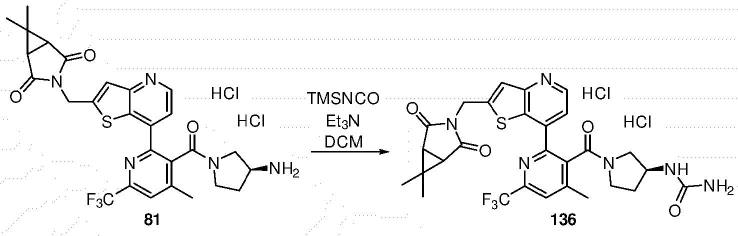

To a stirred solution of **81** (28 mg; 0.045 mmol) in DCM (0.25 mL) TMSNCO (18 µL; 0.135 mmol) and Et₃N (25 µL;0.180 mmol) were added and the resulting mixture was stirred at room temperature for 2 hours. The reaction progress was monitored by LC-MS. When analysis indicated completion of the reaction, the mixture was cooled to 0 °C and quenched with MeOH. The cooling bath was removed and this mixture was stirred to allow to room temperature. Then the solvents were evaporated under reduced pressure. To the residue 1 M HCl (1.5 mL) was added and a whole was washed with AcOEt (2 x). the aqueous phase was then purified twice by preparative reversed-phase column chromatography (first: C-18, water + 0.3‰ HCl (36%)/MeCN + 0.3‰ HCl (36%), 75:25 to 35:65, 30 min, 20 mL/min; second: C-18, water + 0.3‰ HCl (36%)/MeCN + 0.3‰ HCl (36%), 70:30 to 40:60, 30 min, 20 mL/min). The title compound (**136**) was obtained as a dihydrochloride salt in 1% yield (4 mg; 0.006 mmol). ESI-MS m/z for C₂₈H₂₈F₃N₆O₄S found 601.0 [M+H]⁺; Rₜ = 1.28 min; ¹H NMR (700 MHz, Methanol-*d*₄) δ 8.88 - 8.80 (m, 1H), 8.06 - 8.01 (m, 1H), 7.88 - 7.81 (m, 1H), 7.66 - 7.58 (m, 1H), 4.97 - 4.89 (m, 2H), 4.20 - 2.63 (m, 7H), 2.58 - 2.51 (m, 3H), 2.22 - 1.65 (m, 2H), 1.42 - 1.08 (m, 6H).

### Example 137.

Synthesis of 3-((7-(3-(3-amino-3-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**137**).

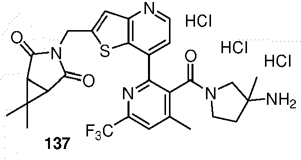

### Step 1.

Synthesis of *tert-*butyl (1-(2-chloro-4-methyl-6-(trifluoromethyl)nicotinoyl)-3-methylpyrrolidin-3-yl)carbamate 2,2,2-trifluoroacetate (**137a**).

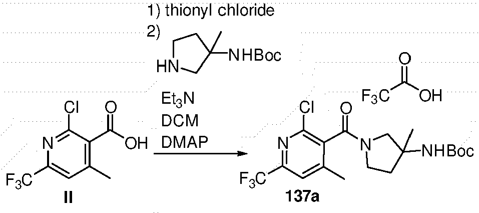

The title compound (**137a**) was obtained as a TFA salt from an acid **II** (99 mg; 0.41 mmol) and from *tert-*butyl (3-methylpyrrolidin-3-yl)carbamate (124 mg; 0.62 mmol) according to the General Procedure **II** in 37% yield (79 mg; 0.15 mmol). The crude product was purified by silica-gel column chromatography (hexane/AcOEt, 100:0 to 20:80, *v*/*v*) and then by preparative reversed-phase column chromatography (C-18, water/MeCN + 1‰ TFA, 70:30 to 30:70, 30 min, 20 mL/min).
ESI-MS m/z for C₁₄H₁₆ClF₃N₃O₃ found 365.9/367.9 [M+H-*t*Bu]⁺; Rt= 1.59 min; ¹H NMR (700 MHz, Methanol-*d*₄) δ 7.88 - 7.77 (m, 1H), 4.34 - 3.67 (m, 2H), 3.51 - 3.35 (m, 1H), 3.29 - 3.10 (m, 1H), 2.49 - 2.36 (m, 3H), 2.33 - 1.94 (m, 2H), 1.53 - 1.35 (m, 12H).

### Step 2.

Synthesis of *tert-*butyl (1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)-3-methylpyrrolidin-3-yl)carbamate (**137b**).

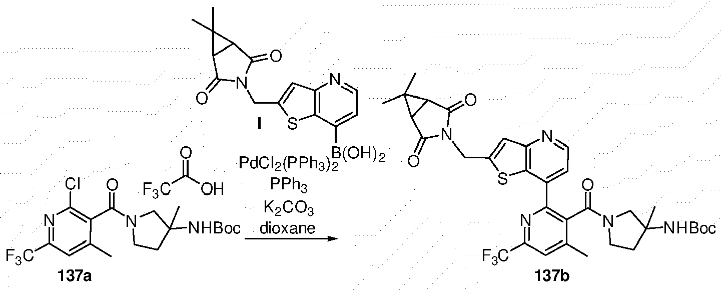

The title compound (**137b**) was obtained from **137a** (75 mg; 0.14 mmol) and from boronic acid **I** (69 mg; 0.21 mmol) according to the General Procedure **Va** in 43% yield (43 mg; 0.06 mmol). The crude product was purified by silica-gel column chromatography (hexane/AcOEt, 100:0 to 0:100, v/v, then AcOEt/MeOH, 95:5 to 90:10, *v*/*v*).
ESI-MS m/z for C₃₃H₃₇F₃N₅O₅S found 672.2 [M+H]⁺; Rₜ = 1.69 min

### Step 3.

Synthesis of 3-((7-(3-(3-amino-3-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**137**).

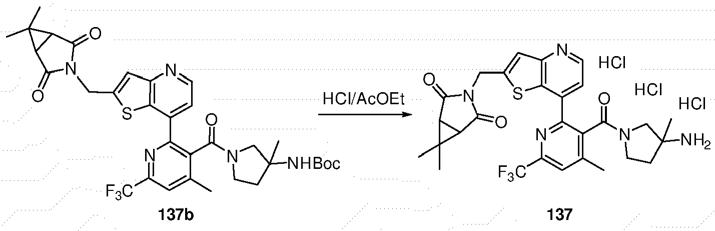

The title compound (**137**) was obtained as a trihydrochloride salt from **137b** (43 mg; 0.06 mmol) according to the General Procedure **IVa** in 33% yield (14 mg; 0.02 mmol). The crude product was purified by preparative reversed-phase column chromatography (C-18, water + 0.3‰ HCl (36%)/MeCN + 0.3‰ HCl (36%), 90:10 to 40:60, 30 min, 20 mL/min).
ESI-MS m/z for C₂₈H₂₉F₃N₅O₃S found 572.0 [M+H]⁺; Rₜ = 1.17 min; ¹H NMR (700 MHz, Methanol-*d*₄) δ 9.06 - 8.96 (m, 1H), 8.17 - 8.10 (m, 1H), 8.06 - 7.98 (m, 1H), 7.79 - 7.69 (m, 1H), 5.04 - 4.96 (m, 2H), 4.00 - 3.68 (m, 2H), 3.29 - 2.74 (m, 3H), 2.65 - 2.51 (m, 3H), 2.24 - 1.80 (m, 3H), 1.55 - 0.94 (m, 9H).

### Example 138.

Synthesis of 3-((7-(3-((3*S*)-3-(1-aminoethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**138**).

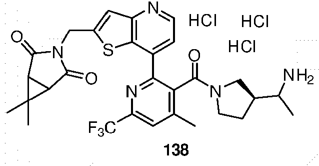

### Step 1.

Synthesis of *tert*-butyl (*S*)-3-formylpyrrolidine-1-carboxylate (**138a**).

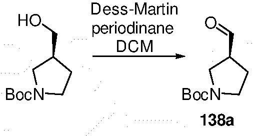

To a solution of *tert-*butyl (*S*)-3-(hydroxymethyl)pyrrolidine-1-carboxylate (3.08 g; 15.32 mmol) in DCM (60 mL) Dess-Martin periodinane (8.45 g; 19.92 mmol) was added at 0 °C and the reaction mixture was warmed to room temperature and stirred for 1 hour. The reaction progress was monitored by TLC. When analysis indicated completion of the reaction, to this mixture 10% Na₂S₂O₃ was added and stirred for 30 minutes, then phases were separated and an organic one was washed with water (1 x), 1 M NaOH (2 x) and water (1 x). An aqueous layer was extracted with DCM (3 x). The combined organic layers were dried over anhydrous Na₂SO₄, filtered and concentrated *in vacuo.* The crude product was purified by silica-gel column chromatography (hexane/AcOEt, 100:0 to 40:60, *v*/*v*). The title compound (**138a**) was obtained in 78% yield (2.38 g; 11.95 mmol).
ESI-MS m/z for C₆H₁₀NO₃ found 144.2 [M+H-*t*Bu]⁺; Rₜ = 0.92 min; ¹H NMR (700 MHz, CDCl₃) δ 9.71 (d, *J =* 1.6 Hz, 1H), 3.80 - 3.65 (m, 1H), 3.60 - 3.47 (m, 1H), 3.47 - 3.34 (m, 2H), 3.08 - 2.97 (m, 1H), 2.27 - 2.08 (m, 2H), 1.48 (s, 9H).

### Step 2.

Synthesis of *tert-butyl* (S)-3-((R)-1-hydroxyethyl)pyrrolidine-1-carboxylate (**138b**).

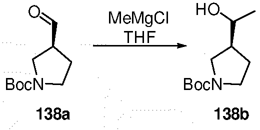

To a cooled to -20 °C solution of **138a** (2.38 g; 11.95 mmol) in anhydrous THF (45 mL) under an argon atmosphere MeMgCl (3 M in THF; 6 mL; 18.00 mmol) was added dropwise. After this reaction was allowed to warm up to room temperature and stirred for 2 hours. The reaction progress was monitored by TLC analysis of small aliquots of the crude reaction mixture. When analyses were indicated completion of the reaction, the mixture was poured into saturated solution of NH₄Cl. An organic phase was separated, and the aqueous phase was extracted with AcOEt (3 x). The combined organic phases were dried over anhydrous MgSO₄, filtered and concentrated *in vacuo.* The crude product was purified by silica-gel column chromatography (hexane/AcOEt, 80:20 to 30:70, *v*/*v*). The title compound (**138b**) was obtained in 82% yield (2.12 g; 9.85 mmol).
¹H NMR (700 MHz, CDCl₃) δ 3.77 - 3.66 (m, 1H), 3.66 - 3.40 (m, 2H), 3.33 - 2.96 (m, 2H), 2.21 - 2.07 (m, 1H), 1.95 - 1.63 (m, 2H), 1.48 (s, 9H), 1.28 - 1.23 (m, 3H).

### Step 3.

Synthesis of *tert-*butyl (*S*)-3-((*R*)-1-((methylsulfonyl)oxy)ethyl)pyrrolidine-1-carboxylate (**138c**).

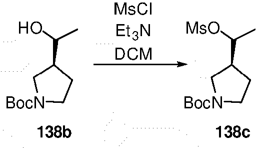

The title compound (**138c**) was obtained from **138b** (2.12 g; 9.85 mmol) according to the General Procedure **VIII** in 99% yield (2.86 g; 9.75 mmol).
ESI-MS m/z for C₁₂H₂₃NO₅SNa found 316.0 [M+Na]⁺; Rt= 1.37 min; ¹H NMR (700 MHz, CDCl₃) δ 4.80 - 4.68 (m, 1H), 3.70 - 2.99 (m, 7H), 2.47 - 1.67 (m, 3H), 1.52 - 1.44 (m, 12H).

### Step 4.

Synthesis of *tert-*butyl (*S*)-3-((*R*)-1-azidoethyl)pyrrolidine-1-carboxylate (**138d**).

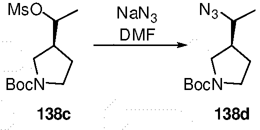

A compound **138c** (2.86 g; 9.75 mmol) and sodium azide (1.26 g; 19.46 mmol) in DMF (50 mL) were stirred at 70 °C overnight. The reaction progress was monitored by LC-MS. When analysis indicated completion of the reaction, the mixture was cooled to room temperature. The obtained precipitate was filtered off and the residue was diluted with Et₂O (250 mL) and washed with water (3 x) and then dried over anhydrous NaSO₄, filtered and concentrated *in vacuo.* The crude product was purified by flash column chromatography on silica (hexane/AcOEt, 100:0 to 20:80, *v*/*v,* 35 min). The title compound (**138d**) was obtained as a single isomer in 36% yield (0.85 g; 3.54 mmol). ¹H NMR (700 MHz, CDCl₃) δ 3.58 - 3.51 (m, 1H), 3.51 - 3.36 (m, 2H), 3.32 - 3.24 (m, 1H), 3.04 - 2.96 (m, 1H), 2.28 - 2.15 (m, 1H), 2.12 - 2.06 (m, 1H), 1.76 - 1.66 (m, 1H), 1.48 (s, 9H), 1.33 (d, *J* = 6.5 Hz, 3H).

### Step 5.

Synthesis of (*S*)-3-((*R*)-1-azidoethyl)pyrrolidine hydrochloride (**138e**).

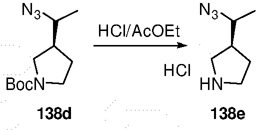

The title compounds (**138e**) was obtained as a hydrochloride salt from **138d** (0.85 g; 3.54 mmol) according to the General Procedure **IVa** in 99% yield (618 mg; 3.50 mmol). ESI-MS m/z for C₆H₁₃N₄ found 141.2 [M+H]⁺; Rt= 0.20 min; ¹H NMR (700 MHz, Methanol-*d*₄) δ 3.79 - 3.72 (m, 1H), 3.45 - 3.37 (m, 2H), 3.29 - 3.22 (m, 1H), 3.08 - 3.02 (m, 1H), 2.52 - 2.40 (m, 1H), 2.22 - 2.13 (m, 1H), 1.93 - 1.80 (m, 1H), 1.37 (d, *J =* 6.6 Hz, 3H).

### Step 6.

Synthesis of ((*S*)-3-((*S*)-1-azidoethyl)pyrrolidin-1-yl)(2-chloro-4-methyl-6-(trifluoromethyl)pyridin-3-yl)methanone (**138f**).

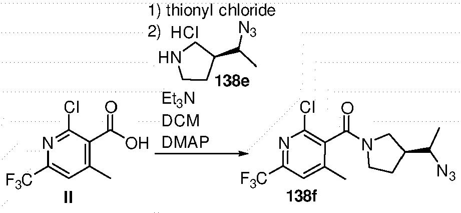

The title compound (**138f**) was obtained from an acid **II** (100 mg; 0.42 mmol) and from **138e** (115 mg; 0.65 mmol) according to the General Procedure **II** in 67% yield (102 mg; 0.28 mmol). The crude product was purified by silica-gel column chromatography (hexane/AcOEt, 80:20 to 30:70, *v*/*v*).
ESI-MS m/z for C₁₄H₁₆ClF₃N₅O found 362.0/364.0 [M+H]⁺; Rt= 1.49 min; ¹H NMR (700 MHz, CDCl₃) δ 7.55 - 7.50 (m, 1H), 3.99 - 3.07 (m, 4H), 2.47 - 2.41 (m, 2H), 2.41 - 2.16 (m, 3H), 1.95 - 1.75 (m, 1H), 1.44 - 1.26 (m, 4H).

### Step 7.

Synthesis of 3-((7-(3-((3*S*)-3-(1-aminoethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**138**).

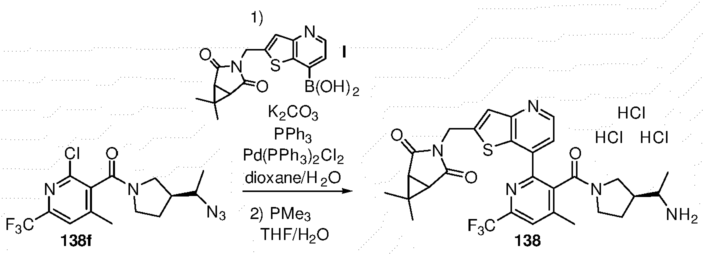

The title compound (**138**) was obtained as a trihydrochloride salt in 4% yield (5 mg; 0.007 mmol) from **138f** (102 mg; 0.28 mmol) and from the boronic acid **I** (139 mg; 0.42 mmol) according to the General Procedure **Va**. Then the crude productwas dissolved in THF/water (5:1; *v*/*v;* 1 mL) and PMe₃ (1 M solution in THF; 0.20 mL; 0.200 mmol) was added to this solution and the mixture was stirred at room temperature for 1 hour. The reaction progress was monitored by LC-MS. When analysis indicated completion of the reaction, the mixture was concentrated *in vacuo* and to the residue 1 M HCl (1.5 mL) was added and then extracted with AcOEt (2 x). The aqueous layer was purified twice by preparative reversed-phase column chromatography (first: C-18, water + 0.3‰ HCl (36%)/MeCN + 0.3‰ HCl (36%), 75:25 to 50:50, 30 min, 20 mL/min, second: C-18, water + 0.3‰ HCl (36%)/MeCN + 0.3‰ HCl (36%), 85:15 to 50:50, 35 min, 20 mL/min).
ESI-MS m/z for C₂₉H₃₁F₃N₅O₃S found 586.2 [M+H]⁺; Rₜ = 1.11 min; ¹H NMR (700 MHz, Methanol-*d*₄) δ 8.88 - 8.84 (m, 1H), 8.09 - 8.02 (m, 1H), 7.91 - 7.77 (m, 1H), 7.68 - 7.61 (m, 1H), 4.99 - 4.92 (m, 2H), 4.03 - 3.60 (m, 2H), 3.45 - 3.36 (m, 1H), 3.25 - 3.05 (m, 1H), 2.91 - 2.64 (m, 1H), 2.59 - 2.51 (m, 3H), 2.43 - 2.28 (m, 1H), 2.18 - 1.84 (m, 2H), 1.76 - 1.45 (m, 2H), 1.37 - 0.83 (m, 9H).

### Example 139.

Synthesis of 3-((7-(3-(3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**139**).

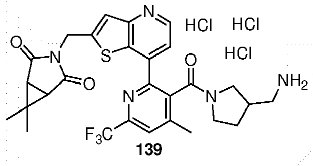

The title compound (**139**) was obtained as a racemate as a trihydrochloride salt in 10% overall yield in a similar way to Example 137 with the exception that, in the first step of the synthesis *tert-*butyl (pyrrolidin-3-ylmethyl)carbamate was used instead of *tert*-butyl (3-methylpyrrolidin-3-yl)carbamate, and in the last step of the synthesis, the General Procedure **IVb** was used instead of the General Procedure **IVa** and the crude product was purified by preparative reversed-phase column chromatography (C-18, water + 0.3‰ HCl (36%)/MeCN + 0.3‰ HCl (36%), 80:20 to 40:60, 30 min, 20 mL/min).
ESI-MS m/z for C₂₈H₂₉F₃N₅O₃S found 572.0 [M+H]⁺; Rₜ = 1.03 min; ¹H NMR (700 MHz, Methanol-*d*₄) δ 9.07 - 8.93 (m, 1H), 8.19 - 8.05 (m, 2H), 7.80 - 7.75 (m, 1H), 5.04 - 4.98 (m, 2H), 4.10 - 3.38 (m, 3H), 3.30 - 2.89 (m, 3H), 2.82 - 2.70 (m, 1H), 2.64 - 2.36 (m, 5H), 2.28 - 1.90 (m, 1H), 1.86 - 1.49 (m, 1H), 1.33 - 1.25 (m, 3H), 1.21 - 1.12 (m, 3H).

### Example 140.

Synthesis of 3-((7-(3-(3-((isopropylamino)methyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**140**).

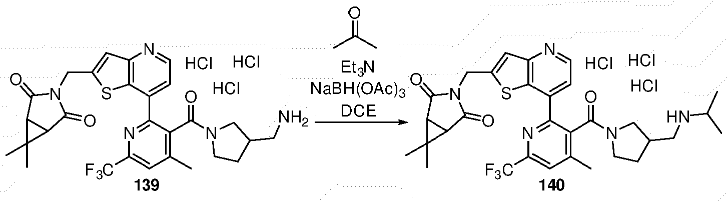

The title compound (**140**) was as a racemate as a trihydrochloride salt obtained from **139** (13 mg, 0.020 mmol) and from acetone (2 µL; 0.027 mmol) according to the General Procedure **VIa** in 70% yield (10 mg; 0.014 mmol) with the exception that in this reaction Et₃N was used instead of AcOH. The crude product was purified by preparative reversed-phase column chromatography (C-18, water + 0.3‰ HCl (36%)/MeCN + 0.3‰ HCl (36%), 80:20 to 40:60, 30 min, 20 mL/min
ESI-MS m/z for C₃₁H₃₅F₃N₅O₃S found 614.1 [M+H]⁺; Rₜ = 0.97 min; ¹H NMR (700 MHz, Methanol-*d*₄) δ 8.97 - 8.87 (m, 1H), 8.11 - 8.05 (m, 1H), 8.01 - 7.88 (m, 1H), 7.70 - 7.64 (m, 1H), 5.02 - 4.91 (m, 2H), 4.10 - 3.49 (m, 4H), 3.28 - 2.75 (m, 3H), 2.70 - 2.48 (m, 3H), 2.47 - 1.59 (m, 5H), 1.37 - 1.12 (m, 12H).

### Example 141.

Synthesis of 3-((7-(3-((2*S*,6*S*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**141**).

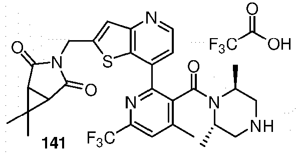

The title compound (**141**) was obtained as a mixture of two isomers (*in ratio* 7:10) as a TFA salt in 4% overall yield in a similar way to Example 137 with the exception that, in the first step of the synthesis *tert-*butyl (3*S*,5*S*)-3,5-dimethylpiperazine-1-carboxylate was used instead of *tert-*butyl (3-methylpyrrolidin-3-yl)carbamate, and in the last step of the synthesis, the General Procedure **IVb** was used instead of the General Procedure **IVa** and the crude product was purified by preparative reversed-phase column chromatography (C-18, water + 0.4‰ TFA/MeCN + 0.4‰ TFA, 90:10 to 50:50, 30 min, 20 mL/min).
ESI-MS m/z for C₂₉H₃₁F₃N₅O₃S found 586.2 [M+H]⁺; Rt= 0.93 min; ¹H NMR (700 MHz, Methanol-*d*₄) δ 8.79 - 8.72 (m, 1H), 8.08 - 7.51 (m, 3H), 4.95 - 4.86 (m, 2H), 4.67 - 4.49 (m, 1H), 3.66 - 3.61 (m, 1H), 3.46 - 3.34 (m, 1H), 3.31 - 2.82 (m, 3H), 2.62 - 2.08 (m, 5H), 1.65 - 0.45 (m, 12H).

### Example 142.

Synthesis of 3-((7-(3-((2*R*,6*R*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione 2,2,2-trifluoroacetate (**142**).

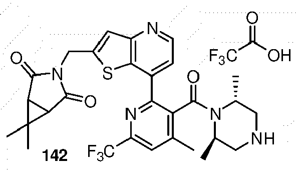

The title compound (**142**) was obtained as a mixture of two isomers (*in ratio* 10:12) as a TFA salt in 1% overall yield in a similar way to Example 137 with the exception that, in the first step of the synthesis *tert-*butyl (3*R*,5*R*)-3,5-dimethylpiperazine-1-carboxylate was used instead of *tert-butyl* (3-methylpyrrolidin-3-yl)carbamate, and in the last step of the synthesis, the General Procedure **IVb** was used instead of the General Procedure **IVa** and the crude product was purified by preparative reversed-phase column chromatography (C-18, water + 0.4‰ TFA/MeCN + 0.4‰ TFA, 80:20 to 40:60, 30 min, 20 mL/min).
ESI-MS m/z for C₂₉H₃₁F₃N₅O₃S found 586.2 [M+H]⁺; Rt= 0.96 min; ¹H NMR (700 MHz, Methanol-*d*₄) δ 8.79 - 8.72 (m, 1H), 8.07 - 7.50 (m, 3H), 4.96 - 4.87 (m, 2H), 4.67 - 4.53 (m, 1H), 3.85 - 3.36 (m, 3H), 3.24 - 2.77 (m, 2H), 2.64 - 2.42 (m, 3H), 2.34 - 2.10 (m, 1H), 1.71 - 0.45 (m, 13H).

### Example 143.

Synthesis of 3-((7-(3-(4-ethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione trihydrochloride (**143**).

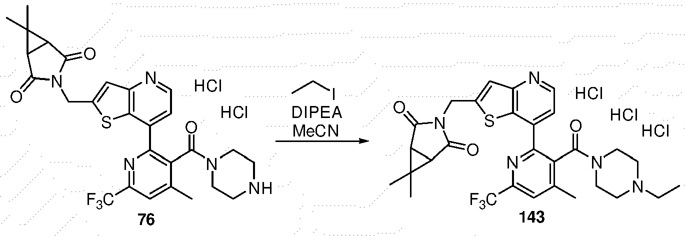

The mixture of compound **76** (10 mg; 0.015 mmol), MeCN (0.5 mL), DIPEA (10 µL; 0.060 mmol) and iodoethane (2 µL; 0.018 mmol) was stirred at room temperature for 2 days. Then the reaction mixture was concentrated under reduced pressure and the residue was purified by preparative reversed-phase column chromatography (C-18, water + 0.3‰ HCl (36%)/MeCN, 90:10 to 40:60, 20 min, 20 mL/min). The title compound (**143**) was obtained as a trihydrochloride salt in 87% yield (9 mg; 0.013 mmol).
ESI-MS m/z for C₂₉H₃₁F₃N₅O₃S found 586.2 [M+H]⁺; Rₜ = 1.04 min; ¹H NMR (700 MHz, Methanol-*d*₄) δ 8.98 - 8.95 (m, 1H), 8.12 (s, 1H), 7.99 - 7.82 (m, 1H), 7.72 (d, *J =* 2.2 Hz, 1H), 5.01 - 4.95 (m, 2H), 3.73 - 3.63 (m, 1H), 3.60 - 3.37 (m, 2H), 3.30 - 3.09 (m, 5H), 2.93 - 2.86 (m, 1H), 2.84 - 2.77 (m, 1H), 2.63 (d, *J =* 10.3 Hz, 2H), 2.56 - 2.52 (m, 3H), 1.33 - 1.26 (m, 6H), 1.17 (s, 3H).

### Example 144.

Synthesis of 6,6-dimethyl-3-((7-(4-methyl-3-((*S*)-3-(methylamino)piperidine-1-carbonyl)-6-(trifluoromethyl)pyridin-2-yl)thieno[3,2-*b*]pyridin-2-yl)methyl)-3-azabicyclo[3.1.0]hexane-2,4-dione trihydrochloride (**144**).

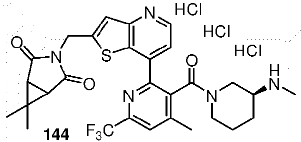

The title compound (**144**) was obtained as a trihydrochloride salt in 15% overall yield in a similar way to Example 45 with the exception that, in the first step of the synthesis an acid **II** and (*S*)-3-*N*-Boc-3-(methylamino)piperidine were used instead of 2-chloro-4-methylnicotinic acid and *tert-*butyl piperazine-1-carboxylate and in the last step of the synthesis the crude product was purified by preparative reversed-phase column chromatography (C-18, water + 0.3‰ HCl (36%)/MeCN, 90:10 to 40:60, 20 min, 20 mL/min).
ESI-MS m/z for C₂₉H₃₁F₃N₅O₃S found 586.10 [M+H]⁺; Rₜ = 0.98 min; ¹H NMR (700 MHz, Methanol-*d*₄) δ 8.99 - 8.83 (m, 1H), 8.11 - 8.02 (m, 1H), 7.97 - 7.77 (m, 1H), 7.71 - 7.61 (m, 1H), 4.99 - 4.94 (m, 2H), 3.29 - 3.14 (m, 1H), 3.08 - 2.93 (m, 2H), 2.85 - 2.76 (m, 3H), 2.66 - 2.59 (m, 2H), 2.57 - 2.50 (m, 4H), 2.46 - 2.20 (m, 1H), 2.01 - 1.66 (m, 1H), 1.54 - 1.31 (m, 2H), 1.20 (d, *J* = 15.1 Hz, 4H), 1.13 (d, *J =* 8.2 Hz, 2H).

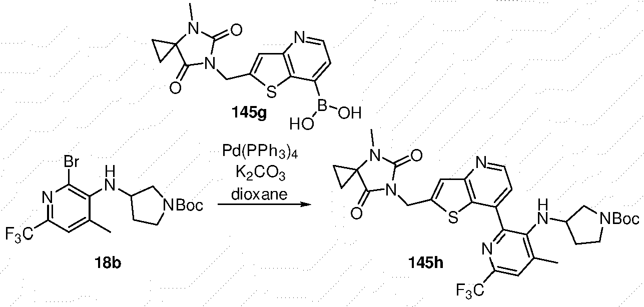

### Example 153.*(this compound is not a part of the present invention)

Synthesis of 3-((7-(3-(((3a*R*,6a*R*)-hexahydropyrrolo[3,4-*b*]pyrrol-1(2*H*)-yl)methyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione trihydrochloride (**153**).

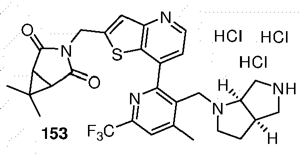

The title compound (**153**) was obtained as a trihydrochloride salt as a racemate in 30% overall yield in a similar way to Example 40 with the exception that, in the first step of the synthesis *tert*-butyl (3a*R*,6a*R*)-hexahydropyrrolo[3,4-*b*]pyrrole-5(1*H*)-carboxylate was used instead of *tert*-butyl 3-oxopiperazine-1-carboxylate. This synthesis step was carried out in a different way (the synthesis was described below). In the last step of the synthesis the crude product was purified by preparative reversed-phase column chromatography (C-18, water + 0.3‰ HCl (36%)/MeCN, 98:2 to 30:70, 30 min, 20 mL/min).
ESI-MS m/z for C₂₉H₃₁F₃N₅O₂S found 570.6 [M+H]⁺; Rt= 0.98 min; ¹H NMR (700 MHz, Methanol-*d*₄) δ 8.92 - 8.87 (m, 1H), 7.92 (s, 1H), 7.80 - 7.76 (m, 1H), 7.71 - 7.66 (m, 1H), 4.95 - 4.92 (m, 2H), 4.12 - 4.03 (m, 1H), 3.78 - 3.68 (m, 1H), 3.21 - 3.14 (m, 1H), 3.13 - 3.04 (m, 2H), 3.04 - 2.95 (m, 2H), 2.94 - 2.85 (m, 1H), 2.78 (s, 3H), 2.65 - 2.58 (m, 1H), 2.54 - 2.49 (m, 2H), 1.99 - 1.86 (m, 2H), 1.46 - 1.36 (m, 1H), 1.26 (s, 3H), 1.11 (s, 3H); ¹⁹F NMR (659 MHz, Methanol-*d*₄) δ -69.32.

### Step 1.

Synthesis of *tert-*butyl (3a*R*,6a*R*)-1-((2-chloro-4-methyl-6-(trifluoromethyl)pyridin-3-yl)methyl)hexahydropyrrolo[3,4-*b*]pyrrole-5(1*H*)-carboxylate (**153a**).

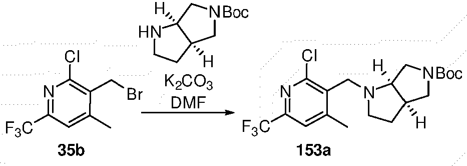

To a solution of **35b** (97 mg; 0.336 mmol) and *tert-*butyl (3a*R*,6a*R*)-hexahydropyrrolo[3,4-*b*]pyrrole-5(1*H*)-carboxylate (60 mg; 0.280 mmol) in DMF (1 mL) K₂CO₃ (58 mg; 0.420 mmol) was added and then the reaction mixture was stirred at 50 °C for 3 hours. The reaction progress was monitored by LC-MS. When analysis indicated completion of the reaction, the mixture was diluted with AcOEt (20 mL) and washed with brine (15 mL). The organic layer was dried over anhydrous NaSO₄, filtered and concentrated *in vacuo.* The title compound **153a** was obtained as a colorless oil in 99% yield (117 mg; 0.279 mmol).
ESI-MS m/z for C₁₉H₂₆ClF₃N₃O₂ found 420.0/422.0 [M+H]⁺; Rt= 1.43 min

### Example 160.

Synthesis of 6,6-dimethyl-3-((7-(4-methyl-3-(((*S*)-3-(methylamino)pyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione hydrochloride (**160**).

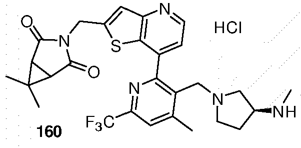

The title compound (**160**) was obtained as a hydrochloride salt in 20% overall yield in a similar way to Example 153 with the exception that, in the first step of the synthesis *tert-*butyl (*S*)-methyl(pyrrolidin-3-yl)carbamate was used instead of *tert-*butyl 3-oxopiperazine-1-carboxylate, and in the last step of the synthesis the crude product was purified by preparative reversed-phase column chromatography (C-18, water + 0.3‰ HCl (36%)/MeCN, 99:1 to 40:60, 30 min, 20 mL/min).
ESI-MS m/z for C₂₈H₃₁F₃N₅O₂S found 558.4 [M+H]⁺; Rt= 0.98 min; ¹H NMR (700 MHz, D₂O) δ 8.85 (s, 1H), 8.03 (s, 1H), 7.71 (s, 1H), 7.68 (s, 1H), 4.99 - 4.95 (m, 2H), 4.20 - 4.09 (m, 2H), 3.66 - 3.58 (m, 1H), 2.97 - 2.90 (m, 1H), 2.76 - 2.70 (m, 5H), 2.68 (s, 2H), 2.57 - 2.50 (m, 4H), 2.21 - 2.11 (m, 1H), 1.83 - 1.75 (m, 1H), 1.27 (s, 3H), 1.07 (s, 3H).

### Example 165.

Synthesis of 6,6-dimethyl-3-((7-(4-methyl-3-((3-oxopiperazin-2-yl)methyl)-6-(trifluoromethyl)pyridin-2-yl)thieno[3,2-*b*]pyridin-2-yl)methyl)-3-azabicyclo[3.1.0]hexane-2,4-dione hydrochloride (**165**).

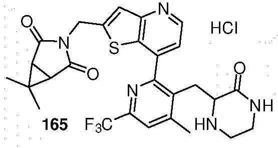

### Step 1.

Synthesis of di-*tert*-butyl 2-oxopiperazine-1,4-dicarboxylate (**165a**).

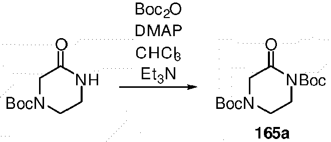

To the suspension of *tert-*butyl 3-oxopiperazine-1-carboxylate (1.00 g; 4.99 mmol) in CHCl₃ (20 mL) Boc₂O (1.30 g; 5.98 mmol), DMAP (183 mg; 1.49 mmol) and Et₃N (0.83 mL; 5.98 mmol) were added and then the reaction mixture was stirred at room temperature overnight. Then Boc₂O (1.30 g; 5.98 mmol) and DMAP (183 mg; 1.49 mmol) were added and the reaction mixture was stirred at room temperature for 3 hours. The reaction progress was monitored by LC-MS. When analysis indicated completion of the reaction, the solvent was evaporated *in vacuo* and the residue was purified by flash column chromatography on silica (hexane/AcOEt, 100:0 to 50:50, *v*/*v,* 35 minutes). Compound **165a** was obtained in 83% yield (1.25 g; 4.16 mmol).
ESI-MS m/z for C₁₄H₂₅N₂O₅ found 301.0 [M+H]⁺; Rₜ = 1.32 min; ¹H NMR (700 MHz, CDCl₃) δ 4.23 - 4.18 (m, 2H), 3.83 - 3.79 (m, 2H), 3.67 - 3.60 (m, 2H), 1.57 (s, 10H), 1.49 (s, 9H).

### Step 2.

Synthesis of di-*tert*-butyl 2-((2-chloro-4-methyl-6-(trifluoromethyl)pyridin-3-yl)methyl)-3-oxopiperazine-1,4-dicarboxylate (**165b**).

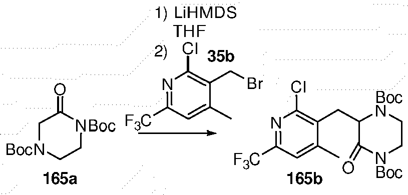

To a cooled to -78 °C solution of **165a** (0.90 g; 3.00 mmol) in THF (50 mL) LiHMDS (1 M in THF; 3.30 mL; 3.30 mmol) was added dropwise and then the reaction mixture was stirred at this temperature for 1 hour. Then to this mixture a solution of **35b** (867 mg; 3.00 mmol) in THF (5 mL) was added and the resulting mixture was stirred at -78 °C for 5 hours and then the mixture was slowly warmed up to room temperature and stirred overnight. The reaction progress was monitored by LC-MS. When analysis indicated completion of the reaction, the mixture was diluted with Et₂O and washed with NH₄Cl. An organic layer was dried over anhydrous MgSO₄, filtered and concentrated *in vacuo* and the crude product was purified by flash column chromatography on silica (hexane/AcOEt, 100:0 to 60:40, *v*/*v,* 25 minutes). The title compound **165b** was obtained as a colorless oil in 52% yield (795 mg; 1.57 mmol). ESI-MS m/z for C₂₂H₂₉ClF₃N₃O₅Na found 530.1/532.1 [M+Na]⁺; Rₜ = 1.84 min

### Step 3.

Synthesis of *tert-*butyl 2-((2-chloro-4-methyl-6-(trifluoromethyl)pyridin-3-yl)methyl)-3-oxopiperazine-1-carboxylate (**165c**).

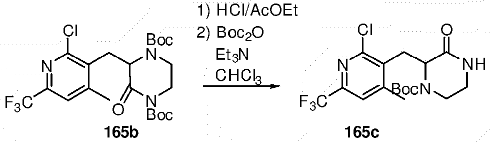

The title compound (**165c**) was obtained from **165b** (730 mg; 1.44 mmol) according to the General Procedure **IVa** and then the General Procedure **III** in 99% yield (per two steps)(578 mg; 1.42 mmol). The crude product was purified by flash column chromatography on silica (DCM/MeOH, 100:0 to 0:100, *v*/*v,* 30 minutes).
ESI-MS m/z for C₁₇H₂₂ClF₃N₃O₃ found 407.9/409.8 [M+H]⁺; Rₜ = 1.34 min; ¹H NMR (700 MHz, CDCl₃) δ 7.45 (s, 1H), 6.22 - 6.08 (m, 1H), 5.03 - 4.86 (m, 1H), 4.38 - 4.07 (m, 1H), 3.55 - 3.31 (m, 4H), 2.60 (s, 3H), 1.20 (s, 9H); ¹⁹F NMR (659 MHz, CDCl₃) δ -68.05.

### Step 4.

Synthesis of *tert-*butyl 2-((2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)methyl)-3-oxopiperazine-1-carboxylate (**165d**).

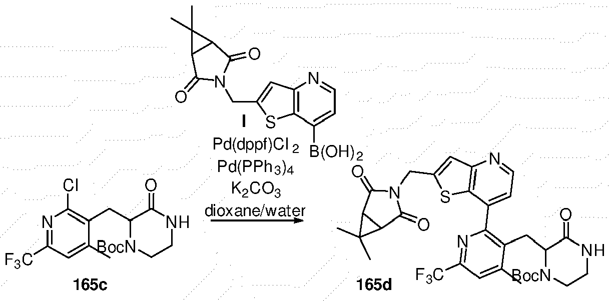

The title compound (**165d**) was obtained from **165c** (40 mg; 0.098 mmol) and from boronic acid **I** (31 mg; 0.098 mmol) according to the General Procedure **Va** and the crude product was used to the next step without additional purification.
ESI-MS m/z for C₃₂H₃₅F₃N₅O₅S found 658.5 [M+H]⁺; Rt= 1.81 min

### Step 5.

Synthesis of 6,6-dimethyl-3-((7-(4-methyl-3-((3-oxopiperazin-2-yl)methyl)-6-(trifluoromethyl)pyridin-2-yl)thieno[3,2-*b*]pyridin-2-yl)methyl)-3-azabicyclo[3.1.0]hexane-2,4-dione hydrochloride (**165**).

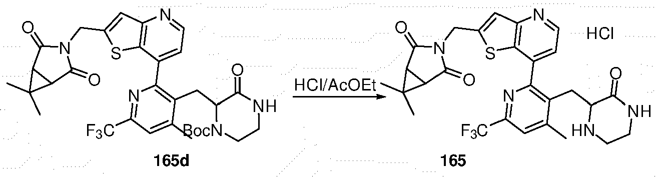

The title compound (**165**) was obtained as a hydrochloride salt from **165d** (the crude product) according to the General Procedure **IVa** in 18% yield (per two steps)(11 mg; 0.018 mmol). The crude product was purified by preparative reversed-phase column chromatography (column: Cosmosil Cholester 20 x 250 mm, water + 0.3‰ HCl (36%)/MeCN, 99:1 to 40:60, 30 min, 20 mL/min).
ESI-MS m/z for C₂₇H₂₇F₃N₅O₃S found 558.5 [M+H]⁺; Rₜ = 0.92 min; ¹H NMR (700 MHz, DMSO-*d*₆+ D₂O, 348 K) δ 8.77 - 8.73 (m, 1H), 7.84 (s, 1H), 7.53 - 7.50 (m, 1H), 7.48 - 7.42 (m, 1H), 4.83 - 4.73 (m, 2H), 3.79 - 3.72 (m, 1H), 3.60 - 3.54 (m, 1H), 3.33 - 3.23 (m, 3H), 3.17 - 3.05 (m, 2H), 2.61 - 2.56 (m, 3H), 2.55 - 2.52 (m, 2H), 1.18 (s, 3H), 1.03 (s, 3H); ¹⁹F NMR (659 MHz, DMSO-*d*₆) δ -66.23.

### Example 168.

Synthesis of 6,6-dimethyl-3-((7-(4-methyl-3-((3R,4S)-3-methyl-4-(methylamino)pyrrolidine-1-carbonyl)-6-(trifluoromethyl)pyridin-2-yl)thieno[3,2-b]pyridin-2-yl)methyl)-3-azabicyclo[3.1.0]hexane-2,4-dione hydrochloride (**168**).

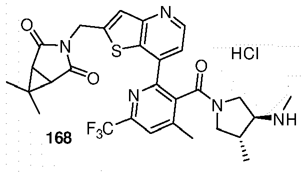

The title compound (**168**) was obtained as a hydrochloride salt in 6% overall yield in a similar way to Example 45 with the exception that, in the first step of the synthesis *tert-*butyl methyl((3*S*,4*R*)-4-methylpyrrolidin-3-yl)carbamate (this compound was synthesized according to the General Procedure **C** with the exception that, in the first step of the synthesis *tert-*butyl ((3*S*,4*R*)-4-methylpyrrolidin-3-yl)carbamate was used instead of *tert-*butyl (*S*)-pyrrolidin-3-ylcarbamate and in the second step of the synthesis, MeI and DMF were used instead of EtI and THF and this reaction was carried out at 0 °C and slowly raised to room temperature and the whole was stirred overnight) was used instead of *tert-*butyl piperazine-1-carboxylate and acid **II** was used instead of 2-chloro-4-methylnicotinic acid and in the last step of the synthesis, the General Procedure **IVb** was used instead of the General Procedure **IVa** and the crude product was purified by preparative reversed-phase column chromatography (C-18, water + 0.3‰ HCl (36%)/MeCN, 100:0 to 45:55, 30 min, 20 mL/min).
ESI-MS m/z for C₂₉H₃₁F₃N₅O₃S found 586.6 [M+H]⁺; Rₜ = 1.07 min; ¹H NMR (700 MHz, D₂O) δ 8.89 - 8.84 (m, 1H), 8.18 - 8.13 (m, 1H), 7.79 - 7.68 (m, 2H), 5.08 - 4.96 (m, 2H), 4.36 - 3.77 (m, 2H), 3.65 - 3.23 (m, 2H), 3.09 - 2.95 (m, 1H), 2.75 - 2.67 (m, 3H), 2.64 - 2.51 (m, 4H), 2.36 - 1.94 (m, 2H), 1.31 - 1.14 (m, 3H), 1.11 - 0.12 (m, 6H); ¹⁹F NMR (376 MHz, D₂O) δ -67.82 - - 67.87 (m).

### Example 169.

Synthesis of 3-((7-(3-((3*R*,4*S*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**169**).

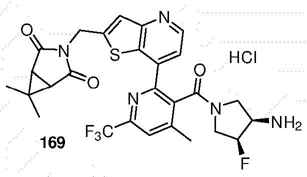

The title compound (**169**) was obtained as a hydrochloride salt in 14% overall yield in a similar way to Example 45 with the exception that, in the first step of the synthesis an acid **II** and *tert-*butyl ((3*R*,4*S*)-4-fluoropyrrolidin-3-yl)carbamate were used instead of 2-chloro-4-methylnicotinic acid and *tert-*butyl piperazine-1-carboxylate and in the last step of the synthesis, the crude product was purified by preparative reversed-phase column chromatography (C-18, water + 0.4‰ HCl (36%)/MeCN, 99:1 to 20:80, 30 min, 18 mL/min).
ESI-MS m/z for C₂₇H₂₆F₄N₅O₃S found 576.5 [M+H]⁺; Rₜ = 1.11 min; ¹H NMR (700 MHz, Methanol-*d*₄) δ 8.98 - 8.90 (m, 1H), 8.15 - 7.89 (m, 2H), 7.73 - 7.69 (m, 1H), 5.46 - 5.06 (m, 1H), 5.02 - 4.92 (m, 2H), 4.32 - 3.93 (m, 2H), 3.89 - 3.35 (m, 2H), 3.26 - 3.06 (m, 1H), 2.61 - 2.56 (m, 2H), 2.56 - 2.50 (m, 3H), 1.28 - 1.23 (m, 3H), 1.20 - 1.09 (m, 3H).

### Example 170.

Synthesis of 3-((7-(3-((3*S*,4*S*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**170**).

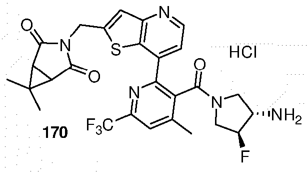

The title compound (**170**) was obtained as a hydrochloride salt in 16% overall yield in a similar way to Example 45 with the exception that, in the first step of the synthesis an acid **II** and *tert-*butyl ((3*S*,4*S*)-4-fluoropyrrolidin-3-yl)carbamate were used instead of 2-chloro-4-methylnicotinic acid and *tert-*butyl piperazine-1-carboxylate and in the last step of the synthesis, the crude product was purified by preparative reversed-phase column chromatography (column: Cosmosil Cholester 20 x 250 mm, water + 0.3‰ HCl (36%)/MeCN, 99:1 to 20:80, 30 min, 20 mL/min).
ESI-MS m/z for C₂₇H₂₆F₄N₅O₃S found 576.3 [M+H]⁺; Rt= 1.14 min; ¹H NMR (700 MHz, Methanol-*d*₄) δ 9.03 - 8.97 (m, 1H), 8.17 - 7.97 (m, 2H), 7.79 - 7.75 (m, 1H), 5.47 - 5.05 (m, 1H), 5.02 - 4.96 (m, 2H), 4.32 - 3.83 (m, 2H), 3.78 - 3.39 (m, 2H), 3.28 - 2.67 (m, 1H), 2.64 - 2.57 (m, 2H), 2.57 - 2.52 (m, 3H), 1.29 - 1.23 (m, 3H), 1.21 - 1.10 (m, 3H).

### Example 171.

Synthesis of 3-((7-(3-((3*S*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**171**).

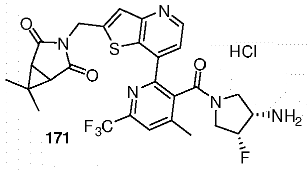

The title compound (**171**) was obtained as a hydrochloride salt in 17% overall yield in a similar way to Example 45 with the exception that, in the first step of the synthesis an acid **II** and *tert-*butyl ((3*S*,4*R*)-4-fluoropyrrolidin-3-yl)carbamate were used instead of 2-chloro-4-methylnicotinic acid and *tert-*butyl piperazine-1-carboxylate and in the last step of the synthesis, the crude product was purified by preparative reversed-phase column chromatography (column: Cosmosil Cholester 20 x 250 mm, water + 0.3‰ HCl (36%)/MeCN, 99:1 to 20:80, 30 min, 20 mL/min).
ESI-MS m/z for C₂₇H₂₆F₄N₅O₃S found 576.5 [M+H]⁺; Rₜ = 1.09 min; ¹H NMR (700 MHz, Methanol-*d*₄) δ 9.03 - 8.97 (m, 1H), 8.17 - 7.96 (m, 2H), 7.79 - 7.75 (m, 1H), 5.47 - 5.05 (m, 1H), 5.02 - 4.96 (m, 2H), 4.32 - 3.39 (m, 4H), 3.28 - 2.66 (m, 1H), 2.63 - 2.57 (m, 2H), 2.57 - 2.52 (m, 3H), 1.29 - 1.23 (m, 3H), 1.21 - 1.10 (m, 3H).

### Example 172.

Synthesis of 3-((7-(3-((3*S*,4*R*)-3-(dimethylamino)-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione hydrochloride (**172**).

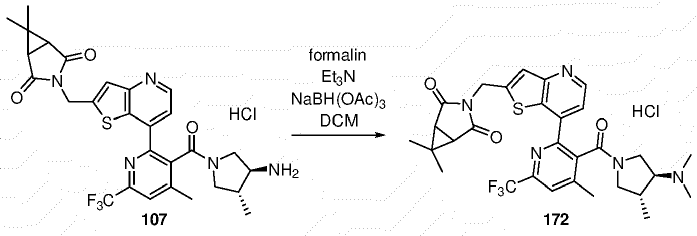

The title compound (**172**) was obtained as a hydrochloride salt from the compound 107 (6.5 mg, 0.0107 mmol) and from the formalin (36 - 38%; 3.3 µL; 0.0424 mmol) according to the General Procedure **VIa** in 82% yield (5.6 mg; 0.0088 mmol) with the exception that in this reaction Et₃N was used instead of AcOH. The crude product was purified by preparative reversed-phase column chromatography (column: Cosmosil Cholester 20 x 250 mm, water + 0.3‰ HCl (36%)/MeCN, 99:1 to 20:80, 30 min, 20 mL/min).
ESI-MS m/z for C₃₀H₃₃F₃N₅O₃3 found 600.4 [M+H]⁺; Rt= 1.18 min; ¹H NMR (700 MHz, Methanol-*d*₄) δ 9.10 - 8.99 (m, 1H), 8.16 - 8.07 (m, 2H), 7.78 - 7.74 (m, 1H), 5.01 - 4.94 (m, 2H), 4.32 - 3.91 (m, 2H), 3.71 - 3.38 (m, 2H), 3.29 - 3.08 (m, 1H), 2.94 - 2.66 (m, 5H), 2.64 - 2.38 (m, 7H), 1.27 - 1.22 (m, 3H), 1.19 - 0.67 (m, 6H).

## Claims

1. A compound of structural Formula (Ia): 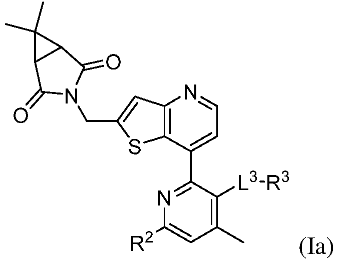 wherein:
R² is selected from hydrogen, methyl, ethyl, chloro, -C≡N, and -CF₃;
L³ represents a single bond, -O-, -CH₂-, -C(=O)-, -NH-, or -NH-C(=O)-; and
R³ is selected from (4-6) membered heterocycloalkyl containing 1 to 2 nitrogen heteroatoms or 1 nitrogen heteroatom and 1 oxygen heteroatom, and optionally substituted with 1 or 2 substituents selected from fluoro, methyl, ethyl, amino, methylamino, dimethylamino, ethylamino, isopropylamino, hydroxy, oxo, aminomethyl, 1-aminoethyl, (isopropylamino)methyl, ureido, alanylamino, valylamino, fluoromethyl, difluoromethyl, trifluoromethyl, and 2,2,2-trifluoroacetyl;
or a tautomer, stereoisomer, a racemic or scalemic mixture of stereoisomers, a pharmaceutically acceptable salt, ester, or solvate thereof.

2. The compound according to claim 1, wherein the compound is:
6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile;
3-((7-(6-chloro-4-methyl-3-(pyrrolidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(((*S*)-piperidin-3-yl)oxy)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-(pyrrolidin-3-ylamino)picolinonitrile;
5-(azetidin-3-ylamino)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
*N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide;
6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione;
3-((7-(3-((3,6-dioxopiperazin-2-yl)methyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-((3-oxomorpholino)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((2-oxopyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((4-methyl-2-oxopiperazin-1-yl)methyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-5-(3-(dimethylamino)azetidine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-((*S*)-3-(dimethylamino)pyrrolidine-1-carbonyl)-4-methylpicolinonitrile;
6,6-dimethyl-3-((7-(4-methyl-3-(piperazine-1-carbonyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(piperazin-1-ylmethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-((*S*)-2-methylpiperazine-1-carbonyl)picolinonitrile;
6,6-dimethyl-3-((7-(4-methyl-3-(morpholine-4-carbonyl)pyridin-2-yl)thieno[3,2-*b*]pyridin-2-yl)methyl)-3-azabicyclo[3.1.0]hexane-2,4-dione;
3-((7-(3-(3,3-difluoropyrrolidine-1-carbonyl)-4-methylpyridin-2-yl)thieno[3,2-*b*]pyridin-2-yl)methyl)-6,6-dimethyl-3-azabicyclo[3.1.0]hexane-2,4-dione;
3-((7-(3-(3,3-difluoroazetidine-1-carbonyl)-4-methylpyridin-2-yl)thieno[3,2-*b*]pyridin-2-yl)methyl)-6,6-dimethyl-3-azabicyclo[3.1.0]hexane-2,4-dione;
5-(3,3-difluoroazetidine-1-carbonyl)-6-(2-((6,6-dimethyl-2,4-dioxo-3-azabicyclo[3.1.0]hexan-3-yl)methyl)thieno[3,2-b]pyridin-7-yl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-((*S*)-2,4-dimethylpiperazine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-5-(3,3-dimethylpiperazine-1-carbonyl)-4-methylpicolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-((*R*)-2-methylpiperazine-1-carbonyl)picolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-(piperazine-1-carbonyl)picolinonitrile;
6-(2-((6,6-dimethyl-2,4-dioxo-3-azabicyclo[3.1.0]hexan-3-yl)methyl)thieno[3,2-*b*]pyridin-7-yl)-4-methyl-5-(morpholine-4-carbonyl)picolinonitrile;
5-(3,3-difluoropyrrolidine-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
3-((7-(3-((*S*)-3-(ethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(isopropylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperazine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((*S*)-3-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((*S*)-2-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-2,4-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(4-methylpiperazine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-3-aminopyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(dimethylamino)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3,3-difluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(morpholine-4-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-(3,3-difluoroazetidine-1-carbonyl)-4,6-dimethylpyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitrile;
6,6-dimethyl-3-((7-(4-methyl-3-(((*S*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*R*)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*S*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((*R*)-4-methylmorpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
3-((7-(3-((*S*)-3-(difluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-(fluoromethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*S*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*,4*R*)-3-amino-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
(2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)-3-methylbutanamide;
(2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)propanamide;
3-((7-(3-(4-aminopiperidine- 1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-hydroxypyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*R*)-3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*R*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(2-oxotetrahydropyrimidin-1(2*H*)-yl)pyridin-2-yl)thieno[3,2-*b*]pyridin-2-yl)methyl)-6,6-dimethyl-3-azabicyclo[3.1.0]hexane-2,4-dione;
3-((7-(3-(azetidin-3-ylamino)-6-chloro-4-methylpyridin-2-yl)thieno[3,2-*b*]pyridin-2-yl)methyl)-6,6-dimethyl-3-azabicyclo[3.1.0]hexane-2,4-dione;
3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-4-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-4-ylamino)-6-(trifluoromethyl)pyridin-2-yl)thieno[3,2-*b*]pyridin-2-yl)methyl)-3-azabicyclo[3.1.0]hexane-2,4-dione;
3-((7-(6-chloro-4-methyl-3-(piperidin-3-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-3-ylamino)-6-(trifluoromethyl)pyridin-2-yl)thieno[3,2-*b*]pyridin-2-yl)methyl)-3-azabicyclo[3.1.0]hexane-2,4-dione;
*N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)azetidine-3-carboxamide;
*N*-(2-(2-((6,6-dimethyl-2,4-dioxo-3-azabicyclo[3.1.0]hexan-3-yl)methyl)thieno[3,2-*b*]pyridin-7-yl)-4,6-dimethylpyridin-3-yl)piperidine-4-carboxamide;
3-((7-(6-chloro-4-methyl-3-(piperazine-1-carbonyl)pyridin-2-yl)thieno[3,2-*b*]pyridin-2-yl)methyl)-6,6-dimethyl-3-azabicyclo[3.1.0]hexane-2,4-dione;
1-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)urea;
3-((7-(3-(3-amino-3-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*S*)-3-(1-aminoethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3-((isopropylamino)methyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((2*S*,6*S*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((2*R*,6*R*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(4-ethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione; or
6,6-dimethyl-3-((7-(4-methyl-3-((*S*)-3-(methylamino)piperidine-1-carbonyl)-6-(trifluoromethyl)pyridin-2-yl)thieno[3,2-*b*]pyridin-2-yl)methyl)-3-azabicyclo[3.1.0]hexane-2,4-dione;
or a tautomer, stereoisomer, a racemic or scalemic mixture of stereoisomers, a pharmaceutically acceptable salt, ester, or solvate thereof.

3. The compound according to claim 2, wherein the compound is:
6-(2-((6,6-dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitrile;
3-((7-(6-chloro-4-methyl-3-(pyrrolidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-3-yloxy)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(((S)-piperidin-3-yl)oxy)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(pyrrolidin-3-ylamino)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-*b*]pyridin-7-yl)-4-methyl-5-(pyrrolidin-3-ylamino)picolinonitrile;
5-(azetidin-3-ylamino)-6-(2-((6,6-dimethyl-2,4-dioxo-3-azabicyclo[3.1.0]hexan-3-yl)methyl)thieno[3,2-*b*]pyridin-7-yl)-4-methylpicolinonitrile;
*N*-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)pyridin-3-yl)azetidine-3-carboxamide;
6,6-dimethyl-3-((7-(4-methyl-3-(piperazin-2-ylmethyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-b]pyridin-7-yl)-4-methyl-5-((*S*)-2-methylpiperazine-1-carbonyl)picolinonitrile;
5-(3,3-difluoroazetidine-1-carbonyl)-6-(2-((6,6-dimethyl-2,4-dioxo-3-azabicyclo[3.1.0]hexan-3-yl)methyl)thieno[3,2-b]pyridin-7-yl)-4-methylpicolinonitrile;
6-(2-((6,6-dimethyl-2,4-dioxo-3-azabicyclo[3.1.0]hexan-3-yl)methyl)thieno[3,2-b]pyridin-7-yl)-5-((*S*)-2,4-dimethylpiperazine-1-carbonyl)-4-methylpicolinonitrile;
6-(2-((6,6-dimethyl-2,4-dioxo-3-azabicyclo[3.1.0]hexan-3-yl)methyl)thieno[3,2-b]pyridin-7-yl)-4-methyl-5-(piperazine-1-carbonyl)picolinonitrile;
5-(3,3-difluoropyrrolidine-1-carbonyl)-6-(2-((6,6-dimethyl-2,4-dioxo-3-azabicyclo[3.1.0]hexan-3-yl)methyl)thieno[3,2-b]pyridin-7-yl)-4-methylpicolinonitrile;
3-((7-(3-((*S*)-3-(ethylamino)pyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((*S*)-3-(isopropylamino)pyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
6,6-dimethyl-3-((7-(4-methyl-3-(piperazine-1-carbonyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((S)-3-(methylamino)pyrrolidine-1-carbonyl)-6-(trifluoromethyl)pyridin-2-yl)thieno[3,2-b]pyridin-2-yl)methyl)-3-azabicyclo[3.1.0]hexane-2,4-dione;
6,6-dimethyl-3-((7-(4-methyl-3-((S)-2-methylpiperazine-1-carbonyl)-6-(trifluoromethyl)pyridin-2-yl)thieno[3,2-b]pyridin-2-yl)methyl)-3-azabicyclo[3.1.0]hexane-2,4-dione;
3-((7-(3-((S)-2,4-dimethylpiperazine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
6,6-dimethyl-3-((7-(4-methyl-3-(4-methylpiperazine-1-carbonyl)-6-(trifluoromethyl)pyridin-2-yl)thieno[3,2-b]pyridin-2-yl)methyl)-3-azabicyclo[3.1.0]hexane-2,4-dione;
3-((7-(3-((S)-3-aminopyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((S)-3-(dimethylamino)pyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-(3,3-difluoropyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
6,6-dimethyl-3-((7-(4-methyl-3-(morpholin-2-ylmethyl)-6-(trifluoromethyl)pyridin-2-yl)thieno[3,2-*b*]pyridin-2-yl)methyl)-3-azabicyclo[3.1.0]hexane-2,4-dione;
6-(2-((6,6-dimethyl-2,4-dioxo-3-azabicyclo[3.1.0]hexan-3-yl)methyl)thieno[3,2-b]pyridin-7-yl)-4-methyl-5-(morpholin-2-ylmethyl)picolinonitrile;
6,6-dimethyl-3-((7-(4-methyl-3-(((S)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((R)-morpholin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-(((R)-4-methylmorpholin-2-yl)methyl)-6-(trifluoromethyl)pyridin-2-yl)thieno[3,2-b]pyridin-2-yl)methyl)-3-azabicyclo[3.1.0]hexane-2,4-dione;
3-((7-(3-((S)-3-(difluoromethyl)pyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((S)-3-(fluoromethyl)pyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((3S,4S)-3-amino-4-methylpyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((3S,4R)-3-amino-4-methylpyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
(2*S*)-2-amino-*N*-((3*S*)-1-(2-(2-((6,6-dimethyl-2,4-dioxo-3-azabicyclo[3.1.0]hexan-3-yl)methyl)thieno[3,2-*b*]pyridin-7-yl)-4-methyl-6-(trifluoromethyl)nicotinoyl)pyrrolidin-3-yl)-3-methylbutanamide;
3-((7-(3-(4-aminopiperidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-hydroxypyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((*S*)-3-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((3*R*,4*R*)-3-amino-4-fluoropyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(azetidin-3-ylamino)-6-chloro-4-methylpyridin-2-yl)thieno[3,2-*b*]pyridin-2-yl)methyl)-6,6-dimethyl-3-azabicyclo[3.1.0]hexane-2,4-dione;
3-((7-(4,6-dimethyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(pyrrolidin-3-ylamino)pyridin-2-yl)thieno[3,2-*b*]pyridin-2-yl)methyl)-6,6-dimethyl-3-azabicyclo[3.1.0]hexane-2,4-dione;
3-((7-(6-chloro-4-methyl-3-(piperidin-4-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-4-ylamino)-6-(trifluoromethyl)pyridin-2-yl)thieno[3,2-*b*]pyridin-2-yl)methyl)-3-azabicyclo[3.1.0]hexane-2,4-dione;
3-((7-(6-chloro-4-methyl-3-(piperidin-3-ylamino)pyridin-2-yl)thieno[3,2-*b*]pyridin-2-yl)methyl)-6,6-dimethyl-3-azabicyclo[3.1.0]hexane-2,4-dione;
6,6-dimethyl-3-((7-(4-methyl-3-(piperidin-3-ylamino)-6-(trifluoromethyl)pyridin-2-yl)thieno[3,2-*b*]pyridin-2-yl)methyl)-3-azabicyclo[3.1.0]hexane-2,4-dione;
3-((7-(3-(3-(aminomethyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-(3-((isopropylamino)methyl)pyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
3-((7-(3-((2*S*,6*S*)-2,6-dimethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione; or
3-((7-(3-(4-ethylpiperazine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
or a tautomer, stereoisomer, a racemic or scalemic mixture of stereoisomers, a pharmaceutically acceptable salt, ester, or solvate thereof.

4. The compound according to claim 1, wherein the compound is:
6,6-dimethyl-3-((7-(4-methyl-3-(((*S*)-3-(methylamino)pyrrolidin-1-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((3-oxopiperazin-2-yl)methyl)-6-(trifluoromethyl)pyridin-2-yl)thieno[3,2-*b*]pyridin-2-yl)methyl)-3-azabicyclo[3.1.0]hexane-2,4-dione;
6,6-dimethyl-3-((7-(4-methyl-3-((3*R*,4*S*)-3-methyl-4-(methylamino)pyrrolidine-1-carbonyl)-6-(trifluoromethyl)pyridin-2-yl)thieno[3,2-*b*]pyridin-2-yl)methyl)-3-azabicyclo[3.1.0]hexane-2,4-dione;
3-((7-(3-((3R,4S)-3-amino-4-fluoropyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((3S,4S)-3-amino-4-fluoropyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione;
3-((7-(3-((3S,4R)-3-amino-4-fluoropyrrolidine-1-carbonyl)-4-methyl-6-(trifluoromethyl)pyridin-2-yl)thieno[3,2-b]pyridin-2-yl)methyl)-6,6-dimethyl-3-azabicyclo[3.1.0]hexane-2,4-dione; or
3-((7-(3-((3*S*,4*R*)-3-(dimethylamino)-4-methylpyrrolidine-1-carbonyl)-4-methyl-6-(trifluoromethyl)pyridin-2-yl)thieno[3,2-*b*]pyridin-2-yl)methyl)-6,6-dimethyl-3-azabicyclo[3.1.0]hexane-2,4-dione;
or a tautomer, stereoisomer, a racemic or scalemic mixture of stereoisomers, a pharmaceutically acceptable salt, ester, or solvate thereof.

5. A pharmaceutical composition comprising (i) a therapeutically effective amount of at least one compound of claim 1, or a pharmaceutically acceptable salt, or solvate thereof; and (ii) a pharmaceutically acceptable carrier, vehicle or excipient therefor.

6. A compound of claim 1, or a pharmaceutically acceptable salt, or solvate thereof, for use in a method for inhibiting USP7 in a cell or a tissue, comprising contacting the cell or the tissue with at least one compound of claim 1, or a pharmaceutically acceptable salt, or solvate thereof.

7. Use of a compound according to claim 1, or a pharmaceutically acceptable salt, or solvate thereof, in the manufacturing of a medicament for the treatment of a disease, disorder, or condition associated with expression of USP7.

8. A compound of claim 1, or a pharmaceutically acceptable salt, or solvate thereof, for use in a method for the treatment or prevention of a disease, disorder, or condition associated with aberrant expression or activity of USP7, comprising administering to the subject in need thereof a therapeutically effective amount of at least one compound of claim 1, or a pharmaceutically acceptable salt, or solvate thereof, or a pharmaceutical composition according to claim 5.

9. A compound for use according to claim 8, wherein the disease, disorder, or condition is selected from the group consisting of cardiovascular disorders, pulmonary disorders, autoimmune disorders, immune disorders, immunoregulatory disorders, neurodegenerative disorders, metabolic disorders, hemolytic disorders, gastrointestinal disorders, sexual disorders, infections, wound healing disorders, and cancers.

10. A compound for use according to claim 9, wherein the disease, disorder, or condition is a cardiovascular disorder selected from the group consisting of systemic hypertension, pulmonary arterial hypertension (PAH), pulmonary arterial hypertension in high altitude, ischemia reperfusion (IR) injury, myocardial infarction, and atherosclerosis.

11. A compound for use according to claim 10, wherein the cardiovascular disorder is pulmonary arterial hypertension (PAH).

12. A compound for use according to claim 10, wherein the cardiovascular disorder is ischemia reperfusion (IR) injury selected from the group consisting of liver IR, kidney IR, and myocardial IR.

13. A compound for use according to claim 10, wherein the cardiovascular disorder is myocardial infarction or atherosclerosis.

14. A compound for use according to claim 9, wherein the disease, disorder, or condition is a pulmonary disorder selected from the group consisting of chemically-induced lung fibrosis, idiopathic pulmonary fibrosis, cystic fibrosis, chronic obstructive pulmonary disease (COPD), and asthma.

15. A compound for use according to claim 9, wherein the disease, disorder, or condition is an autoimmune disorder selected from the group consisting of encephalomyelitis, multiple sclerosis, anti-phospholipid syndrome 1, autoimmune hemolytic anemia, chronic inflammatory demyelinating polyradiculoneuropathy, psoriasis, dermatitis herpetiformis, dermatomyositis, myasthenia gravis, pemphigus, rheumatoid arthritis, stiff-person syndrome, type 1 diabetes, ankylosing spondylitis, paroxysmal nocturnal hemoglobinuria (PNH), paroxysmal cold hemoglobinuria, severe idiopathic autoimmune hemolytic anemia, and Goodpasture's syndrome.

16. A compound for use according to claim 9, wherein the disease, disorder, or condition is an immune disorder selected from the group consisting of T-cell dysfunction mediated by myeloid-derived suppressor cells (MDSC), human immunodeficiency virus (HIV) infection, autoimmune encephalomyelitis, and AB0 mismatch transfusion reaction.

17. A compound for use according to claim 16, wherein the immune disorder is T-cell dysfunction mediated by myeloid-derived suppressor cells (MDSC).

18. A compound for use according to claim 9, wherein the disease, disorder, or condition is a disease resulting from an immunoregulatory disorder selected from the group consisting of renal disease inflammation, hepatic fibrosis, leishmaniosis, neurodegenerative diseases, wound healing, human immunodeficiency virus (HIV) infection, hepatitis B virus (HBV) infection, *Helicobacter pylori* infection, fibrotic disorders, arthritis, candidiasis, periodontal disease, keloids, adenotonsilar disease, African sleeping sickness, Chagas' disease, and transplant rejection.

19. A compound for use according to claim 9, wherein the disease, disorder, or condition is a neurodegenerative disorder selected from the group consisting of Alzheimer's disease, Parkinson's disease, Huntington's disease, extrapyramidal syndrome, dystonia, akathisia, epilepsy, periodic limb movement, and dementia.

20. A compound for use according to claim 9, wherein the disease, disorder, or condition is a metabolic disorder selected from the group consisting of diabetes, non-alcoholic steatohepatitis (NASH), and non-alcoholic fatty liver disease (NAFLD).

21. A compound for use according to claim 9, wherein the disease, disorder, or condition is a hemolytic disorder selected from the group consisting of sickle-cell disease, thalassemias, hereditary spherocytosis, stomatocytosis, microangiopathic hemolytic anemias, pyruvate kinase deficiency, infection-induced anemia, cardiopulmonary bypass, mechanical heart valve-induced anemia, and chemical-induced anemia.

22. A compound for use according to claim 21, wherein the hemolytic disorder is sickle-cell disease.

23. A compound for use according to claim 9, wherein the disease, disorder, or condition is a gastrointestinal disorder selected from the group consisting of gastrointestinal motility disorders, gastric cancers, inflammatory bowel disease, Crohn's disease, ulcerative colitis, and gastric ulcers.

24. A compound for use according to claim 9, wherein the disease, disorder, or condition is a sexual disorder selected from the group consisting of Peyronie's disease, and erectile dysfunction.

25. A compound for use according to claim 9, wherein the disease, disorder, or condition is a wound healing disorder selected from the group consisting of infected and uninfected wound healing.

26. A compound for use according to claim 9, wherein the disease, disorder, or condition is a cancer selected from the group consisting of oesophagic, gastric, colon, ovary, breast, pancreatic, head-and-neck, bladder, and lung cancers (including squamous and non-small cell lung carcinoma), renal cell carcinoma, prostate carcinoma, multiple myeloma, neuroblastoma, glioblastoma, astrocytoma, mesothelioma and melanoma, B cells, T cells and NK cells lymphomas, acute and chronic, myeloid leukemia, and lymphoid leukemia.

27. A compound for use according to claim 9, wherein the disease, disorder, or condition is a cancer selected from the group consisting of gastric cancer (including, but not limited to, gastric or gastroesophageal junction cancer), colorectal cancer, pancreatic cancer, liver cancer, breast cancer, lung cancers (including, but not limited to, non-small cell lung carcinoma), renal cell carcinoma, prostate carcinoma, multiple myeloma, acute and chronic leukemias, T cell, B cell and NK cell lymphomas, brain tumors (including, but not limited to, neuroblastoma, glioblastoma, astrocytoma), squamous-cell carcinomas of the head and neck, and melanoma.

28. A compound for use according to claim 9, wherein the disease, disorder, or condition is a cancer selected from the group consisting of chronic lymphocytic leukemia, acute lymphoblastic leukemia, chronic myelogenous leukemia, acute myeloid leukemia, adrenocortical carcinoma, anal cancer, appendix cancer, atypical teratoid/rhabdoid tumor, basal cell carcinoma, bile duct cancer, bladder cancer, bone cancer, brain tumor, brain and spinal cord tumor, brain stem glioma, central nervous system atypical teratoid/rhabdoid tumor, central nervous system embryonal tumors, breast cancer, bronchial tumors, Burkitt lymphoma, carcinoid tumor, carcinoma of unknown primary, central nervous system cancer, cervical cancer, childhood cancers, chordoma, chronic myeloproliferative disorders, colon cancer, craniopharyngioma, cutaneous T-cell lymphoma, ductal carcinoma in situ, embryonal tumors, endometrial cancer, ependymoblastoma, ependymoma, esophageal cancer, esthesioneuroblastoma, Ewing sarcoma, extracranial germ cell tumor, extrahepatic bile duct cancer, eye cancer, fibrous histiocytoma of bone, gallbladder cancer, gastric cancer, gastrointestinal carcinoid tumor, gastrointestinal stromal tumors, germ cell tumor, extracranial germ cell tumor, extragonadal germ cell tumor, ovarian germ cell tumor, gestational trophoblastic tumor, glioma, hairy cell leukemia, head and neck cancer, heart cancer, hepatocellular cancer, histiocytosis, Langerhans cell cancer, Hodgkin's lymphoma, hypopharyngeal cancer, intraocular melanoma, islet cell tumors, Kaposi sarcoma, kidney cancer, Langerhans cell histiocytosis, laryngeal cancer, lip and oral cavity cancer, lobular carcinoma in situ, lymphoma, AIDS-related lymphoma, macroglobulinemia, male breast cancer, medulloblastoma, medulloepithelioma, Merkel cell carcinoma, malignant mesothelioma, metastatic squamous neck cancer, midline tract carcinoma involving NUT gene, mouth cancer, multiple endocrine neoplasia syndrome, plasma cell neoplasm, mycosis fungoides, myeloma, chronic myeloproliferative disorder, nasal cavity cancer, paranasal sinus cancer, nasopharyngeal cancer, non-Hodgkin's lymphoma, oral cancer, oral cavity cancer, lip cancer, oropharyngeal cancer, osteosarcoma, ovarian cancer, papillomatosis, paraganglioma, paranasal sinus cancer, nasal cavity cancer, parathyroid cancer, penile cancer, pharyngeal cancer, pheochromocytoma, pineal parenchymal tumors of intermediate differentiation, pineoblastoma, pituitary tumor, plasma cell neoplasm, pleuropulmonary blastoma, primary central nervous system lymphoma, rectal cancer, renal cell cancer, renal pelvis cancer, ureter cancer, transitional cell cancer, retinoblastoma, rhabdomyosarcoma, salivary gland cancer, sarcoma, Sézary syndrome, skin cancer, small cell lung cancer, small intestine cancer, soft tissue sarcoma, squamous cell carcinoma, squamous neck cancer with occult primary, supratentorial primitive neuroectodermal tumors, testicular cancer, throat cancer, thymoma, thymic carcinoma, thyroid cancer, transitional cell cancer of the renal pelvis and ureter, gestational trophoblastic tumor, unknown primary, unusual cancer of childhood, urethral cancer, uterine cancer, uterine sarcoma, Waldenströms macroglobulinemia, or Wilms' tumor.

29. A compound for use according to claim 9, wherein the at least one compound of claim 1 is administered simultaneously or sequentially with a therapeutically effective amount of one or more other therapeutic agent(s) selected from the group consisting of anti-viral agents, chemotherapeutic agents (including alkylating antineoplastic agents, antimetabolites, anti-microtubule agents), immunosuppressants, anti-tumor vaccines, antiviral vaccines, cytokine therapy, tyrosine kinase inhibitors, immunotherapeutic agents, including checkpoint inhibitors such as PD-1, PD-L1 or CTLA-4 inhibitors and IDO/TDO inhibitors, adenosine A2A receptor antagonists, ectonucleotidase (CD73 and CD39) inhibitors, agent affecting interleukins, cytokines and chemokines, topoisomerase inhibitors, and cytotoxic antibiotics, or targeted therapies comprising antibodies, antibody drug conjugates, cell-based immunotherapy, nanoparticles, and radiotherapy.

30. A compound for use according to claim 29, wherein the antibodies comprise a therapeutically effective amount of anti-PD-1, anti-PD-L1 or anti-CTLA4 antibodies.

31. A compound of claim 1 for use in a method for T lymphocyte cell *in vitro* or *ex vivo* activation for releasing cytokines, comprising contacting the cell with at least one compound of claim 1, or a pharmaceutically acceptable salt, or solvate thereof.

32. Use of a compound of claim 1, or a pharmaceutically acceptable salt, or solvate thereof, for protecting an organ outside the human or animal body during transport.

## Patentansprüche

1. Eine Verbindung mit der Strukturformel (Ia): 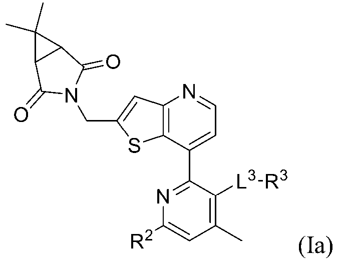 wobei:
R² aus Wasserstoff, Methyl, Ethyl, Chlor, -C≡N und -CF₃ ausgewählt ist;
L³ für eine Einfachbindung, -O-, -CH₂-, -C(=O)-, -NH-, oder -NH-C(=O)- steht; und
R³ aus einem (4-6)-gliedrigen Heterozykloalkyl ausgewählt wird, der 1 bis 2 Stickstoff-Heteroatome oder 1 Stickstoff-Heteroatom und 1 Sauerstoff-Heteroatom enthält und optional mit 1 oder 2 Substituenten ersetzt ist, die wiederrum aus Fluor, Methyl, Ethyl, Amin, Methylamin, Dimethylamin, Ethylamin, Isopropylamin, Hydroxy, Oxo, Aminomethyl, 1-Aminoethyl, (Isopropylamino)methyl, Ureido, Alanylamin, Valylamin, Fluormethyl, Difluormethyl, Trifluormethyl und 2,2,2-Trifluoracetyl ausgewählt sind;
oder ein Tautomer, ein Stereoisomer, ein racemisches oder
enantiomerenangereichertes (skalemisches) Gemisch von Stereoisomeren, ein pharmazeutisch zulässiges Salz, ein Ester oder ein Solvat davon.

2. Eine Verbindung gemäß Anspruch 1, wobei diese Verbindung ist:
6-(2-((6,6-Dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitril;
3-((7-(6-Chlor-4-methyl-3-(pyrrolidin-3-yloxy)pyridin-2-yl)thieno[3,*2-b*]pyridin-2-yl)methyl)-6,6-dimethyl-3-azabicyclo[3.1.0]hexan-2,4-dion;
3-((7-(6-Chlor-4-methyl-3-(piperidin-3-yloxy)pyridin-2-yl)thieno[3,*2-b*]pyridin-2-yl)methyl)-6,6-dimethyl-3-azabicyclo[3.1.0]hexan-2,4-dion;
3-((7-(6-Chlor-4-methyl-3-(((*S*)-piperidin-3-yl)oxy)pyridin-2-yl)thieno[3,*2-b*]pyridin-2-yl)methyl)-6,6-dimethyl-3-azabicyclo[3.1.0]hexan-2,4-dion;
6,6-Dimethyl-3-((7-(4-methyl-3-(pyrrolidin-3-ylamino)-6-(trifluormethyl)pyridin-2-yl)thieno[3,*2-b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,*2-b*]pyridin-7-yl)-4-methyl-5-(pyrrolidin-3-ylamino)picolinonitril;
5-(Azetidin-3-ylamino)-6-(2-((6,6-dimethyl-2,4-dioxo-3-azabicyclo[3.1.0]hexan-3-yl)methyl)thieno[3,*2-b*]pyridin-7-yl)-4-methylpicolinnitril;
*N-*(2-(2-((6,6-Dimethyl-2,4-dioxo-3-azabicyclo[3.1.0]hexan-3-yl)methyl)thieno[3,2-*b*]pyridin-7-yl)-4-methyl-6-(trifluormethyl)pyridin-3-yl)azetidin-3-carboxamid;
6,6-Dimethyl-3-((7-(4-methyl-3-(piperazin-2-ylmethyl)-6-(trifluormethyl)pyridin-2-yl)thieno[3,*2-b*]pyridin-2-yl)methyl)-3-azabicyclo[3.1.0]hexan-2,4-dion;
3-((7-(3-((3,6-Dioxopiperazin-2-yl)methyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-((3-oxomorpholino)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-Methyl-3-((2-Oxopyrrolidin-1-yl)methyl)-6-(trifluormethyl)pyridin-2-yl)thieno[3,2-*b*]pyridin-2-yl)methyl)-3-Azabicyclo[3.1.0]hexan-2,4-dion;
6,6-Dimethyl-3-((7-(4-Methyl-3-((2-Oxopiperazin-1-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-Methyl-3-((4-Methyl-2-oxopiperazin-1-yl)methyl)-6-(trifluormethyl)pyridin-2-yl)thieno[3,2-*b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,2-*b*]pyridin-7-yl)-5-(3-(dimethylamino)azetidin-1-carbonyl)-4-methylpicolinnitril;
6-(2-((6,6-Dimethyl-2,4-dioxo-3-azabicyclo[3.1.0]hexan-3-yl)methyl)thieno[3,2-*b*]pyridin-7-yl)-5-((*S*)-3-(Dimethylamino)pyrrolidin-1-carbonyl)-4-methylpicolinnitril;
6,6-Dimethyl-3-((7-(4-methyl-3-(piperazin-1-carbonyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-(piperazin-1-ylmethyl)pyridin-2-yl)thieno[3,2-*b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,2-*b*]pyridin-7-yl)-4-methyl-5-((*S*)-2-methylpiperazin-1-carbonyl)picolinnitril;
6,6-Dimethyl-3-((7-(4-methyl-3-(morpholin-4-carbonyl)pyridin-2-yl)thieno[3,2-*b*]pyridin-2-yl)methyl)-3-azabicyclo[3.1.0]hexan-2,4-dion;
3-((7-(3-(3,3-Difluorpyrrolidin-1-carbonyl)-4-methylpyridin-2-yl)thieno[3,2-*b*]pyridin-2-yl)methyl)-6,6-dimethyl-3-azabicyclo[3.1.0]hexan-2,4-dion;
3-((7-(3-(3,3-Difluorazetidin-1-carbonyl)-4-methylpyridin-2-yl)thieno[3,2-*b*]pyridin-2-yl)methyl)-6,6-dimethyl-3-azabicyclo[3.1.0]hexan-2,4-dion;
5-(3,3-Difluorazetidin-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinnitril;
6-(2-((6,6-Dimethyl-2,4-dioxo-3-azabicyclo[3.1.0]hexan-3-yl)methyl)thieno[3,2-*b*]pyridin-7-yl)-5-((*S*)-2,4-Dimethylpiperazin-1-carbonyl)-4-methylpicolinnitril;
6-(2-((6,6-Dimethyl-2,4-dioxo-3-azabicyclo[3.1.0]hexan-3-yl)methyl)thieno[3,2-*b*]pyridin-7-yl)-5-(3,3-Dimethylpiperazin-1-carbonyl)-4-methylpicolinnitril;
6-(2-((6,6-Dimethyl-2,4-dioxo-3-azabicyclo[3.1.0]hexan-3-yl)methyl)thieno[3,2-*b*]pyridin-7-yl)-4-methyl-5-((*R*)-2-methylpiperazin-1-carbonyl)picolinonitril;
6-(2-((6,6-Dimethyl-2,4-dioxo-3-azabicyclo[3.1.0]hexan-3-yl)methyl)thieno[3,2-*b*]pyridin-7-yl)-4-methyl-5-(piperazin-1-carbonyl)picolinonitril;
6-(2-((6,6-Dimethyl-2,4-dioxo-3-azabicyclo[3.1.0]hexan-3-yl)methyl)thieno[3,2-*b*]pyridin-7-yl)-4-methyl-5-(morpholin-4-carbonyl)picolinonitril;
5-(3,3-Difluorpyrrolidin-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinnitril;
3-((7-(3-((*S*)-3-(Ethylamino)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-(Isopropylamino)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(piperazin-1-carbonyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-((*S*)-3-(Methylamino)pyrrolidin-1-carbonyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-((*S*)-2-Methylpiperazin-1-carbonyl)-6-(trifluormethyl)pyridin-2-yl)thieno[3,2-*b*]pyridin-2-yl)methyl)-3-azabicyclo[3.1.0]hexan-2,4-dion;
3-((7-(3-((*S*)-2,4-Dimethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(4-methylpiperazin-1-carbonyl)-6-(trifluormethyl)pyridin-2-yl)thieno[3,2-*b*]pyridin-2-yl)methyl)-3-azabicyclo[3.1.0]hexan-2,4-dion;
3-((7-(3-((*S*)-3-Aminopyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-(Dimethylamino)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(3,3-Difluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(morpholin-4-carbonyl)-6-(trifluormethyl)pyridin-2-yl)thieno[3,2-*b*]pyridin-2-yl)methyl)-3-azabicyclo[3.1.0]hexan-2,4-dion;
3-((7-(3-(3,3-Difluorazetidin-1-carbonyl)-4,6-dimethylpyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(morpholin-2-ylmethyl)-6-(trifluormethyl)pyridin-2-yl)thieno[3,2-*b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitril;
6,6-Dimethyl-3-((7-(4-methyl-3-(((*S*)-Morpholin-2-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-(((*R*)-Morpholin-2-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-(((*S*)-4-Methylmorpholin-2-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-(((*R*)-4-Methylmorpholin-2-yl)methyl)-6-(trifluormethyl)pyridin-2-yl)thieno[3,2-*b*]pyridin-2-yl)methyl)-3-azabicyclo[3.1.0]hexan-2,4-dion;
3-((7-(3-((*S*)-3-(Difluormethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-(Fluormethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*,*4S*)-3-Amino-4-methylpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*,*4R*)-3-Amino-4-methylpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
(*2S*)*-2-Amino-N-*((*3S*)-1-(2-(2-((6,6-Dimethyl-2,4-dioxo-3-azabicyclo[3.1.0]hexan-3-yl)methyl)thieno[3,2-*b*]pyridin-7-yl)-4-methyl-6-(trifluormethyl)nicotinoyl)pyrrolidin-3-yl)-3-methylbutanamid;
(*2S*)*-2-Amino-N-*((*3S*)-1-(2-(2-((6,6-Dimethyl-2,4-dioxo-3-azabicyclo[3.1.0]hexan-3-yl)methyl)thieno[3,2-*b*]pyridin-7-yl)-4-methyl-6-(trifluormethyl)nicotinoyl)pyrrolidin-3-yl)propanamid;
3-((7-(3-(4-Aminopiperidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-Hydroxypyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-Fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*R*)-3-(Aminomethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3R*,*4R*)-3-Amino-4-fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(6-Chlor-4-methyl-3-(2-oxotetrahydropyrimidin-1(*2H*)-yl)pyridin-2-yl)thieno[3,2-*b*]pyridin-2-yl)methyl)-6,6-dimethyl-3-azabicyclo[3.1.0]hexan-2,4-dion;
3-((7-(3-(Azetidin-3-ylamino)-6-chlor-4-methylpyridin-2-yl)thieno[3,2-*b*]pyridin-2-yl)methyl)-6,6-dimethyl-3-azabicyclo[3.1.0]hexan-2,4-dion;
3-((7-(4,6-Dimethyl-3-(pyrrolidin-3-ylamino)pyridin-2-yl)thieno[3,2-*b*]pyridin-2-yl)methyl)-6,6-dimethyl-3-azabicyclo[3.1.0]hexan-2,4-dion;
3-((7-(6-Chlor-4-methyl-3-(pyrrolidin-3-ylamino)pyridin-2-yl)thieno[3,2-*b*]pyridin-2-yl)methyl)-6,6-dimethyl-3-azabicyclo[3.1.0]hexan-2,4-dion;
3-((7-(6-Chlor-4-methyl-3-(piperidin-4-ylamino)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(piperidin-4-ylamino)-6-(trifluormethyl)pyridin-2-yl)thieno[3,2-*b*]pyridin-2-yl)methyl)-3-azabicyclo[3.1.0]hexan-2,4-dion;
3-((7-(6-Chlor-4-methyl-3-(piperidin-3-ylamino)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(piperidin-3-ylamino)-6-(trifluormethyl)pyridin-2-yl)thieno[3,2-*b*]pyridin-2-yl)methyl)-3-azabicyclo[3.1.0]hexan-2,4-dion;
*N-*(2-(2-((6,6-Dimethyl-2,4-dioxo-3-azabicyclo[3.1.0]hexan-3-yl)methyl)thieno[3,2-*b*]pyridin-7-yl)-4,6-dimethylpyridin-3-yl)azetidin-3-carboxamid;
*N-*(2-(2-((6,6-Dimethyl-2,4-dioxo-3-azabicyclo[3.1.0]hexan-3-yl)methyl)thieno[3,2-*b*]pyridin-7-yl)-4,6-dimethylpyridin-3-yl)piperidin-4-carboxamid;
3-((7-(6-Chlor-4-methyl-3-(piperazin-1-carbonyl)pyridin-2-yl)thieno[3,2-*b*]pyridin-2-yl)methyl)-6,6-dimethyl-3-azabicyclo[3.1.0]hexan-2,4-dion;
1-((*3S*)-1-(2-(2-((6,6-Dimethyl-2,4-dioxo-3-azabicyclo[3.1.0]hexan-3-yl)methyl)thieno[3,2-*b*]pyridin-7-yl)-4-methyl-6-(trifluormethyl)nicotinoyl)pyrrolidin-3-yl)harnstoff;
3-((7-(3-(3-Amino-3-methylpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*)-3-(1-Aminoethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(3-(Aminomethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(3-((Isopropylamino)methyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*2S*,*6S*)-2,6-Dimethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*2R*,*6R*)-2,6-Dimethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(4-Ethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion; oder
6,6-Dimethyl-3-((7-(4-methyl-3-((*S*)-3-(Methylamino)piperidin-1-carbonyl)-6-(trifluormethyl)pyridin-2-yl)thieno[3,2-*b*]pyridin-2-yl)methyl)-3-azabicyclo[3.1.0]hexan-2,4-dion;
oder ein Tautomer, ein Stereoisomer, ein racemisches oder
enantiomerenangereichertes (skalemisches) Gemisch von Stereoisomeren, ein pharmazeutisch zulässiges Salz, ein Ester oder ein Solvat davon.

3. Eine Verbindung gemäß Anspruch 2, wobei diese Verbindung ist:
6-(2-((6,6-Dimethyl-2,4-dioxo-3-azabicyclo[3.1.0]hexan-3-yl)methyl)thieno[3,2-*b*]pyridin-7-yl)-4-methyl-5-(piperidin-3-yloxy)picolinonitril;
3-((7-(6-Chlor-4-methyl-3-(pyrrolidin-3-yloxy)pyridin-2-yl)thieno[3,*2-b*]pyridin-2-yl)methyl)-6,6-dimethyl-3-azabicyclo[3.1.0]hexan-2,4-dion;
3-((7-(6-Chlor-4-methyl-3-(piperidin-3-yloxy)pyridin-2-yl)thieno[3,*2-b*]pyridin-2-yl)methyl)-6,6-dimethyl-3-azabicyclo[3.1.0]hexan-2,4-dion;
3-((7-(6-Chlor-4-methyl-3-(((*S*)-piperidin-3-yl)oxy)pyridin-2-yl)thieno[3,*2-b*]pyridin-2-yl)methyl)-6,6-dimethyl-3-azabicyclo[3.1.0]hexan-2,4-dion;
6,6-Dimethyl-3-((7-(4-methyl-3-(pyrrolidin-3-ylamino)-6-(trifluormethyl)pyridin-2-yl)thieno[3,*2-b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,*2-b*]pyridin-7-yl)-4-methyl-5-(pyrrolidin-3-ylamino)picolinonitril;
5-(Azetidin-3-ylamino)-6-(2-((6,6-dimethyl-2,4-dioxo-3-azabicyclo[3.1.0]hexan-3-yl)methyl)thieno[3,*2-b*]pyridin-7-yl)-4-methylpicolinnitril;
*N-*(2-(2-((6,6-Dimethyl-2,4-dioxo-3-azabicyclo[3.1.0]hexan-3-yl)methyl)thieno[3,2-*b*]pyridin-7-yl)-4-methyl-6-(trifluormethyl)pyridin-3-yl)azetidin-3-carboxamid;
6,6-Dimethyl-3-((7-(4-methyl-3-(piperazin-2-ylmethyl)-6-(trifluormethyl)pyridin-2-yl)thieno[3,*2-b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,2-*b*]pyridin-7-yl)-4-methyl-5-((*S*)-2-methylpiperazin-1-carbonyl)picolinnitril;
5-(3,3-Difluorazetidin-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinnitril;
6-(2-((6,6-Dimethyl-2,4-dioxo-3-azabicyclo[3.1.0]hexan-3-yl)methyl)thieno[3,2-*b*]pyridin-7-yl)-5-((*S*)-2,4-Dimethylpiperazin-1-carbonyl)-4-methylpicolinnitril;
6-(2-((6,6-Dimethyl-2,4-dioxo-3-azabicyclo[3.1.0]hexan-3-yl)methyl)thieno[3,2-*b*]pyridin-7-yl)-4-methyl-5-(piperazin-1-carbonyl)picolinonitril;
5-(3,3-Difluorpyrrolidin-1-carbonyl)-6-(2-((6,6-dimethyl-2,4-dioxo-3-azabicyclo[3.1.0]hexan-3-yl)methyl)thieno[3,2-*b*]pyridin-7-yl)-4-methylpicolinnitril;
3-((7-(3-((*S*)-3-(Ethylamino)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-(Isopropylamino)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(piperazin-1-carbonyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-((*S*)-3-(Methylamino)pyrrolidin-1-carbonyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-((*S*)-2-Methylpiperazin-1-carbonyl)-6-(trifluormethyl)pyridin-2-yl)thieno[3,2-*b*]pyridin-2-yl)methyl)-3-azabicyclo[3.1.0]hexan-2,4-dion;
3-((7-(3-((*S*)-2,4-Dimethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(4-methylpiperazin-1-carbonyl)-6-(trifluormethyl)pyridin-2-yl)thieno[3,2-*b*]pyridin-2-yl)methyl)-3-azabicyclo[3.1.0]hexan-2,4-dion;
3-((7-(3-((*S*)-3-Aminopyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-(Dimethylamino)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(3,3-Difluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(morpholin-2-ylmethyl)-6-(trifluormethyl)pyridin-2-yl)thieno[3,2-*b*]pyridin-2-yl)methyl)-3-azabicyclo[3.1.0]hexan-2,4-dion;
6-(2-((6,6-Dimethyl-2,4-dioxo-3-azabicyclo[3.1.0]hexan-3-yl)methyl)thieno[3,2-*b*]pyridin-7-yl)-4-methyl-5-(morpholin-2-ylmethyl)picolinonitril;
6,6-Dimethyl-3-((7-(4-methyl-3-(((*S*)-Morpholin-2-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-(((*R*)-Morpholin-2-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-(((*R*)-4-Methylmorpholin-2-yl)methyl)-6-(trifluormethyl)pyridin-2-yl)thieno[3,2-*b*]pyridin-2-yl)methyl)-3-azabicyclo[3.1.0]hexan-2,4-dion;
3-((7-(3-((*S*)-3-(Difluormethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-(Fluormethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*,*4S*)-3-Amino-4-methylpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*,*4R*)-3-Amino-4-methylpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
(*2S*)*-2-Amino-N-*((*3S*)-1-(2-(2-((6,6-Dimethyl-2,4-dioxo-3-azabicyclo[3.1.0]hexan-3-yl)methyl)thieno[3,2-*b*]pyridin-7-yl)-4-methyl-6-(trifluormethyl)nicotinoyl)pyrrolidin-3-yl)-3-methylbutanamid;
3-((7-(3-(4-Aminopiperidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-Hydroxypyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*S*)-3-Fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3R*,*4R*)-3-Amino-4-fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(Azetidin-3-ylamino)-6-chlor-4-methylpyridin-2-yl)thieno[3,2-*b*]pyridin-2-yl)methyl)-6,6-dimethyl-3-azabicyclo[3.1.0]hexan-2,4-dion;
3-((7-(4,6-Dimethyl-3-(pyrrolidin-3-ylamino)pyridin-2-yl)thieno[3,2-*b*]pyridin-2-yl)methyl)-6,6-dimethyl-3-azabicyclo[3.1.0]hexan-2,4-dion;
3-((7-(6-Chlor-4-methyl-3-(pyrrolidin-3-ylamino)pyridin-2-yl)thieno[3,2-*b*]pyridin-2-yl)methyl)-6,6-dimethyl-3-azabicyclo[3.1.0]hexan-2,4-dion;
3-((7-(6-Chlor-4-methyl-3-(piperidin-4-ylamino)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(piperidin-4-ylamino)-6-(trifluormethyl)pyridin-2-yl)thieno[3,2-*b*]pyridin-2-yl)methyl)-3-azabicyclo[3.1.0]hexan-2,4-dion;
3-((7-(6-Chlor-4-methyl-3-(piperidin-3-ylamino)pyridin-2-yl)thieno[3,2-*b*]pyridin-2-yl)methyl)-6,6-dimethyl-3-azabicyclo[3.1.0]hexan-2,4-dion;
6,6-Dimethyl-3-((7-(4-methyl-3-(piperidin-3-ylamino)-6-(trifluormethyl)pyridin-2-yl)thieno[3,2-*b*]pyridin-2-yl)methyl)-3-azabicyclo[3.1.0]hexan-2,4-dion;
3-((7-(3-(3-(Aminomethyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-(3-((Isopropylamino)methyl)pyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*2S*,*6S*)-2,6-Dimethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion; oder
3-((7-(3-(4-Ethylpiperazin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
oder ein Tautomer, ein Stereoisomer, ein racemisches oder
enantiomerenangereichertes (skalemisches) Gemisch von Stereoisomeren, ein pharmazeutisch zulässiges Salz, ein Ester oder ein Solvat davon.

4. Eine Verbindung gemäß Anspruch 1, wobei diese Verbindung ist:
6,6-Dimethyl-3-((7-(4-methyl-3-(((*S*)-3-(Methylamino)pyrrolidin-1-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-Methyl-3-((3-Oxopiperazin-2-yl)methyl)-6-(trifluormethyl)pyridin-2-yl)thieno[3,2-*b*]pyridin-2-yl)methyl)-3-azabicyclo[3.1.0]hexan-2,4-dion;
6,6-Dimethyl-3-((7-(4-methyl-3-((*3R*,*4S*)-3-Methyl-4-(methylamino)pyrrolidin-1-carbonyl)-6-(trifluormethyl)pyridin-2-yl)thieno[3,2-*b*]pyridin-2-yl)methyl)-3-azabicyclo[3.1.0]hexan-2,4-dion;
3-((7-(3-((*3R*,*4S*)-3-Amino-4-fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*,*4S*)-3-Amino-4-fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
3-((7-(3-((*3S*,*4R*)-3-Amino-4-fluorpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion; oder
3-((7-(3-((*3S*,*4R*)-3-(Dimethylamino)-4-methylpyrrolidin-1-carbonyl)-4-methyl-6-(trifluormethyl)pyridin-2-yl)thieno[3,2-*b*]pyridin-2-yl)methyl)-6,6-dimethyl-3-azabicyclo[3.1.0]hexan-2,4-dion;
oder ein Tautomer, ein Stereoisomer, ein racemisches oder
enantiomerenangereichertes (skalemisches) Gemisch von Stereoisomeren, ein pharmazeutisch zulässiges Salz, ein Ester oder ein Solvat davon.

5. Ein pharmazeutisches Gemisch, das (i) eine therapeutisch wirksame Menge von mindestens einer Verbindung gemäß Anspruch 1 oder eines pharmazeutisch zulässigen Salzes oder Solvats davon; und (ii) einen pharmazeutisch zulässigen Trägerstoff, ein zulässiges Vehikel oder einen zulässigen Hilfsstoff dafür enthält.

6. Eine Verbindung gemäß Anspruch 1 oder ein pharmazeutisch zulässiges Salz oder Solvat davon, zur Verwendung in einem Verfahren zur Unterdrückung von USP7 in einer Zelle oder einem Gewebe, wobei die Zelle oder das Gewebe mit mindestens einer Verbindung gemäß Anspruch 1 oder einem pharmazeutisch zulässigen Salz oder Solvat davon in Kontakt gebracht wird.

7. Verwendung einer Verbindung gemäß Anspruch 1 oder eines pharmazeutisch zulässigen Salzes oder Solvats davon zur Herstellung eines Arzneimittels zur Behandlung einer Krankheit, einer Störung oder eines Zustands, der mit der Expression von USP7 in Zusammenhang steht.

8. Eine Verbindung gemäß Anspruch 1 oder ein pharmazeutisch zulässiges Salz oder Solvat davon, zur Verwendung in einem Verfahren zur Behandlung oder Vorbeugung einer Krankheit, einer Störung oder eines Zustands, der mit einer aberrierenden Expression oder Aktivität von USP7 in Zusammenhang steht, wobei dem betroffenen Patienten eine therapeutisch wirksame Menge mindestens einer Verbindung gemäß Anspruch 1 oder eines pharmazeutisch zulässigen Salzes oder Solvats davon oder eines pharmazeutischen Gemischs gemäß Anspruch 5 verabreicht wird.

9. Eine Verbindung zur Verwendung gemäß Anspruch 8, wobei die Krankheit, die Störung oder der Zustand aus einer Gruppe ausgewählt wird, die aus kardiovaskulären Erkrankungen, Lungen-, Autoimmun-, Immun-, immunregulatorischen, neurodegenerativen Erkrankungen, Stoffwechselstörungen, hämolytischen Störungen, Magen-Darm-Erkrankungen, sexuellen Funktionsstörungen, Infektionen, Wundheilungsstörungen und Krebserkrankungen besteht.

10. Eine Verbindung zur Verwendung gemäß Anspruch 9, wobei die Krankheit, die Störung oder der Zustand eine kardiovaskuläre Erkrankung ist, ausgewählt aus einer Gruppe, die systemische Hypertonie, pulmonale arterielle Hypertonie (PAH), pulmonale arterielle Hypertonie in der Höhenkrankheit, Ischämie-Reperfusionsschaden (IR), Myokardinfarkt und Atherosklerose umfasst.

11. Eine Verbindung zur Verwendung gemäß Anspruch 10, wobei die kardiovaskuläre Erkrankung eine pulmonale arterielle Hypertonie (PAH) ist.

12. Eine Verbindung zur Verwendung gemäß Anspruch 10, wobei die kardiovaskuläre Erkrankung ein Ischämie-Reperfusionsschaden (IR) ist, ausgewählt aus einer Gruppe, die IR der Leber, IR der Niere und IR des Myokards umfasst.

13. Eine Verbindung zur Verwendung gemäß Anspruch 10, wobei die kardiovaskuläre Erkrankung ein Myokardinfarkt oder Atherosklerose ist.

14. Eine Verbindung zur Verwendung gemäß Anspruch 9, wobei die Erkrankung, die Störung oder der Zustand eine Lungenerkrankung ist, ausgewählt aus einer Gruppe, die chemisch induzierte Lungenfibrose, idiopathische Lungenfibrose, Mukoviszidose, chronisch obstruktive Lungenerkrankung (COPD) und Asthma umfasst.

15. Eine Verbindung zur Verwendung gemäß Anspruch 9, wobei die Erkrankung, die Störung oder der Zustand eine Autoimmunerkrankung ist, ausgewählt aus einer Gruppe, die Enzephalomyelitis, Multiple Sklerose, Antiphospholipid-Syndrom 1, autoimmune hämolytische Anämie, chronisch-entzündliche demyelinisierende Polyradikuloneuropathie, Psoriasis, Dermatitis herpetiformis, Dermatomyositis, Myasthenia gravis, Pemphigus, rheumatoide Arthritis, Stiff-Person-Syndrom, Typ-1-Diabetes, ankylosierende Spondylitis, paroxysmale nächtliche Hämoglobinurie (PNH), paroxysmale Kältehämoglobinurie, schwere idiopathische autoimmune hämolytische Anämie und Goodpasture-Syndrom umfasst.

16. Eine Verbindung zur Verwendung gemäß Anspruch 9, wobei die Erkrankung, die Störung oder der Zustand eine Immunstörung ist, ausgewählt aus einer Gruppe, die eine durch myeloide Suppressorzellen (MDSC) vermittelte T-Zell-Dysfunktion, eine Infektion mit dem humanen Immundefizienz-Virus (HIV), eine autoimmune Enzephalomyelitis und eine Transfusionsreaktion aufgrund einer AB0-Unverträglichkeit umfasst.

17. Eine Verbindung zur Verwendung gemäß Anspruch 16, wobei die Immunerkrankung eine durch myeloide Suppressorzellen (MDSC) vermittelte T-Zell-Dysfunktion ist.

18. Eine Verbindung zur Verwendung gemäß Anspruch 9, wobei die Erkrankung, die Störung oder der Zustand eine Erkrankung ist, die auf eine immunregulatorische Störung zurückzuführen ist, ausgewählt aus einer Gruppe, die Nierenentzündungen, Leberfibrose, Leishmaniose, neurodegenerative Erkrankungen, Wundheilung, Infektionen mit dem Humanen Immundefizienz-Virus (HIV), Infektionen mit dem Hepatitis-B-Virus (HBV), Infektionen mit *Helicobacter pylori* , fibrotische Erkrankungen, Arthritis, Candidiasis, Parodontitis, Keloidbildung, Erkrankungen der Mandeln und des Rachenraums, Afrikanische Schlafkrankheit, Chagas-Krankheit und Transplantatabstoßung umfasst.

19. Eine Verbindung zur Verwendung gemäß Anspruch 9, wobei die Erkrankung, die Störung oder der Zustand eine neurodegenerative Störung ist, ausgewählt aus einer Gruppe, die Alzheimer-, Parkinson-, Huntington-Krankheit, extrapyramidales Syndrom, Dystonie, Akathisie, Epilepsie, periodische Beinbewegungen und Demenz umfasst.

20. Eine Verbindung zur Verwendung gemäß Anspruch 9, wobei die Erkrankung, die Störung oder der Zustand eine Stoffwechselstörung ist, ausgewählt aus einer Gruppe, die Diabetes, nichtalkoholischen Steatohepatitis (NASH) und nichtalkoholische Fettlebererkrankung (NAFLD) umfasst.

21. Eine Verbindung zur Verwendung gemäß Anspruch 9, wobei die Erkrankung, die Störung oder der Zustand eine hämolytische Störung ist, ausgewählt aus einer Gruppe, die Sichelzellenanämie, Thalassämien, hereditäre Sphärozytose, Stomatozytose, mikroangiopathische hämolytische Anämien, Pyruvatkinase-Mangel, infektionsbedingte Anämie, durch kardiopulmonalen Bypass und durch mechanische Herzklappen verursachte Anämie und chemisch induzierte Anämie umfasst.

22. Eine Verbindung zur Verwendung gemäß Anspruch 21, wobei die hämolytische Erkrankung die Sichelzellenanämie ist.

23. Eine Verbindung zur Verwendung gemäß Anspruch 9, wobei die Erkrankung, die Störung oder der Zustand eine Magen-Darm-Erkrankung ist, ausgewählt aus einer Gruppe, die Störungen der Magen-Darm-Motilität, Magenkrebs, entzündliche Darmerkrankungen, Crohn-Krankheit, Colitis ulcerosa und Magengeschwüre umfasst.

24. Eine Verbindung zur Verwendung gemäß Anspruch 9, wobei die Erkrankung, die Störung oder der Zustand eine sexuelle Störung ist, ausgewählt aus einer Gruppe, die Peyronie-Krankheit und erektile Dysfunktion umfasst.

25. Eine Verbindung zur Verwendung gemäß Anspruch 9, wobei die Erkrankung, die Störung oder der Zustand eine Wundheilungsstörung ist, ausgewählt aus einer Gruppe, die infizierte und nicht infizierte Wunden umfasst.

26. Eine Verbindung zur Verwendung gemäß Anspruch 9, wobei die Erkrankung, die Störung oder der Zustand eine Krebserkrankung ist, ausgewählt aus einer Gruppe, die Speiseröhren-, Magen-, Dickdarm-, Eierstock-, Brust-, Bauchspeicheldrüsen-, Kopf- und Hals-, Blasen- und Lungenkrebs (einschließlich Plattenepithelkarzinom und nichtkleinzelliger Lungenkarzinom), Nierenzellkarzinom, Prostatakarzinom, multiples Myelom, Neuroblastom, Glioblastom, Astrozytom, Mesotheliom und Melanom, B-Zell-, T-Zell- und NK-Zell-Lymphome, akute und chronische myeloische Leukämie, sowie lymphoide Leukämie umfasst.

27. Eine Verbindung zur Verwendung gemäß Anspruch 9, wobei die Erkrankung, die Störung oder der Zustand eine Krebserkrankung ist, ausgewählt aus einer Gruppe, die Magenkrebs (einschließlich, aber nicht beschränkt auf Magenkrebs oder Karzinom des gastroösophagealen Übergangs), Darm-, Bauchspeicheldrüsen-, Leber-, Brust-, Lungenkrebs (einschließlich, aber nicht beschränkt auf nicht-kleinzelliges Lungenkarzinom), Nierenzell-, Prostatakarzinom, multiples Myelom, akute und chronische Leukämie, T-Zell-, B-Zell- und NK-Zell-Lymphom, Hirntumor (einschließlich, aber nicht beschränkt auf Neuroblastom, Glioblastom, Astrozytom), Plattenepithelkarzinom im Kopf- und Halsbereich sowie Melanomen umfasst.

28. Eine Verbindung zur Verwendung gemäß Anspruch 9, wobei die Erkrankung, die Störung oder der Zustand eine Krebserkrankung ist, ausgewählt aus einer Gruppe, die chronische lymphatische Leukämie, akute lymphoblastische Leukämie, chronische myeloische Leukämie, akute myeloische Leukämie, Nebennierenrindenkarzinom, Analkrebs, Blinddarmkrebs, atypischen teratoiden/rhabdoiden Tumor, Basalzellkarzinom, Gallengangskrebs, Harnblasenkarzinom, Knochenkrebs, Hirntumor, Hirn- und Rückenmarkstumor, Hirnstammgliom, atypischen teratoiden/rhabdoiden Tumor des Zentralnervensystems, embryonale Tumoren des Zentralnervensystems, Brustkrebs, Bronchialtumoren, Burkitt-Lymphom, neuroendokrinen Tumor, Karzinom unbekannter Primärherkunft, Krebs des Zentralnervensystems, Gebärmutterhalskrebs, Krebserkrankungen im Kindesalter, Chordom, chronische myeloproliferative Erkrankungen, Dickdarmkrebs, Kraniopharyngeom, kutanes T-Zell-Lymphom, duktales Karzinom in situ, embryonale Tumoren, Gebärmutterschleimhautkrebs, Ependymoblastom, Ependymom, Speiseröhrenkrebs, Ästhesioneuroblastom, Ewing-Sarkom, extrakranialen Keimzelltumor, extrahepatischen Gallengangskrebs, Augenkrebs, fibröses Histiozytom des Knochens, Gallenblasen-, Magenkrebs, gastrointestinalen Karzinoidtumor, gastrointestinale Stromatumoren, Keimzelltumor, extrakraniellen, extragonadalen und ovariellen Keimzelltumor, gestationellen Trophoblastentumor, Gliom, Haarzell-Leukämie, Kopf-Hals-Krebs, Herzkrebs, Leberzellkarzinom, Histiozytose, Langerhans-Zell-Karzinom, Hodgkin-Lymphom, Hypopharynxkarzinom, intraokulares Melanom, Inselzelltumore, Kaposi-Sarkom, Nierenkrebs, Langerhans-Zell-Histiozytose, Kehlkopfkrebs, Lippen- und Mundhöhlenkrebs, lobuläres Carcinoma in situ, Lymphom, AIDS-assoziiertes Lymphom, Makroglobulinämie, Brustkrebs beim Mann, Medulloblastom, Medulloepitheliom, Merkelzellkarzinom, malignes Mesotheliom, metastasierenden Plattenepithelkarzinom des Halses, NUT-Mittellinienkarzinom, Mundkrebs, Syndrom der multiplen endokrinen Neoplasien, Plasmazellneoplasie, Mycosis fungoides, Myelom, chronische myeloproliferative Erkrankung, Nasenhöhlenkrebs, Nasennebenhöhlenkrebs, Nasen-Rachen-Krebs, Non-Hodgkin-Lymphom, Mundkrebs, Mundhöhlenkrebs, Lippenkrebs, Mund-Rachen-Krebs, Osteosarkom, Eierstockkrebs, Papillomatose, Paragangliom, Nasennebenhöhlenkrebs, Nasenhöhlenkrebs, Nebenschilddrüsenkrebs, Peniskrebs, Rachenkrebs, Phäochromozytom, mitteldifferenzierte Pinealisparenchymtumoren, Pineoblastom, Hypophysentumor, Plasmazellneoplasie, pleuropulmonales Blastom, primäres Lymphom des Zentralnervensystems, Rektumkarzinom, Nierenzellkarzinom, Nierenbeckenkarzinom, Harnleiterkarzinom, Übergangszellkarzinom, Retinoblastom, Rhabdomyosarkom, Speicheldrüsenkarzinom, Sarkom, Sézary-Syndrom, Hautkrebs, kleinzelliges Lungenkarzinom, Dünndarmkrebs, Weichteilsarkom, Plattenepithelkarzinom, Plattenepithelkarzinom des Halses mit okkultem Primärtumor, supratentoriale primitive neuroektodermale Tumoren, Hodenkrebs, Kehlkopfkrebs, Thymom, Thymuskarzinom, Schilddrüsenkrebs, Übergangszellkarzinom des Nierenbeckens und des Harnleiters, gestationsbedingten Trophoblasttumor, unbekannten Primärtumor, seltene Krebserkrankungen im Kindesalter, Harnröhrenkrebs, Gebärmutterkrebs, Gebärmuttersarkom, Waldenström-Makroglobulinämie oder Wilms-Tumor umfasst.

29. Eine Verbindung zur Verwendung gemäß Anspruch 9, wobei die mindestens eine Verbindung gemäß Anspruch 1 gleichzeitig oder nacheinander mit einer therapeutisch wirksamen Menge eines oder mehrerer anderer therapeutischer Wirkstoffe verabreicht wird, ausgewählt aus einer Gruppe, die antivirale Wirkstoffe, Chemotherapeutika (einschließlich Alkylanzien, Antimetaboliten, Antimitotika), Immunsuppressiva, Krebsimpfungen, Vakzine, zytokinetische Therapie, Tyrosinkinase-Inhibitoren, Immuntherapeutika, einschließlich Checkpoint-Inhibitoren, wie PD-1, PD-L1- oder CTLA-4-Inhibitoren sowie IDO/TDO-Inhibitoren, Adenosin-A2A-Rezeptor-Antagonisten, Ectonucleotidaser-Inhibitoren (CD73 und CD39), Wirkstoffe, die auf Interleukine, Zytokine und Chemokine einwirken, Topoisomerase-Inhibitoren und zytotoxische Antibiotika, oder zielgerichtete Therapien, die Antikörper, Antikörper-Wirkstoff-Konjugate, zellbasierte Immuntherapie, Nanopartikeln und Strahlentherapie umfassen.

30. Eine Verbindung zur Verwendung gemäß Anspruch 29, wobei die Antikörper eine therapeutisch wirksame Menge an Anti-PD-1-, Anti-PD-L1- oder Anti-CTLA-4-Antikörpern enthalten.

31. Eine Verbindung gemäß Anspruch 1 zur Verwendung in einem Verfahren zur *In-vitro-* oder *Ex-vivo-*Aktivierung von T-Lymphozyten zur Freisetzung von Zytokinen, wobei die Zelle mit mindestens einer Verbindung gemäß Anspruch 1 oder einem pharmazeutisch zulässigen Salz oder Solvat davon in Kontakt gebracht wird.

32. Verwendung einer Verbindung gemäß Anspruch 1 oder eines pharmazeutisch zulässigen Salzes oder Solvats davon zum Schutz eines Organs außerhalb des menschlichen oder tierischen Körpers während Beförderung.

## Revendications

1. Composé de formule structurelle (Ia) : 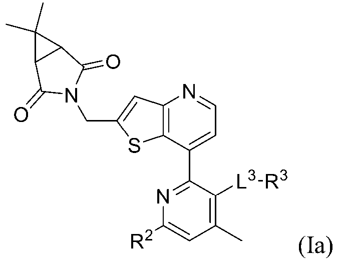 dans laquelle :
R² est choisi parmi l'hydrogène, le méthyle, l'éthyle, le chlore, -C≡N et -CF₃;
L³ représente une liaison simple, -O-, -CH₂-, -C(=O)-, -NH- ou -NH-C(=O)- ; et
est choisi parmi les hétérocycloalkyles à 4 à 6 chaînons contenant 1 ou 2 hétéroatomes d'azote ou 1 hétéroatome d'azote et 1 hétéroatome d'oxygène,
éventuellement substitués par 1 ou 2 substituants choisis parmi le fluor, le méthyle, l'éthyle, l'amino, le méthylamino, le diméthylamino, l'éthylamino, l'isopropylamino, l'hydroxy, l'oxo, l'aminométhyle, le 1-aminoéthyle, le (isopropylamino)méthyle, l'uréido, l'alanylamino, le valylamino, le fluorométhyle, le difluorométhyle, le trifluorométhyle et le 2,2,2-trifluoroacétyle ;
ou un tautomère, un stéréoisomère, un mélange racémique ou scalémique de stéréoisomères, un sel, un ester ou un solvate pharmaceutiquement acceptable de celui-ci.

2. Composé selon la revendication 1, dans lequel le composé est :
6-(2-((6,6-diméthyl-2,4-dioxo-3-azabicyclo[3.1.0]hexan-3-yl)méthyl)thieno[3,2-*b*]pyridin-7-yl)-4-méthyl-5-(pipéridin-3-yloxy)picolinonitrile ;
3-((7-(6-chloro-4-méthyl-3-(pyrrolidin-3-yloxy)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(pipéridin-3-yloxy)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(((*S*)-pipéridin-3-yl)oxy)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pyrrolidin-3-ylamino)-6-(trifluorométhyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(pyrrolidin-3-ylamino)picolinonitrile ;
5-(azétidin-3-ylamino)-6-(2-((6,6-diméthyl-2,4-dioxo-3-azabicyclo[3.1.0]hexan-3-yl)méthyl)thieno[3,2-*b*]pyridin-7-yl)-4-méthylpicolinonitrile ;
*N*-(2-(2-((6,6-diméthyl-2,4-dioxo-3-azabicyclo[3.1.0]hexan-3-yl)méthyl)thieno[3,2-*b*]pyridin-7-yl)-4-méthyl-6-(trifluorométhyl)pyridin-3-yl)azétidine-3-carboxamide ;
6,6-diméthyl-3-((7-(4-méthyl-3-(pipérazin-2-ylméthyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((3,6-dioxopipérazin-2-yl)méthyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-((3-oxomorpholino)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((2-oxopyrrolidin-1-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((2-oxopipérazin-1-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((4-méthyl-2-oxopipérazin-1-yl)méthyl)-6-(trifluorométhyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-5-(3-(diméthylamino)azétidine-1-carbonyl)-4-méthylpicolinonitrile ;
6-(2-((6,6-diméthyl-2,4-dioxo-3-azabicyclo[3.1.0]hexan-3-yl)méthyl)thieno[3,2-*b*]pyridin-7-yl)-5-((*S*)-3-(diméthylamino)pyrrolidine-1-carbonyl)-4-méthylpicolinonitrile ;
6,6-diméthyl-3-((7-(4-méthyl-3-(pipérazine-1-carbonyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-(pipérazin-1-ylméthyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-4-méthyl-5-((*S*)-2-méthylpipérazine-1-carbonyl)picolinonitrile ;
6,6-diméthyl-3-((7-(4-méthyl-3-(morpholine-4-carbonyl)pyridin-2-yl)thieno[3,2-*b*]pyridin-2-yl)méthyl)-3-azabicyclo[3.1.0]hexane-2,4-dione ;
3-((7-(3-(3,3-difluoropyrrolidine-1-carbonyl)-4-méthylpyridin-2-yl)thieno[3,2-*b*]pyridin-2-yl)méthyl)-6,6-diméthyl-3-azabicyclo[3.1.0]hexane-2,4-dione ;
3-((7-(3-(3,3-difluoroazétidine-1-carbonyl)-4-méthylpyridin-2-yl)thieno[3,2-*b*]pyridin-2-yl)méthyl)-6,6-diméthyl-3-azabicyclo[3.1.0]hexane-2,4-dione ;
5-(3,3-difluoroazétidine-1-carbonyl)-6-(2-((6,6-diméthyl-2,4-dioxo-3-azabicyclo[3.1.0]hexan-3-yl)méthyl)thieno[3,2-*b*]pyridin-7-yl)-4-méthylpicolinonitrile ;
6-(2-((6,6-diméthyl-2,4-dioxo-3-azabicyclo[3.1.0]hexan-3-yl)méthyl)thieno[3,2-*b*]pyridin-7-yl)-5-((S)-2,4-diméthylpipérazine-1-carbonyl)-4-méthylpicolinonitrile ;
6-(2-((6,6-diméthyl-2,4-dioxo-3-azabicyclo[3.1.0]hexan-3-yl)méthyl)thieno[3,2-*b*]pyridin-7-yl)-5-(3,3-diméthylpipérazine-1-carbonyl)-4-méthylpicolinonitrile ;
6-(2-((6,6-diméthyl-2,4-dioxo-3-azabicyclo[3.1.0]hexan-3-yl)méthyl)thieno[3,2-*b*]pyridin-7-yl)-4-méthyl-5-((*R*)-2-méthylpipérazine-1-carbonyl)picolinonitrile ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(pipérazine-1-carbonyl)picolinonitrile ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(morpholine-4-carbonyl)picolinonitrile ;
5-(3,3-difluoropyrrolidine-1-carbonyl)-6-(2-((6,6-diméthyl-2,4-dioxo-3-azabicyclo[3.1.0]hexan-3-yl)méthyl)thieno[3,2-*b*]pyridin-7-yl)-4-méthylpicolinonitrile ;
3-((7-(3-((*S*)-3-(éthylamino)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-(isopropylamino)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pipérazine-1-carbonyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((*S*)-3-(méthylamino)pyrrolidine-1-carbonyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((*S*)-2-méthylpipérazine-1-carbonyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((*S*)-2,4-diméthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(4-méthylpipérazine-1-carbonyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((*S*)-3-aminopyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-(diméthylamino)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(3,3-difluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(morpholine-4-carbonyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-(3,3-difluoroazétidine-1-carbonyl)-4,6-diméthylpyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(morpholin-2-ylméthyl)-6-(trifluorométhyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(morpholin-2-ylméthyl)picolinonitrile ;
6,6-diméthyl-3-((7-(4-méthyl-3-(((*S*)-morpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-(((*R*)-morpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-(((*S*)-4-méthylmorpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-(((*R*)-4-méthylmorpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((*S*)-3-(difluorométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-(fluorométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*S*)-3-amino-4-méthylpyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*R*)-3-amino-4-méthylpyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
(2*S*)-2-amino-N-((3*S*)-1-(2-(2-((6,6-diméthyl-2,4-dioxo-3-azabicyclo[3.1.0]hexan-3-yl)méthyl)thieno[3,2-*b*]pyridin-7-yl)-4-méthyl-6-(trifluorométhyl)nicotinoyl)pyrrolidin-3-yl)-3-méthylbutanamide ;
(2*S*)-2-amino-N-((3*S*)-1-(2-(2-((6,6-diméthyl-2,4-dioxo-3-azabicyclo[3.1.0]hexan-3-yl)méthyl)thieno[3,2-*b*]pyridin-7-yl)-4-méthyl-6-(trifluorométhyl)nicotinoyl)pyrrolidin-3-yl)propanamide ;
3-((7-(3-(4-aminopipéridine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-hydroxypyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*R*)-3-(aminométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*R*,4*R*)-3-amino-4-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(2-oxotétrahydropyrimidin-1(2*H*)-yl)pyridin-2-yl)thieno[3,2-b]pyridin-2-yl)méthyl)-6,6-diméthyl-3-azabicyclo[3.1.0]hexane-2,4-dione ;
3-((7-(3-(azétidin-3-ylamino)-6-chloro-4-méthylpyridin-2-yl)thieno[3,2-*b*]pyridin-2-yl)méthyl)-6,6-diméthyl-3-azabicyclo[3.1.0]hexane-2,4-dione ;
3-((7-(4,6-diméthyl-3-(pyrrolidin-3-ylamino)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(pyrrolidin-3-ylamino)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(pipéridin-4-ylamino)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pipéridin-4-ylamino)-6-(trifluorométhyl)pyridin-2-yl)thieno[3,2-*b*]pyridin-2-yl)méthyl)-3-azabicyclo[3.1.0]hexane-2,4-dione ;
3-((7-(6-chloro-4-méthyl-3-(pipéridin-3-ylamino)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pipéridin-3-ylamino)-6-(trifluorométhyl)pyridin-2-yl)thieno[3,2-*b*]pyridin-2-yl)méthyl)-3-azabicyclo[3.1.0]hexane-2,4-dione ;
*N*-(2-(2-((6,6-diméthyl-2,4-dioxo-3-azabicyclo[3.1.0]hexan-3-yl)méthyl)thieno[3,2-*b*]pyridin-7-yl)-4,6-diméthylpyridin-3-yl)azétidine-3-carboxamide ;
*N*-(2-(2-((6,6-diméthyl-2,4-dioxo-3-azabicyclo[3.1.0]hexan-3-yl)méthyl)thieno[3,2-*b*]pyridin-7-yl)-4,6-diméthylpyridin-3-yl)pipéridine-4-carboxamide ;
3-((7-(6-chloro-4-méthyl-3-(pipérazine-1-carbonyl)pyridin-2-yl)thieno[3,2-*b*]pyridin-2-yl)méthyl)-6,6-diméthyl-3-azabicyclo[3.1.0]hexane-2,4-dione ;
1-((3*S*)-1-(2-(2-((6,6-diméthyl-2,4-dioxo-3-azabicyclo[3.1.0]hexan-3-yl)méthyl)thieno[3,2-*b*]pyridin-7-yl)-4-méthyl-6-(trifluorométhyl)nicotinoyl)pyrrolidin-3-yl)urée ;
3-((7-(3-(3-amino-3-méthylpyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*)-3-(1-aminoéthyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(3-(aminométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(3-((isopropylamino)méthyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((2*S*,6*S*)-2,6-diméthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((2*R*,6*R*)-2,6-diméthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(4-éthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ; ou
6,6-diméthyl-3-((7-(4-méthyl-3-((*S*)-3-(méthylamino)pipéridine-1-carbonyl)-6-(trifluorométhyl)pyridin-2-yl)thieno[3,2-*b*]pyridin-2-yl)méthyl)-3-azabicyclo[3.1.0]hexane-2,4-dione ;
ou un tautomère, un stéréoisomère, un mélange racémique ou scalémique de stéréoisomères, un sel, un ester ou un solvate pharmaceutiquement acceptable de celui-ci.

3. Composé selon la revendication 2, dans lequel le composé est :
6-(2-((6,6-diméthyl-2,4-dioxo-3-azabicyclo[3.1.0]hexan-3-yl)méthyl)thieno[3,2-*b*]pyridin-7-yl)-4-méthyl-5-(pipéridin-3-yloxy)picolinonitrile ;
3-((7-(6-chloro-4-méthyl-3-(pyrrolidin-3-yloxy)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(pipéridin-3-yloxy)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(((*S*)-pipéridin-3-yl)oxy)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pyrrolidin-3-ylamino)-6-(trifluorométhyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(pyrrolidin-3-ylamino)picolinonitrile ;
5-(azétidin-3-ylamino)-6-(2-((6,6-diméthyl-2,4-dioxo-3-azabicyclo[3.1.0]hexan-3-yl)méthyl)thieno[3,2-*b*]pyridin-7-yl)-4-méthylpicolinonitrile ;
*N*-(2-(2-((6,6-diméthyl-2,4-dioxo-3-azabicyclo[3.1.0]hexan-3-yl)méthyl)thieno[3,2-*b*]pyridin-7-yl)-4-méthyl-6-(trifluorométhyl)pyridin-3-yl)azétidine-3-carboxamide ;
6,6-diméthyl-3-((7-(4-méthyl-3-(pipérazin-2-ylméthyl)-6-(trifluorométhyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-4-méthyl-5-((*S*)-2-méthylpipérazine-1-carbonyl)picolinonitrile ;
5-(3,3-difluoroazétidine-1-carbonyl)-6-(2-((6,6-diméthyl-2,4-dioxo-3-azabicyclo[3.1.0]hexan-3-yl)méthyl)thieno[3,2-*b*]pyridin-7-yl)-4-méthylpicolinonitrile ;
6-(2-((6,6-diméthyl-2,4-dioxo-3-azabicyclo[3.1.0]hexan-3-yl)méthyl)thieno[3,2-*b*]pyridin-7-yl)-5-((S)-2,4-diméthylpipérazine-1-carbonyl)-4-méthylpicolinonitrile ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(pipérazine-1-carbonyl)picolinonitrile ;
5-(3,3-difluoropyrrolidine-1-carbonyl)-6-(2-((6,6-diméthyl-2,4-dioxo-3-azabicyclo[3.1.0]hexan-3-yl)méthyl)thieno[3,2-*b*]pyridin-7-yl)-4-méthylpicolinonitrile ;
3-((7-(3-((*S*)-3-(éthylamino)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-(isopropylamino)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pipérazine-1-carbonyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((*S*)-3-(méthylamino)pyrrolidine-1-carbonyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((*S*)-2-méthylpipérazine-1-carbonyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((*S*)-2,4-diméthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(4-méthylpipérazine-1-carbonyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((*S*)-3-aminopyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-(diméthylamino)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(3,3-difluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(morpholin-2-ylméthyl)-6-(trifluorométhyl)pyridin-2-yl)thieno[3,2-*b*]pyridin-2-yl)méthyl)-3-azabicyclo[3.1.0]hexane-2,4-dione ;
6-(2-((6,6-diméthyl-2,4-dioxo-3-azabicyclo[3.1.0]hexan-3-yl)méthyl)thieno[3,2-*b*]pyridin-7-yl)-4-méthyl-5-(morpholin-2-ylméthyl)picolinonitrile ;
6,6-diméthyl-3-((7-(4-méthyl-3-(((*S*)-morpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-(((*R*)-morpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-(((*R*)-4-méthylmorpholin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((*S*)-3-(difluorométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-(fluorométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*S*)-3-amino-4-méthylpyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*R*)-3-amino-4-méthylpyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
(2*S*)-2-amino-N-((3*S*)-1-(2-(2-((6,6-diméthyl-2,4-dioxo-3-azabicyclo[3.1.0]hexan-3-yl)méthyl)thieno[3,2-*b*]pyridin-7-yl)-4-méthyl-6-(trifluorométhyl)nicotinoyl)pyrrolidin-3-yl)-3-méthylbutanamide ;
3-((7-(3-(4-aminopipéridine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-hydroxypyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((*S*)-3-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*R*,4*R*)-3-amino-4-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(azétidin-3-ylamino)-6-chloro-4-méthylpyridin-2-yl)thieno[3,2-*b*]pyridin-2-yl)méthyl)-6,6-diméthyl-3-azabicyclo[3.1.0]hexane-2,4-dione ;
3-((7-(4,6-diméthyl-3-(pyrrolidin-3-ylamino)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(pyrrolidin-3-ylamino)pyridin-2-yl)thieno[3,2-*b*]pyridin-2-yl)méthyl)-6,6-diméthyl-3-azabicyclo[3.1.0]hexane-2,4-dione ;
3-((7-(6-chloro-4-méthyl-3-(pipéridin-4-ylamino)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pipéridin-4-ylamino)-6-(trifluorométhyl)pyridin-2-yl)thieno[3,2-*b*]pyridin-2-yl)méthyl)-3-azabicyclo[3.1.0]hexane-2,4-dione ;
3-((7-(6-chloro-4-méthyl-3-(pipéridin-3-ylamino)pyridin-2-yl)thieno[3,2-*b*]pyridin-2-yl)méthyl)-6,6-diméthyl-3-azabicyclo[3.1.0]hexane-2,4-dione ;
6,6-diméthyl-3-((7-(4-méthyl-3-(pipéridin-3-ylamino)-6-(trifluorométhyl)pyridin-2-yl)thieno[3,2-*b*]pyridin-2-yl)méthyl)-3-azabicyclo[3.1.0]hexane-2,4-dione ;
3-((7-(3-(3-(aminométhyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-(3-((isopropylamino)méthyl)pyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((2*S*,6*S*)-2,6-diméthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ; ou
3-((7-(3-(4-éthylpipérazine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
ou un tautomère, un stéréoisomère, un mélange racémique ou scalémique de stéréoisomères, un sel, un ester ou un solvate pharmaceutiquement acceptable de celui-ci.

4. Composé selon la revendication 1, dans lequel le composé est :
6,6-diméthyl-3-((7-(4-méthyl-3-(((*S*)-3-(méthylamino)pyrrolidin-1-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((3-oxopipérazin-2-yl)méthyl)-6-(trifluorométhyl)pyridin-2-yl)thieno[3,2-*b*]pyridin-2-yl)méthyl)-3-azabicyclo[3.1.0]hexane-2,4-dione ;
6,6-diméthyl-3-((7-(4-méthyl-3-((3*R*,4*S*)-3-méthyl-4-(méthylamino)pyrrolidine-1-carbonyl)-6-(trifluorométhyl)pyridin-2-yl)thieno[3,2-*b*]pyridin-2-yl)méthyl)-3-azabicyclo[3.1.0]hexane-2,4-dione ;
3-((7-(3-((3*R*,4*S*)-3-amino-4-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*S*)-3-amino-4-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*R*)-3-amino-4-fluoropyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
3-((7-(3-((3*S*,4*R*)-3-(diméthylamino)-4-méthylpyrrolidine-1-carbonyl)-4-méthyl-6-(trifluorométhyl)pyridin-2-yl)thieno[3,2-*b*]pyridin-2-yl)méthyl)-6,6-diméthyl-3-azabicyclo[3.1.0]hexane-2,4-dione ;
ou un tautomère, un stéréoisomère, un mélange racémique ou scalémique de stéréoisomères, un sel, un ester ou un solvate pharmaceutiquement acceptable de celui-ci.

5. Composition pharmaceutique comprenant (i) une quantité thérapeutiquement efficace d'au moins un composé selon la revendication 1, ou d'un sel ou solvate pharmaceutiquement acceptable de celui-ci ; et (ii) un support, un véhicule ou un excipient pharmaceutiquement acceptable pour celui-ci.

6. Composé selon la revendication 1, ou un sel ou solvate pharmaceutiquement acceptable de celui-ci, destiné à être utilisé dans une méthode d'inhibition de l'USP7 dans une cellule ou un tissu, ladite méthode comprenant la mise en contact de la cellule ou du tissu avec au moins un composé selon la revendication 1, ou un sel ou solvate pharmaceutiquement acceptable de celui-ci.

7. Utilisation d'un composé selon la revendication 1, ou d'un sel ou solvate pharmaceutiquement acceptable de celui-ci, pour la fabrication d'un médicament destiné au traitement d'une maladie, d'un trouble ou d'une affection associé(e) à l'expression de l'USP7.

8. Composé selon la revendication 1, ou un sel ou solvate pharmaceutiquement acceptable de celui-ci, destiné à être utilisé dans une méthode de traitement ou de prévention d'une maladie, d'un trouble ou d'une affection associé(e) à une expression ou une activité aberrante de l'USP7, ladite méthode comprenant l'administration à un sujet qui en a besoin d'une quantité thérapeutiquement efficace d'au moins un composé selon la revendication 1, ou d'un sel ou solvate pharmaceutiquement acceptable de celui-ci, ou d'une composition pharmaceutique selon la revendication 5.

9. Composé destiné à être utilisé selon la revendication 8, dans lequel la maladie, le trouble ou l'affection est choisi(e) dans le groupe constitué par les troubles cardiovasculaires, les troubles pulmonaires, les troubles auto-immuns, les troubles immunitaires, les troubles immunorégulateurs, les troubles neurodégénératifs, les troubles métaboliques, les troubles hémolytiques, les troubles gastro-intestinaux, les troubles sexuels, les infections, les troubles de la cicatrisation et les cancers.

10. Composé destiné à être utilisé selon la revendication 9, dans lequel la maladie, le trouble ou l'affection est un trouble cardiovasculaire choisi dans le groupe constitué par l'hypertension systémique, l'hypertension artérielle pulmonaire (HTAP), l'hypertension artérielle pulmonaire en haute altitude, les lésions d'ischémie-reperfusion (IR), l'infarctus du myocarde et l'athérosclérose.

11. Composé destiné à être utilisé selon la revendication 10, dans lequel le trouble cardiovasculaire est l'hypertension artérielle pulmonaire (HTAP).

12. Composé destiné à être utilisé selon la revendication 10, dans lequel le trouble cardiovasculaire est une lésion d'ischémie-reperfusion (IR) choisie dans le groupe constitué par l'ischémie-reperfusion hépatique, l'ischémie-reperfusion rénale et l'ischémie-reperfusion myocardique.

13. Composé destiné à être utilisé selon la revendication 10, dans lequel le trouble cardiovasculaire est un infarctus du myocarde ou une athérosclérose.

14. Composé destiné à être utilisé selon la revendication 9, dans lequel la maladie, le trouble ou l'affection est un trouble pulmonaire choisi dans le groupe constitué par la fibrose pulmonaire induite chimiquement, la fibrose pulmonaire idiopathique, la mucoviscidose, la bronchopneumopathie chronique obstructive (BPCO) et l'asthme.

15. Composé destiné à être utilisé selon la revendication 9, dans lequel la maladie, le trouble ou l'affection est un trouble auto-immun choisi dans le groupe constitué par l'encéphalomyélite, la sclérose en plaques, le syndrome des antiphospholipides de type 1, l'anémie hémolytique auto-immune, la polyradiculonévrite inflammatoire démyélinisante chronique, le psoriasis, la dermatite herpétiforme, la dermatomyosite, la myasthénie grave, le pemphigus, la polyarthrite rhumatoïde, le syndrome de l'homme raide, le diabète de type 1, la spondylarthrite ankylosante, l'hémoglobinurie paroxystique nocturne (HPN), l'hémoglobinurie paroxystique a frigore, l'anémie hémolytique auto-immune idiopathique sévère et le syndrome de Goodpasture.

16. Composé destiné à être utilisé selon la revendication 9, dans lequel la maladie, le trouble ou l'affection est un trouble immunitaire choisi dans le groupe constitué par le dysfonctionnement des lymphocytes T médié par les cellules suppressives dérivées de la lignée myéloïde (MDSC), l'infection par le virus de l'immunodéficience humaine (VIH), l'encéphalomyélite auto-immune et la réaction transfusionnelle liée à une incompatibilité AB0.

17. Composé destiné à être utilisé selon la revendication 16, dans lequel le trouble immunitaire est un dysfonctionnement des lymphocytes T médié par les cellules suppressives dérivées de la lignée myéloïde (MDSC).

18. Composé destiné à être utilisé selon la revendication 9, dans lequel la maladie, le trouble ou l'affection est une maladie résultant d'un trouble immunorégulateur choisie dans le groupe constitué par l'inflammation des maladies rénales, la fibrose hépatique, la leishmaniose, les maladies neurodégénératives, la cicatrisation des plaies, l'infection par le virus de l'immunodéficience humaine (VIH), l'infection par le virus de l'hépatite B (VHB), l'infection par *Helicobacter pylori*, les troubles fibrotiques, l'arthrite, la candidose, les maladies parodontales, les chéloïdes, les maladies adéno-amygdaliennes, la trypanosomiase africaine, la maladie de Chagas et le rejet de greffe.

19. Composé destiné à être utilisé selon la revendication 9, dans lequel la maladie, le trouble ou l'affection est a trouble neurodégénératif choisi dans le groupe constitué par la maladie d'Alzheimer, la maladie de Parkinson, la maladie de Huntington, le syndrome extrapyramidal, la dystonie, l'akathisie, l'épilepsie, les mouvements périodiques des membres et la démence.

20. Composé destiné à être utilisé selon la revendication 9, dans lequel la maladie, le trouble ou l'affection est un trouble métabolique choisi dans le groupe constitué par le diabète, la stéatohépatite non alcoolique (NASH) et la stéatose hépatique non alcoolique (NAFLD).

21. Composé destiné à être utilisé selon la revendication 9, dans lequel la maladie, le trouble ou l'affection est un trouble hémolytique choisi dans le groupe constitué par la drépanocytose, les thalassémies, la sphérocytose héréditaire, la stomatocytose, les anémies hémolytiques microangiopathiques, le déficit en pyruvate kinase, l'anémie induite par une infection, le pontage cardiopulmonaire, l'anémie induite par une valve cardiaque mécanique et l'anémie induite chimiquement.

22. Composé destiné à être utilisé selon la revendication 21, dans lequel le trouble hémolytique est la drépanocytose.

23. Composé destiné à être utilisé selon la revendication 9, dans lequel la maladie, le trouble ou l'affection est un trouble gastro-intestinal choisi dans le groupe constitué par les troubles de la motilité gastro-intestinale, les cancers gastriques, les maladies inflammatoires chroniques de l'intestin, la maladie de Crohn, la rectocolite hémorragique et les ulcères gastriques.

24. Composé destiné à être utilisé selon la revendication 9, dans lequel la maladie, le trouble ou l'affection est un trouble sexuel choisi dans le groupe constitué par la maladie de La Peyronie et la dysfonction érectile.

25. Composé destiné à être utilisé selon la revendication 9, dans lequel la maladie, le trouble ou l'affection est un trouble de la cicatrisation choisi dans le groupe constitué par la cicatrisation des plaies infectées et non infectées.

26. Composé destiné à être utilisé selon la revendication 9, dans lequel la maladie, le trouble ou l'affection est un cancer choisi dans le groupe constitué par les cancers de l'œsophage, de l'estomac, du côlon, de l'ovaire, du sein, du pancréas, de la tête et du cou, de la vessie et du poumon (y compris le carcinome pulmonaire épidermoïde et le carcinome pulmonaire non à petites cellules), le carcinome à cellules rénales, le carcinome de la prostate, le myélome multiple, le neuroblastome, le glioblastome, l'astrocytome, le mésothéliome et le mélanome, les lymphomes des cellules B, des cellules T et des cellules NK, les leucémies myéloïdes aiguës et chroniques et les leucémies lymphoïdes.

27. Composé destiné à être utilisé selon la revendication 9, dans lequel la maladie, le trouble ou l'affection est un cancer choisi dans le groupe constitué par le cancer gastrique (y compris, notamment, le cancer gastrique ou le cancer de la jonction gastro-œsophagienne), le cancer colorectal, le cancer du pancréas, le cancer du foie, le cancer du sein, les cancers du poumon (y compris, notamment, le carcinome pulmonaire non à petites cellules), le carcinome à cellules rénales, le carcinome de la prostate, le myélome multiple, les leucémies aiguës et chroniques, les lymphomes des cellules T, des cellules B et des cellules NK, les tumeurs cérébrales (y compris, notamment, le neuroblastome, le glioblastome et l'astrocytome), les carcinomes épidermoïdes de la tête et du cou et le mélanome.

28. Composé destiné à être utilisé selon la revendication 9, dans lequel la maladie, le trouble ou l'affection est un cancer choisi dans le groupe constitué par la leucémie lymphoïde chronique, la leucémie lymphoblastique aiguë, la leucémie myéloïde chronique, la leucémie myéloïde aiguë, le carcinome corticosurrénalien, le cancer de l'anus, le cancer de l'appendice, la tumeur tératoïde/rhabdoïde atypique, le carcinome basocellulaire, le cancer des voies biliaires, le cancer de la vessie, le cancer des os, la tumeur cérébrale, la tumeur du cerveau et de la moelle épinière, le gliome du tronc cérébral, la tumeur tératoïde/rhabdoïde atypique du système nerveux central, les tumeurs embryonnaires du système nerveux central, le cancer du sein, les tumeurs bronchiques, le lymphome de Burkitt, la tumeur carcinoïde, le carcinome primitif de siège inconnu, le cancer du système nerveux central, le cancer du col de l'utérus, les cancers pédiatriques, le chordome, les syndromes myéloprolifératifs chroniques, le cancer du côlon, le craniopharyngiome, le lymphome cutané à cellules T, le carcinome canalaire in situ, les tumeurs embryonnaires, le cancer de l'endomètre, l'épendymoblastome, l'épendymome, le cancer de l'œsophage, l'esthésioneuroblastome, le sarcome d'Ewing, la tumeur extracrânienne à cellules germinales, le cancer des voies biliaires extrahépatiques, le cancer de l'œil, l'histiocytome fibreux de l'os, le cancer de la vésicule biliaire, le cancer gastrique, la tumeur carcinoïde gastro-intestinale, les tumeurs stromales gastro-intestinales, la tumeur à cellules germinales, la tumeur extracrânienne à cellules germinales, la tumeur extragonadique à cellules germinales, la tumeur ovarienne à cellules germinales, la tumeur trophoblastique gestationnelle, le gliome, la leucémie à tricholeucocytes, le cancer de la tête et du cou, le cancer du cœur, le carcinome hépatocellulaire, l'histiocytose, le cancer des cellules de Langerhans, le lymphome de Hodgkin, le cancer de l'hypopharynx, le mélanome intraoculaire, les tumeurs des cellules des îlots pancréatiques, le sarcome de Kaposi, le cancer du rein, l'histiocytose à cellules de Langerhans, le cancer du larynx, le cancer de la lèvre et de la cavité buccale, le carcinome lobulaire in situ, le lymphome, le lymphome lié au SIDA, la macroglobulinémie, le cancer du sein chez l'homme, le médulloblastome, le médulloépithéliome, le carcinome à cellules de Merkel, le mésothéliome malin, le cancer métastatique épidermoïde du cou, le carcinome de la ligne médiane impliquant le gène NUT, le cancer de la bouche, le syndrome de néoplasie endocrinienne multiple, le néoplasme plasmocytaire, la mycose fongoïde, le myélome, le syndrome myéloprolifératif chronique, le cancer de la cavité nasale, le cancer des sinus paranasaux, le cancer du nasopharynx, le lymphome non hodgkinien, le cancer buccal, le cancer de la cavité buccale, le cancer de la lèvre, le cancer de l'oropharynx, l'ostéosarcome, le cancer de l'ovaire, la papillomatose, le paragangliome, le cancer des sinus paranasaux, le cancer de la cavité nasale, le cancer des glandes parathyroïdes, le cancer du pénis, le cancer du pharynx, le phéochromocytome, les tumeurs du parenchyme pinéal de différenciation intermédiaire, le pinéoblastome, la tumeur hypophysaire, le néoplasme plasmocytaire, le blastome pleuropulmonaire, le lymphome primitif du système nerveux central, le cancer du rectum, le cancer du rein à cellules rénales, le cancer du bassinet du rein, le cancer de l'uretère, le carcinome transitionnel, le rétinoblastome, le rhabdomyosarcome, le cancer des glandes salivaires, le sarcome, le syndrome de Sézary, le cancer de la peau, le cancer du poumon à petites cellules, le cancer de l'intestin grêle, le sarcome des tissus mous, le carcinome épidermoïde, le cancer épidermoïde cervical avec primitif occulte, les tumeurs neuroectodermiques primitives supratentorielles, le cancer du testicule, le cancer de la gorge, le thymome, le carcinome thymique, le cancer de la thyroïde, le carcinome transitionnel du bassinet du rein et de l'uretère, la tumeur trophoblastique gestationnelle, le cancer primitif de siège inconnu, les cancers pédiatriques rares, le cancer de l'urètre, le cancer de l'utérus, le sarcome utérin, la macroglobulinémie de Waldenström ou la tumeur de Wilms.

29. Composé destiné à être utilisé selon la revendication 9, dans lequel ledit au moins un composé selon la revendication 1 est administré simultanément ou séquentiellement avec une quantité thérapeutiquement efficace d'un ou plusieurs autres agents thérapeutiques choisis dans le groupe constitué par les agents antiviraux, les agents chimiothérapeutiques (y compris les agents antinéoplasiques alkylants, les antimétabolites et les agents antimicrotubules), les immunosuppresseurs, les vaccins antitumoraux, les vaccins antiviraux, les cytokines thérapeutiques, les inhibiteurs de tyrosine kinase, les agents immunothérapeutiques, y compris les inhibiteurs de points de contrôle immunitaire tels que les inhibiteurs de PD-1, de PD-L1 ou de CTLA-4 et les inhibiteurs d'IDO/TDO, les antagonistes du récepteur A2A de l'adénosine, les inhibiteurs des ectonucléotidases (CD73 et CD39), les agents agissant sur les interleukines, les cytokines et les chimiokines, les inhibiteurs de la topoisomérase et les antibiotiques cytotoxiques, ou les thérapies ciblées comprenant des anticorps, des conjugués anticorps-médicament, une immunothérapie cellulaire, des nanoparticules et la radiothérapie.

30. Composé destiné à être utilisé selon la revendication 29, dans lequel les anticorps comprennent une quantité thérapeutiquement efficace d'anticorps anti-PD-1, anti-PD-L1 ou anti-CTLA-4.

31. Composé selon la revendication 1 destiné à être utilisé dans une méthode d'activation *in vitro* ou *ex vivo* de lymphocytes T en vue de la libération de cytokines, ladite méthode comprenant la mise en contact de la cellule avec au moins un composé selon la revendication 1, ou un sel ou solvate pharmaceutiquement acceptable de celui-ci.

32. Utilisation d'un composé selon la revendication 1, ou d'un sel ou solvate pharmaceutiquement acceptable de celui-ci, pour protéger un organe situé à l'extérieur du corps humain ou animal pendant son transport.
